# EUROPÄISCHE PATENTANMELDUNG

(11) **EP 1 847 540 A1**
(43) Veröffentlichungstag der Anmeldung: **24.10.2007**
(21) Anmeldenummer: 07013545.4
(22) Anmeldetag: 10.08.1998
(51) Int. Cl.: C07D 405/06, C07D 417/06, C07D 493/04, C07D 313/00, C07C 49/185, C07C 49/17, C07C 59/185, C07C 69/66, C07F 7/18, A61K 31/335, A61K 31/425, A61K 31/44, C07D 303/00

(54) **Neue Epothilonderivate, Herstellungsverfahren dafür und ihre pharmazeutische Verwendung**

(30) Priorität: 09.08.1997 DE 19735574; 09.08.1997 DE 19735575; 09.08.1997 DE 19735578; 24.10.1997 DE 19748928; 31.10.1997 DE 19749717; 13.11.1997 DE 19751200; 20.03.1998 DE 19813821
(62) Teilanmeldung aus: 98946309.6
(71) Anmelder: Bayer Schering Pharma Aktiengesellschaft, 13353 Berlin (DE)
(72) Erfinder: Klar, Ulrich, 13503 Berlin (DE); Schwede, Wolfgang, 16548 Glienicke (DE); Skuballa, Werner, 13465 Berlin (DE); Buchmann, Bernd, 16540 Hohen Neuendorf (DE); Schirner, Michael, 13158 Berlin (DE)

(57) **Zusammenfassung**

Die vorliegende Erfindung betrifft die neuen Epothilon-Derivate der allgemeinen Formel I, 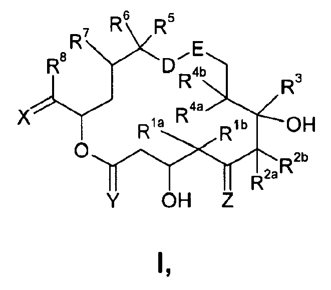 worin
die Substituenten Y, Z, R^{2a}, R^{2b}, R³, R^{4a}, R^{4b}, D-E, R⁵, R⁶, R⁷, R⁸ und X die in der Beschreibung näher angegebenen Bedeutungen haben.

Die neuen Verbindungen interagieren mit Tubulin, indem sie gebildete Mikrotubuli stabilisieren. Sie sind in der Lage, die Zellteilung phasenspezifisch zu beeinflussen und sind zur Behandlung maligner Tumoren geeignet, beispielsweise Ovarial-, Magen-, Colon-, Adeno-, Brust-, Lungen-, Kopf- und Nacken-Karzinome, malignes Melanom, akute lymphozytäre und myelocytäre Leukämie. Außerdem sind sie zur Anti-Angiogenese-Therapie sowie zur Behandlung chronischer entzündlicher Erkrankungen (Psoriasis, Arthritis) geeignet. Zur Vermeidung unkontrollierter Zellwucherungen an sowie der besseren Verträglichkeit von medizinischen Implantaten lassen sie sich in polymere Materialien auf- bzw. einbringen.

Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.

## Beschreibung

Von Höfle et al. wird die cytotoxische Wirkung der Naturstoffe Epothilon A (R = Wasserstoff) und Epothilon B (R = Methyl) 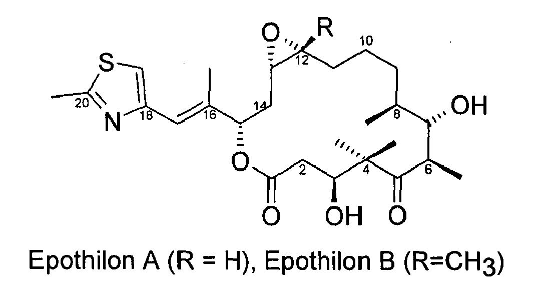 z.B. in Angew. Chem. 1996, 108, 1671-1673, beschrieben. Wegen der in-vitro-Selektivität gegenüber Brust- und Darmzelllinien und ihrer im Vergleich zu Taxol deutlich höheren Aktivität gegen P-Glycoprotein-bildende, multiresistente Tumorlinien sowie ihre gegenüber Taxol verbesserten physikalischen Eigenschaften, z.B eine um den Faktor 30 höhere Wasserlöslichkeit, ist diese neuartige Strukturklasse für die Entwicklung eines Arzneimittels zur Therapie maligner Tumoren besonders interessant.

Die Naturstoffe sind sowohl chemisch als auch metabolisch für eine Arzneimittelentwicklung nicht ausreichend stabil. Zur Beseitigung dieser Nachteile sind Modifikationen an dem Naturstoff nötig. Derartige Modifikationen sind nur auf totalsynthetischem Wege möglich und setzen Synthesestrategien voraus, die eine breite Modifikation des Naturstoffes ermöglichen. Ziel der Strukturveränderungen ist es auch, die therapeutische Breite zu erhöhen. Dies kann durch eine Verbesserung der Selektivität der Wirkung und/oder eine Reduktion unerwünschter toxischer Nebenwirkungen und/oder eine Erhöhung der Wirkstärke erfolgen.

Die Totalsynthese von Epothilon A ist von Schinzer et al. in Chem. Eur. J. 1996, 2, No. 11, 1477-1482 und in Angew. Chem. 1997, 109, Nr. 5, S. 543-544) beschrieben.
Epothilon-Derivate wurden bereits von Höfle et al. in der WO 97/19086 beschrieben. Diese Derivate wurden ausgehend vom natürlichen Epothilon A oder B hergestellt.
Eine weitere Synthese von Epothilon und Epothilonderivaten wurde von Nicolaou et al. in Angew. Chem. 1997, 109, Nr. 1/2, S. 170 - 172 beschrieben. Die Synthese von Epothilon A und B und einiger Epothilon-Analoga wurde in Nature, Vol. 387, 1997, S. 268-272, die Synthese von Epothilon A und seinen Derivaten in J. Am. Chem. Soc., Vol. 119, No. 34, 1997, S. 7960 - 7973 sowie die Synthese von Epothilon A und B und einiger Epothilon-Analoga in J. Am. Chem. Soc., Vol. 119, No. 34, 1997, S. 7974 - 7991 ebenfalls von Nicolaou et al. beschrieben.
Ebenfalls Nicolaou et al. beschreiben in Angew. Chem. 1997, 109, Nr. 19, S. 2181-2187 die Herstellung von Epothilon A-Analoga mittels kombinatorischer Festphasensynthese. Auch einige Epothilon B-Analoga sind dort beschrieben.

Die Aufgabe der vorliegenden Erfindung besteht darin, neue Epothilon-Derivate zur Verfügung zu stellen, die sowohl chemisch als auch metabolisch für eine Arzneimittelentwicklung ausreichend stabil sind und die hinsichtlich ihrer therapeutischen Breite, ihrer Selektivität der Wirkung und/oder unerwünschter toxischer Nebenwirkungen und/oder ihrer Wirkstärke den natürlichen Derivaten überlegen sind.

Die vorliegende Erfindung beschreibt die neuen Epothilon-Derivate der allgemeinen Formel l, 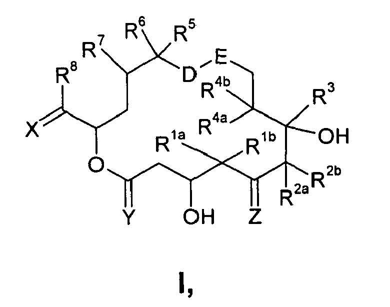 worin
- R^{1a}, R^{1b}: gleich oder verschieden sind und Wasserstoff, C₁-C₁₀-Alkyl, Aryl, C₇-C₂₀-Aralkyl, oder gemeinsam eine -(CH₂)ₘ-Gruppe mit m = 2, 3, 4 oder 5,
- R^{2a}, R^{2b}: gleich oder verschieden sind und Wasserstoff, C₁-C₁₀-Alkyl, Aryl, C₇-C₂₀-Aralkyl oder gemeinsam eine -(CH₂)ₙ-Gruppe mit n = 2, 3, 4 oder 5, wobei, falls -D-E- für -CH₂-CH₂- oder Y für ein Sauerstoffatom steht, R^{2a} /R^{2b} nicht Wasserstoff / Methyl sein können,
- R³: Wasserstoff, C₁-C₁₀-Alkyl, Aryl, C₇-C₂₀-Aralkyl,
- R^{4a}, R^{4b}: gleich oder verschieden sind und Wasserstoff, C₁-C₁₀-Alkyl, Aryl, C₇-C₂₀-Aralkyl oder gemeinsam eine -(CH₂)ₚ-Gruppe mit p = 2, 3, 4 oder 5,
- D-E: eine Gruppe H₂C-CH₂ , HC=CH, C≡C, 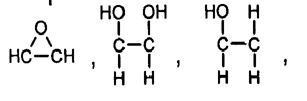
- R⁵: Wasserstoff, C₁-C₁₀-Alkyl, Aryl, C₇-C₂₀-Aralkyl,
- R⁶, R⁷: je ein Wasserstoffatom, gemeinsam eine zusätzliche Bindung oder ein Sauerstoffatom,
- R⁸: Wasserstoff, C₁-C₂₀-Alkyl, Aryl, C₇-C₂₀-Aralkyl, die alle substituiert sein können,
- X: ein Sauerstoffatom, zwei Alkoxygruppen OR²³, eine C₂-C₁₀-Alkylen-α,ω-dioxygruppe, die geradkettig oder verzweigt sein kann, H/OR⁹ oder eine Gruppierung CR¹⁰R¹¹,
wobei
R²³ für einen C₁-C₂₀-Alkylrest,
R⁹ für Wasserstoff oder eine Schutzgruppe PG^{x},
R¹⁰, R¹¹ gleich oder verschieden sind und für Wasserstoff, einen C₁-C₂₀-Alkyl-, Aryl-, C₇-C₂₀-Aralkylrest oder
R¹⁰ und R¹¹ zusammen mit dem Methylenkohlenstoffatom gemeinsam für einen 5- bis 7-gliedrigen carbocyclischen Ring stehen,
- Y: ein Sauerstoffatom oder zwei Wasserstoffatome,
- Z: ein Sauerstoffatom oder H/OR¹²,
wobei
R¹² Wasserstoff oder eine Schutzgruppe PG^{z} ist,
bedeuten.

Die Darstellung der neuen Epothilon-Derivate basiert auf der Verknüpfung dreier Teilfragmente A, B und C. Die Schnittstellen liegen wie in der allgemeinen Formel l' angedeutet.

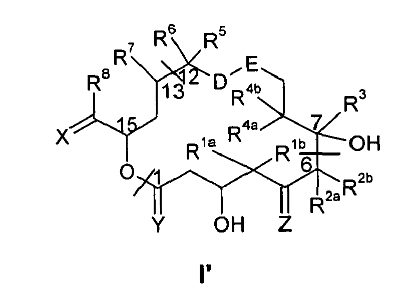

A bedeutet ein C1-C6-Fragment (Epothilon-Zählweise) der allgemeinen Formel 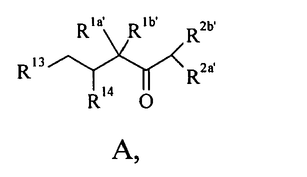 worin
- R^{1a'}, R^{1b'}, R^{2a'} und R^{2b'}: die bereits für R^{1a}, R^{1b}, R^{2a} und R^{2b} genannten Bedeutungen haben und
- R¹³: CH₂OR^{13a}, CH₂-Hal, CHO; CO₂R^{13b}, COHal,
- R¹⁴: Wasserstoff, OR^{14a}, Hal, OSO₂R^{14b},
- R^{13a}, R^{14a}: Wasserstoff, SO₂-Alkyl, SO₂-Aryl, SO₂-Aralkyl oder gemeinsam eine-(CH₂)ₒ-Gruppe oder gemeinsam eine CR^{15a}R^{15b}-Gruppe,
- R^{13b}, R^{14b}: Wasserstoff, C₁-C₂₀-Alkyl, Aryl, C₇-C₂₀-Aralkyl,
- R^{15a}, R^{15b} gleich: oder verschieden sind und Wasserstoff, C₁-C₁₀-Alkyl, Aryl, C₇-C₂₀-Aralkyl, oder gemeinsam eine -(CH₂)q-Gruppe,
- Hal: Halogen,
- o: 2 bis 4,
- q: 3 bis 6,
einschließlich aller Stereoisomeren sowie deren Gemische
bedeuten sowie
freie Hydroxylgruppen in R¹³ und R¹⁴ verethert oder verestert, freie Carbonylgruppen in A und R¹³ ketalisiert, in einen Enolether überführt oder reduziert sowie freie Säuregruppen in A in deren Salze mit Basen überführt sein können.

B steht für ein C7-C12-Fragment (Epothilon-Zählweise) der allgemeinen Formel 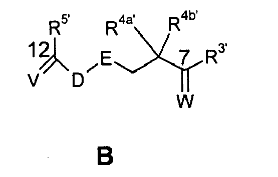 worin
- R^{3'}, R^{4a',} R^{4b'} und R^{5'}: die bereits für R³, R^{4a}, R^{4b} und R⁵ genannten Bedeutungen haben,und
- V: ein Sauerstoffatom, zwei Alkoxygruppen OR¹⁷, eine C₂-C₁₀-Alkylen-α,ω-dioxygruppe, die geradkettig oder verzweigt sein kann oder H/OR¹⁶,
- W ein: Sauerstoffatom, zwei Alkoxygruppen OR¹⁹, eine C₂-C₁₀-Alkylen-α,ω-dioxygruppe, die geradkettig oder verzweigt sein kann oder H/OR¹⁸,
- R¹⁶, R¹⁸: unabhängig voneinander Wasserstoff oder eine Schutzgruppe PG¹
- R¹⁷, R¹⁹: unabhängig voneinander C₁-C₂₀-Alkyl,
bedeuten.

C steht für ein C13-C16-Fragment (Epothilon-Zählweise) der allgemeinen Formel 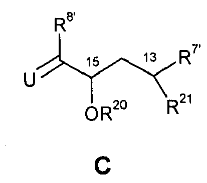 worin
- R^{8'}: die bereits in der allgemeinen Formel 1 für R⁸ genannte Bedeutung hat und
- R^{7'}: ein Wasserstoffatom,
- R²⁰: ein Wasserstoffatom oder eine Schutzgruppe PG²
- R²¹: eine Hydroxygruppe, Halogen, eine geschützte Hydroxygruppe OPG³, ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
- U: ein Sauerstoffatom, zwei Alkoxygruppen OR²³, eine C₂-C₁₀-Alkylen-α,ω-dioxygruppe, die geradkettig oder verzweigt sein kann, H/OR⁹ oder eine Gruppierung CR¹⁰R¹¹,
wobei
R²³ für einen C₁-C₂₀-Alkylrest,
R⁹ für Wasserstoff oder eine Schutzgruppe PG³,
R¹⁰, R¹¹ gleich oder verschieden sind und für Wasserstoff, einen C₁-C₂₀-Alkyl-, Aryl-, C₇-C₂₀-Aralkylrest oder
R¹⁰ und R¹¹ zusammen mit dem Methylenkohlenstoffatom gemeinsam für einen 5- bis 7-gliedrigen carbocyclischen Ring stehen,
bedeuten.

Als Alkylgruppen R^{1a}, R^{1b}, R^{2a}, R^{2b}, R³, R⁴, R⁵, R⁸, R⁹, R¹⁰, R¹¹, R¹², R^{13b}, R^{14b}, R^{15a}, R^{15b}, R¹⁷ und R²³ sind gerad- oder verzweigtkettige Alkylgruppen mit 1-20 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl. Die Alkylgruppen R^{1a}, R^{1b}, R^{2a}, R^{2b}, R³, R⁴, R⁵, R⁸, R⁹, R¹⁰, R¹¹, R¹², R^{13b}, R^{14b}, R^{15a}, R^{15b}, R¹⁷ und R²³ können perfluoriert oder substituiert sein durch 1-5 Halogenatome, Hydroxygruppen, C₁-C₄-Alkoxygruppen, C₆-C₁₂-Arylgruppen (die durch 1-3 Halogenatome substituiert sein können).
Als Arylrest R^{1a}, R^{1b}, R^{2a}, R^{2b}, R³, R⁴, R⁵, R⁸, R⁹, R¹⁰, R¹¹, R¹², R^{13b}, R^{14b}, R^{15a} und R^{15b} kommen substituierte und unsubstituierte carbocyclische oder heterocyclische Reste mit einem oder mehreren Heteroatomen wie z.B. Phenyl, Naphthyl, Furyl, Thienyl, Pyridyl, Pyrazolyl, Pyrimidinyl, Oxazolyl, Pyridazinyl, Pyrazinyl, Chinolyl, Thiazolyl, die einfach oder mehrfach substituiert sein können durch Halogen, OH, O-Alkyl, CO₂H, CO₂-Alkyl, -NH₂, -NO₂, -N₃, -CN, C₁-C₂₀-Alkyl, C₁-C₂₀-Acyl, C₁-C₂₀-Acyloxy-Gruppen, in Frage. Heteroatome in den Heteroarylresten können oxidiert sein; so kann beispielsweise der Thiazolring in Form des N-Oxids vorliegen.
Die Aralkylgruppen in R^{1a}, R^{1b}, R^{2a}, R^{2b}, R³, R⁴, R⁵, R⁸, R⁹, R¹⁰, R¹¹, R¹² R^{13b}, R^{14b}, R^{15a} und R^{15b} können im Ring bis 14 C-Atome, bevorzugt 6 bis 10 und in der Alkylkette 1 bis 8, bevorzugt 1 bis 4 Atome enthalten. Als Aralkylreste kommen beispielweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H, CO₂-Alkyl -NO₂, -N₃, -CN, C₁-C₂₀-Alkyl, C₁-C₂₀-Acyl, C₁-C₂₀-Acyloxy-Gruppen.
Die in X in der allgemeinen Formel I enthaltenen Alkoxygruppen sollen jeweils 1 bis 20 Kohlenstoffatome enthalten, wobei Methoxy-, Ethoxy- Propoxy- lsopropoxy- und t-Butyloxygruppen bevorzugt sind.
Als Vertreter für die Schutzgruppen PG sind Alkyl- und/oder Aryl-substituiertes Silyl, C₁-C₂₀-Alkyl, C₄-C₇-Cycloalkyl, das im Ring zusätzlich ein Sauerstoffatom enthalten kann, Aryl, C₇-C₂₀-Aralkyl, C₁-C₂₀-Acyl sowie Aroyl zu nennen.
Als Alkyl-, Silyl- und Acylreste für die Schutzgruppen PG kommen die dem Fachmann bekannten Reste in Betracht. Bevorzugt sind aus den entsprechenden Alkyl- und Silylethern leicht abspaltbare Alkyl- bzw. Silylreste, wie beispielsweise der Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-Rest sowie Alkylsulfonyl- und Arylsulfonylreste. Als Acylreste kommen z.B. Formyl, Acetyl, Propionyl, Isopropionyl, Pivalyl-, Butyryl oder Benzoyl, die mit Amino- und/oder Hydroxygruppen substituiert sein können, in Frage.
Die Acylgruppen PG^{x} bzw. PG^{z} in R⁹ und R¹² können 1 bis 20 Kohlenstoffatome enthalten, wobei Formyl-, Acetyl-, Propionyl-, Isopropionyl und Pivalylgruppen bevorzugt sind.

Der Index m in der aus R^{1a} und R^{1b} gebildeten Alkylengruppe steht vorzugsweise für 2, 3 oder 4.

Die für X mögliche C₂-C₁₀-Alkylen-α,ω-dioxygruppe ist vorzugsweise eine Ethylenketal- oder Neopentylketalgruppe.

Die Substituenten können in den Verbindungen der allgemeinen Formel I so gewählt sein, daß
Y, Z, R^{1a}, R^{1b}, R^{2a} und R^{2b} alle die in der allgemeinen Formel I angegebenen Bedeutungen haben können, und der Rest des Moleküls identisch ist mit dem natürlich vorkommenden Epothilon A oder B, oder
R³, R^{4a}, R^{4b}, D-E, R⁵, R⁶ und R⁷ alle die in der allgemeinen Formel 1 angegebenen Bedeutungen haben können, und der Rest des Moleküls identisch ist mit dem natürlich vorkommenden Epothilon A oder B. oder
R⁶, R⁷, R⁸ und X alle die in der allgemeinen Formel I angegebenen Bedeutungen haben können, und der Rest des Moleküls identisch ist mit dem natürlich vorkommenden Epothilon A oder B oder
Y, Z, R^{1a}, R^{1b}, R^{2a}, R^{2b}, R³, R^{4a}, R^{4b}, D-E, R⁵, R⁶ und R⁷ alle die in der allgemeinen Formel I angegebenen Bedeutungen haben können, und der Rest des Moleküls identisch ist mit dem natürlich vorkommenden Epothilon A oder B oder
Y, Z, R^{1a}, R^{1b}, R^{2a}, R^{2b}, R⁶, R⁷, R⁸ und X alle die in der allgemeinen Formel I angegebenen Bedeutungen haben können, und der Rest des Moleküls identisch ist mit dem natürlich vorkommenden Epothilon A oder B oder
R³, R^{4a}, R^{4b}, D-E, R⁵, R⁶, R⁷, R⁸ und X alle die in der allgemeinen Formel I angegebenen Bedeutungen haben können, und der Rest des Moleküls identisch ist mit dem natürlich vorkommenden Epothilon A oder B.

Die nachstehen genannten Verbindungen sind erfindungsgemäß bevorzugt:
(4S,7R,8S,9S,13(Z),16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion und
   (4S,7R,8S,9S,13E,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion
(1S,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
   (1R,3S(E),7S,10R,11S,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(1S,3S(E),7S,10R,11S,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
   (1R,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(4S,7S,8R,9S,13Z,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion und
   (4S,7S,8R,9S,13E,16S(E))-4,8-Dihydroxy-7-ethyl-16-(1-methy)-2-(2-methyl-4-thiazolyl)ethenyl)-1-oxa-5,5,9,13-tetramethyl-cyclohexadec-13-en-2,6-dion
(1S,3S(E),7S,10S,11R,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
   (1R,3S(E),7S,10S,11R,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(1S,3S(E),7S,10S,11R,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
   (1R,3S(E),7S,10S,11R,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(4S,7R,8S,9S,13(Z),16S(E))-4,8-Dihydroxy-5,5,7,9,13-pentamethyl-16-((3-pyridyl)ethenyl)-1-oxa-cyclohexadec-13-en-2,6-dion und
   (4S,7R,8S,9S,13E,16S(E))-4,8-Dihydroxy-5,5,7,9,13-pentamethyl-16-((3-pyridyl)ethenyl)-1-oxa-cyclohexadec-13-en-2,6-dion
(1S,3S(E),7S,10R,11S,12S,16R)-7,11-Dihydroxy-8,8,10,12,16-pentamethyl-3-((3-pyridyl)ethenyl)-4,17-dioxabicyclo[14.1.0]heptadecan-5,9-dion und
   (1S,3S(E),7S,10R,11S,12S,16S)-7,11-Dihydroxy-8,8,10,12,16-pentamethyl-3-((3-pyridyl)ethenyl)-4,17-dioxabicyclo[14.1.0]heptadecan-5,9-dion
(4S,7R,8S,9S,13(Z),16S(E))-4, 8-Dihydroxy-5,5,7,9,13-pentamethyl-16-((4-pyridyl)ethenyl)-1-oxa-cyclohexadec-13-en-2,6-dion und
   (4S,7R,8S,9S,13E,16S(E))-4,8-Dihydroxy-5,5,7,9,13-pentamethyl-16-((4-pyridyl)ethenyl)-1-oxa-cyclohexadec-13-en-2,6-dion
(1S,3S(E),7S,10R,11S,12S,16R)-7,11-Dihydroxy-8,8,10,12,16-pentamethyl-3-((4-pyridyl)ethenyl)-4,17-dioxabicyclo[14.1.0]heptadecan-5,9-dion und
   (1S,3S(E),7S,10R,11S,12S,16S)-7,11-Dihydroxy-8,8,10,12,16-pentamethyl-3-((4-pyridyl)ethenyl)-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-1-oxa-7-phenyl-5,5,9,13-tetramethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-10-phenyl-8,8,12,16-tetramethyl-4,17-dioxabicyclo[14.10]heptadecann-5,9-dion
(1(R oder S),3S(E),7S,10R,11S,12S,16S)-7,11-Dihydroxy-3-(1-methyl-2-(2-methyl-4-thiazolyl)ethenyl)-10-phenyl-8,8,12,16-tetramethyl-4,17-dioxabicyclo[14.1.0]heptadecan-5,9-dion
(4S,7R,8S,9S,13(E oder Z),16S(E))-7-Benzyl-4,8-dihydroxy-16-(1-methyl-2-(2-methyl-4-thiazolyl)ethenyl)-1-oxa-5,5,9,13-tetramethyl-cyclohexadec-13-en-2,6-dion
(1(S oder R),3S(E),7S,10R,11S,12S,16R)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5,9-dion
(1(R oder S),3S(E),7S,10R,11S,12S,16S)-10-Benzyl-7,11-dihydroxy-3-(1-methyl-2-(2-methyl-4-thiazolyl)ethenyl)-8,8,10,12,16-tetramethyl-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-1 -oxa-5,5,7,13-tetramethyl-9-trifluormethyl-cyclohexadec-l 3-en-2,6-dion
(1(S oder R),3S(E),7S,10R,11S,12S,16R)-7,11-Dihydroxy-3-(1-methyl-2-(2-methyl-4-thiazolyl)ethenyl)-8, 8,10,16-tetramethyl-12-trifluormethyl-4,17-dioxabicyclo[14.1.0]heptadecan-5,9-dion
(1(R oder S),3S(E),7S,10R,11S,12S,16S)-7,11-Dihydroxy-3-(1-methyl-2-(2-methyl-4-thiazolyl)ethenyl)-8, 8,10,16-tetramethyl-12-trifluormethyl-4,17-dioxabicyclo[14.1.0]heptadecan-5,9-dion
(4S,7R,8S,9S,11E/Z,13(E oder Z),16S(E))-4,8-Dihydroxy-16-(1-methyl-2-(2-methyl-4-thiazolyl)ethenyl)-1-oxa-5,5,7,9,13-pentamethyl-cyclohexadec-11,13-dien-2,6-dion
(1(S oder R),3S(E),7S,10R,11S,12S,14E/Z,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-en-5,9-dion
(1(R oder S),3S(E),7S,10R,11S,12S,14E/Z,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-en-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5,7,9,13-pentamethyl-cyclohexadec-13-en-11-in-2,6-dion
(1(S oder R),3S(E),7S,10R,11S,12S,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-in-5,9-dion
(1(R oder S),3S(E),7S,10R,11S,12S,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-in-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5,7,9-tetramethyl-13-trifluormethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-8,8,10,12-tetramethyl-16-trifluormethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-8,8,10,12-tetramethyl-16-trifluormethyl-4,17-dioxabicyclo[14.1.0]heptadeca-5,9-dion
(4S,7R,8S,9S,13(E oder Z),16S(E))-4,8-Dihydroxy-16-(1-methyl-2-(2-methyl-4-thiazolyl)ethenyl)-1-oxa-13-pentafluorethyl-5,5,7,9-tetramethyl-cyclohexadec-13-en-2,6-dion
(1(S oder R),3S(E),7S,1OR,11S,128,16R)-7,11-Dihydroxy-3-(1-methyl-2-(2-methyl-4-thiazolyl)ethenyl)-16-pentafluorethyl-8,8,10,12-tetramethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-16-pentafluorethyl-8,8,10,12-tetramethyl-4,17-dioxabicyclo[14.1.0]heptadeca-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5-(1,3-trimethylen)-7,9,13-trimethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-8,8-(1,3-trimethylen)-10,12,16-trimethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-8,8-(1, 3-trimethylen)-10,12,16-trimethyl-4,17-dioxabicyclo[14.1.0]heptadeca-5,9-dion
(4S,7R,8S,9S,11E/Z,13(E oder Z),16S(E))-4,8-Dihydroxy-13-ethyl-16-(1-methyl-2-(2-methyl-4-thiazolyl)ethenyl)-1-oxa-5,5,7,9-tetramethyl-cyclohexadec-11,13-dien-2,6-dion
(1(S oder R),3S(E),7S,10R,11S,12S,14E/Z,16R)-7,11-Dihydroxy-16-ethyl-3-(1-methyl-2-(2-methyl-4-thiazolyl)ethenyl)-8,8,10,12-tetramethyl-4,17-dioxabicyclo[14.1.0]heptadec-14-en-5,9-dion
(1(R oder S),3S(E),7S,10R,11S,12S,14E/Z,16S)-7,11-Dihydroxy-16-ethyl-3-(1-methyl-2-(2-methyl-4-thiazolyl)ethenyl)-8,8,10,12-tetramethyl-4,17-dioxabicyclo[14.1.0]heptadec-14-en-5,9-dion
(4S,7R,8S,9S,11E/Z,13(E oder Z),16S(E))-4,8-Dihydroxy-16-(1-methyl-2-(2-methyl-4-thiazolyl)ethenyl)-1-oxa-13-propyl-5,5,7,9-tetramethyl-cyclohexadec-11,13-dien-2,6-dion
(1(S oder R),3S(E),7S,10R,11S,12S,14E/Z,16R)-7,11-Dihydroxy-3-(1-methyl-2-(2-methyl-4-thiazolyl)ethenyl)-16-propyl-8,8,10,12-tetramethyl-4,17-dioxabicyclo[14.1.0]heptadec-14-en-5,9-dion
(1(R oder S),3S(E),7S,10R,11S,12S,14E/Z,16S)-7,11-Dihydroxy-3-(1-methyl-2-(2-methyl-4-thiazolyl)ethenyl)-16-propyl-8,8,10,12-tetramethyl-4,17-dioxabicyclo[14.1.0]heptadec-14-en-5,9-dion
(4S,7R,8S,9S,13(E oder Z),16S(E))-4,8-Dihydroxy-16-(1-methyl-2-(2-pyridyl)ethenyl)-1-oxa-5,5,7,9,13-pentamethyl-cyclohexadec-13-en-2,6-dion
(1(S oder R),3S(E),7S,10R,11S,12S,16R)-7,11-Dihydroxy-3-(1-methy)-2-(2-pyridyl)ethenyl)-8,8,10,12,16-pentamethyl-4,17-dioxabicyclo[14.1.0]heptadecan-5,9-dion
(1(R oder S),3S(E),7S,10R,11S,12S,16S)-7,11-Dihydroxy-3-(1-methyl-2-(2-pyridyl)ethenyl)-8,8,10,12,16-pentamethyl-4,17-dioxabicyclo[14.1.0]heptadecan-5,9-dion
(4S,7R,8S,9S,13(E oder Z),16S(E))-4,8-Dihydroxy-16-(1-methyl-2-(4-pyridyl)ethenyl)-1-oxa-5,5,7,9,13-pentamethyl-cyclohexadec-13-en-2,6-dion
(1(S oder R),3S(E),7S,10R,11S,12S,16R)-7,11-Dihydroxy-3-(1-methyl-2-(4-pyridyl)ethenyl)-8,8,10,12,16-pentamethyl-4,17-dioxabicyclo[14.1.0]heptadecan-5,9-dion
(1(R oder S),3S(E),7S,10R,11S,12S,16S)-7,11-Dihydroxy-3-(1-methyl-2-(4-pyridyl)ethenyl)-8,8,10,12,16-pentamethyl-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-5,5,7,913-pentamethyl-cyclohexadec-13-en-6-on
(1(S oder R),3S(E),7S,10R,11S,12S,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-9-on
(1(R oder S),3S(E),7S,10R,11S,12S,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-9-on

### Darstellung der Teilfragmente A:

Es ist bekannt, daß die Verbindung der folgenden Formel 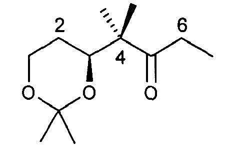 zur Synthese des C1-C6-Fragmentes (Epothilon-Zählweise) von Epothilon A verwendet werden kann (Schinzer et al, Chem. Eur. J. 1996, 2, No. 11, 1477-1482; Schinzer et al., Angew. Chem. 1997, 109, Nr. 5, S. 543-544).
Diese Synthese besitzt den Nachteil, daß die Gesamtausbeute mit 10,5% sehr niedrig, die notwendige Einführung der Chiralität an C-Atom 3 die Synthese eines teuren, chemisch instabilen, in äquimolaren Mengen einzusetzenden und nicht wiedergewinnbaren chiralen Hilfsstoffes erfordert und die damit erzielte optische Induktion mit ca. 80%ee unvollständig ist.

Für eine industriell verwertbare Synthese sind jedoch hohe Ausbeuten und hohe optische Reinheit notwendig.

In Angew. Chem. 1997, 109, Nr. 1/2, S. 170 - 172 wird die Synthese eines (C1-C6)-Bausteins mit einer Carboxylgruppe an C-1, der für die Synthese von Epothilon oder Epothilonderivaten verwendet werden kann, 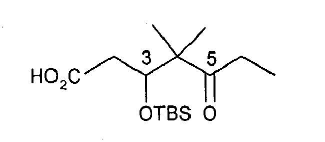 (TBS =*tert*.-Butyldimethylsilyl) von Nicolaou et al. beschrieben. Die Stereochemie am C3 wird durch die Reaktion mit dem Brown Reagenz Allylisopinocamphenylboran (+)-lpc₂B(allyl) gesteuert, das äquimolar in die Reaktion eingesetzt werden muß und nicht wiedergewinnbar ist.
Ebenso wird die Verwendung dieses Bausteins zur Synthese von Epothilon A und B und einiger Epothilon-Analoga in Nature, Vol. 387, 1997, S. 268-272, zur Synthese von Epothilon A und seinen Derivaten in J. Am. Chem. Soc., Vol. 119, No. 34, 1997, S. 7960- 7973 sowie zur Synthese von Epothilon A und B und einiger Epothilon-Analoga in J. Am. Chem. Soc., Vol. 119, No. 34, 1997, S. 7974 - 7991 von Nicolaou et al. beschrieben. Ebenfalls von Nicolaou et al. wird in Angew. Chem. 1997, 109, Nr. 19, S. 2181-2187 die Herstellung von Epothilon A-Analoga mittels kombinatorischer Festphasensynthese beschrieben. Aus dieser Fundstelle gehen auch Epothilon B-Analoga hervor. Als C1-C6-Bausteine werden die nachstehenden Verbindungen eingesetzt:

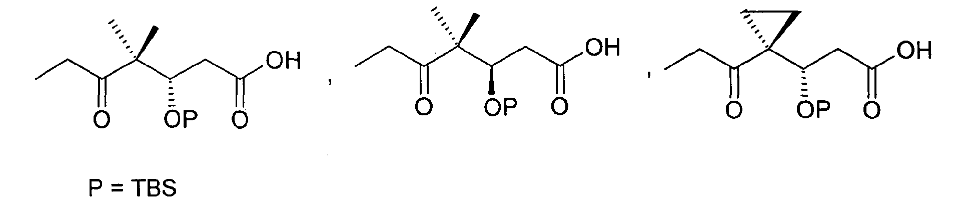

Für eine industriell verwertbare Synthese ist es von Vorteil, wenn die Synthese ohne teure chirale Auxiliare durchgeführt werden kann.

Es bestand daher die Aufgabe, eine geeignete Synthese zu finden, die hohe Ausbeuten ergibt, das gewünschte Produkt in hoher optischer Reinheit ergibt und ohne teure chirale Auxiliare auskommt.
Außerdem sollte die neue Synthese eine breitere Variation von Substituenten in diesem Baustein und somit letztendlich in den daraus herzustellenden Epothilonderivaten zulassen.

Die Teilfragmente (Synthesebausteine) der allgemeinen Formel A lassen sich leicht aus
a) einem Pantolacton der allgemeinen Formel lla 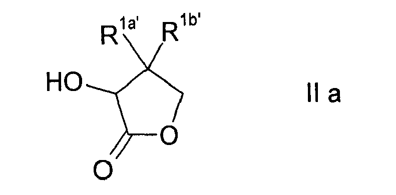 worin
   R^{1a'}, R^{1b'} jeweils für eine Methylgruppe stehen
   oder
b) einem Malonsäuredialkylester der allgemeinen Formel XXVIII 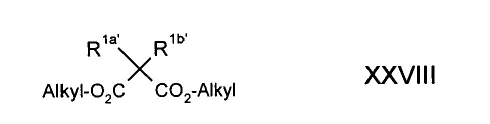 worin
   R^{1a}', R^{1b}' die in der allgemeinen Formel A angegebene Bedeutung haben, und
   Alkyl unabhängig voneinander einen C₁-C₂₀-Alkyl-, C₃-C₁₀-Cycloalkyl- oder C₄-C₂₀-Alkylcycloalkylrest bedeuten.
als Ausgangsprodukt herstellen.

Die Teilfragmente A, in denen R^{1a'}=R^{1b'}=Methyl ist, können aus wohlfeilem Pantolacton auf effiziente Weise mit einer optischen Reinheit >98%ee hergestellt werden.

Die Synthese wird im folgenden Schema 1 am Beispiel des D-(-)-Pantolactons beschrieben. Aus L-(+)-Pantolacton erhält man die entsprechenden, zu A-II bis A-XIV enantiomeren Verbindungen ent-A-II bis ent-A-XIV und aus racemischem DL-Pantolacton die entsprechenden racemischen Verbindungen rac-A-II bis rac-A-XIV: 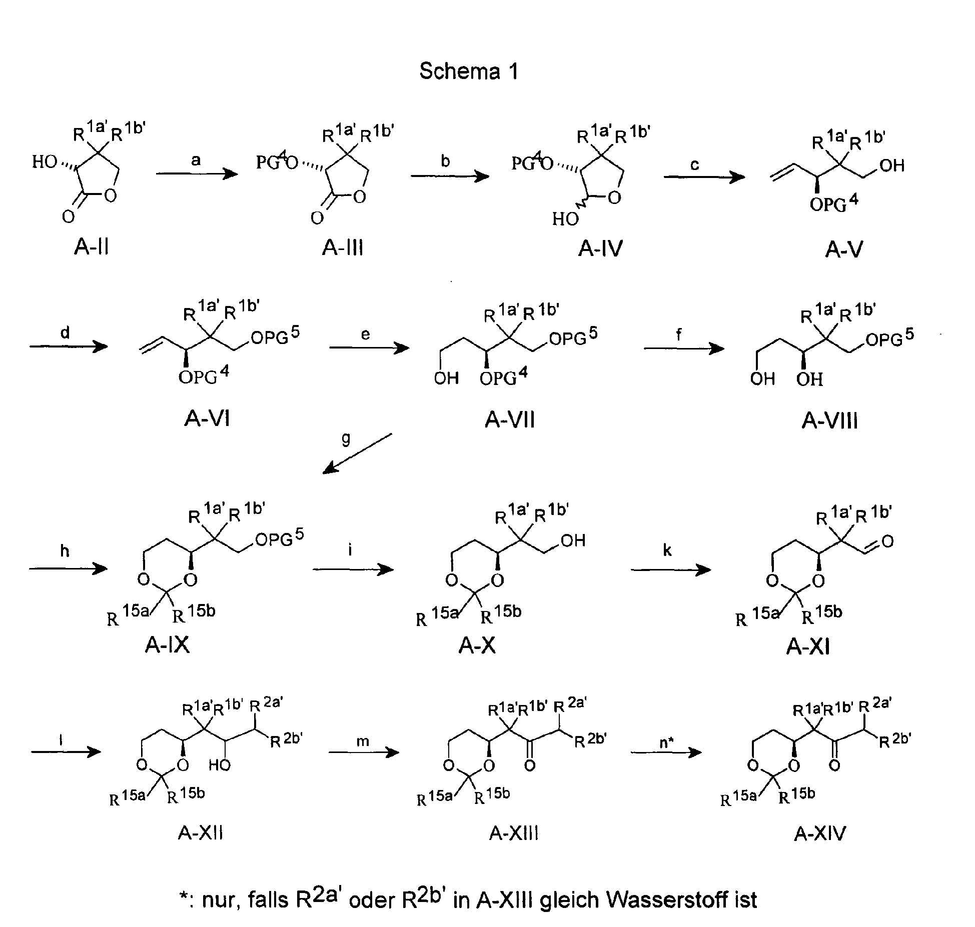
Schritt a (A-II → A-III):
   Die freie Hydroxygruppe des Pantolactons (A-II) wird nach den, dem Fachmann bekannten Methoden geschützt. Als Schutzgruppe PG⁴ kommen die, dem Fachmann bekannten Schutzgruppen wie z.B. der Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-, Formyl-, Acetyl-, Propionyl-, Isopropionyl-, Pivalyl-, Butyryl- oder Benzoylrest in Frage.
   Eine Übersicht befindet sich z.B. in "Protective Groups in Organic Synthesis" Theodora W. Green, John Wiley and Sons).
   Bevorzugt sind solche Schutzgruppen, die unter sauren Reaktionsbedingungen gespalten werden können, wie z.B. der Methoxymethyl-, Tetrahydropyranyl-, Tetrahydrofuranyl-, Trimethylsilyl-Rest.
   Besonders bevorzugt ist der Tetrahydropyranyl-Rest.
Schritt b (A-III → A-IV):
   Das geschützte Lacton A-III wird zum Lactol A-IV reduziert. Als Reduktionsmittel eignen sich in ihrer Reaktivität modifizierte Aluminiumhydride wie z.B. Diisobutylaluminiumhydrid. Die Reaktion erfolgt in einem inerten Lösungsmittel wie z.B. Toluol, vorzugsweise bei niedrigen Temperaturen.
Schritt c (A-IV → A-V):
   Das Lactol A-IV wird unter Erweiterung um ein C-Atom zum Hydroxyolefin A-V geöffnet. Hierzu eignen sich die, dem Fachmann bekannten Methoden wie z.B. die Olefinierung nach Tebbe, die Wittig- oder Wittig/Horner-Reaktion, die Addition einer metallorganischen Verbindung unter Abspaltung von Wasser. Bevorzugt ist die Wittigreaktion unter Verwendung von Methyltriarylphosphoniumhalogeniden wie z.B. Methyltriphenylphosphoniumbromid mit starken Basen wie z.B. n-Butyllithium, Kaliumtert.-butanolat, Natriumethanolat, Natriumhexamethyldisilazan; als Base bevorzugt ist n-Butyllithium.
Schritt d (A-V→ A-VI):
   Die freie Hydroxygruppe in A-V wird nach den, dem Fachmann bekannten Methoden geschützt. Als Schutzgruppe PG⁵ kommen die, dem Fachmann bekannten Schutzgruppen, wie sie schon vorstehend für PG⁴ im Schritt a (A-II ### A-III) genannt wurden, in Frage.
   Bevorzugt sind solche Schutzgruppen, die unter Einwirkung von Fluorid gespalten werden können, wie z.B. der Trimethylsilyl-, tert.-Butyldimethylsilyl-, tert.-Butyldiphenylsilyl-, Tribenzylsilyl-, Triisopropylsilyl-Rest.
   Besonders bevorzugt ist der tert.-Butyldimethylsilyl-, der Triisopropylsilyl- und der tert.-Butyldiphenylsilyl-Rest.
Schritt e (A-VI → A-VII):
   An die Doppelbindung in A-VI wird nach anti-Markovnikov Wasser addiert. Hierzu eignen sich die dem Fachmann bekannten Verfahren wie z.B. die Umsetzung mit Boranen, deren anschließende Oxidation zu den entsprechenden Borsäureestern und deren Verseifung. Als Borane bevorzugt sind z.B. der Boran-Tetrahydrofuran-Komplex, der Boran-Dimethylsulfid-Komplex, 9-Borabicyclo[3.3.1]nonan in einem inerten Lösungsmittel wie beispielsweise Tetrahydrofuran oder Diethylether. Als Oxidationsmittel wird vorzugsweise Wasserstoffperoxid verwendet, zur Verseifung der Borester vorzugsweise Alkalihydroxide wie z.B. Natriumhydroxid.
Schritt f (A-VI → A-VII):
   Die unter Schritt a) eingeführte Schutzgruppe PG⁴ wird nun nach den dem Fachmann bekannten Verfahren gespalten. Handelt es sich um eine sauer spaltbare Schutzgruppe, so eignen sich für die Spaltung verdünnte Mineralsäuren in wässrig-alkoholischen Lösungen, die Verwendung von katalytischen Mengen Säuren wie z.B. para-Toluolsulfonsäure, para-Toluolsulfonsäure-pyridiniumsalz, Camphersulfonsäure in alkoholischen Lösungen, vorzugsweise in Ethanol oder Isopropanol.
Schritt g (A-VII → A-IX):
   Ein gemeinsamer Schutz beider Alkoholfunktionen des monogeschützten 1.3-Diols in A-VII ist durch direkte Ketalisierung mit einer Carbonylverbindung der allgemeinen Formel R^{15a}CO-R^{15b}, oder durch Umketalisierung mit einem Ketal der allgemeinen Formeln, R^{15a}-C(OC₂H₅)₂-R^{15b}, R^{15a}-C(OC₂H₄)₂-R^{15b}, R^{15a}-C(OCH₂C(CH₃)₂CH₂O)-R^{15b} worin jeweils R^{15a} und R^{15b} die oben angegebenen Bedeutungen haben, unter Säurekatalyse möglich. Als Säuren eignen sich die bereits unter Schritt f) genannten Säuren, bevorzugt ist die Verwendung von para-Toluolsulfonsäure gegebenenfalls unter Zusatz von Kupfer(II)- oder Kobalt(II)-Salzen wie z.B. Kupfer(II)sulfat.
Schritt h (A-VIII → A-IX):
   Ein Schutz beider Alkoholfunktionen des 1.3-Diols in A-VIII ist durch direkte Ketalisierung mit einer Carbonylverbindung der allgemeinen Formel R^{15a}-CO-R^{15b}, oder durch Umketalisierung mit einem Ketal der allgemeinen Formeln, R^{15a}-C(OC₂H₅)₂-R^{15b}, R^{15a}-C(OC₂H₄)₂-R^{15b}, R^{15a}-C(OCH₂C(CH₃)₂CH₂O)-R^{15b} worin jeweils R^{15a} und R^{15b} die oben angegebenen Bedeutungen haben, unter Säurekatalyse möglich. Bevorzugt ist die Umketalisierung vorzugsweise mit 2,2-Dimethoxypropan. Als Säuren eignen sich die bereits unter Schritt f) genannten Säuren, bevorzugt ist die Verwendung von Camphersulfonsäure.
Schritt i (A-lX → A-X):
   Die unter Schritt d) eingeführte Schutzgruppe PG⁵ wird nun nach den dem Fachmann bekannten Verfahren gespalten. Handelt es sich um einen Silylether, so eignet sich für die Spaltung die Umsetzung mit Fluoriden wie beispielsweise Tetrabutylammoniumfluorid, dem Fluorwasserstoff-Pyridin-Komplex, Kaliumfluorid oder die Anwendung verdünnter Mineralsäuren, die Verwendung von katalytischen Mengen Säuren wie z.B. para-Toluolsulfonsäure, para-Toluolsulfonsäure-pyridiniumsalz, Camphersulfonsäure in alkoholischen Lösungen, vorzugsweise in Ethanol oder Isopropanol.
Schritt k (A-X → A-XI):
   Die Oxidation des primären Alkohols in A-X zum Aldehyd erfolgt nach den, dem Fachmann bekannten Methoden. Beispielsweise genannt sei die Oxidation mit Pyridiniumchlorochromat, Pyridiniumdichromat, Chromtrioxid-Pyridin-Komplex, die Oxidation nach Swern oder verwandter Methoden z.B. unter Verwendung von Oxalylchlorid in Dimethylsulfoxid, die Verwendung des Dess-Martin-Periodinans, die Verwendung von Stickstoffoxiden wie z.B. N-Methyl-morpholino-N-oxid in Gegenwart geeigneter Katalysatoren wie z.B. Tetrapropylammoniumperruthenat in inerten Lösungsmitteln. Bevorzugt ist die Oxidation nach Swern sowie mit N-Methyl-morpholino-N-oxid unter Verwendung von Tetrapropylammoniumperruthenat.
Schritt I (A-XI → A-XII):
   Die Umsetzung der Aldehyde A-XI zu Alkoholen der Formel A-Xll erfolgt mit metallorganischen Verbindungen der allgemeinen Formel M-CHR^{2a'}R^{2b'}, worin M für ein Alkalimetall, vorzugsweise Lithium oder ein zweiwertiges Metall MX, worin X ein Halogen repräsentiert und die Reste R^{2a'} und R^{2b'} jeweils die oben genannten Bedeutungen aufweisen. Als zweiwertiges Metall ist bevorzugt Magnesium und Zink, als Halogen X ist bevorzugt Chlor, Brom und lod.
Schritt m (A-XII → A-XIII):
   Die Oxidation des sekundären Alkohols in A-XII zum Keton A-XIII erfolgt nach den, unter Schritt k) genannten Bedingungen. Bevorzugt ist die Oxidation mit N-Methyl-morpholino-N-oxid unter Verwendung von Tetrapropylammoniumperruthenat.
Schritt n (A-XIII → A-XIV):
   Für den Fall, daß R^{2a'} in A-XIII gleich Wasserstoff ist, besteht die Möglichkeit, hierfür einen zweiten Rest R^{2a'}, der die oben genannten Bedeutungen, ausgenommen Wasserstoff besitzt, einzuführen. Hierzu wird unter Anwendung starker Basen wie z.B. Lithiumdiisopropylamid das Keton in A-XIII in das Enolat überführt und mit einer Verbindung der allgemeinen Formel X-R^{2a'}, worin X ein Halogen repräsentiert umgesetzt. Als Halogen X ist bevorzugt Chlor, Brom und lod.

Der zuvor beschriebene Weg kann ebenfalls dazu benutzt werden, C1-C6-Epothilon-Bausteine zu synthetisieren, die an C-1 eine Carbonsäure oder deren Ester enthalten (R¹³=CO₂R^{13b} in A).

Die Synthese des Bausteins A-XXII wird im folgenden Schema 2 am Beispiel der von D-(-)-Pantolacton abgeleiteten Zwischenstufe A-V beschrieben. Aus L-(+)-Pantolacton erhält man die entsprechenden, zu A-V bis A-XXVII enantiomeren Verbindungen ent-A-V bis ent-A-XXVII und aus racemischem DL-Pantolacton die entsprechenden racemischen Verbindungen rac-A-V bis rac-A-XXVII: 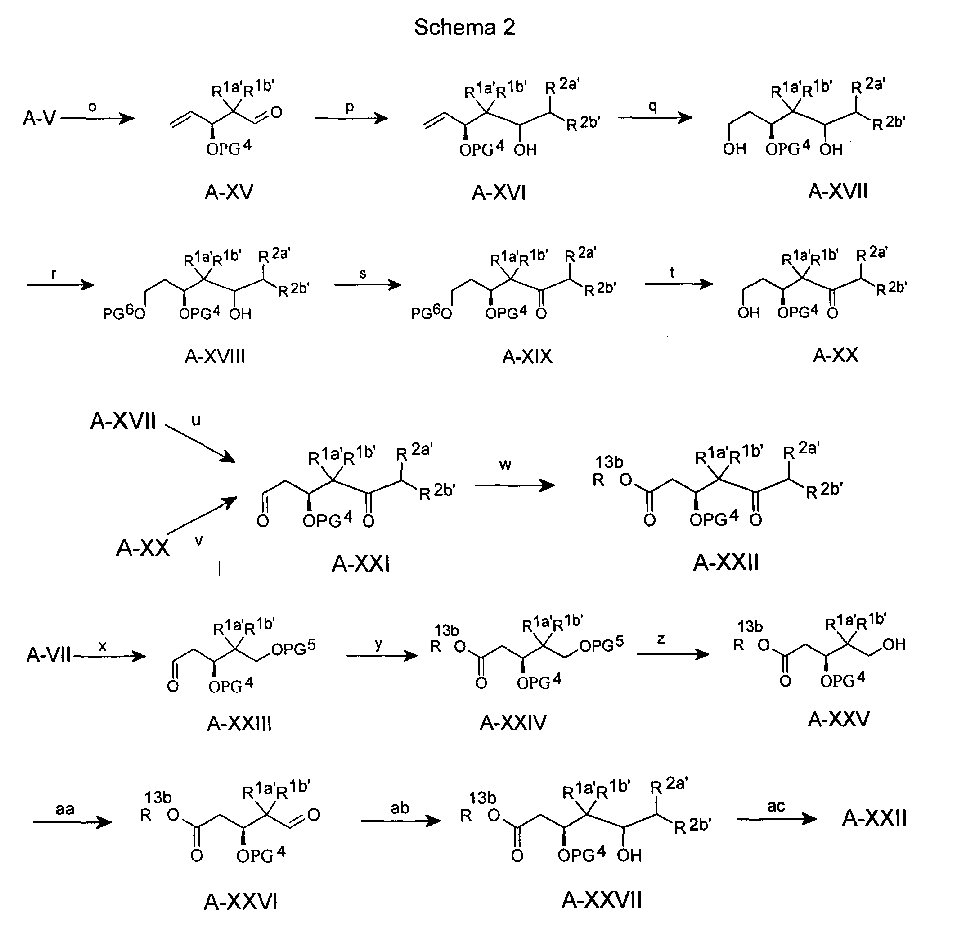
Schritt o (A-V → A-XV):
   Die Oxidation des primären Alkohols in A-V zum Aldehyd A-XV erfolgt nach den, unter Schritt k) genannten Bedingungen. Bevorzugt ist das Oxidationsverfahren nach Swern.
Schritt p (A-XV → A-XVI):
   Die Umsetzung der Aldehyde A-XV zu Alkoholen der Formel A-XVI erfolgt mit metallorganischen Verbindungen der allgemeinen Formel M-CHR^{2a'}R^{2b'}, worin M für ein Alkalimetall, vorzugsweise Lithium oder ein zweiwertiges Metall MX, worin X ein Halogen repräsentiert und die Reste R^{2a}' und R^{2b}' jeweils die oben genannten Bedeutungen aufweisen. Als zweiwertiges Metall ist bevorzugt Magnesium und Zink, als Halogen X ist bevorzugt Chlor, Brom und lod.
Schritt q (A-XVI → A-XVII):
   An die Doppelbindung in A-XVI wird nach anti-Markovnikov Wasser addiert. Hierzu eignen sich die unter e) beschriebenen Verfahren.
Schritt r (A-XVII → A-XVIII):
   Die freie Hydroxygruppe in A-XVII wird nach den, dem Fachmann bekannten Methoden geschützt. Als Schutzgruppe PG⁶ kommen die, dem Fachmann bekannten Schutzgruppen, wie sie schon vorstehend für PG⁴ im Schritt a (A-II ### A-III) genannt wurden, in Frage.
   Bevorzugt sind solche Schutzgruppen, die unter basischen oder hydrogenolytischen Reaktionsbedingungen gespalten werden können, wie z.B. Benzyl-, para-Nitrobenzyl-, Acetyl-, Propionyl-, Butyryl-, Benzoyl -Rest.
   Besonders bevorzugt ist der Benzoyl-Rest.
Schritt s (A-XVIII → A-XIX):
   Die Oxidation des sekundären Alkohols in A-XVII zum Keton A-XIX erfolgt nach den, unter Schritt k) genannten Bedingungen. Bevorzugt ist die Oxidation mit N-Methyl-morpholino-N-oxid unter Verwendung von Tetrapropylammoniumperruthenat.
Schritt t (A-XIX → A-XX):
   Die Schutzgruppe PG⁶ in XIX wird nun selektiv gespalten. Handelt es sich um eine hydrogenolytisch spaltbare Schutzgruppe, so wird vorzugsweise in Gegenwart von Palladium- oder Platin-Katalysatoren in inerten Lösungsmitteln wie beispielsweise Ethylacetat oder Ethanol hydriert. Handelt es sich um eine basisch spaltbare Schutzgruppe, so findet vorzugsweise Verwendung die Verseifung mit Carbonaten in alkoholischer Lösung wie z.B. Kaliumcarbonat in Methanol, die Verseifung mit wässrigen Lösungen von Alkalihydroxiden wie z.B. Lithiumhydroxid oder Natriumhydroxid unter Verwendung von organischen, mit Wasser mischbaren Lösungsmitteln wie z.B. Methanol, Ethanol, Tetrahydrofuran oder Dioxan.
Schritt u (A-XVII → A-XXI):
   Die Oxidation der Alkohole in A-XVII zum Ketoaldehyd A-XXI erfolgt nach den, unter Schritt k) genannten Bedingungen. Bevorzugt ist die Oxidation mit N-Methyl-morpholino-N-oxid unter Verwendung von Tetrapropylammoniumperruthenat sowie die Methode nach Swern.
Schritt v (A-XX → A-XXI):
   Die Oxidation des primären Alkohols in A-XX zum Ketoaldehyd A-XXI erfolgt nach den, unter Schritt k) genannten Bedingungen. Bevorzugt ist die Oxidation mit N-Methyl-morpholino-N-oxid unter Verwendung von Tetrapropylammoniumperruthenat.
Schritt w (A-XXI → A-XXII):
   Die Oxidation des Aldehydes in A-XXI zur Carbonsäure A-XXII (R^{13b}=Wasserstoff) erfolgt nach den, dem Fachmann bekannten Methoden. Beispielsweise genannt sei die Oxidation nach Jones, die Oxidation mit Kaliumpermanganat beispielsweise in einem wässrigen System aus tert.-Butanol und Natriumdihydrogenphosphat, die Oxidation mit Natriumchlorit in wässrigem tert.-Butanol gegebenenfalls in Gegenwart eines Chlorfängers wie z.B. 2-Methyl-2-buten.
   Die Oxidation des Aldehydes in A-XXI zum Ester A-XXII, worin R^{13b} die oben genannten Bedeutungen hat und ungleich Wasserstoff ist, kann beispielsweise mit Pyridiniumdichromat und dem gewünschten Alkohol HO-R^{13b} in einem inerten Lösungsmittel wie z.B. Dimethylformamid erfolgen.
Schritt x (A-VII → A-XXIII):
   Die Oxidation des primären Alkohols in A-VII zum Aldehyd A-XXIII erfolgt nach den, unter Schritt k) genannten Bedingungen. Bevorzugt ist die Oxidation mit N-Methyl-morpholino-N-oxid unter Verwendung von Tetrapropylammoniumperruthenat sowie die Methode nach Swern.
Schritt y (A-XXIII → A-XXIV):
   Die Oxidation des Aldehyds A-XXIII zur Carbonsäure bzw. deren Ester A-XXIV erfolgt nach den bereits unter w) beschriebenen Bedingungen.
Schritt z (A-XXIV → A-XXV):
   Die unter Schritt d) eingeführte Schutzgruppe PG⁵ wird wie unter Schritt i beschrieben gespalten.
Schritt aa (A-XXV→ A-XXVI):
   Die Oxidation des primären Alkohols in A-XXV zum Aldehyd A-XXVI erfolgt nach den, unter Schritt k) genannten Bedingungen. Bevorzugt ist die Oxidation mit N-Methyl-morpholino-N-oxid unter Verwendung von Tetrapropylammoniumperruthenat sowie die Methode nach Swern.
Schritt ab (A-XXVI → A-XXVII):
   Die Umsetzung der Aldehyde A-XXVI zu Alkoholen der Formel A-XXVII erfolgt nach den, unter Schritt I) genannten Bedingungen.
Schritt ac (A-XXVII → A-XXII):
   Die Oxidation des sekundären Alkohols in A-XXVII zum Keton A-XXII erfolgt nach den, unter Schritt k) genannten Bedingungen. Bevorzugt ist die Oxidation mit N-Methyl-morpholino-N-oxid unter Verwendung von Tetrapropylammoniumperruthenat.

Die Verbindungen der Formel A, in der R^{1a'} und R^{1b'} alle die in der allgemeinen Formel A angegebenen Bedeutungen haben können, lassen sich ferner aus wohlfeilen oder leicht zugänglichen Malonsäuredialkylestern auf effiziente Weise mit hoher optischer Reinheit herstellen.

Die Synthese wird im folgenden Schema 3 beschrieben: 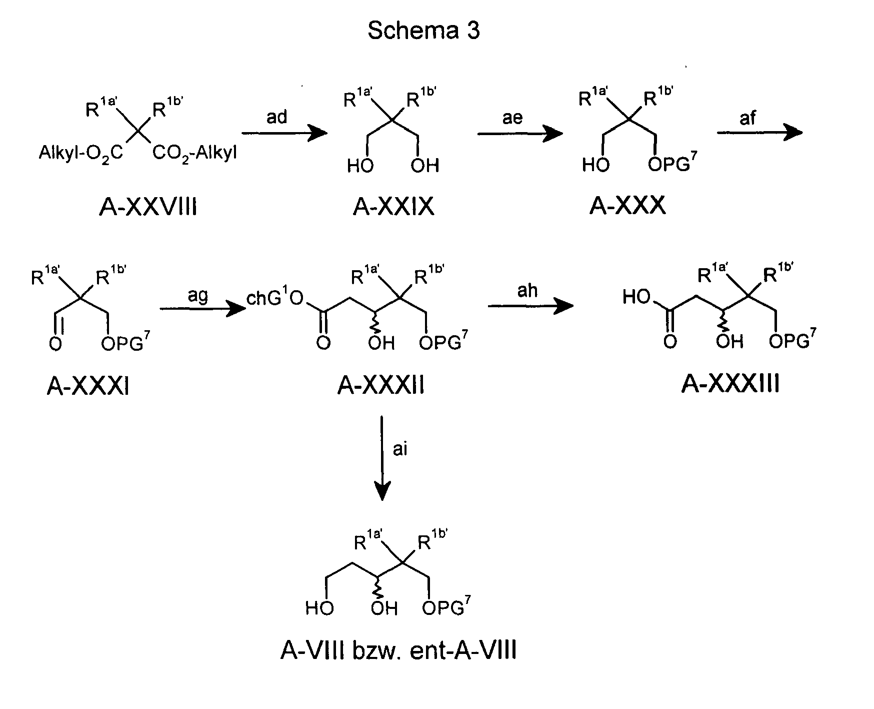
Schritt ad (A-XXVIII→A-XXIX):
   Entsprechend substituierte Malonsäuresterderivate A-XXVIII, die entweder käuflich sind oder nach den, dem Fachmann bekannten Verfahren aus Malonsäuren oder deren Alkylestern hergestellt werden können, werden zu Diolen A-XXIX reduziert. Hierzu eignen sich die, dem Fachmann bekannten Reduktionsmittel wie z.B. Diisobutylaluminiumhydrid, komplexe Metallhydride wie z.B. Lithiumaluminiumhydrid.
Schritt ae (A-XXIX→A-XXX) :
   Eine freie Hydroxylgruppe in A-XXIX wird nach den, dem Fachmann bekannten Methoden selektiv geschützt. Als Schutzgruppe PG⁷ kommen die, dem Fachmann bekannten Schutzgruppen, wie sie schon vorstehend für PG⁴ im Schritt a (A-II ### A-III) genannt wurden, in Frage.
   Bevorzugt sind Silizium-haltige Schutzgruppen.
Schritt af (A-XXX→A-XXXI) :
   Die Oxidation der verbliebenen, primären Hydroxylgruppe in A-XXX zum Aldehyd A-XXXI erfolgt nach den, unter Schritt k) genannten Bedingungen. Bevorzugt ist die Oxidation mit N-Methyl-morpholino-N-oxid unter Verwendung von Tetrapropylammoniumper-ruthenat, die Verwendung von Pyridiniumchlorochromat, Pyridiniumdichromat sowie die Methode nach Swern.
Schritt ag (A-XXXI→A-XXXII):
   Die Aldehyde A-XXXI werden mit einem Ester der Essigsäure chG¹OC(O)CH3, worin chG¹ eine chirale Hilfsgruppe bedeutet, im Sinne einer Aldolreaktion umgesetzt. Die Verbindungen chG¹OC(O)CH₃ werden in optisch reiner Form in die Aldolreaktion eingesetzt. Die Art der chiralen Hilfsgruppe bestimmt, ob die Aldolreaktion mit hoher Diastereoselektivität verläuft oder ein mit physikalischen Methoden trennbares Diastereomerengemisch ergibt. Eine Übersicht über vergleichbare diastereoselektive Aldolreaktionen findet sich in Angew. Chem. 99 (1987), 24-37. Als chirale Hilfsgruppen chG¹-OH eignen sich beispielsweise optisch reines 2-Phenyl-cyclohexanol, Pulegol, 2-Hydroxy-1,2,2-triphenylethanol, 8-Phenylmenthol.
Schritt ah (A-XXXII→A-XXXIII):
   Die diastereomerenreinen Verbindungen A-XXXII können dann nach dem Fachmann bekannten Verfahren durch Verseifung der Estereinheit unter gleichzeitiger Freisetzung der wiederverwendbaren chiralen Hilfskomponente chG¹-OH in enantiomerenreine Verbindungen des Typs A-XXXIII oder ent-A-XXXIII überführt werden. Für die Verseifung geeignet sind Carbonate in alkoholischer Lösung wie z.B. Kaliumcarbonat in Methanol, wässrige Lösungen von Alkalihydroxiden wie z.B. Lithiumhydroxid oder Natriumhydroxid unter Verwendung von organischer, mit Wasser mischbaren Lösungsmitteln wie z.B. Methanol, Ethanol, Tetrahydrofuran oder Dioxan.
Schritt ai (A-XXXII→A-VIII):
   Alternativ zum Schritt ah kann die chirale Hilfsgruppe auch reduktiv entfernt werden. Auf diese Weise werden die enantiomerenreinen Verbindungen des Typs A-VIII bzw. ent-A-VIII erhalten. Die Reduktion kann nach den, dem Fachmann bekannten Verfahren durchgeführt werden. Als Reduktionsmittel kommen z.B. Düsobutyl-aluminiumhydrid und komplexe Metallhydride wie z.B. Lithiumaluminiumhydrid in Frage.

Die Verbindungen A-VIII bzw. ent-A-VIII können wie zuvor beschrieben in Verbindungen des Typs A-XIII bzw. ent-A-XIII überführt werden. Entsprechend lassen sich Verbindungen des Typs A-XXXIII bzw. ent-A-XXXIII gemäß oben beschriebenen Verfahren in Verbindungen des Typs A-XXII bzw. ent-A-XXII überführen.

Alternativ zum oben geschilderten Weg kann die Sequenz auch ohne Verwendung einer chiralen Hilfsgruppe chG¹ durchgeführt werden. Auf diese Weise werden dann racemische Mischungen von Verbindungen des Typs rac-A-VIII bzw. rac-A-XXXIII über die entsprechenden, racemischen Vorstufen erhalten. Diese Mischungen können wiederum nach den, dem Fachmann bekannten Verfahren zur Racematspaltung, z.B. Chromatographie an chiralen Säulen, getrennt werden. Die Fortsetzung der Synthese kann aber auch mit den racemischen Gemischen erfolgen.

Die vorliegende Erfindung betrifft somit auch ein Verfahren zur Herstellung der Verbindungen der allgemeinen Formel A, welches dadurch gekennzeichnet ist, daß
a) ein Pantolacton der allgemeinen Formel lla oder
b) ein Malonsäuredialkylester der allgemeinen Formel XXVIII
als Ausgangsprodukt verwendet wird.

Die vorliegende Erfindung betrifft somit außerdem die neuen C1-C6-Epothilon-Bausteine der allgemeinen Formel A' 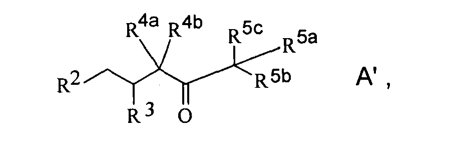 worin
- R²: CH₂OR^{2a}, CHO, CO₂R^{2b}, COX,
- R^{2a}, R^{2b}: Wasserstoff, C₁-C₂₀-Alkyl, Aryl, C₇-C₂₀-Aralkyl,
- R³: Wasserstoff, OR^{3a}, X, OSO₂R^{3b},
- R^{3a}: Wasserstoff oder gemeinsam mit R^{2a} eine -(CH₂)ₙ-Gruppe oder eine CR^{6a}R^{6b}-Gruppe,
- R^{3b}: C₁-C₄-Alkyl, Aryl,
- X: Halogen,
- n: 2 bis 4,
- R^{6a}, R^{6b}: gleich oder verschieden sind und C₁-C₈-Alkyl, C₆-C₁₀-Aryl, oder gemeinsam eine -(CH₂)ₒ-Gruppe,
- o: 3 bis 6,
- R^{6a}: zusätzlich die Bedeutung von Wasserstoff annehmen kann,
- R^{4a}, R^{4b}: gleich oder verschieden sind und Wasserstoff, C₁-C₁₀-Alkyl, C₇-C₂₀-Aralkyl, oder gemeinsam eine -(CH₂)ₘ-Gruppe,
- m: 2 bis 5,
- R^{5a}, R^{5b}: gleich oder verschieden sind und Wasserstoff, C₁-C₁₀-Alkyl, C₇-C₂₀-Aralkyl, oder gemeinsam eine -(CH₂)ₚ-Gruppe,
- p: 2 bis 5,
- R^{5c}: Wasserstoff,
einschließlich aller Stereoisomeren sowie deren Gemische
bedeuten sowie
freie Hydroxylgruppen in R² und R³ verethert oder verestert, freie Carbonylgruppen in A und R² ketalisiert, in einen Enolether überführt oder reduziert sowie freie Säuregruppen in A in deren Salze mit Basen überführt sein können,
ausgenommen der Verbindungen

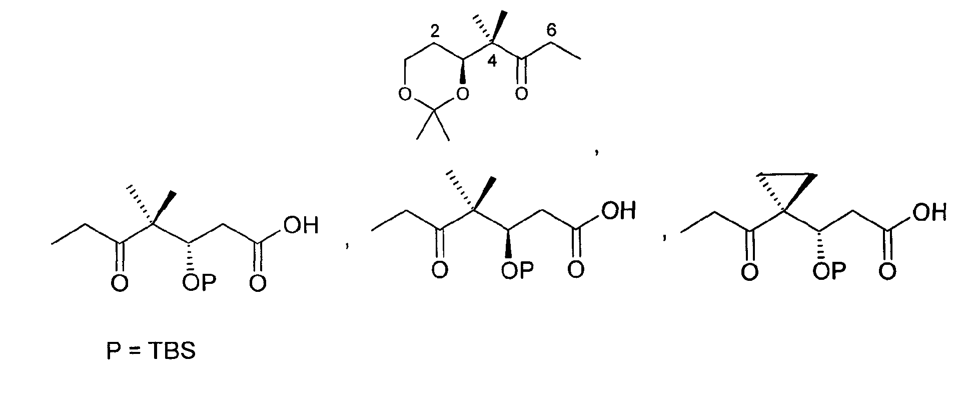

Es wurde ferner gefunden, daß Synthesebausteine der allgemeinen Formel A" 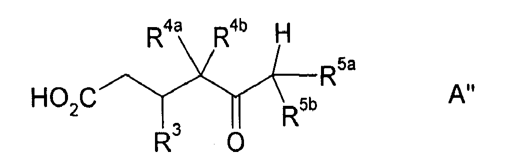 worin
- R³: OR^{3a} und
- R^{3a}: Wasserstoff oder eine Schutzgruppe PG
- R^{4a}, R^{4b}: gleich oder verschieden sind und Wasserstoff, C₁-C₁₀-Alkyl, C₇-C₂₀-Aralkyl, oder gemeinsam eine -(CH₂)ₘ-Gruppe,
- m: 2 bis 5,
- R^{5a}, R^{5b}: gleich oder verschieden sind und Wasserstoff, C₁-C₁₀-Alkyl, C₇-C₂₀-Aralkyl, oder gemeinsam eine -(CH₂)ₚ-Gruppe,
- p: 2 bis 5,
einschließlich aller Stereoisomeren sowie deren Gemische
bedeuten sowie
freie Carbonylgruppen in I ketalisiert sein können,

leicht durch Umsetzung einer Verbindung der allgemeinen Formel II 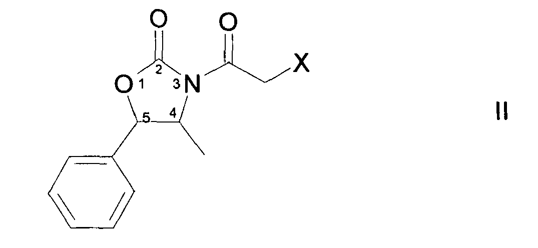 worin
X ein Chlor- oder Bromatom ist, und der 2-Oxazolidinon-Ring entweder (4R,5S)- oder (4S,5R)-Konformation aufweist,
mit einer Verbindung der allgemeinen Formel III 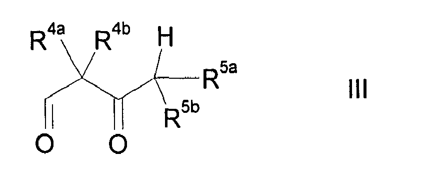 worin
- R^{4a}, R^{4b}: gleich oder verschieden sind und Wasserstoff, C₁-C₁₀-Alkyl, C₇-C₂₀-Aralkyl, oder gemeinsam eine -(CH₂)ₘ-Gruppe,
- m: 2 bis 5,
- R^{5a}, R^{5b}: gleich oder verschieden sind und Wasserstoff, C₁-C₁₀-Alkyl, C₇-C₂₀-Aralkyl, oder gemeinsam eine -(CH₂)p-Gruppe,
- p: 2 bis 5,
bedeuten,
zu einer Verbindung der allgemeinen Formel IV 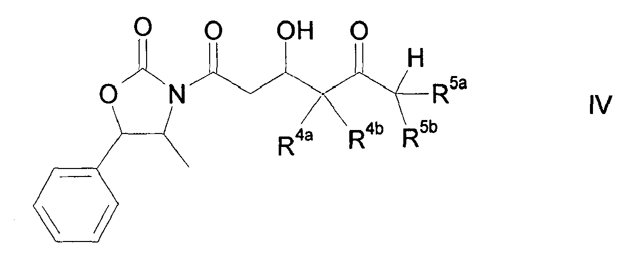 worin
der 2-Oxazolidinon-Ring (4R,5S)- und das 3'-Kohlenstoffatom R-Konformation oder
der 2-Oxazolidinon-Ring (4S,5R)- und das 3'-Kohlenstoffatom S-Konformation aufweisen,
sowie nach Schutz der 3'-Hydroxygruppe in IV mit einer Schutzgruppe PG, durch Abspaltung des Oxazolidinon-Restes und gegebenenfalls Abspaltung der Schutzgruppe PG hergestellt werden können.

Die Umsetzung einer Verbindung der allgemeinen Formel II mit einer Verbindung der allgemeinen Formel III gelingt nach Überführung der Verbindung der allgemeinen Formel II in ein Metallenolat durch Insertion eines Metalls oder Metallsalzes in die Kohlenstoff-Halogen-Bindung der Verbindung der allgemeinen Formel II.
Als Metall oder Metallsalz kommen generell alle dem Fachmann bekannten Metalle oder Metallsalze in Frage, die für eine Reformatzky-Reaktion geeignet sind (siehe z.B. A. Fürstner, Synthesis 1989, 571 - 590).
Erfindungsgemäß wird vorzugsweise Chrom(II)chlorid verwendet.

Der Oxazolidon-Ring wird bei der Abspaltung aus den Verbindungen der allgemeinen Formel IV fast quantitativ und ohne Verlust der optischen Aktivität zurückgewonnen.

Als Alkylgruppen R^{4a}, R^{4b}, R^{5a} und R^{5b} sind gerad- oder verzweigtkettige Alkylgruppen mit 1 bis maximal 10 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R^{4a}, R^{4b}, R^{5a} und R^{5b} können perfluoriert oder substituiert sein durch 1-5 Halogenatome, Hydroxygruppen, C₁-C₄-Alkoxygruppen und C₆-C₁₂-Arylgruppen (die durch 1-3 Halogenatome substituiert sein können).

Die Aralkylgruppen in R^{4a}, R^{4b}, R^{5a} und R^{5b} können im Ring bis 14 C-Atome, bevorzugt 6 bis 10 enthalten und in der Alkylkette 1 bis 8, bevorzugt 1 bis 4 Atome. Als Aralkylreste kommen beispielweise in Betracht Benzyl, Phenylethyl, Naphthylmethyl, Naphthylethyl, Furylmethyl, Thienylethyl, Pyridylpropyl. Die Ringe können ein- bis dreifach substituiert sein durch Halogen, OH, O-Alkyl, NH₂, CO₂H, CO₂-Alkyl -NO₂, -N₃, -CN, C₁-C₂₀-Alkyl, C₁-C₂₀-Acyl, C₁-C₂₀-Acyloxy-Gruppen.

Als Schutzgruppe PG kommen alle, dem Fachmann als derartige Schutzgruppen bekannten Reste in Betracht. Bevorzugt sind hierbei silylhaltige Schutzgruppen, wie beispielsweise der Trimethylsilyl-, Triethylsilyl-, tert.-Butyldimethylsilyl-, tert.-Butyldiphenylsilyl-, Tribenzylsilyl-, Trüsopropylsilyl-Rest.
Eine Übersicht über Schutzgruppen findet sich z.B. in "Protective Groups in Organic Synthesis" Theodora W. Green, John Wiley and Sons).

Halogen bedeutet Fluor, Chlor, Brom und lod.

Die für das erfindungsgemäße Verfahren benötigten Verbindungen der allgemeinen Formel II sind durch Acetylierung von (4R,5S)- bzw. (4S,5R)-4-Methyl-5-phenyl-2-oxazolidinon mit Brom- oder Chloracetylchlorid in Gegenwart einer starken Base, wie beispielsweise n-Butyllithium, zugänglich.
Durch die Wahl des chiralen Auxiliars wird später die Stereochemie der Hydroxygruppe in Position 3 gesteuert.

Die für das erfindungsgemäße Verfahren benötigten Verbindungen der allgemeinen Formeln III sind käuflich oder lassen sich einfach herstellen.
Sofern die Verbindungen der allgemeinen Formel III nicht käuflich sind, lassen sie sich beispielsweise nach den in Abb. 1 und 2 angegebenen Methoden herstellen.

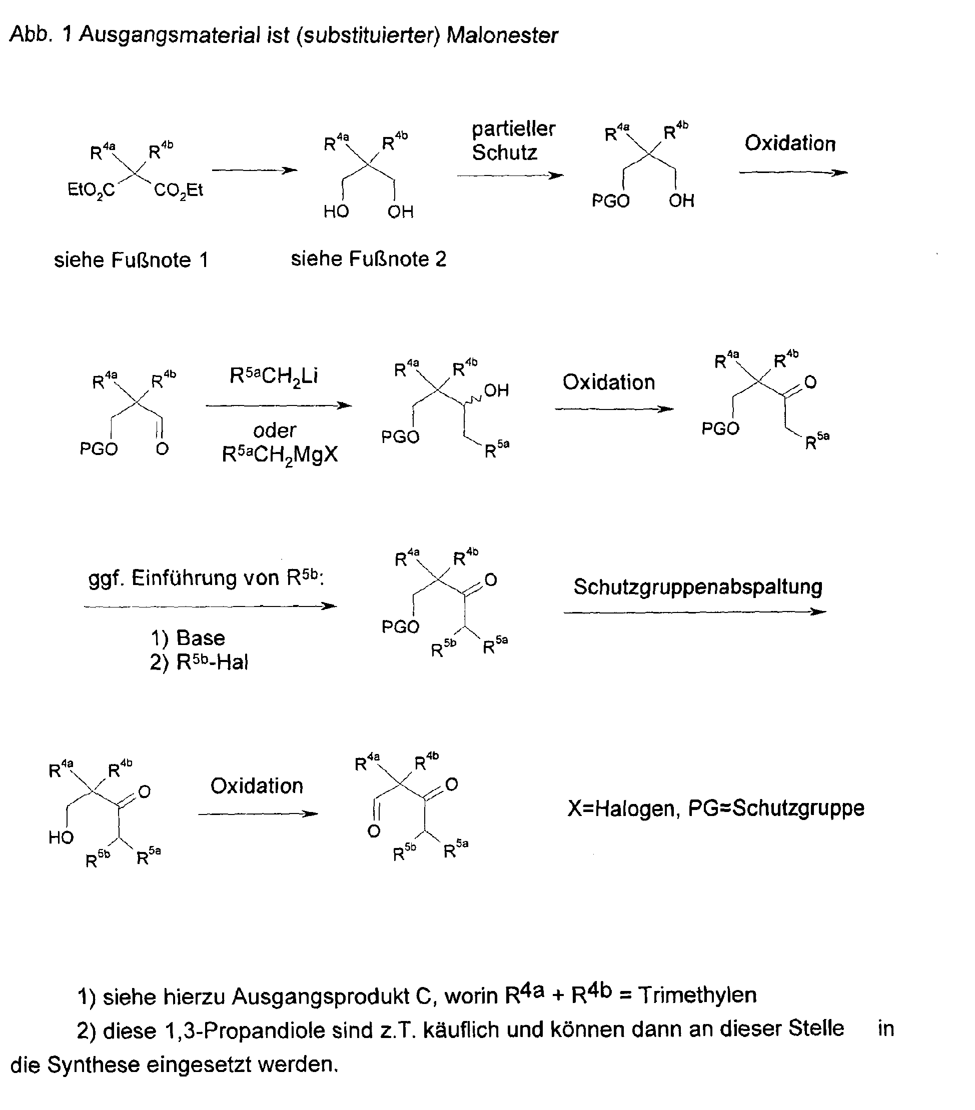

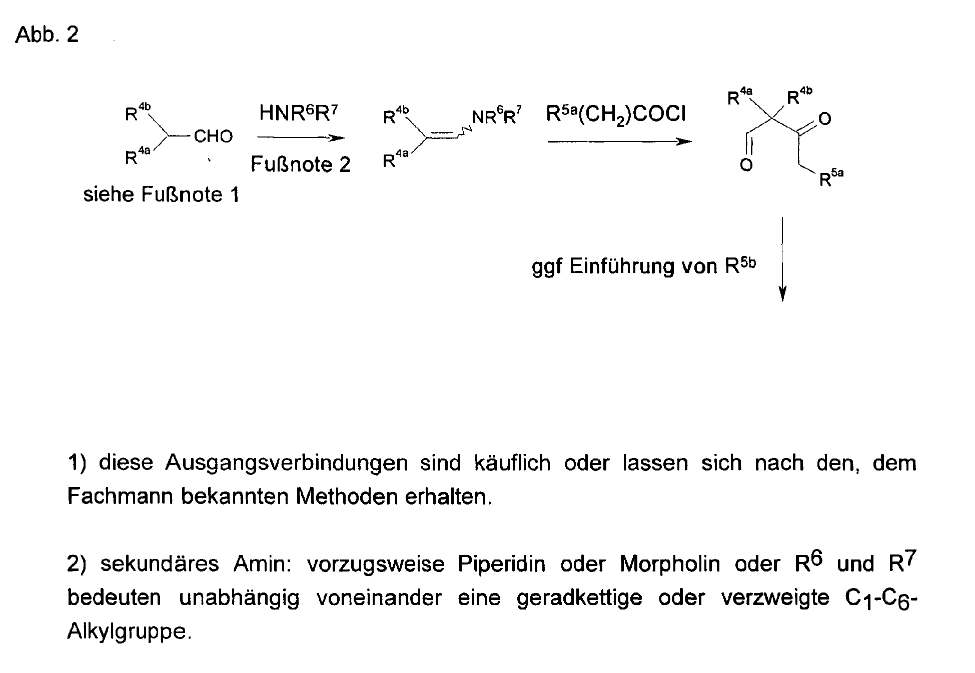

Die gemäß vorliegender Erfindung hergestellten Bausteine der allgemeinen Formel I können analog zu beschriebenen, beispielsweise aus den auf der Seite 2 dieses Anmeldetextes (Schinzer et al.: Chem.Eur.J. 1996, 2, No. 11, 1477-1482; Angew. Chem. 1997, 109, Nr. 5, S. 543-544; Nicolaou et al.: Angew. Chem. 1997, 109, Nr. 1/2, S. 170-172; Nature, Vo. 387, 1997, S. 268-272; J.Am.Chem.Soc., Vol. 119, No. 34, 1997, S. 7960-7973; J.Am.Chem.Soc., Vol. 119, No. 34, 1997, S. 7974-7991; Angew. Chem. 1997, 109, Nr. 19, S. 2181-2187) hervorgehenden Methoden zur Synthese von Epothilon A und B sowie von im C₁-C₆-Abschnitt des Epothilongerüstes entsprechend modifizierten Epothilonderivaten verwendet werden.

Mit den Verbindungen der allgemeinen Formel A" wird somit die eingangs geforderte Variabilität der Substituenten erreicht.
Ein großer Vorteil des erfindungsgemäßen Verfahrens liegt auch darin, daß sich das verwendete chirale Auxiliar (4R,5S)- bzw. (4S,5R)-4-Methyl-5-phenyl-2-oxazolidinon nach seiner Abspaltung aus der geschützten Verbindung der allgemeinen Formel IV einfach wiedergewinnen und erneut ohne Verlust an optischer Induktion in die Synthese wieder einsetzen läßt.

Die auf diesen Wegen erhaltenen Bausteine, auch deren Enantiomere oder Gemische aus diesen Enantiomeren, eignen sich für die Aldokondensation mit einem Epothilonbaustein, der an C-7 (Epothilon-Zählweise) eine Carbonylfunktion trägt , wie dies bei den oben genannten Totalsynthesen von Epothilon A und Epothilon B der Fall ist.
Die Bausteine A, deren Enantiomere oder Gemische aus diesen Enantiomeren eignen sich darüber hinaus für die Veresterung mit einem Epothilonbaustein, der an C-15 (Epothilion-Zählweise) eine Hydroxylfunktion trägt, wie dies bei den oben genannten Totalsynthesen von Epothilon A und B der Fall ist.

### Darstellung der Teilfragmente B:

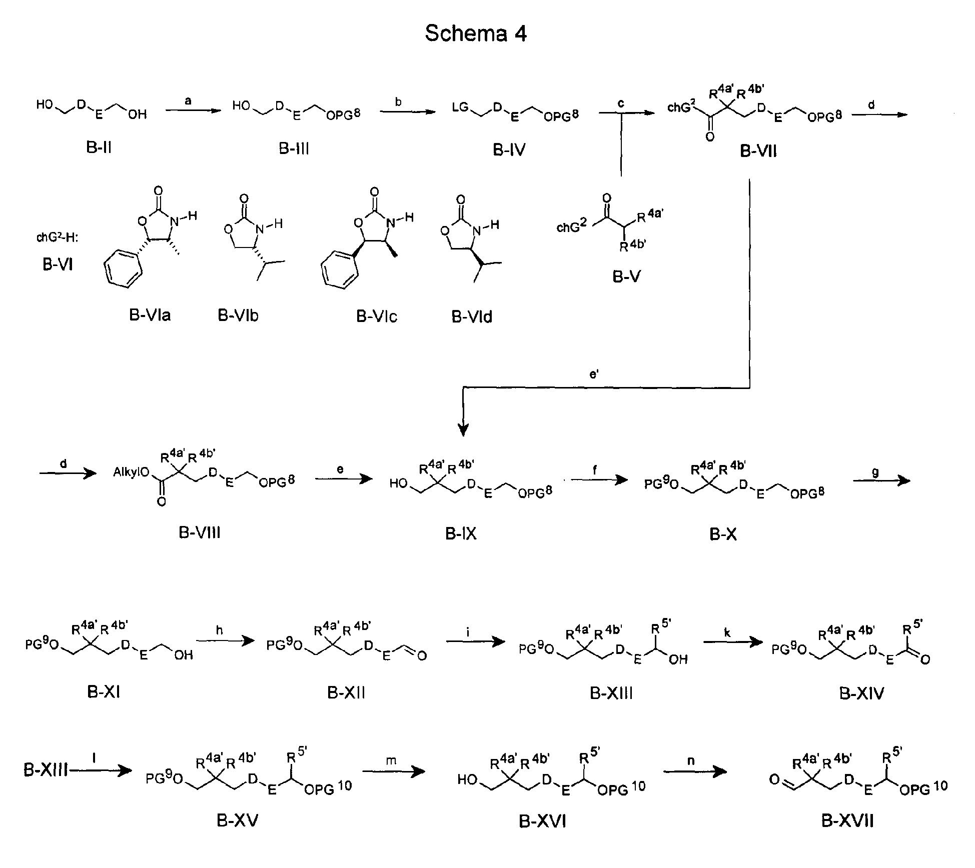

Schritt a (B-II → B-III):
   Eine Hydroxylgruppe in B-II wird nach den, dem Fachmann bekannten Methoden geschützt. Als Schutzgruppe PG⁸ kommen die, dem Fachmann bekannten Schutzgruppen, wie sie schon vorstehend für PG⁴ im Schritt a (A-II ### A-III) genannt wurden, in Frage.
   Bevorzugt sind Silizium haltige Schutzgruppen, die unter sauren Reaktions-bedingungen oder Anwendung von Fluorid gespalten werden können, wie z.B. der Trimethylsilyl-, Triethylsilyl-, tert.-Butyldimethylsilyl-, tert.-Butyldiphenylsilyl-, Tribenzylsilyl-, Triisopropylsilyl-Rest.
   Besonders bevorzugt ist der tert.-Butyldimethylsilyl-Rest.
Schritt b (B-III → B-IV):
   Die freie Hydroxylgruppe in B-lll wird nach den, dem Fachmann bekannten Methoden in eine Abgangsgruppe LG überführt. Als Abgangsgruppe LG eignen sich beispielsweise Halogene wie z.B. Brom oder lod oder Alkyl- bzw. Arylsulfonate, die aus den entsprechenden Sulfonsäurehalogeniden bzw. Sulfonsäureanhydriden nach den, dem Fachmann bekannten Methoden hergestellt werden.
   Als Abgangsgruppe LG bevorzugt ist das Trifluormethansulfonat.
Schritt c (B-IV → B-VII):
   Die Verbindung B-IV wird mit dem Enolat einer Carbonylverbindung der allgemeinen Formel B-V, worin chG² eine einfache Alkoxygruppe oder aber eine chirale Hilfsgruppe sein kann, nach den, dem Fachmann bekannten Methoden alkyliert. Das Enolat wird durch Einwirkung starker Basen wie z.B. Lithiumdiisopropylamid, Lithiumhexamethyldisilazan bei niedrigen Temperaturen hergestellt. Als chirale Hilfsgruppe chG²-H (B-VI) eignen sich chirale, optisch rein herstellbare und wohlfeile Alkohole wie z.B. Pulegol, 2-Phenylcyclohexanol, 2-Hydroxy-1,2,2-triphenylethanol, 8-Phenylmenthol oder optisch rein herstellbare und wohlfeile, reaktive NH-Gruppen enthaltende Verbindungen wie z.B. Amine, Aminosäuren, Lactame oder Oxazolidinone. Bevorzugt sind Oxazolidinone, besonders bevorzugt die Verbindungen der Formeln B-Vla bis B-Vld. Durch die Wahl des jeweiligen Antipoden wird die absolute Stereochemie am α-Carbonylkohlenstoff der Verbindung der allgemeinen Formel B-VII festgelegt. Auf diesem Wege lassen sich die Verbindungen der allgemeinen Formeln B-VII bis B-XVII bzw. deren jeweilige Enantiomere ent-B-VII bis ent-B-XVII enatiomerenrein erhalten. Wird als chG²-H (B-VI) ein achiraler Alkohol wie z.B. Ethanol eingesetzt, so erhält man die racemischen Verbindungen rac-B-VII bis rac-B-XVII.
Schritt d (B-VII → B-VIII):
   Repräsentiert die Gruppe chG² eine der unter Schritt c erwähnten chiralen Hilfsgruppen, so wird diese durch Umesterung von B-VII in einen Alkylester der allgemeinen Formel B-VIII wiedergewonnen. Die Umesterung erfolgt nach den, dem Fachmann bekannten Methoden. Bevorzugt ist die Umesterung mit einfachen Alkoholen wie z.B. Methanol oder Ethanol in Gegenwart entsprechender Titan(IV)alkoholate.
Schritt e (B-VIII → B-IX):
   Der Ester in B-VIII wird zum Alkohol B-IX reduziert. Als Reduktionsmittel eignen sich die, dem Fachmann bekannten Reduktionsmittel wie z.B. Aluminiumhydride wie z.B. Lithiumaluminiumhydrid oder Düsobutylaluminium-hydrid. Die Reaktion erfolgt in einem inerten Lösungsmittel wie z.B. Diethylether, Tetrahydrofuran, Toluol.
Schritt e' (B-VII → B-IX):
   Alternativ zu den Schritten d) und e) kann die Carbonylgruppe in B-VII nach den unter Schritt e) genannten Bedingungen direkt zu den Alkoholen der allgemeinen Formel B-IX reduziert werden. Auch hier kann die chirale Hilfskomponente chG²-H wiedergewonnen werden.
Schritt f (B-IX → B-X):
   Die freie Hydroxylgruppe in B-IX wird nach den, dem Fachmann bekannten Methoden geschützt. Als Schutzgruppe PG⁹ kommen die, dem Fachmann bekannten Schutzgruppen, wie sie schon vorstehend für PG⁴ im Schritt a (A-II ### A-III) genannt wurden, in Frage.
   Bevorzugt sind solche Schutzgruppen, die unter sauren Reaktionsbedingungen gespalten werden können, wie z.B. der Methoxymethyl-, Tetrahydropyranyl-, Tetrahydrofuranyl-, Trimethylsilyl-Rest.
   Besonders bevorzugt ist der Tetrahydropyranyl-Rest.
Schritt g (B-X → B-XI):
   Die unter Schritt a) eingeführte Schutzgruppe PG⁸ wird nun nach den, dem Fachmann bekannten Verfahren gespalten. Handelt es sich um einen Silylether, so eignet sich für die Spaltung die Umsetzung mit Fluoriden wie beispielsweise Tetrabutylammoniumfluorid, dem Fluorwasserstoff-Pyridin-Komplex, Kaliumfluorid oder die Anwendung verdünnter Mineralsäuren, die Verwendung von katalytischen Mengen Säuren wie z.B. para-Toluolsulfonsäure, para-Toluolsulfonsäure-pyridiniumsalz, Camphersulfonsäure in alkoholischen Lösungen, vorzugsweise in Ethanol oder Isopropanol.
Schritt h (B-XI → B-XII):
   Die Oxidation des primären Alkohols in B-XI zum Aldehyd der allgemeinen Formel B-XII erfolgt nach den, dem Fachmann bekannten Verfahren. Beispielsweise genannt sei die Oxidation mit Pyridiniumchlorochromat, Pyridiniumdichromat, Chromtrioxid-Pyridin-Komplex, die Oxidation nach Swern oder verwandter Methoden z.B. unter Verwendung von Oxalylchlorid in Dimethylsulfoxid, die Verwendung des Dess-Martin-Periodinans, die Verwendung von Stickstoffoxiden wie z.B. N-Methyl-morpholino-N-oxid in Gegenwart geeigneter Katalysatoren wie z.B. Tetrapropylammoniumperruthenat in inerten Lösungsmitteln. Bevorzugt ist die Oxidation nach Swern sowie mit N-Methyl-morpholino-N-oxid unter Verwendung von Tetrapropylammoniumperruthenat.
Schritt i (B-XII → B-XIII):
   Die Umsetzung der Aldehyde B-XII zu Alkoholen der allgemeinen Formel B-XIII erfolgt nach den, dem Fachmann bekannten Methoden mit metallorganischen Verbindungen der allgemeinen Formel M-R^{5'} worin M für ein Alkalimetall, vorzugsweise Lithium oder ein zweiwertiges Metall MX, worin X ein Halogen repräsentiert und der Rest R^{5'} die oben genannte Bedeutung aufweist. Als zweiwertiges Metall ist bevorzugt Magnesium und Zink, als Halogen X ist bevorzugt Chlor, Brom und lod.
Schritt k (B-XIII → B-XIV):
   Die Oxidation des Alkohols B-XIII zum Keton der allgemeinen Formel B-XIV erfolgt nach den unter h) genannten Verfahren. Bevorzugt ist die Oxidation mit N-Methyl-morpholino-N-oxid unter Verwendung von Tetrapropylammoniumperruthenat.
Schritt l (B-XIII → B-XV):
   Die Hydroxylgruppe in B-XIII kann nach den unter a) genannten Verfahren mit einer Schutzgruppe PG¹⁰ versehen werden. Bevorzugt sind Silizium haltige Schutzgruppen, die unter sauren Reaktionsbedingungen oder Anwendung von Fluorid gespalten werden können, wie z.B. der Trimethylsilyl-, Triethylsilyl-, tert.-Butyldimethylsilyl-, tert.-Butyldiphenylsilyl-, Tribenzylsilyl-, Triisopropylsilyl-Rest. Besonders bevorzugt ist der tert.-Butyldiphenylsilyl-Rest.
Schritt m (B-XV → B-XVI):
   Die unter Schritt f) eingeführte Schutzgruppe PG⁹ wird nach den unter Schritt g) beschriebenen Verfahren gespalten.
Schritt n (B-XVI → B-XVII):
   Die Oxidation des Alkohols B-XVI zum Aldehyd der allgemeinen Formel B-XVII erfolgt nach den unter h) genannten Verfahren. Bevorzugt ist die Oxidation nach Swern.

Alternativ können die Verbindungen der allgemeinen Formel B-XIII über den in Schema 5 beschriebenen Weg hergestellt werden. 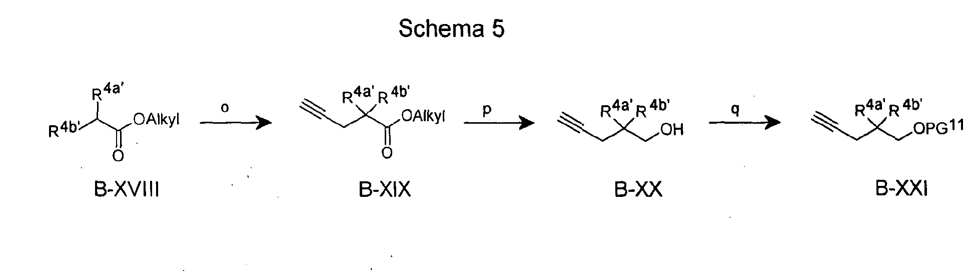 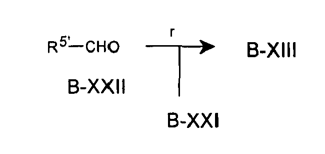
Schritt o (B-XVIII → B-XIX):
   Ausgehend von wohlfeil erhältlichen Essigesterderivaten der allgemeinen Formel B-XVIII, in denen R^{4a'} und R^{4b'} die oben genannten Bedeutungen haben, wird das Esterenolat durch Einwirkung starker Basen wie z.B. Lithiumdiisopropylamid, Lithiumhexamethyldisilazan bei niedrigen Temperaturen hergestellt und mit 3-Halogen-1-propin, vorzugsweise 3-Brom-1-propin zu Verbindungen der allgeminen Formel B-XIX umgesetzt.
Schritt p (B-XIX →B-XX):
   Die Reduktion des Esters B-XIX zum Akohol B-XX erfolgt nach den unter Schritt e) beschriebenen Methoden, vorzugsweise unter Verwendung von Diisobutylaluminiumhydrid.
Schritt q (B-XX → B-XXI):
   Die Hydroxylgruppe in B-XX kann nach den unter a) genannten Bedindungen mit einer Schutzgruppe PG¹¹ versehen werden. Bevorzugt sind Silizium haltige Schutzgruppen, die unter sauren Reaktionsbedingungen oder Anwendung von Fluorid gespalten werden können, wie z.B. der Trimethylsilyl-, Triethylsilyl-, tert.-Butyldimethylsilyl-, tert.-Butyldiphenylsilyl-, Tribenzylsilyl-, Triisopropylsilyl-Rest.
   Besonders bevorzugt ist der tert.-Butyldimethylsilyl-Rest.
Schritt r (B-XXI → B-XIII):
   Das Acetylen B-XXI kann nach den, dem Fachmann bekannten Verfahren deprotoniert und das erhaltene Acetylid mit Carbonylverbindungen der allgemeinen Formel B-XXII, in der R^{5'} die oben genannte Bedeutung hat, zu einem Alkohol der allgemeinen Formel XIII umgesetzt werden. Zur Deprotonierung eignen sich Alkylalkaliverbindungen wie z.B. Buthyllithium oder andere starke Basen wie z.B. Alkalihexamethyldisilazane oder Lithiumdiisopropylamid. Bevorzugt wird n-Buthyllithium.
   Auf dem in Schema 5 beschriebenen Weg werden zunächst die racemischen Verbindungen rac-B-XIII erhalten. Optional bieten die durchlaufenen Stufen rac-B-XIX bzw. rac-B-XX gemäß Schema 6 die Möglichkeit zur chemischen Racematspaltung und somit auch einen Zugang zu den enantiomerenreinen Verbindungen B-XX bzw. ent-B-XX, sofern R^{4a'} nicht identisch ist mit R^{4b'}.

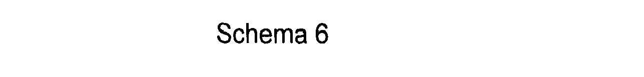

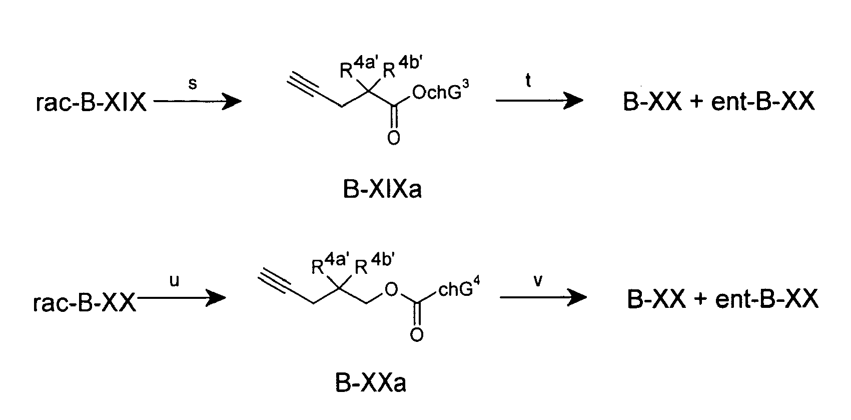

Schritt s (rac-B-XIX → B-XIXa):
   Die racemische Verbindung rac-B-XIX läßt sich mit einem chiralen, optisch rein erhältlichen Alkohol chG³-OH nach den, dem Fachmann bekannten Methoden, bespielsweise dem unter Schritt d) genannten Verfahren zu einem Gemisch der diastereomeren Ester B-XlXa umestern und mit einfachen chromatographischen Methoden trennen. Als chirale Alkohole kommen beispielsweise Pulegol, 2-Phenylcyclohexanol, 2-Hydroxy-1,2,2-triphenylethanol, 8-Phenylmenthol in Betracht.
Schritt t (B-XlXa → B-XX und ent-B-XX):
   Die diastereomereinreinen Ester B-XlXa lassen sich jeweils nach dem unter Schritt e beschriebenen Verfahren zu den Alkoholen B-XX bzw. ent-B-XX reduzieren, wobei die unter Schritt s beschriebene Hilfskomponente chG³-OH wiedergewonnen werden kann.
Schritt u (rac-B-XX → B-XXa):
   Die racemische Verbindung rac-B-XX läßt sich mit einer chiralen, optisch rein erhältlichen Säure chG⁴-CO₂H, deren Ester, Anhydrid oder Säurehalogenid nach den, dem Fachmann bekannten Methoden zu einem Gemisch der diastereomeren Ester XXa umsetzen und mit einfachen chromatographischen Methoden trennen. Als chirale Säuren kommen beispielsweise Äpfelsäure, Weinsäure bzw. deren Derivate in Betracht.
Schritt v (B-XXa→ B-XX und ent-B-XX):
   Die diastereomereinreinen Ester B-XXa lassen sich jeweils nach dem unter Schritt e beschriebenen Verfahren zu den Alkoholen B-XX bzw. ent-B-XX reduzieren, oder nach den, dem Fachmann bekannten Methoden verseifen wobei im letztgenannten Fall die unter Schritt u beschriebene Hilfskomponente chG⁴-CO₂H wiedergewonnen werden kann.

### Darstellung der Teilfragmente C:

Es ist bekannt, daß die Verbindung der Formel 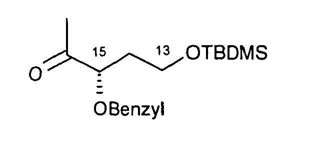 (TBDMS steht für einen tert.-Butyldimethylsilylrest) zur Synthese des C13-C16-Fragments (Epothilon-Zählweise) von Epothilon A verwendet werden kann (Schinzer et. al. Chem. Eur. J. 1996, 2, No. 11, 1477-1482). Die von Schinzer et al. beschriebene Synthese führt die benötigte Chiralität über eine kinetische Racematspaltung nach Sharpless ein. Eine notwendige chromatographische Trennung, ein ungenügender Enantiomerenüberschuß (80% ee) und eine geringe Gesamtausbeute disqualifizieren diesen Weg für eine industrielle Synthese, die hohe Ausbeuten und hohe optische Reinheit der Syntheseprodukte erfordert.

Es ist weiterhin bekannt, daß der oben genannte Synthesebaustein mit dem Phosphonat der Formel 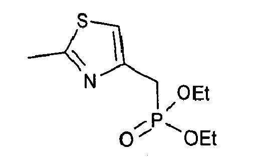 durch Wittig-Reaktion in eine Verbindung der Formel 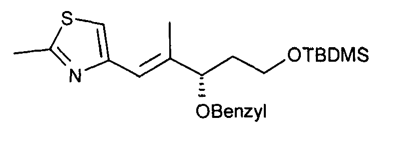 überführt werden kann, die dann zur Einführung des C13-C20-Fragments für die Epothilonsynthese genutzt werden kann.

Teilfragmente der Formel C können aus wohlfeiler, preiswert erhältlicher Äpfelsäure in effizienter Weise mit hoher optischer Reinheit (>99,5%ee) hergestellt werden.

Die Synthese wird im folgenden Schema 7 am Beispiel der L-(-)-Äpfelsäure (C-1) beschrieben. Ausgehend von D(+)-Äpfelsäure (ent-C-1) erhält man die entsprechenden enantiomeren Verbindungen (ent-C-II bis ent-C-XI) und ausgehend von racemischer Äpfelsäure (rac-C-1) die entsprechenden racemischen Verbindungen (rac-C-11 bis rac-C-Xi). 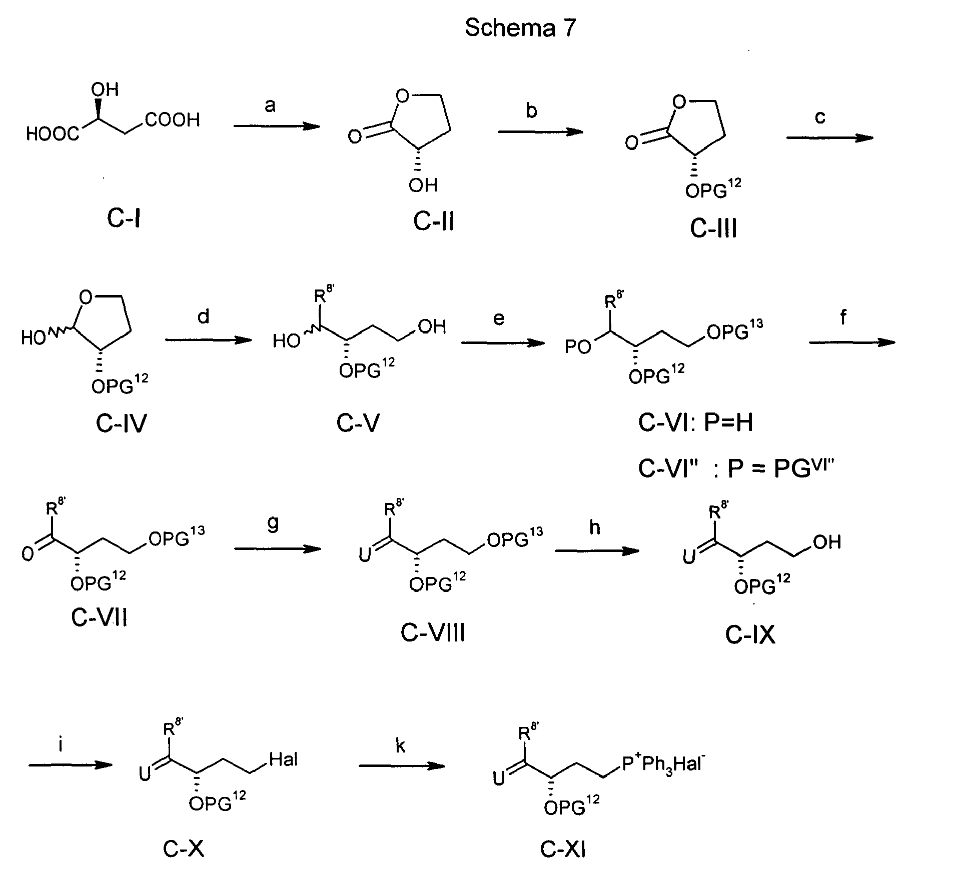
Schritt a (Äpfelsäure C-I ⇒ C-II):
   L-(-)-Äpfelsäure wird nach einem literaturbekannten Verfahren (Liebigs Ann. Chem. 1993, 1273-1278) in das Hydroxylacton C-II überführt.
Schritt b (C-II → C-III):
   Die freie Hydroxygruppe in Verbindung C-II wird nach den, dem Fachmann bekannten Methoden geschützt. Als Schutzgruppe PG¹² kommen die, dem Fachmann bekannten Schutzgruppen, wie sie schon vorstehend für PG⁴ im Schritt a (A-II ### A-III) genannt wurden, in Frage.
   Bevorzugt sind solche Schutzgruppen, die unter Einwirkung von Fluorid gespalten werden können, aber unter schwach sauren Reaktionsbedingungen stabil sind, wie z.B. der tert.-Butyldiphenylsilyl-, tert.-Butyldimethylsilyl-, oder Triisopropylsilyl-Rest.
   Besonders bevorzugt sind der tert.-Butyldiphenylsilyl- und der tert.-Butyldimethylsilyl-Rest.
Schritt c (C-III → C-IV):
   Das Lacton C-III wird zum Lactol C-IV nach den dem Fachmann bekannten Methoden reduziert. Als Reduktionsmittel eignen sich in ihrer Reaktivität modifizierte Aluminiumhydride wie z.B. Diisobutylaluminium-hydrid. Die Reaktion erfolgt in einem inerten Lösungsmittel wie z.B. Toluol, vorzugsweise bei niedrigen Temperaturen (-20 bis -100°C).
Schritt d (C-IV → C-V):
   Die Umsetzung des Lactols C-IV zu Verbindungen der Formel C-V erfolgt mit metallorganischen Verbindungen der allgemeinen Formel M-R^{8'} worin M für ein Alkalimetall, vorzugsweise Lithium oder ein zweiwertiges Metall MX steht, worin X ein Halogen repräsentiert und R^{8'} die oben genannten Bedeutungen aufweist. Als zweiwertiges Metall ist bevorzugt Magnesium und Zink, als Halogen X ist bevorzugt Chlor, Brom und lod.
Schritt e (C-V → C-VI):
   Die primäre Hydroxylgruppe in Verbindung C-V wird nach den, dem Fachmann bekannten Methoden selektiv gegenüber der sekundären Hydroxylgruppe geschützt.
   Die sekundäre Hydroxygruppe wird gegebenenfalls anschließend ebenfalls nach bekannten, dem Fachmann geläufigen Methoden geschützt.
   Als Schutzgruppen PG¹³ und PG^{VI}" kommen die, dem Fachmann bekannten Schutzgruppen, wie sie schon vorstehend für PG⁴ im Schritt a (A-II ### A-III) genannt wurden, in Frage.
   Bevorzugt sind solche Schutzgruppen, die unter schwach sauren Reaktionsbedingungen selektiv in Gegenwart der Schutzgruppe PG10, die aus dem Baustein A in die Synthese der Verbindung der allgemeinen Formel I eingebracht wird, gespalten werden können, wie z.B. der Trimethylsilyl-, Triethylsilyl, -tert.-Butyldimethylsilyl-Rest.
   Besonders bevorzugt ist der tert.-Butyldimethylsilyl-Rest.
Schritt f (C-VI → C-VII):
   Die Oxidation des sekundären Alkohols in C-VI zum Keton C-VII erfolgt nach den, dem Fachmann bekannten Methoden. Beispielsweise genannt sei die Oxidation mit Pyridiniumchlorochromat, Pyridiniumdichromat, Chromtrioxid-Pyridin-Komplex, die Oxidation nach Swern oder verwandter Methoden z.B. unter Verwendung von Oxalylchlorid in Dimethylsulfoxid, die Verwendung des Dess-Martin-Periodinans, die Verwendung von Stickstoffoxiden wie z.B. N-Methyl-morpholino-N-oxid in Gegenwart geeigneter Katalysatoren wie z.B. Tetrapropylammoniumperruthenat in inerten Lösungsmitteln. Bevorzugt ist die Oxidation nach Swern.
Schritt g (C-VII → C-VIII):
   Für Verbindungen in denen U gleich CR10'R11' ist, wird diese Gruppierung nach den dem Fachmann bekannten Verfahren etabliert. Hierzu eignen sich Methoden wie z.B. die Wittig- oder Wittig/Horner-Reaktion, die Addition einer metallorganischen Verbindung MCHR10'R11' unter Abspaltung von Wasser. Bevorzugt ist die Wittig- und Wittig/Horner-Reaktion unter Verwendung von Phosphoniumhalogeniden des Typs CR10'R11'P(Ph)3⁺Hal⁻ oder Phosphonaten des Typs CR10'R11'P(O)(OAlkyl)2 mit Ph gleich Phenyl, R10', R11' und Halogen in den bereits genannten Bedeutungen mit starken Basen wie z.B. n-Butyllithium, Kalium-tert.-butanolat, Natriumethanolat, Natriumhexamethyldisilazan; als Base bevorzugt ist n-Butyllithium.
   Für Verbindungen, in denen U zwei Alkoxygruppen OR²³ oder eine C₂-C₁₀-Alkylen-α,ω-dioxygruppe darstellt, wird das Keton nach den dem Fachmann bekannten Methoden beispielsweise unter Verwendung eines Alkohols HOR²³ oder eines C₂-C₁₀-Alkylen-α,ω-diols unter Säurekatalyse ketalisiert.
Schritt h (C-VIII → C-IX):
   Die unter e eingeführte Schutzgruppe PG¹³ wird nun nach den dem Fachmann bekannten Verfahren selektiv in Gegenwart von PG¹² gespalten. Handelt es sich um eine sauer abspaltbare Schutzgruppe so erfolgt die Spaltung bevorzugt unter schwach sauren Bedindungen, wie z.B. durch Umsetzung mit verdünnten organischen Säuren in inerten Lösungsmittel. Bevorzugt ist Essigsäure.
Schritt i (C-IX → C-X):
   Gegebenenfalls wird die freie primäre Hydroxylgruppe nach den dem Fachmann bekannten Verfahren in ein Halogenid überführt. Bevorzugte Halogenide sind Chlor, besonders aber Brom und lod. Die Substitution der Hydroxylgruppe gegen ein Brom kann z.B. mittels Triphenylphosphin/Tetrabrommethan aber auch nach jedem anderen dem Fachmann bekannten Verfahren erfolgen. Die Etablierung eines lodatoms kann aus dem Bromid durch Substitution z.B. nach Finkelstein mit Natriumiodid in Aceton erfolgen.
   Auch die direkte Überführung der Hydroxylgruppe in das lodid ist möglich, z.B. unter Verwendung von elementarem lod, lmidazol und Triphenylphosphin in Dichlormethan.
   Soll U letztendlich für H/OR⁹ mit R⁹ in der Bedeutung eines Wasserstoffatoms stehen, wird die Umwandlung der primären Hydroxygruppe in ein Halogenatom auf der Stufe der Verbindung C-VI' nach selektiver Entschützung der primären Hydroxygruppe vorgenommen.
Schritt k (C-X → C-XI):
   Soll die Verknüpfung der C13-C16-Einheit mit der Position 12 des Epothilonrestes bzw. von Epothilonbruchstücken, z.B. einer C7-C12-Einheit durch Wittigreaktion erfolgen, wie z.B. in Nature Vol. 387, 268-272 (1997) beschrieben, so werden ausgehend von den Halogeniden C-X nach den dem Fachmann bekannten Verfahren die Triphenylphosphonium-halogenide (R²¹ = P(Ph)₃⁺Hal⁻), Alkyl- bzw. Arylphoshonate (R²¹ =P(O)(OQ)₂) oder Phosphinoxide (R²¹ = P(O)Ph₂) des Typs C-XI hergestellt. Ph bedeutet dabei Phenyl; Hal steht für F, Cl, Br oder 1 und Q ist ein C₁-C₁₀-Alkyl- oder Phenylrest.
   Zur Darstellung der Phosphoniumsalze eignet sich z.B. die Umsetzung der entsprechenden Halogenide mit Triphenylphosphin in Lösungsmitteln wie Toluol oder Benzol.
   Die Darstellung der Phosphonate kann z.B. durch Reaktion der Halogenide C-X mit einem metallierten Dialkylphosphit erfolgen. Die Metallierung erfolgt üblicherweise mit starken Basen wie z.B. Butyllithium.
   Die Darstellung der Phosphinoxide kann z.B. durch Umsetzung der Halogenide C-X mit metalliertem Diphenylphosphin und anschließender Oxidation erfolgen. Für die Metallierung eignen sich ebenfalls starke Basen wie Butyllithium. Die anschließende Oxidation zum Phosphinoxid kann dann z.B. mit verdünnter wäßriger Wasserstoffperoxid-Lösung erfolgen.

Es wurde gefunden, daß Verbindungen der Formel C' aus wohlfeiler, preiswert erhältlicher, enantiomerenreiner Äpfelsäure überaschenderweise in effizienter Weise mit hoher optischer Reinheit (>99,5%ee) hergestellt werden können, obwohl prinzipiell bei dem beschriebenen erfindungsgemäßen Verfahren die Möglichkeit zur vollständigen oder teilweisen Racemisierung bestehen würde.
Wie eingangs erwähnt, liefert das bekannte Verfahren diejenigen Verbindungen, worin R¹ eine Methylgruppe, R² ein tert.-Butyldimethylsilyl- oder Benzylrest, R³ ein O-tert.-Butyldimethylsilylrest und X ein Sauerstoffatom oder ein (2-Methylthiazol-4-yl)methylenrest ist, nur in einer optischen Reinheit von ca. 80% ee.

Außerdem sind die chemischen Ausbeuten des erfindungsgemäßen Verfahrens wesentlich höher als die bei den von Schinzer et al. beschriebenen Verfahren angegebenen Ausbeuten. Beispielsweise ist die Ausbeute an nach dem erfindungsgemäßen Verfahren hergestelltem (3S)-5-[[Dimethyl(1,1-dimethylethyl)silyl]oxy]-3-[[(1,1-dimethylethyl)diphenylsilyl]oxy]-2-pentanon ausgehend von L-(-)-Äpfelsäure mit 26,5% fast doppelt so hoch wie die von Schinzer et al. bei der Herstellung von (3S)-3-Benzyloxy-5-[[dimethyl(1,1-dimethylethyl)silyl]oxy]-2-pentanon (14,35%; Chem. Eur. J. 1996, 2, No. 11, 1477-1482) angegebenen bzw. bei der Herstellung von (3S)-3-[[Dimethyl(1,1-dimethylethyl)silyl]oxy]-5-[[dimethyl(1,1-dimethylethyl)silyl]oxy]-2-pentanon (20,58%; Angew. Chem. 1997, 109, Nr. 5, 543-544) erzielten Ausbeute.
Dieser Vergleich beruht auf den in den genannten Literaturstellen angegebenen Ausbeuten, wobei -wie schon vorstehend erwähnt- zu berücksichtigen ist, daß die nach den bekannten Verfahren erhaltenen Verbindungen nicht enantiomerenrein anfallen, so daß die tatsächliche Ausbeute der betreffenden enantiomerenreinen Verbindung niedriger liegt und zur Gewinnung einer enantiomerenreinen Verbindung ein weiterer Reinigungsschritt auf dieser oder einer späteren Verfahrenstufe nötig wird.

Darüber hinaus ermöglicht das erfindungsgemäße Verfahren eine sehr breite Variation der Substituenten in diesem C13-C16-Baustein.

Die vorliegende Erfindung betrifft somit ein Verfahren zur Herstellung der Verbindungen der allgemeinen Formel C', welches dadurch gekennzeichnet ist, daß L-(-)-Äpfelsäure, D(+)-Äpfelsäure oder racemische Äpfelsäure als Ausgangsprodukt verwendet wird.

Bevorzugt wird optisch reine D-(+)- oder L-(-)-Äpfelsäure verwendet.

Die Erfindung betrifft auch die in dem Verfahren auftretenden Zwischenverbindungen der allgemeinen Formel V, VI und VI' (nachstehend zusammengefaßt als VI") 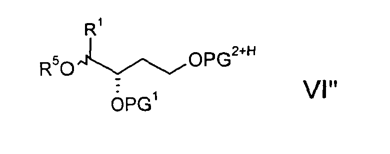 worin
R¹, PG¹ und R⁵ die in der allgemeinen Formel C' angegebene Bedeutung haben und
PG^{2+H} für ein Wasserstoffatom oder eine Schutzgruppe PG² stehen.

Diese Verbindungen werden erfindungsgemäß dadurch hergestellt, daß an eine Verbindung der allgemeinen Formel IV 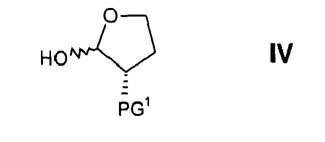 worin
PG¹ die in der allgemeinen Formel C angegebene Bedeutung hat,
unter Öffnung des Lactolringes eine Organometallverbindung der allgemeinen Formel

R¹Y

- worin: R¹ die in der allgemeinen Formel C' angegebene Bedeutung hat, und Y für ein Alkalimetallatom oder MZ steht, wobei M ein zweiwertiges. Metallatom und Z ein Halogenatom ist,
addiert wird.

Als Alkaliatom ist Lithium bevorzugt.
Im Falle von MZ ist für das zweiwertige Metallatom Magnesium und Zink bevorzugt; als Halogenatom kommt in erster Linie Chlor, Brom und Jod in Betracht.

Die vorliegende Erfindung betrifft außerdem die neuen C13-C16-Epothilon-Bausteine der allgemeinen Formel C 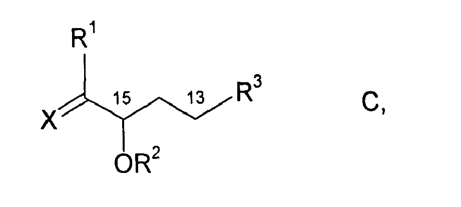 worin
- R¹: Wasserstoff, C₁-C₂₀-Alkyl, Aryl, C₇-C₂₀-Aralkyl, die alle substituiert sein können,
- R²: Wasserstoff oder eine Schutzgruppe PG¹,
- R³: eine Hydroxygruppe, Halogen, eine geschützte Hydroxygruppe OPG², ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
- X: ein Sauerstoffatom, zwei Alkoxygruppen OR⁴, eine C₂-C₁₀-Alkylen-□,□-dioxygruppe, die geradkettig oder verzweigt sein kann, H/OR⁵ oder eine Gruppierung CR⁶R⁷,
wobei
R⁴ für einen C₁-C₂₀-Alkylrest,
R⁵ für Wasserstoff oder eine Schutzgruppe PG³,
R⁶, R⁷ gleich oder verschieden sind und für Wasserstoff, einen C₁-C₂₀-Alkyl-, Aryl-, C₇-C₂₀-Aralkylrest oder R⁶ und R⁷ zusammen mit dem Methylenkohlenstoffatom gemeinsam für einen 5- bis 7-gliedrigen carbocyclischen Ring stehen,
bedeuten,
- wobei: nicht gleichzeitig
R¹ eine Methylgruppe, R² ein tert.-Butyldimethylsilyl- oder Benzylrest, R³ ein O-tert.-Butyldimethylsilylrest und X ein (2-Methylthiazol-4-yl)methylen-rest oder
R¹ eine Methylgruppe, R² ein tert.-Butyldimethylsilylrest, R³ ein Triphenylphosphoniumiodidrest und X ein (2-Methylthiazol-4-yl)methylen-rest
sein können.

Durch den ersten Disclaimer werden diejenigen Verbindungen ausgenommen, die bereits von Schinzer et al. nach einem anderen, als dem erfindungsgemäßen Verfahren hergestellt wurden (Chem. Eur. J. 1996, 2, No. 11, 1477-1482 und Angew. Chem. 1997, 109, Nr. 5, 543-544).

Der zweite Disclaimer berücksichtigt das von K. C. Nicolaou et al. in Nature, Vol. 387, 1997, 268-272, erwähnte (5E,3S)-[3-[[(1,1-Dimethylethyl)dimethylsilyl]oxy]-4-methyl-5-(2-methylthiazol-4-yl)-pent-4-en-1-yl]-triphenylphosphoniumiodid.

Für die nähere Erklärung der in den Verbindungen der allgemeinen Formel C vorkommenden Substituenten R¹, R⁴, R⁶, R⁷, PG¹, PG² und PG³ gelten die vorstehend für die Substituenten der allgemeinen Formel C' gemachten Ausführungen.

Erfindungsgemäß sind solche Verbindungen der allgemeinen Formel C bevorzugt, worin
R¹ für ein Wasserstoffatom, einen gegebenenfalls substituierten C₁-C₄-Alkylrest, einen gegebenenfalls mit 1 bis 3 Resten, ausgewählt aus der Gruppe der Substituenten Halogen, freie Hydroxygruppe oder geschützte Hydroxygruppe OPG⁴, C₁-C₄-Alkyl, Azido, Nitro, Nitril und Amino (NH₂), substituierten Phenylrest steht, und/oder
X für ein Sauerstoffatom steht, und/oder
der für R⁶ und/oder R⁷ stehende Arylrest für einen gegebenenfalls mit 1 bis 3 Resten, ausgewählt aus der Gruppe der Substituenten Halogen, freie Hydroxygruppe oder geschützte Hydroxygruppe OPG⁵, CO₂H, CO₂-Alkyl C₁-C₄-Alkyl, Azido, Nitro, Nitril, Amino (NH₂), substituierten Phenylrest oder für einen gegebenenfalls mit 1 bis 2 C₁-C₄-Alkylresten substituierten 5- oder 6-gliedrigen Heteroarylrest,
insbesondere für einen aus der Gruppe 2-, 3-Furanyl-, 2-, 3-, 4-Pyridinyl-, 2-, 4-, 5-Thiazolyl-, 2-, 4- und 5-Imidazolylrest, der gegebenenfalls durch 1 oder 2 C₁-C₄-Alkylreste substituiert ist, ausgewählten Substituenten steht und/oder
PG¹, PG² und PG³ aus der Gruppe der Substituenten Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl-, para-Nitrobenzyl-, para-Methoxybenzyl-, Acetyl-, Propionyl-, Butyryl- und Benzoylrest ausgewählt sind,
insbesondere PG¹ ein tert.-Butyldiphenylsilyl-, tert.-Butyldimethylsilyl-, oder Triisopropylsilyl- und
insbesondere PG² ein tert.-Butyldimethylsilyl-, Acetyl, Benzoyl-, Benzyl-, Tetrahydropyranyl-Rest ist.

Als Schutzgruppen PG⁴ und PG⁵ kommen alle schon vorstehend für PG¹, PG² und PG³ angegebenen Schutzgruppen in Frage.

Darstellung der Teilfragmente ABC und deren Zyklisierung zu 1:
Teilfragmente der allgemeinen Formel AB 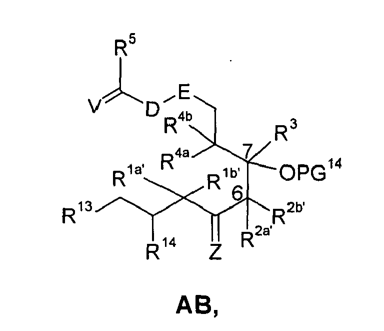 worin R^{1a'}, R^{1b'}, R^{2a'}, R^{2b'}, R³, R^{4a}, R^{4b}, R⁵, R¹³, R¹⁴, D, E, V und Z die bereits genannten Bedeutungen haben und PG¹⁴ ein Wasserstoffatom oder eine Schutzgruppe PG darstellt, werden aus den zuvor beschriebenen Fragmenten A und B nach dem in Schema 8 gezeigten Verfahren erhalten. 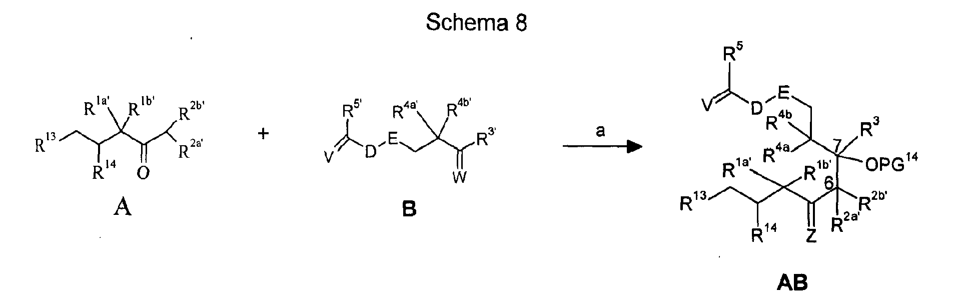
Schritt a (A + B → AB):
   Die Verbindung B, worin W die Bedeutung eines Sauerstoffatomes hat und eventuell vorhandene zusätzliche Carbonylgruppen geschützt sind, wird mit dem Enolat einer Carbonylverbindung der allgemeinen Formel A alkyliert. Das Enolat wird durch Einwirkung starker Basen wie z.B. Lithiumdiisopropylamid, Lithiumhexamethyldisilazan bei niedrigen Temperaturen hergestellt.
Teilfragmente der allgemeinen Formel ABC 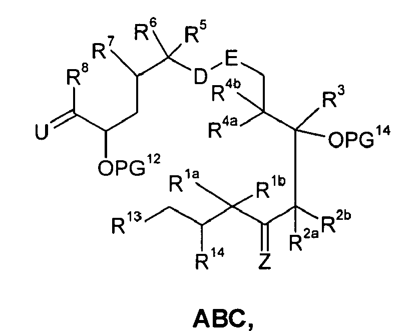 worin R^{1a'}, R^{1b'}, R^{2a'}, R^{2b'}, R³, R^{4a}, R^{4b}, R⁵, R⁶, R⁷, R⁸, R¹³, R¹⁴, D, E, U und Z die bereits genannten Bedeutungen haben, werden aus den zuvor beschriebenen Fragmenten AB und C nach dem in Schema 9 gezeigten Verfahren erhalten. 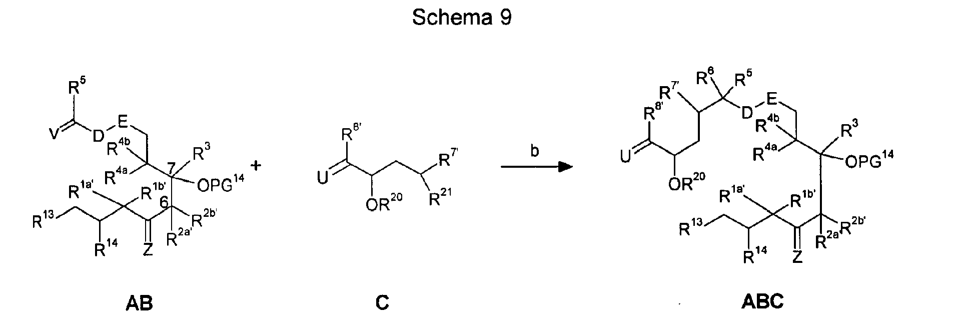
Schritt b (AB + C→ ABC):
   Die Verbindung C, in der R²¹ die Bedeutung eines Wittigsalzes hat und eventuell vorhandene zusätzliche Carbonylgruppen geschützt sind, wird durch eine geeignete Base wie z.B. n-Butyllithium, Lithiumdiisopropylamid, Kalium-tert.butanolat, Natrium- oder Lithium-hexamethyldisilazid deprotoniert und mit einer Verbindung AB, worin V die Bedeutung eines Sauerstoffatomes hat, umgesetzt.
Schritt c (ABC → I):
   Die Verbindungen ABC, in denen R¹³ eine Carbonsäure CO₂H und R²⁰ ein Wasserstoffatom darstellt, setzt man nach den, dem Fachmann bekannten Methoden für die Bildung großer Macrolide zu Verbindungen der Formel I, in denen Y die Bedeutung eines Sauerstoffatomes besitzt, um. Bevorzugt wird die in "Reagents for Organic Synthesis, Vol. 16, p 353" beschriebene Methode unter Verwendung von 2,4,6-Trichlorbenzoesäurechlorid und geeigneten Basen wie z.B. Triethylamin, 4-Dimethylaminopyridin, Natriumhydrid.
Schritt d (ABC → I):
   Die Verbindungen ABC, in denen R¹³ eine Gruppe CH₂OH und R²⁰ ein Wasserstoffatom darstellt, lassen sich vorzugsweise unter Verwendung von Triphenylphosphin und Azodiestern wie beispielsweise Azodicarbonsäurediethylester zu Verbindungen der Formel I, in denen Y die Bedeutung zweier Wasserstoffatome hat, umsetzen.
   Die Verbindungen ABC, in denen R¹³ eine Gruppe CH₂OSO₂Alkyl oder CH₂OSO₂Aryl oder CH₂OSO₂Aralkyl und R²⁰ ein Wasserstoffatom darstellt, lassen sich nach Deprotonierung mit geeigneten Basen wie beispielsweise Natriumhydrid, n-Buthyllithium, 4-Dimethylaminopyridin, Hünig-Base, Alkylihexamethyldisilazanen zu Verbindungen der Formel I, in denen Y die Bedeutung zweier Wasserstoffatome hat, zyklisieren.

Die flexible Funktionalisierung der beschriebenen Bausteine A, B und C gewährleistet auch eine von dem oben beschriebenen Verfahren abweichende Verknüpfungsreihenfolge, die zu den Bausteinen ABC führt. Diese Verfahren sind in der folgenden Tabelle zusammengestellt:

| Verknüpfungsmöglichkeiten | Verknüpfungsmethoden a bis e | Voraussetzungen |
|---|---|---|
| A + B ### A-B | a: Aldol (siehe Schema 8) | Z = W = Sauerstoff |
| B + C ### B-C | b: Wittig (analog Schema 9) e: McMurry | U =Sauerstoff und R²¹ = Wittigsalz oder Phosphinoxid oder Phosphonat U = V = Sauerstoff |
| A + C ### A-C | c: Veresterung (z. B. 2,4,6-Trichlorbenzoylchlorid/4-Dimethylaminopyridin) d: Veretherung (z.B. Mitsunobu) | R¹³ = CO₂R^{13b} oder COHal und R²⁰ = Wasserstoff R¹³ = CH₂OH und R²⁰ = Wasserstoff oder SO₂-Alky oder SO₂-Aryl oder SO₂-Aralkyl |

Nach diesen Verfahren lassen sich die Bausteine A, B und C, wie in Schema 10 angegeben, verknüpfen:

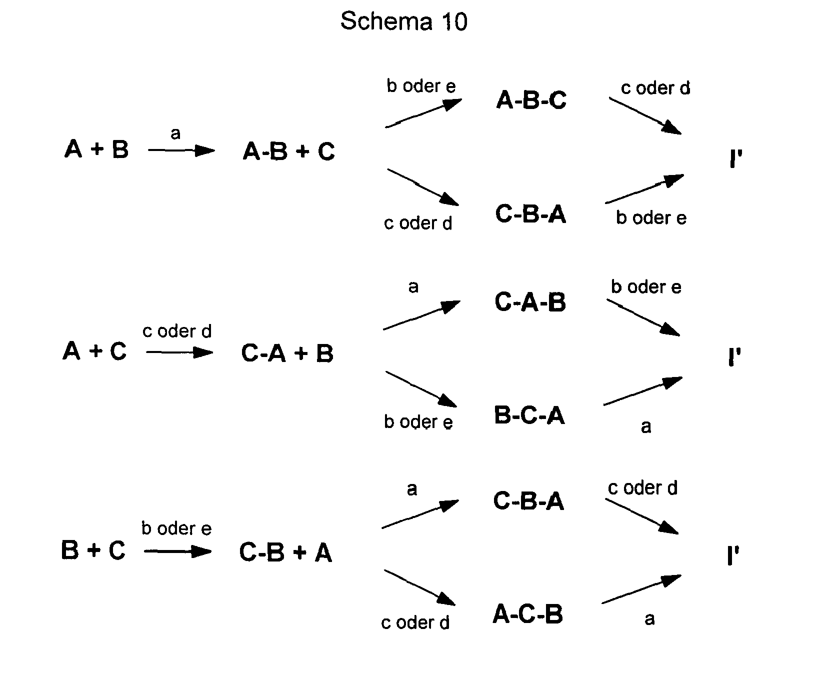

Freie Hydroxylgruppen in I, A, B, C, AB, ABC können durch Veretherung oder Veresterung, freie Carbonylgruppen durch Ketalisierung, Enoletherbildung oder Reduktion weiter funktionell abgewandelt sein.
Die Erfindung betrifft alle Stereoisomeren dieser Verbindungen und auch deren Gemische.

Biologische Wirkungen und Anwendungsbereiche der neuen Derivate:

Die neuen Verbindungen der Formel I sind wertvolle Pharmaka. Sie interagieren mit Tubulin, indem sie gebildete Mikrotubuli stabilisieren und sind somit in der Lage, die Zellteilung phasenspezifisch zu beeinflussen. Dies betrifft vor allem schnell wachsende, neoplastische Zellen, deren Wachstum durch interzelluläre Regelmechnismen weitgehend unbeeinflußt ist. Wirkstoffe dieser Art sind prinzipiell geeignet zur Behandlung maligner Tumoren. Als Anwendungsbereich seien beispielweise genannt die Therapie von Ovarial-, Magen-, Colon-, Adeno-, Brust-, Lungen-, Kopf- und Nacken-Karzinomen, dem malignen Melanom, der akuten lymphozytären und myelocytären Leukämie. Die erfindungsgemäßen Verbindungen eignen sich aufgrund ihrer Eigenschaften prinzipiell zur Anti-Angiogenese-Therapie sowie zur Behandlung chronischer entzündlicher Erkrankungen wie beispielsweise der Psoriasis oder der Arthritis. Zur Vermeidung unkontrollierter Zellwucherungen an sowie der besseren Verträglichkeit von medizinischen Implantaten lassen sie sich prinzipiell in die hierfür verwendeten polymeren Materialien auf- bzw. einbringen. Die erfindungsgemäßen Verbindungen können alleine oder zur Erzielung additiver oder synergistischer Wirkungen in Kombination mit weiteren in der Tumortherapie anwendbaren Prinzipien und Substanzklassen verwendet werden.
Als Beispiele seien genannt die Kombination mit
○ Platinkomplexen wie z.B. Cisplatin, Carboplatin,
○ interkalierenden Substanzen z.B. aus der Klasse der Anthracycline wie z.B. Doxorubicin oder aus der Klasse der Antrapyrazole wie z.B. CI-941,
○ mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin oder andere Verbindungen wie z.B. Colchicin, Combretastatin A-4,
○ DNA Topoisomeraseinhibitoren wie z.B. Camptothecin, Etoposid, Topotecan, Teniposid,
○ Folat- oder Pyrimidin-Antimetaboliten wie z.B. Lometrexol, Gemcitubin,
○ DNA alkylierenden Verbindungen wie z.B. Adozelesin, Dystamycin A,
○ Inhibitoren von Wachstumsfaktoren (z.B. von PDGF, EGF, TGFb, EGF) wie z.B. Somatostatin, Suramin, Bombesin-Antagonisten,
○ Inhibitoren der Protein Tyrosin Kinase oder der Protein Kinasen A oder C wie z.B. Erbstatin, Genistein, Staurosporin, Ilmofosin, 8-Cl-cAMP,
○ Antihormonen aus der Klasse der Antigestagene wie z.B. Mifepriston, Onapriston oder aus der Klasse der Antiöstrogene wie z.B. Tamoxifen oder aus der Klasse der Antiandrogene wie z.B. Cyproteronacetat,
○ Metastasen inhibierenden Verbindungen z.B. aus der Klasse der Eicosanoide wie z.B. PGl₂, PGE₁, 6-Oxo-PGE₁ sowie deren stabiler Derivate (z.B. Iloprost, Cicaprost, Misoprostol),
○ Inhibitoren onkogener RAS-Proteine, welche die mitotische Signaltransduktion beeinflussen wie beispielsweise Inhibitoren der Farnesyl-Protein-Transferase,
○ natürlichen oder künstlich erzeugten Antikörpern, die gegen Faktoren bzw. deren Rezeptoren, die das Tumorwachstum fördern, gerichtet sind wie beispielsweise der erbB2-Antikörper.

Die Erfindung betrifft auch Arzneimittel auf Basis der pharmazeutisch verträglichen, d.h. in den verwendeten Dosen nicht toxischen Verbindungen der allgemeinen Formel 1, gegebenenfalls zusammen mit den üblichen Hilfs- und Trägerstoffen.
Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.
Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.
Die Erfindung betrifft somit auch pharmazeutische Zusammensetzungen, die als Wirkstoff zumindest eine erfindungsgemäße Verbindung enthalten. Eine Dosiseinheit enthält etwa 0,1-100 mg Wirkstoff(e). Die Dosierung der erfindungsgemäßen Verbindungen liegt beim Menschen bei etwa 0,1-1000 mg pro Tag.

Die nachfolgenden Beispiele dienen der näheren Erläuterung der Erfindung, ohne sie darauf einschränken zu wollen:

### Herstellung der Bausteine der allgemeinen Formel A aus Pantolacton bzw. aus Malonsäuredialkylestern:

### Beispiel 1

### (3S)-1-Oxa-2-oxo-3-(tetrahydropyran-2(RS)-yloxy)-4,4-dimethyl-cyclopentan

Die Lösung von 74,1 g (569 mmol) D-(-)-Pantolacton in 11 wasserfreiem Dichlormethan versetzt man unter einer Atmosphäre aus trockenem Argon mit 102 ml 3,4-Dihydro-2H-pyran, 2 g p-Toluolsulfonsäure-Pyridiniumsalz und rührt 16 Stunden bei 23°C. Man gießt in eine gesättigte Natriumhydrogencarbonatlösung, trennt die organische Phase ab und trocknet über Natriumsulfat. Nach Filtration und Lösungsmittelabzug chromatographiert man den Rückstand an ca. 5 kg feinem Kieselgel mit einem Gemisch aus n-Hexan und Ethylacetat. Isoliert werden 119,6 g (558 mmol, 98%) der Titelverbindung als farbloses Öl.
¹H-NMR (CDCl₃): δ = 1,13 (3H), 1,22 (3H), 1,46-1,91 (6H), 3,50-3,61 (1H), 3,86 (1H), 3,92 (1H), 4,01 (1H), 4,16 (1H), 5,16 (1H) ppm.

### Beispiel 2 (2RS,3S)-1-Oxa-2-hydroxy-3-(tetrahydropyran-2(RS)-yloxy)-4,4-dimethyl-cyclopentan

Die Lösung von 117,5 g (548 mmol) der nach Beispiel 1 dargestellten Verbindung in 2,4 I wasserfreiem Toluol kühlt man unter einer Atmosphäre aus trockenem Argon auf -70°C, versetzt innerhalb 1 Stunde mit 540 ml einer 1,2 molaren Lösung von Diisobutylaluminiumhydrid in Toluol und rührt noch 3 Stunden bei -70°C. Man läßt auf - 20°C erwärmen, versetzt mit gesättigter Ammoniumchloridlösung, Wasser und trennt die ausgefallenen Aluminiumsalze durch Filtration über Celite ab. Das Filtrat wird mit Wasser und gesättigter Natriumchloridlösung gewaschen und über Magnesiumsulfat getrocknet. Isoliert werden nach Filtration und Lösungsmittelabzug 111,4 g (515 mmol, 94%) der Titelverbindung als farbloses Öl, das man ohne Reinigung weiter umsetzt.
IR (CHCl₃): 3480, 3013, 2950, 2874, 1262, 1133, 1074, 1026 und 808 cm⁻¹.

### Beispiel 3

### (3S)-2,2-Dimethyl-3-(tetrahydropyran-2(R)-yloxy)-pent-4-en-1-ol und (3S)-2,2-Dimethyl-3-(tetrahydropyran-2(S)-yloxy)-pent-4-en-1-ol

Die Aufschlämmung von 295 g Methyl-triphenylphosphoniumbromid in 2,5 I wasserfreiem Tetrahydrofuran versetzt man unter einer Atmosphäre aus trockenem Argon bei -60°C mit 313 ml einer 2,4 molaren Lösung von n-Butyllithium in n-Hexan, läßt auf 23°C erwärmen, eine Stunde nachrühren und kühlt auf 0°C. Man versetzt mit der Lösung von 66,2 g (306 mmol) der nach Beispiel 2 dargestellten Verbindung in 250 ml Tetrahydrofuran, läßt auf 23°C erwärmen und 18 Stunden rühren. Man gießt in eine gesättigte Natriumhydrogencarbonatlösung, extrahiert mehrfach mit Dichlormethan und trocknet die vereinigten organischen Extrakte über Natriumsulfat. Nach Filtration und Lösungsmittelabzug chromatographiert man den Rückstand an ca. 5 1 feinem Kieselgel mit einem Gradientensystem aus n-Hexan und Ethylacetat. Isoliert werden 36,5 g (170 mmol, 56%) des unpolaren, 14,4 g (67,3 mmol, 22%) des polaren THP-Isomeren der Titelverbindung, sowie 7,2 g (33,3 mmol; 11%) des Ausgangsmaterials jeweils als farbloses Öl.
¹H-NMR (CDCl₃), unpolares Isomer: δ = 0,78 (3H), 0,92 (3H), 1,41-1,58 (4H), 1,63-1,87 (2H), 3,18 (1H), 3,41 (1H), 3,48 (1H), 3,68 (1H), 3,94 (1H), 4,00 (1H), 4,43 (1H), 5,19 (1H), 5,27 (1H), 5,75 (1H) ppm.
¹H-NMR (CDCl₃), polares Isomer: δ = 0,83 (3H), 0,93 (3H), 1,42-1,87 (6H), 2,76 (1H), 3,30 (1H), 3,45 (1H), 3,58 (1H), 3,83 (1H), 3,89 (1H), 4,65 (1H), 5,12-5,27 (2H), 5,92 (1H) ppm.

### Beispiel 4

### (3S)-1-(tert.-Butyldiphenylsilyloxy)-2,2-dimethyl-pentan-3-(tetrahydropyran-2-yloxy)-pent-4-en

Die Lösung von 59,3 g (277 mmol) des nach Beispiel 3 dargestellten THP-IsomerenGemisches in 1000 ml wasserfreiem Dimethylformamid versetzt man unter einer Atmosphäre aus trockenem Argon mit 28 g lmidazol, 85 ml tert.-Butyldiphenylchlorsilan und rührt 16 Stunden bei 23°C. Man gießt in Wasser, extrahiert mehrfach mit Dichlormethan, wäscht die vereinigten organischen Extrakte mit Wasser und trocknet über Natriumsulfat. Nach Filtration und Lösungsmittelabzug chromatographiert man den Rückstand an feinem Kieselgel mit einem Gradientensystem aus n-Hexan und Ethylacetat. Isoliert werden 106,7 g (236 mmol, 85%) der Titelverbindung als farbloses Öl.
¹H-NMR (CDCl₃): δ = 0,89 (3H), 0,99 (3H), 1,08 (9H), 1,34-1,82 (6H), 3,40 (1H), 3,51 (2H), 3,76 (1H), 4,02 (1H), 4,67 (1H), 5,18 (1H), 5,23 (1H), 5,68 (1H), 7,30-7,48 (6H), 7,60-7,73 (4H) ppm.

### Beispiel 5

### (3S)-1 -(tert.-Butyldiphenylsilyloxy)2,2-dimethyl-3-(tetrahydropyran-2-yloxy)-pentan-5-ol

Die Lösung von 3,09 g (6,83 mmol) der nach Beispiel 4 dargestellten Verbindung in 82 ml Tetrahydrofuran versetzt man man unter einer Atmosphäre aus trockenem Argon bei 23°C mit 13,1 ml einer 1 molaren Lösung von Boran in Tetrahydrofuran und läßt 1 Stunde reagieren. Anschließend versetzt man unter Eiskühlung mit 16,4 ml einer 5%- igen Natronlauge sowie 8,2 ml einer 30%-igen Wasserstoffperoxidlösung und rührt weitere 30 Minuten. Man gießt in Wasser, extrahiert mehrfach mit Ethylacetat, wäscht die vereinigten organischen Extrakte mit Wasser, gesättigter Natriumchloridlösung und trocknet über Magnesiumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 1,78 g (3,78 mmol, 55%) der Titelverbindung als chromatographisch trennbares Gemisch der beiden THP-Epimeren sowie 0,44g (1,14 mmol, 17%) der Titelverbindung aus Beispiel 6 jeweils als farbloses Öl.
¹H-NMR (CDCl₃), unpolares THP-Isomer: δ = 0,80 (3H), 0,88 (3H), 1,10 (9H), 1,18-1,80 (9H), 3,27 (1H), 3,39 (1H), 3,48 (1H), 3,64 (1H), 3,83 (1H), 3,90-4,08 (2H), 4,49 (1H), 7,31-7,50 (6H), 7,58-7,73 (4H) ppm.
¹H-NMR (CDCl₃), polares THP-Isomer: δ = 0,89 (3H), 0,98 (3H), 1,08 (9H), 1,36-1,60 (4H), 1,62-1,79 (3H), 1,88 (1H), 2,03 (1H), 3,37 (1H), 3,50 (1H), 3,57 (1H), 3,62-3,83 (4H), 4,70 (1H), 7,30-7,48 (6H), 7,61-7,73 (4H) ppm.

### Beispiel 6

### (3S)-1-(tert.-Butyldiphenylsilyloxy)-2,2-dimethyl-pentan-3,5-diol

Die Lösung von 570 mg (1,55 mmol) der nach Beispiel 12 dargestellten Verbindung setzt man in Analogie zu Beispiel 5 um und isoliert nach Aufarbeitung und Reinigung 410 mg (1,06 mmol, 68%) der Titelverbindung als farbloses Öl.
¹H-NMR (CDCl₃): δ = 0,82 (3H), 0,93 (3H), 1,08 (9H), 1,56-1,79 (2H), 3,11 (1H), 3,50 (2H), 3,78-3,92 (3H), 4,02 (1H), 7,34-7,51 (6H), 7,61-7,71 (4H) ppm.

### Beispiel 7, Variante I

### 4(S)-[2-Methyl-1-(tert.-butyldiphenylsilyloxy)-prop-2-yl]-2,2-dimethyl-[1,3]dioxan

Die Lösung von 100 mg (0,212 mmol) der nach Beispiel 5 dargestellten Verbindungen in 2,6 ml wasserfreiem Aceton versetzt man unter einer Atmosphäre aus trockenem Argon mit 78,9 mg Kupfer(II)sulfat, einer Spatelspitze p-Toluolsulfonsäure-Monohydrat und rührt 16 Stunden bei 23°C. Man versetzt mit gesättigter Natriumhydrogencarbonatlösung, extrahiert mehrfach mit Diethylether, wäscht mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 24 mg (56 µmol, 27%) der Titelverbindung als farbloses Öl.
¹H-NMR (CDCl₃): δ = 0,83 (3H), 0,89 (3H), 1,07 (9H), 1,30 (1H), 1,36 (3H), 1,44 (3H), 1,71 (1H), 3,24 (1H), 3,62 (1H), 3,86 (1H), 3,91-4,03 (2H), 7,31-7,48 (6H), 7,61-7,74 (4H) ppm.

### Variante II

320 mg (0,88 mmol) der nach Beispiel 6 dargestellten Verbindung setzt man in Analogie zu Beispiel 7;Variante I um und isoliert nach Aufarbeitung und Reinigung 234 mg (0,548 mmol, 62%) der Titelverbindung:

### Variante III

Die Lösung von 5,60 g (14,5 mmol) der nach Beispiel 6 dargestellten Verbindung in 250 ml wasserfreiem Dichlormethan versetzt man unter einer Atmosphäre aus trockenem Argon mit 10 ml 2,2-Dimethoxypropan, 145 mg Campher-10-sulfonsäure und rührt 6 Stunden bei 23°C. Man versetzt mit Triethylamin, verdünnt mit Ethylacetat, wäscht mit gesättigter Natriumhydrogencarbonatlösung und trocknet über Natriumsulfat. Nach Filtration und Lösungsmittelabzug chromatographiert man den Rückstand an feinem Kieselgel mit einem Gemisch aus n-Hexan und Ethylacetat. Isoliert werden 5,52 g (12,9 mmol, 89%) der Titelverbindung als farbloses Öl.

### Beispiel 8

### (4S)-4-(2-Methyl-1-hydroxy-prop-2-yl)-2,2-dimethyl-[1,3]dioxan

Die Lösung von 5,6 g (13,1 mmol) der nach Beispiel 7 dargestellten Verbindung in 75 ml wasserfreiem Tetrahydrofuran versetzt man unter einer Atmosphäre aus trockenem Argon mit 39 ml einer 1 molaren Lösung von Tetrabutylammoniumfluorid in Tetrahydrofuran und erwärmt 16 Stunden auf 50°C. Man versetzt mit gesättigter Natriumhydrogencarbonatlösung, extrahiert mehrfach mit Ethylacetat, wäscht mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 2,43 g (12,9 mmol, 99%) der Titelverbindung als farbloses Öl.
¹H-NMR (CDCl₃): δ = 0,87 (3H), 0,90 (3H), 1,35 (1H), 1,37 (3H), 1,43 (3H), 1,77 (1H), 2,93 (1H), 3,36 (1H), 3,53 (1H), 3,79 (1H), 3,87 (1H), 3,96 (1H) ppm.

### Beispiel 9

### (4S)-4-(2-Methyl-1-oxo-prop-2-yl)-2,2-dimethyl-[1,3]dioxan

Die Lösung von 0,13 ml Oxalylchlorid in 5,7 ml wasserfreiem Dichlormethan kühlt man unter einer Atmosphäre aus trockenem Argon auf -70°C, versetzt mit 0,21 ml Dimethylsulfoxid, der Lösung von 200 mg (1,06 mmol) der nach Beispiel 8 dargestellten Verbindung in 5,7 ml wasserfreiem Dichlormethan und rührt 0,5 Stunden. Anschließend versetzt man mit 0,65 ml Triethylamin, läßt 1 Stunde bei -30°C reagieren und versetzt mit n-Hexan und gesättigter Natriumhydrogencarbonatlösung. Die organische Phase wird abgetrennt, die wässrige noch mehrfach mit n-Hexan extrahiert, die vereinigten organischen Extrakte mit Wasser gewaschen und über Magnesiumsulfat getrocknet. Den nach Filtration und Lösungsmittelabzug erhaltenen Rückstand setzt man ohne Reinigung weiter um.

### Beispiel 10

### (4S)-4-(2-Methyl-3(RS)-hydroxy-pent-2-yl)-2,2-dimethyl-[1,3]dioxan

Die Lösung von 900 mg (4,83 mmol) der nach Beispiel 9 dargestellten Verbindung in 14 ml wasserfreiem Diethylether versetzt man unter einer Atmosphäre aus trockenem Argon bei 0°C mit 2,42 ml einer 2,4 molaren Lösung von Ethylmagnesiumbromid in Diethylether, läßt auf 23°C erwärmen und 16 Stunden rühren. Man versetzt mit gesättigter Ammoniumchloridlösung, trennt die organische Phase ab und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 321 mg (1,48 mmol, 31%) des unpolaren 3R- oder 3S- Epimeren der Titelverbindung, 542 mg (2,51 mmol, 52%) des polaren 3S- oder 3R- Epimeren der Titelverbindung sowie 77 mg der in Beispiel 8 beschriebenen Titelverbindung jeweils als farbloses Öl.
¹H-NMR (CDCl₃) unpolares Isomer: δ = 0,86 (3H), 0,89 (3H), 1,03 (3H), 1,25-1,37 (2H), 1,37 (3H), 1,46 (3H), 1,49 (1H), 1,84 (1H), 3,35 (1H), 3,55 (1H), 3,81-4,02 (3H) ppm.
¹H-NMR (CDCl₃) polares Isomer: δ = 0,72 (3H), 0,91 (3H), 0,99 (3H), 1,25-1,44 (2H), 1,38 (3H), 1,43-1,60 (1H), 1,49 (3H), 1,76 (1H), 3,39 (1H), 3,63 (1H), 3,79-4,03 (3H) ppm.

### Beispiel 11

### (4S)-4-(2-Methyl-3-oxo-pent-2-yl)-2,2-dimethyl-[1,3]dioxan

Die Lösung von 850 mg (3,93 mmol) eines Gemisches der nach Beispiel 10 dargestellten Verbindungen in 63 ml wasserfreiem Dichlormethan versetzt man mit Molekularsieb (4A, ca. 80 Kugeln), 690 mg N-Methylmorpholino-N-oxid, 70 mg Tetrapropylammoniumperruthenat und rührt 16 Stunden bei 23°C unter einer Atmosphäre aus trockenem Argon. Man engt ein und reinigt das erhaltene Rohprodukt durch Chromatographie an ca. 200 ml feinem Kieselgel mit einem Gradientensystem aus n-Hexan und Ethylacetat. Isoliert werden 728 mg (3,39 mmol, 86%) der Titelverbindung als farbloses Öl.
¹H-NMR (CDCl₃): δ = 1,00 (3H), 1,07 (3H), 1,11 (3H), 1,31 (1H), 1,32 (3H), 1,41 (3H), 1,62 (1H), 2,52 (2H), 3,86 (1H), 3,97 (1H), 4,05 (1H) ppm.

### Beispiel 12

### (3S)-1-(tert-Butyldiphenylsilyloxy)-2,2-dimethyl-3-hydroxy-pent-4-en

Die Lösung von 106,7 g (236 mmol) der nach Beispiel 4 dargestellten Verbindung in 1,5 l wasserfreiem Ethanol versetzt man unter einer Atmosphäre aus trockenem Argon mit 5,9 g Pyridinium-p-Toluolsulfonat und erhitzt 6 Stunden auf 50°C. Nach Lösungsmittelabzug chromatographiert man den Rückstand an feinem Kieselgel mit einem Gemisch aus n-Hexan und Ethylacetat. Isoliert werden 82,6 g (224 mmol, 95%) der Titelverbindung als farbloses Öl, in dem noch zusätzlich ca. 5g Ethoxy-tetrahydropyran enthalten sind.
¹H-NMR (CDCl₃) einer analytischen Probe: δ = 0,89 (6H), 1,08 (9H), 3,45 (1H), 3,49 (1H), 3,58 (1H), 4,09 (1H), 5,21 (1H), 5,33 (1H), 5,93 (1H), 7,34-7,51 (6H), 7,63-7,73 (4H) ppm.

### Beispiel 13

### (4S)-4-((2RS)-3-Methyl-2-hydroxy-prop-3-yl)-2,2-dimethyl-[1,3]dioxan

In Analogie zu Beispiel 10 werden 450 mg (2,42 mmol) der nach Beispiel 9 dargestellten Verbindung unter Verwendung von Methylmagnesiumbromid umgesetzt. Nach Aufarbeitung und Reinigung isoliert man 431 mg (2,13 mmol, 88%) eines chromatographisch trennbaren Gemisches der epimeren Titelverbindungen als farbloses Öl.

### Beispiel 14

### (4S)-4-(3-Methyl-2-oxo-prop-3-yl)-2,2-dimethyl-[1,3]dioxan

In Analogie zu Beispiel 11 werden 420 mg (2,08 mmol) der nach Beispiel 13 dargestellten Verbindungen umgesetzt. Nach Aufarbeitung und Reinigung isoliert man 388 mg (1,94 mmol, 93%) der Titelverbindung als farbloses Öl.
¹ H-NMR (CDCl₃): δ = 1,08 (3H), 1,12 (3H), 1,33 (3H), 1,35 (1H), 1,42 (3H), 1,63 (1H), 2,17 (3H), 3,87 (1H), 3,98 (1H), 4,04 (1H) ppm.

### Beispiel 15 (4S)-4-((3RS)-2-Methyl-3-hydroxy-hex-2-yl)-2,2-dimethyl-[1,3]dioxan

In Analogie zu Beispiel 10 werden 450 mg (2,42 mmol) der nach Beispiel 9 dargestellten Verbindung unter Verwendung von n-Propylmagnesiumbromid umgesetzt. Nach Aufarbeitung und Reinigung isoliert man insgesamt 244 mg (1,06 mmol, 44%) eines trennbaren Gemisches der epimeren Titelverbindungen sowie 191 mg der in Beispiel 8 beschriebenen Titelverbindung jeweils als farbloses Öl.
¹H-NMR (CDCl₃) unpolares Isomer: δ = 0,87 (3H), 0,89 (3H), 0,94 (3H), 1,25-1,52 (4H), 1,38 (3H), 1,45 (3H), 1,66 (1H), 1,85 (1H), 3,46 (1H), 3,80-4,02 (4H) ppm.
¹H-NMR (CDCl₃) polares Isomer: δ = 0,73 (3H), 0,92 (3H), 0,95 (3H), 1,19-1,84 (6H), 1,37 (3H), 1,49 (3H), 3,49 (1H), 3,60 (1H), 3,80-4,03 (3H) ppm.

### Beispiel 16

### (4S)-4-(2-Methyl-3-oxo-hex-2-yl)-2,2-dimethyl-[1,3]dioxan

In Analogie zu Beispiel 11 werden 230 mg (1,00 mmol) der nach Beispiel 15 dargestellten Verbindungen umgesetzt. Nach Aufarbeitung und Reinigung isoliert man 185 mg (0,81 mmol, 81%) der Titelverbindung als farbloses Öl.
¹H-NMR (CDCl₃): δ = 0,88 (3H), 1,04 (3H), 1,12 (3H), 1,22-1,37 (1H), 1,31 (3H), 1,40 (3H), 1,48-1,71 (3H), 2,46 (2H), 3,83 (1H), 3,96 (1H), 4,04 (1H) ppm.

### Beispiel 17

### (4R)-4-(2-Methyl-3-oxo-pent-2-yl)-2,2-dimethyl-[1,3]dioxan

Ausgehend von L-(+)-Pantolacton wird in Analogie zu den in den Beispielen 1 bis 9 und 12 beschriebenen Verfahren über die jeweils enantiomeren Zwischenstufen die Titelverbindung hergestellt.
¹H-NMR (CDCl₃): δ = 1,00 (3H), 1,07 (3H), 1,12 (3H), 1,24-1,37 (1H), 1,31 (3H), 1,40 (3H), 1,61 (1H), 2,50 (2H), 3,84 (1H), 3,95 (1H), 4,03 (1H) ppm.

### Beispiel 18

### (4R)-4-(3-Methyl-2-oxo-prop-3-yl)-2,2-dimethyl-[1,3]dioxan

Ausgehend von L-(+)-Pantolacton wird in Analogie zu den in den Beispielen 1 bis 9 und 12 bis 14 beschriebenen Verfahren über die jeweils enantiomeren Zwischenstufen die Titelverbindung hergestellt.
¹H-NMR (CDCl₃): δ = 1,07 (3H), 1,12 (3H), 1,30-1,39 (1H), 1,33 (3H), 1,43 (3H), 1,62 (1H), 2,17 (3H), 3,86 (1H), 3,96 (1H), 4,03 (1H) ppm.

### Beispiel 19

### (4R)-4-(2-Methyl-3-oxo-hex-2-yl)-2,2-dimethyl-[1,3]dioxan

Ausgehend von L-(+)-Pantolacton wird in Analogie zu den in den Beispielen 1 bis 9, 12, 15 und 16 beschriebenen Verfahren über die jeweils enantiomeren Zwischenstufen die Titelverbindung hergestellt.
¹H-NMR (CDCl₃): δ = 0,88 (3H), 1,04 (3H), 1,12 (3H), 1,22-1,37 (1H), 1,31 (3H), 1,41 (3H), 1,48-1,72 (3H), 2,47 (2H), 3,84 (1H), 3,96 (1H), 4,05 (1H) ppm.

### Beispiel 20

### (2S,4S)-2-(2-Cyanophenyl)-4-[2-methyl-1-(tert.-butyldiphenylsilyloxy)-prop-2-yl]-[1,3]dioxan

Die Lösung von 1,00 g (2,59 mmol) der nach Beispiel 6 dargestellten Verbindung in 50 ml Benzol versetzt man mit 850 mg 2-Cyanobenzaldehyd, einer Spatelspitze p-Toluolsulfonsäure-Monohydrat und refluxiert 16 Stunden am Wasserabscheider unter einer Atmosphäre aus trockenem Argon. Man versetzt mit 0,5 ml Triethylamin, verdünnt mit Ethylacetat, wäscht mit gesättigter Natriumhydrogencarbonatlösung und trocknet über Natriumsulfat. Nach Filtration und Lösungsmittelabzug chromatographiert man den Rückstand an feinem Kieselgel mit einem Gemisch aus n-Hexan und Ethylacetat. Isoliert werden 1,22 g (2,44 mmol, 94%) der Titelverbindung als farbloses Öl
¹H-NMR (CDCl₃): δ = 0,99 (6H), 1,05 (9H), 1,47 (1H), 1,98 (1H), 3,34 (1H), 3,63 (1H), 3,96-4,09 (2H), 4,31 (1H), 5,75 (1H), 7,17 (2H), 7,24-7,51 (5H), 7,51-7,74 (7H) ppm.

### Beispiel 21

### (2S,4S)-2-(2-Cyanophenyl)-4-(2-methyl-1 -hydroxy-prop-2-yl)-[1,3]dioxan

In Analogie zu Beispiel 8 setzt man 1,22 g (2,44 mmol) der nach Beispiel 20 dargestellten Verbindung um und isoliert nach Aufarbeitung und Reinigung 593 mg (2,27 mmol, 93%) der Titelverbindung als farbloses Öl.
¹H-NMR (CDCl₃): δ = 0,89 (3H), 0,97 (3H), 1,51 (1H), 2,01 (1H), 2,42 (1H), 3,31 (1H), 3,72 (1H), 3,97 (1H), 4,02 (1H), 4,39 (1H), 5,78 (1H), 7,46 (1H), 7,63 (1H), 7,69 (1H), 7,75 (1H) ppm.

### Beispiel 22

### (2S,4S)-2-(2-Cyanophenyl)-4-(2-methyl-1-oxo-prop-2-yl)-[1,3]dioxan

In Analogie zu Beispiel 9 setzt man 570 mg (2,18 mmol) der nach Beispiel 21 dargestellten Verbindung um und isoliert nach Aufarbeitung 780 mg der Titelverbindung als gelbes Öl, die man ohne Reinigung weiter umsetzt.

### Beispiel 23 (2S,4S)-2-(2-Cyanophenyl)-4-((3RS)-2-methyl-3-hydroxy-pent-2-yl)-[1,3]dioxan

In Analogie zu Beispiel 10 setzt man 780 mg (max. 2,18 mmol) des nach Beispiel 22 dargestellten Rohproduktes um und isoliert nach Aufarbeitung und Reinigung 468 mg (1,62 mmol, 74%) der epimeren Titelverbindungen als farbloses Öl.
¹H-NMR (CDCl₃): δ = 0,81-1,09 (9H), 1,22-1,43 (1H), 1,43-1,70 (2H), 2,04 (1H), 2,35 (0,55H), 2,89 (0,45H), 3,41-3,59 (1H), 3,89-4,13 (2H), 4,36 (1H), 5,78 (0,45H), 5,81 (0,55H), 7,45 (1H), 7,54-7,78 (3H) ppm.

### Beispiel 24

### (2S,4S)-2-(2-Cyanophenyl)-4-(2-methyl-3-oxo-pent-2-yl)-[1,3]dioxan

In Analogie zu Beispiel 11 setzt man 463 mg (1,60 mmol) der nach Beispiel 23 dargestellten Verbindung um und isoliert nach Aufarbeitung und Reinigung 420 mg (1,46 mmol, 91 %) der Titelverbindung als farbloses Öl.
¹ H-NMR (CDCl₃): δ = 1,00 (3H), 1,19 (3H), 1,24 (3H), 1,49 (1H), 1,92 (1H), 2,56 (2H), 4,03 (1H), 4,16 (1H), 4,32 (1H), 5,78 (1H), 7,44 (1H), 7,60 (1H), 7,64-7,72 (2H) ppm.

### Beispiel 25

### (4S,2S)-4-[2-Methyl-1-(tert.-butyldiphenylsilyloxy)-prop-2-yl]-2-phenyl-[1,3]dioxan

In Analogie zu Beispiel 20 setzt man 1,00 g (2,59 mmol) der nach Beispiel 6 dargestellten Verbindung in 50 ml Toluol unter Verwendung von Benzaldehyd um und isoliert nach Aufarbeitung und Reinigung 1,2 g (2,53 mmol, 98%) der Titelverbindung als farbloses Öl.
¹H-NMR (CDCl₃): δ = 0,93 (3H), 1,00 (3H), 1,07 (9H), 1,43 (1H), 1,92 (1H), 3,30 (1H), 3,72 (1H), 3,95 (1H), 4,00 (1H), 4,30 (1H), 5,53 (1H), 7,18 (2H), 7,29-7,49 (9H), 7,61 (2H), 7,67 (2H) ppm.

### Beispiel 26

### (4S,2S)-4-(2-Methyl-1-hydroxy-prop-2-yl)-2-phenyl-[1,3]dioxan

In Analogie zu Beispiel 8 setzt man 1,20 g (2,53 mmol) der nach Beispiel 25 dargestellten Verbindung um und isoliert nach Aufarbeitung und Reinigung 518 mg (2,19 mmol, 87%) der Titelverbindung als farbloses Öl.
¹H-NMR (CDCl₃): δ = 0,98 (6H), 1,49 (1H), 2,00 (1H), 2,49 (1H), 3,46 (1H), 3,62 (1H), 3,81 (1H), 3,98 (1H), 4,33 (1H), 5,51 (1H), 7,30-7,41 (3H), 7,41-7,51 (2H) ppm.

### Beispiel 27

### (2S,4S)-4-(2-Methyl-1 -oxo-prop-2-yl)-2-phenyl-[1,3]dioxan

In Analogie zu Beispiel 9 setzt man 500 mg (2,12 mmol) der nach Beispiel 26 dargestellten Verbindung um und isoliert nach Aufarbeitung 715 mg der Titelverbindung als gelbes Öl, die man ohne Reinigung weiter umsetzt.

### Beispiel 28

### (2S,4S)-4-((3RS)-2-Methyl-3-hydroxy-pent-2-yl)-2-phenyl-[1,3]dioxan

In Analogie zu Beispiel 10 setzt man 715 mg (max. 2,12 mmol) des nach Beispiel 27 dargestellten Rohproduktes um und isoliert nach Aufarbeitung und Reinigung 440 mg (1,66 mmol, 79%) der epimeren Titelverbindungen als farbloses Öl.
¹H-NMR (CDCl₃): δ = 0,80-1,10 (9H), 1,23-1,42 (1H), 1,42-1,70 (2H), 1,90-2,16 (1H), 2,92 (0,6 H), 3,07 (0,4H), 3,40-3,53 (1H), 3,86 (1H), 3,98 (1H), 4,32 (1H), 5,49 (0,4H), 5,55 (0,6H), 7,28-7,40 (3H), 7,40-7,51 (2H) ppm.

### Beispiel 29 .

### (2S,4S)-4-(2-Methyl-3-oxo-pent-2-yl)-2-phenyl-[1,3]dioxan

In Analogie zu Beispiel 11 setzt man 435 mg (1,65 mmol) der nach Beispiel 28 dargestellten Verbindung um und isoliert nach Aufarbeitung und Reinigung 410 mg (1,56 mmol, 95%) der Titelverbindung als farbloses Öl.
¹H-NMR (CDCl₃): δ = 1,02 (3H), 1,17 (3H), 1,23 (3H), 1,44 (1H), 1,84 (1H), 2,58 (2H), 3,97 (1H), 4,06 (1H), 4,30 (1H), 5,50 (1H), 7,28-7,49 (5H) ppm.

### Beispiel 30

### (4S)-4-[2-Methyl-1-(tert.-butyldiphenylsilyloxy)-prop-2-yl]-2,2-pentamethylen-[1,3]dioxan

In Analogie zu Beispiel 20 setzt man 1,00 g (2,59 mmol) der nach Beispiel 6 dargestellten Verbindung in 50 ml Toluol unter Verwendung von Cyclohexanon um und isoliert nach Aufarbeitung und Reinigung 1,09 g (2,34 mmol, 90%) der Titelverbindung als farbloses Öl.
¹H-NMR (CDCl₃): δ = 0,84 (3H), 0,89 (3H), 0,97-1,10 (10H), 1,20-1,64 (9H), 1,71 (1H), 2,13 (1H), 3,33 (1H), 3,56 (1H), 3,81 (1H), 3,89 (1H), 3,99 (1H), 7,32-7,49 (6H), 7,60-7,74 (4H) ppm.

### Beispiel 31

### (4S)-4-(2-Methyl-1 -hydroxy-prop-2-yl)-2,2-pentamethylen-[1,3]dioxan

In Analogie zu Beispiel 8 setzt man 1,09 g (2,34 mmol) der nach Beispiel 30 dargestellten Verbindung um und isoliert nach Aufarbeitung und Reinigung 470 mg (2,06 mmol, 88%) der Titelverbindung als farbloses Öl.
¹H-NMR (CDCl₃): δ = 0,88 (3H), 0,94 (3H), 1,24-1,71 (10H), 1,81 (1H), 2,18 (1H), 3,09 (1H), 3,39 (1H), 3,60 (1H), 3,80 (1H), 3,87 (1H), 4,02 (1H) ppm.

### Beispiel 32

### (4S)-4-(2-Methyl-1-oxo-prop-2-yl)-2,2-pentamethylen-[1,3]dioxan

In Analogie zu Beispiel 9 setzt man 450 mg (1,97 mmol) der nach Beispiel 31 dargestellten Verbindung um und isoliert nach Aufarbeitung 678 mg der Titelverbindung als gelbes Öl, die man ohne Reinigung weiter umsetzt.

### Beispiel 33

### (4S)-4-(2-Methyl-3-hydroxy-pent-2-yl)-2,2-pentamethylen-[1,3]dioxan

In Analogie zu Beispiel 10 setzt man 678 mg (max 1,97 mmol) des nach Beispiel 32 dargestellten Rohproduktes um und isoliert nach Aufarbeitung und Reinigung 391 mg (1,54 mmol, 77%) der epimeren Titelverbindungen als farbloses Öl.
¹H-NMR (CDCl₃): δ = 0,70-1,08 (9H), 1,23-1,98 (13H), 2,01-2,13 (1H), 3,37-3,50 (1H), 3,61 (0,5H), 3,80-4,06 (3,5H) ppm.

### Beispiel 34

### (4S)-(2-Methyl-3-oxo-pent-2-yl)-2,2-pentamethylen-[1,3]dioxan

In Analogie zu Beispiel 11 setzt man 386 mg (1,51 mmol) der nach Beispiel 33 dargestellten Verbindung um und isoliert nach Aufarbeitung und Reinigung 376 mg (1,48 mmol, 98%) der Titelverbindung als farbloses Öl.
¹H-NMR (CDCl₃): δ = 1,01 (3H), 1,09 (3H), 1,17 (3H), 1,22-1,38 (3H), 1,40-1,72 (8H), 2,15 (1H), 2,57 (2H), 3,81 (1H), 3,92-4,07 (2H) ppm.

### Beispiel 35

### (4S)-4-[2-Methyl-1-(tert.-butyldiphenylsilyloxy)-prop-2-yl]-2,2-tetramethylen-[1,3]dioxan

In Analogie zu Beispiel 20 setzt man 1,00 g (2,59 mmol) der nach Beispiel 6 dargestellten Verbindung in 50 ml Toluol unter Verwendung von Cyclopentanon um und isoliert nach Aufarbeitung und Reinigung 997 mg (2,20 mmol, 85%) der Titelverbindung als farbloses Öl.
¹H-NMR (CDCl₃): δ = 0,84 (3H), 0,88 (3H), 0,99-1,10 (10H), 1,30 (1H), 1,50-1,99 (8H), 3,23 (1H), 3,60 (1H), 3,80-3,98 (3H), 7,31-7,49 (6H), 7,61-7,73 (4H) ppm.

### Beispiel 36

### (4S)-4-(2-Methyl-1-hydroxy-prop-2-yl)-2,2-tetramethylen-[1,3]dioxan

In Analogie zu Beispiel 8 setzt man 997 mg (2,20 mmol) der nach Beispiel 35 dargestellten Verbindung um und isoliert nach Aufarbeitung und Reinigung 415 mg (1,94 mmol, 88%) der Titelverbindung als farbloses Öl.
¹H-NMR (CDCl₃): δ = 0,90 (6H), 1,36 (1H), 1,53-2,02 (9H), 2,93 (1H), 3,39 (1H), 3,55 (1H), 3,70 (1H), 3,87 (1H), 3,96 (1H) ppm.

### Beispiel 37

### (4S)-4-(2-Methyl-1-oxo-prop-2-yl)-2,2-tetramethylen-[1,3]dioxan

In Analogie zu Beispiel 9 setzt man 400 mg (1,87 mmol) der nach Beispiel 36 dargestellten Verbindung um und isoliert nach Aufarbeitung 611 mg der Titelverbindung als gelbes Öl, die man ohne Reinigung weiter umsetzt.

### Beispiel 38

### (4S)-4-(2-Methyl-3-hydroxy-pent-2-yl)-2,2-tetramethylen-[1,3]dioxan

In Analogie zu Beispiel 10 setzt man 611 mg (max. 1,87 mmol) der nach Beispiel 37 dargestellten Verbindung um und isoliert nach Aufarbeitung und Reinigung 353 mg (1,46 mmol, 78%) der epimeren Titelverbindungen als farbloses Öl.
¹H-NMR (CDCl₃): δ = 0,71-1,09 (9H), 1,20-1,44 (2H), 1,44-1,78 (5H), 1,78-2,02 (5H), 3,32-3,44 (1H), 3,51-3,60 (1H), 3,76 (1H), 3,80-4,02 (2H) ppm.

### Beispiel 39

### (4S)-4-(2-Methyl-3-oxo-pent-2-yl)-2,2-tetramethylen-[1,3]dioxan

In Analogie zu Beispiel 11 setzt man 348 mg (1,44 mmol) der nach Beispiel 38 dargestellten Verbindung um und isoliert nach Aufarbeitung und Reinigung 332 mg (1,38 mmol, 96%) der Titelverbindung als farbloses 01.
¹H-NMR (CDCl₃) : δ = 1,00 (3H), 1,07 (3H), 1,17 (3H), 1,31 (1H), 1,50-2,00 (9H), 2,52 (2H), 3,84 (1H), 3,88-3,99 (2H) ppm.

### Beispiel 40

### 1,1-Cyclobutandimethanol

Zu einer Lösung von 20 g (99,9 mmol) 1,1-Cyclobutandicarbonsäurediethylester in 200 ml absolutem Tetrahydrofuran werden bei 0°C 170 ml einer 1,2 molaren Lösung von Diisobutylaluminiumhydrid getropft. Man läßt eine Stunde bei 0°C nachrühren und addiert dann 30 ml Wasser. Es wird über Celite filtriert. Das Filtrat wird mit Natriumsulfat getrocknet und im Vakuum eingeengt. Das erhaltene Rohprodukt (9,9 g, 85,2 mmol, 85%) wird ohne Aufreinigung in die Folgestufe eingesetzt.

### Beispiel 41

### 1-[[[Dimethyl(1,1-dimethylethyl)silyl]oxy]methyl]cyclobutanmethanol

Zu einer Suspension von 3,4 g Natriumhydrid (60%ig in Öl) in 35 ml absolutem Tetrahydrofuran wird bei 0°C eine Lösung von 9,9 g (85 mmol) der nach Beispiel 40 dargestellten Verbindung in 100 ml absolutem Tetrahydrofuran gegeben. Man läßt 30 Minuten nachrühren und addiert dann eine Lösung von 12,8 g *tert*.Butyldimethylsilylchlorid in 50 ml Tetrahydrofuran. Man läßt eine Stunde bei 25°C nachrühren und gießt dann das Reaktionsgemisch auf gesättigte wäßrige Natriumhydrogencarbonatlösung. Es wird mit Ethylacetat extrahiert. Die organische Phase wird mit gesättigter Natriumchloridlösung gewaschen und über Natriumsulfat getrocknet. Nach Abziehen des Lösungsmittels im Vakuum wird das erhaltene Rohprodukt durch Säulenchromatographie an Kieselgel mit einem Gemisch aus Hexan/Ethylacetat gereinigt. Man erhält 13,5 g (58,6 mmol, 69%) der Titelverbindung.
¹H-NMR (CDCl₃): δ = 0,04 (6H), 0,90 (9H), 1,70-2,00 (6H), 3,70 (4H) ppm.

### Beispiel 42

### 1-[[[Dimethyl(1,1-dimethylethyl)silyl]oxy]methyl]cyclobutancarbaldehyd

8 ml Oxalylchlorid werden in 100 ml Dichlormethan gelöst. Man kühlt auf -78°C und addiert 13 ml Dimethylsulfoxid. Man läßt 3 Minuten nachrühren und addiert dann eine Lösung von 13,5 g (58,6 mmol) der nach Beispiel 41 dargestellten Verbindung in 80 ml Dichlormethan. Nach weiteren 15 Minuten Nachrührzeit werden 58 ml Triethylamin hinzugetropft. Anschließend läßt man auf 0°C erwärmen. Dann wird das Reaktionsgemisch auf gesättigte Natriumhydrogencarbonatlösung gegossen. Man extrahiert mit Dichlormethan, wäscht die organische Phase mit gesättigter Natriumchloridlösung, trocknet über Natriumsulfat und engt im Vakuum ein. Nach Chromatographie des Rohprodukts an Kieselgel mit einem Gemisch aus Hexan/Ethylacetat erhält man 7,7 g (33,7 mmol, 58%) der Titelverbindung.
¹H-NMR (CDCl₃): δ = 9,70 s (1H), 3,83 s (2H), 2,20-2,30 m (2H), 1,85-2,00 m (4H), 0,90 s (9H), 0,03 s (6H) ppm.

### Beispiel 43

### [1R-[1α(R*),2β]]-2-Phenylcyclohexyl 3-[1-[[[dimethyl(1,1-dimethylethyl)silyl]oxy]methyl]cyclobutyl]3-hydroxypropanoat (A) und [1R-[1(α(S*),2β]]-2-Phenylcyclohexyl 3-[1-[[dimethyl(1,1-dimethylethyl)silyl]oxy]methyl]cyclobutyl]3-hydroxypropanoat (B)

Aus 7,2 ml Diisopropylamin und Butyllithium (32 ml einer 1,6 molaren Lösung in Hexan) wird in absolutem Tetrahydrofuran Lithiumdiisopropylamid hergestellt. Dann addiert man bei -78°C eine Lösung von 11,2 g (1*R*-*trans*)-2-Phenylcyclohexyl acetat in 100 ml absolutem Tetrahydrofuran und läßt 30 Minuten bei dieser Temperatur nachrühren. Anschließend wird eine Lösung von 7,7 g (33,7 mmol) der nach Beispiel 42 dargestellten Verbindung in 50 ml Tetrahydrofuran addiert. Man läßt 1,5 Stunden bei -78°C nachrühren und gießt danach das Reaktionsgemisch auf gesättigte wäßrige Ammoniumchloridlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat erhält man 6,34 g (14,2 mmol, 42%) der Titelverbindung A und 4,22 g (9,4 mmol, 28%) der Titelverbindung B.
¹H-NMR (CDCl₃) von A: δ = 0,04 (6H), 0,98 (9H), 2,69 (1H), 3,08 (1H), 3,60 (1H), 3,67 (1H), 3,78-3,84 (1H), 4,97 (1H), 7,15-7,30 (5H) ppm.
¹H-NMR (CDCl₃) von B: δ = 0,03 (6H) 0,90 (9H), 2,68 (1H), 2,80 (1H), 3,56 (2H), 3,68-3,72 (1H), 4,99 (1H), 7,18-7,30 m (5H) ppm.

### Beispiel 44

### (S)-1-[1-[[[Dimethyl(1,1-dimethylethyl)silyl]oxy]methyl]cyclobutyl]-1,3-propandiol

Zu einer Lösung von 1 g (2,24 mmol) der nach Beispiel 43 dargestellten Verbindung A in 10 ml absolutem Toluol werden bei 0°C 4 ml einer 1,2 molaren Lösung von Diisobutylaluminiumhydrid in Toluol getropft. Man läßt 1,5 Stunden bei 0°C nachrühren und addiert dann 5 ml Wasser. Es wird über Celite filtriert. Das Filtrat wird über Natriumsulfat getrocknet und im Vakuum eingeengt. Man erhält nach Säulenchromatographie des Rohprodukts an Kieselgel mit einem Gemisch aus Hexan/Ethylacetat 370 mg (1,35 mmol, 60%) der Titelverbindung.
¹H-NMR (CDCl₃): δ = 0,05 (6H), 0,90 (9H), 1,55- 1,60 (2H), 1,80 (2H), 1,90 (3H), 2,10 (1H), 3,75 (1H), 3,85-3,95 (4H) ppm.

### Beispiel 45

### (S)-2,2-Dimethyl-4-[1-[[[dimethyl(1,1-dimethylethyl)silyl]oxy]methyl]cyclobutyl]-1,3-dioxan

370 mg (1,35 mmol) der nach Beispiel 44 dargestellten Verbindung werden in 10 ml Aceton gelöst. Man addiert eine Spatelspitze p-Toluolsulfonsäure und läßt 2 Stunden bei 25°C nachrühren. Anschließend wird das Reaktionsgemisch auf gesättigte Natriumhydrogencarbonatlösung gegossen. Man extrahiert mit Ethylacetat, wäscht die organische Phase mit gesättigter Natriumchloridlösung, trocknet über Natriumsulfat und engt im Vakuum ein. Nach Säulenchromatographie an Kieselgel mit einem Gemisch aus Hexan/Ethylacetat werden 338 mg (1,07 mmol, 79%) der Titelverbindung erhalten.
¹H-NMR (CDCl₃): δ = 0,03 (6H), 0,88 (9H), 1,38 (3H), 1,42 (3H), 1,50-1,80 (4H), 2,00 (1H), 3,52 (1H), 3,62 (1H), 3,85-4,00 (3H) ppm.

### Beispiel 46

### (R)-1-[1-[[[Dimethyl(1,1-dimethylethyl)silyl]oxy]methyl]cyclobutyl]-1,3-propandiol

In Analogie zu Beispiel 44 setzt man 700 mg (1,57 mmol) der nach Beispiel 43 hergestellten Verbindung B um und isoliert nach Aufarbeitung und Reinigung 250 mg (0,91 mmol, 58%) der Titelverbindung.
Das 1H-NMR-Spektrum ist deckungsgleich mit dem in Beispiel 44 beschriebenen.

### Beispiel 47

### (R)-2,2-Dimethyl-4-[1-[[[dimethyl(1,1-dimethylethyl)silyl]oxy]methyl]cyclobutyl]1,3-dioxan

In Analogie zu Beispiel 45 setzt man 250 mg (0,91 mmol) der nach Beispiel 46 hergestellten Verbindung um und isoliert nach Aufarbeitung und Reinigung 228 mg (0,72 mmol, 60%) der Titelverbindung.
Das 1H-NMR-Spektrum ist deckungsgleich mit dem in Beispiel 45 beschriebenen.

### Beispiel 48

### 1-[1-[[[Dimethyl(1,1-dimethylethyl)silyl]oxy]methyl]cyclobutyl]-1,3-propandiol

In Analogie zu Beispiel 44 setzt man 500 mg (1,12 mmol) eines Gemisches der nach Beispiel 43 hergestellten Verbindungen A und B um und isoliert nach Aufarbeitung und Reinigung 190 mg (0,69 mmol, 62%) der Titelverbindung.
Das ¹ H-NMR-Spektrum ist deckungsgleich mit dem in Beispiel 44 beschriebenen.

### Beispiel 49

### 2,2-Dimethyl-4-[1-[[[dimethyl(1,1-dimethylethyl)silyl]oxy]methyl]cyclobutyl]-1,3-dioxan

In Analogie zu Beispiel 45 setzt man 190 mg (0,69 mmol) der nach Beispiel 48 hergestellten Verbindung um und isoliert nach Aufarbeitung und Reinigung 171 mg (0,54 mmol, 79%) der Titelverbindung.
Das ¹ H-NMR-Spektrum ist deckungsgleich mit dem in Beispiel 45 beschriebenen.

### Beispiel 50

### [1R-[1α(3S*),2β]]-2-Phenylcyclohexyl 3-[1-[[[dimethyl(1,1-dimethylethyl)silyl]oxy]methyl]cyclobutyl]3-[(tetrahydro-2H-pyran-2-yl)oxy]propanoat

In Analogie zu Beispiel 1 setzt man 460 mg (1,03 mmol) der nach Beispiel 43 dargestellten Verbindung um und isoliert nach Aufarbeitung und Reinigung 398 mg (0,75 mmol, 73%) der Titelverbindung als farbloses Öl.
¹H-NMR (CDCl₃) : δ = 0,01 (6H), 0,89 (9H), 1,24-1,97 (19H), 2,15-2,27 (3H), 2,66 (1H), 3,12 (1H), 3,50 (2H), 3,58 (1H), 3,98 (1H), 4,52 (1H), 4,87 (1H), 7,09-7,27 (5H) ppm.

### Beispiel 51

### (S)-3-[1-[[[Dimethyl(1,1-dimethylethyl)silyl]oxy]methyl]cyclobutyl]3-[(tetrahydro-2H-pyran-2-yl)oxy]propansäure

420 mg (3,75 mmol) Kaliumtert.butylat werden in 5 ml Diethylether suspendiert. Man addiert 16 µl Wasser und läßt 5 Minuten nachrühren. Anschließend wird eine Lösung von 398 mg (0,75 mmol) der nach Beispiel 50 dargestellten Verbindung in 5 ml Diethylether addiert. Man läßt 3 Stunden nachrühren. Danach wird die Reaktionslösung mit Wasser verdünnt und mit 10%iger Salzsäure neutralisiert. Man extrahiert mit Dichlormethan, wäscht die organische Phase mit gesättigter wäßriger Natriumchloridlösung, trocknet über Natriumsulfat und engt im Vakuum ein. Säulenchromatographie des Rohprodukts an Kieselgel mit einem Gemisch aus Hexan/Ethylacetat ergibt 112 mg (0,3 mmol).
¹H-NMR (CDCl₃): δ = 0,01 (6H), 0,90 (9H), 1,30-2,25 (10H), 3,12 (1H), 3,50 (2H), 3,58 (1H), 3,98 (1H), 4,45 (1H) ppm.
Das Reaktionsprodukt kann nach Spaltung der Silylschutzgruppe durch Oxidation analog zu Beispiel 9 in den Aldehyd überführt, analog zu Beispiel 10 mit einer metallorganischen Verbindung wie z.B. XMgCHR^{5a}R^{5b}, beispielsweise mit Ethylmagnesiumbromid, zur Reaktion gebracht und durch anschließende Oxidation des erhaltenen Alkoholgemisches analog zu Beispiel 11 in Verbindungen gemäß Anspruch 1 überführt werden.
Ersetzt man in Beispiel 40 das Ausgangsmaterial 1,1-Cyclobutandicarbonsäurediethylester durch andere 2-substituierte- oder 2,2-disubstituierte Malonesterderivate, so lassen sich in Analogie zu den Beispielen 9, 10 und 40-51 beispielsweise folgende Verbindungen herstellen:

| R^{4a} | R^{4b} | R^{5a} | R^{5b} |
|---|---|---|---|
| -(CH₂)₂- | | H | CH₃ |
| -(CH₂)₂- | | H | CH₂-CH₃ |
| -(CH₂)₂- | | H | (CH₂)₂-CH₃ |
| -(CH₂)₂- | | H | CH₂-C₆H₅ |
| -(CH₂)₂- | | H | (CH₂)₂-C₆H₅ |
| -(CH₂)₂- | | CH₃ | CH₃ |
| -(CH₂)₂- | | CH₃ | CH₂-CH₃ |
| -(CH₂)₃- | | H | CH₃ |
| -(CH₂)₃- | | H | CH₂-CH₃ |
| -(CH₂)₃- | | H | (CH₂)₂-CH₃ |
| -(CH₂)₃- | | H | CH₂-C₆H₅ |
| -(CH₂)₃- | | H | (CH₂)₂-C₆H₅ |
| -(CH₂)₃- | | CH₃ | CH₃ |
| -(CH₂)₃- | | CH₃ | CH₂-CH₃ |
| -(CH₂)₄- | | H | CH₃ |
| -(CH₂)₄- | | H | CH₂-CH₃ |
| -(CH₂)₄- | | H | (CH₂)₂-CH₃ |
| -(CH₂)₄- | | H | CH₂-C₆H₅ |
| -(CH₂)₄- | | H | (CH₂)₂-C₆H₅ |
| -(CH₂)₄- | | CH₃ | CH₃ |
| -(CH₂)₄- | | CH₃ | CH₂-CH₃ |
| CH₃ | CH₃ | H | CH₃ |
| CH₃ | CH₃ | CH₂- CH₃ | CH₂-CH₃ |
| CH₃ | CH₃ | H | (CH₂)₂-CH₃ |
| CH₃ | CH₃ | H | CH₂-C₆H₅ |
| CH₃ | CH₃ | H | (CH₂)₂-C₆H₅ |
| CH₂-CH₃ | CH₂-CH₃ | H | CH₃ |
| CH₂-CH₃ | CH₂-CH₃ | H | CH₂-CH₃ |
| CH₂-CH₃ | CH₂-CH₃ | H | (CH₂)₂-CH₃ |
| CH₂-CH₃ | CH₂-CH₃ | H | CH₂-C₆H₅ |
| CH₂-CH₃ | CH₂-CH₃ | H | (CH₂)₂-C₆H₅ |
| CH₃ | CH₂-CH₃ | H | CH₃ |
| CH₃ | CH₂-CH₃ | H | CH₂-CH₃ |
| CH₃ | CH₂-CH₃ | H | (CH₂)₂-CH₃ |
| CH₃ | CH₂-CH₃ | H | CH₂-C₆H₅ |
| CH₃ | CH₂-CH₃ | H | (CH₂)₂-C₆H₅ |

### Beispiel 52

### (3S)-4,4-Dimethyl-5-oxo-3-(tetrahydropyran-2-yloxy)-pent-1-en

In Analogie zu Beispiel 9 setzt man 5,0 g (23,3 mmol) der nach Beispiel 3 dargestellten Verbindung um und isoliert nach Aufarbeitung 6,1 g der Titelverbindung als farbloses Öl, die man ohne Reinigung weiter umsetzt.

### Beispiel 53

### (3S,5RS)-4,4-Dimethyl-5-hydroxy-3-(tetrahydropyran-2-yloxy)-hept-1-en

In Analogie zu Beispiel 10 setzt man 6,1 g (max. 23,3 mmol) des nach Beispiel 52 dargestellten Rohproduktes um und isoliert nach Aufarbeitung und Reinigung 1,59 g (6,56 mmol, 28%) des unpolaren Diastereomers sowie 1,67 g (6,89 mmol, 30%) des polaren Diastereomers jeweils als farbloses Öl.
¹H-NMR (CDCl₃) unpolares Isomer: δ = 0,79 (3H), 0,84 (3H), 1,03 (3H), 1,23-1,62 (6H), 1,62-1,88 (2H), 3,41-3,58 (2H), 3,88-4,01 (2H), 4,08 (1H), 4,47 (1H), 5,20 (1H), 5,29 (1H), 5,78 (1H) ppm.
¹H-NMR (CDCl₃) polares Isomer: δ = 0,78 (3H), 0,93 (3H), 1,01 (3H), 1,38 (1H), 1,47-1,85 (7H), 3,39-3,57 (3H), 3,90 (1H), 4,04 (1H), 4,62 (1H), 5,21 (1H), 5,32 (1H), 5,69 (1H) ppm.

### Beispiel 54

### (3S,5S)-4,4-Dimethyl-3-(tetrahydropyran-2-yloxy)-heptan-1,5-diol und/oder (3S,5R)-4,4-Dimethyl-3-(tetrahydropyran-2-yloxy)-heptan-1,5-diol

In Analogie zu Beispiel 5 setzt man 1,59 g (6,56 mmol) des nach Beispiel 53 dargestellten unpolaren Alkohols um und isoliert nach Aufarbeitung und 1,14 g (4,38 mmol, 67%) der Titelverbindung als farbloses Öl.
¹H-NMR (CDCl₃): δ = 0,78 (6H), 1,01 (3H), 1,28 (1H), 1,36-1,64 (6H), 1,64-1,93 (4H), 3,41-3,55 (2H), 3,61-3,82 (2H), 3,87 (1H), 3,99 (1H), 4,28 (1H), 4,56 (1H) ppm.

### Beispiel 55

### (3S,5R oder 5S)-1-Benzoyloxy-4,4-dimethyl-3-(tetrahydropyran-2-yloxy)-heptan-5-ol

Die Lösung von 1,04g (3,99 mmol) der nach Beispiel 54 dargestellten Verbindung in 20 ml wasserfreiem Pyridin versetzt man unter einer Atmosphäre aus trockenem Argon mit 476 µl Benzoylchlorid und rührt 16 Stunden bei 23°C. Man gießt in eine gesättigte Natriumhydrogencarbonatlösung, extrahiert mit Dichlormethan und trocknet über Natriumsulfat. Den nach Filtration und Lösungsmittelabzug erhaltenen Rückstand reinigt man durch Chromatographie an ca. 300 ml feinem Kieselgel mit einem Gradientensystem aus n-Hexan und Ethylacetat. Isoliert werden 785 mg (2,15 mmol, 54%) der Titelverbindung als farbloses Öl sowie 352 mg Startmaterial.
¹H-NMR (CDCl₃): δ = 0,83 (6H), 1,04 (3H), 1,31 (1H), 1,38-1,58 (5H), 1,74-1,99 (3H), 2,12 (1H), 3,40 (1H), 3,52 (1H), 3,90-4,03 (2H), 4,28-4,56 (4H), 7,45 (2H), 7,58 (1H), 8,05 (2H) ppm.

### Beispiel 56

### (3S)-1-Benzoyloxy-4,4-dimethyl-3-(tetrahydropyran-2-yloxy)-heptan-5-on.

In Analogie zu Beispiel 11 setzt man 780 mg (2,14 mmol) des nach Beispiel 55 dargestellten Verbindung um und isoliert nach Aufarbeitung und Reinigung 641 mg (1,77 mmol, 83%) der Titelverbindung als farbloses Öl.
¹H-NMR (CDCl₃): δ = 1,02 (3H), 1,11 (3H), 1,23 (3H), 1,40-1,56 (4H), 1,65-1,87 (3H), 1,93 (1H), 2,59 (2H), 3,36 (1H), 3,80 (1H), 4,13 (1H), 4,32 (1H), 4,45 (1H), 4,53 (1H), 7,45 (2H), 7,58 (1H), 8,05 (2H) ppm.

### Beispiel 57

### (3S)-1-Hydroxy-4,4-dimethyl-3-(tetrahydropyran-2-yloxy)-heptan-5-on.

Die Lösung von 636 mg (1,75 mmol) der nach Beispiel 56 dargestellten Verbindung in 25 ml Methanol versetzt man mit 738 mg Kaliumcarbonat und rührt 2 Stunden bei 23°C. Man versetzt mit Dichlormethan, filtriertab, wäscht mit Wasser und trocknet die organische Phase über Natriumsulfat. Den nach Filtration und Lösungsmittelabzug erhaltenen Rückstand reinigt man durch Chromatographie an ca. 100 ml feinem Kieselgel mit einem Gradientensystem aus n-Hexan und Ethylacetat. Isoliert werden 311 mg (1,20 mmol, 69%) der Titelverbindung als farbloses Öl.
¹H-NMR (CDCl₃): δ = 0,98 (3H), 1,07 (3H), 1,18 (3H), 1,44-1,90 (10H), 2,00 (1H), 3,50-3,68 (2H), 3,74 (1H), 3,83-4,06 (2H), 4,79 (1H) ppm.

### Herstellung der Bausteine der allgemeinen Formel A" mit der 2-Oxazolidinon-Hilfsgruppe

### Ausgangsprodukte

### A) 2,2-Dimethyl-3-oxopentanal

### Aa) 4-(2-Methylprop-1-enyl)morpholin

In einem 250 ml-Dreihalsrundkolben werden 43,6 g Morpholin vorgelegt. Unter Eisbadkühlung werden bei einer Temperatur von 5°C innerhalb von 20 Minuten 46 ml Isobutylaldehyd zugetropft. Dabei war eine starke Temperaturerhöhung zu beobachten (stark exotherme Reaktion). Nach beendeter Zugabe wird der Ansatz über einen Wasserabscheider 4 Stunden refluxiert Das Volumen des Wasserabscheiders wird mit Isobutylaldehyd gefüllt. Es werden 7,5 ml H₂O abgeschieden. Nach Ablauf der Reaktion wird das Reaktionsgemisch im Vakuum destilliert.
Ölbadtemperatur: 85° - 90°C
Hauptlauf m = 58,37 g 82,03 %
Siedepunkt: 59° C bei 11 mbar
Ausbeute: 58,37 g 82,03 % Aa)

### A) 2,2-Dimethyl-3-oxopentanal

In einem 1000 ml Dreihalsrundkolben wird die Lösung von 77,14 g Propionsäurechlorid in 200 ml Ether p.a. vorgelegt. Unter Eisbadkühlung wird innerhalb von 30 Minuten bei einer Reaktionstempertur von 6°C eine Lösung von 117,73 g der unter Aa) erhaltenen Verbindung in 200 ml Ether p. A. zugetropft. Ausfällung, weißer Niederschlag entsteht. Nach beendeter Zugabe wird der Ansatz 5 Stunden am Rückfluß gekocht und anschließend über Nacht bei Raumtemperatur gerührt. Der entstehende weiße Niederschlag, feuchtigkeitsempfindlich, wird abgesaugt, mit Ether gewaschen und an der Ölpumpe getrocknet.
Rohprodukt: m = 65,26 g Hydrochlorid.
Im Filtrat ist eine Nachfällung zu beobachten.
Rohprodukt m = 35,49 g Gesamt: m = 100,75 g.
Die 100,75 g Hydrochlorid werden in 150 ml H₂O gelöst. Anschließend wird die Wasserphase mit NaHCO₃ insgesamt auf pH 0 5 eingestellt und dann 4 mal mit je 150 ml Ether extrahiert. Die organische Phase wird einmal mit Sole gewaschen und dann über Na₂SO₄ getrocknet. Der Ether wird bei Normaldruck abdestilliert und der Rückstand wird im Vakuum über eine kleine Vigreux-Kolonne (6 Böden destilliert.
Hauptlauf: m = 29,65 g 27,75 %
Siedepunkt: 62°C bei 15 mbar
Ausbeute: 29,65 g 27,75 % A)

### B) 2,2-Dimethyl-3-oxo-butanal

Durchführung analog A).
   Ansatz: 58,37 g = 413,36 mMol Aa), M = 141,21 g/mol
   100 ml Diethylether p.A.
   32,45 g = 413,38 mMol Acetylchlorid, M = 0 78,5 g/mol
   = 1,104 g/ml
   100 ml Diäthylether p. A.
   übers Wochenende bei Raumtemperatur gerührt.
   Rohprodukt m = 72,07 g Hydrochlorid
Aufarbeitung siehe Ab)
   Ölbadtemperatur: 75°C bis 80°C
   Hauptlauf: m = 18,75 g 39,74 %
   Siedepunkt: 50°C bei 11 mbar
   Ausbeute m = 18,7 g 39,6 % B)

### C) 1-(1-Oxopropyl)cyclobutancarbaldehyd

### Ca) 1,1-Cyclobutandimethanol

Zu einer Lösung von 20 g (100 mmol) 1,1-Cyclobutandicarbonsäurediethylester in 200 ml absolutem Tetrahydrofuran werden bei 0°C 170 ml einer 1,2 molaren Lösung von Diisobutylaluminiumhydrid getropft. Man läßt eine Stunde bei 0°C nachrühren und addiert dann 30 ml Wasser. Es wird über Celite filtriert. Das Filtrat wird mit Natriumsulfat getrocknet und im Vakuum eingeengt. Das erhaltene Rohprodukt (9,9 g) wird ohne Aufreinigung in die Folgestufe eingesetzt.

### Cb) 1-[[[Dimethyl(1,1-dimethylethyl)silyl]oxy]methyl]cyclobutanmethanol

Zu einer Suspension von 3,4 g Natriumhydrid (60%ig in Öl, 85 mmol)) in 35 ml absolutem Tetrahydrofuran wird bei 0°C eine Lösung von 9,9 g Ca) (85 mmol) in 100 ml absolutem Tetrahydrofuran gegeben. Man läßt 30 Minuten nachrühren und addiert dann eine Lösung von 12,8 g *tert*.Butyldimethylsilylchlorid (85 mmol) in 50 ml Tetrahydrofuran. Man läßt eine Stunde bei 25°C nachrühren und gießt dann das Reaktionsgemisch auf gesättigte wäßrige Natriumhydrogencarbonatlösung. Es wird mit Ethylacetat extrahiert. Die organische Phase wird mit gesättigter Natriumchloridlösung gewaschen und über Natriumsulfat getrocknet. Nach Abziehen des Lösungsmittels im Vakuum wird das erhaltene Rohprodukt durch Säulenchromatographie an Kieselgel mit einem Gemisch aus Hexan/Ethylacetat gereinigt. Man erhält 13,5 g (69 %) der Titelverbindung.
¹H-NMR (CDCl₃): δ = 0,04 (6H), 0,90 (9H), 1,70-2,00 (6H), 3,70 (4H) ppm.

### Cc) 1-[[[Dimethyl(1,1-dimethylethyl)silyl]oxyl]methyl]cyclobutancarbaldehyd

8 ml Oxalylchlorid werden in 100 ml Dichlormethan gelöst. Man kühlt auf -78°C und addiert 13 ml Dimethylsulfoxid. Man läßt 3 Minuten nachrühren und addiert dann eine Lösung von 13,5 g Cb) (58,6 mmol) in 80 ml Dichlormethan. Nach weiteren 15 Minuten Nachrührzeit werden 58 ml Triethylamin hinzugetropft. Anschließend läßt man auf 0°C erwärmen. Dann wird das Reaktionsgemisch auf gesättigte Natriumhydrogencarbonatlösung gegossen. Man extrahiert mit Dichlormethan, wäscht die organische Phase mit gesättigter Natriumchloridlösung, trocknet über Natriumsulfat und engt im Vakuum ein. Nach Chromatographie des Rohprodukts an Kieselgel mit einem Gemisch aus Hexan/Ethylacetat erhält man 7,7 g (58 %) der Titelverbindung.
¹ H-NMR (CDCl₃): δ = 0,03 (6H), 0,90 (9H), 1,85-2,00 (4H), 2,20-2,30 (2H), 3,83 (2H), 9,70 (1H) ppm.

### Cd) 1-[[[Dimethyl(1,1-dimethylethyl)silyl]oxy]methyl]-α-ethylcyclobutanmethanol

Eine Lösung von 7,7 g (33,7 mmol) der unter Cc) beschriebenen Verbindung in 80 ml Tetrahydrofuran wird bei 0°C zu 20 ml einer 2 molaren Lösung von Ethylmagnesiumchlorid (40 mmol) in Tetrahydrofuran getropft. Man läßt 30 Minuten bei 0°C nachrühren und gießt dann das Reaktionsgemisch auf gesättigte Ammonium-chloridlösung. Es wird mit Ethylacetat extrahiert. Die organische Phase wird mit gesättigter Natriumchloridlösung gewaschen und über Natriumsulfat getrocknet. Nach dem Abziehen des Lösungsmittels wird das erhaltene Rohprodukt durch Säulenchromatographie an Kieselgel gereinigt. Man erhält 7,93 g (91,5%) der Titelverbindung.
¹H-NMR (CDCl₃): δ = 0,09 s (6H), 0,90 s (9H), 1,05 (3H), 1,30-1,50 (3H), 1,70-1,90 (4H), 2,09 (1H), 3,19 (1H), 3,46 (1H), 3,72 (1H), 3,85 (1H) ppm.

### Ce) 1-[1-[[[Dimethyl(1,1-dimethylethyl)silyl]oxy]methyl]cyclobut-1-yl]-1-propanon

Zu 3,76 ml (43,8 mmol) Oxalylchlorid in 80 ml Dichlormethan werden bei -78°C 6 ml (85,7 mmol) Dimethylsulfoxid addiert. Man läßt 3 Minuten nachrühren und addiert dann eine Lösung von 7,93 g (30,7 mmol) der unter Cd) beschriebenen Verbindung in 80 ml Dichlormethan. Es wird weitere 15 Minuten bei -78°C nachgerührt. Anschließend wird eine Mischung aus 19 ml (136 mmol) Triethylamin und 40 ml Dichlormethan hinzugetropft. Man läßt auf -25°C erwärmen und rührt bei dieser Temperatur 30 Minuten nach. Anschließend das Reaktiongemisch auf gesättigte eiskalte Natriumhydrogencarbonatlösung gegossen. Es wird mit Dichlormethan extrahiert. Die organische Phase wird mit gesättigter Natriumchloridlösung gewaschen und über Natriumsulfat getrocknet. Nach dem Abziehen des Lösungsmittels wird das erhaltene Rohprodukt über Kieselgel filtriert. Man erhält 7,87 g (100%) der Titelverbindung.
¹H-NMR (CDCl₃): δ = 0,05 (6H), 0,88 (9H), 1,04 (3H), 1,82-1,95 (4H), 2,33-2,47 (2H), 2,45-2,54 (2H), 3,81 (2H) ppm.

### Cf) 1-[1-(Hydroxymethyl)cyclobut-1-yl]-1-propanon

7,87 g (30,7 mmol) der unter Ce) beschriebenen Verbindung werden in 100 ml Tetrahydrofuran gelöst. Man addiert 15 ml einer 1 molaren lösung von Tetrabutylammoniumfluorid und läßt 12 Stunden bei 25°C nachrüren. Danach wird das Reaktionsgemisch auf gesättigte Natriumhydrogencarbonatlösung gegossen. Man extrahiert mit Ethylacetat. Die organische Phase wird mit gesättigter Natriumchloridlösung gewaschen und über Natriumsulfat getrocknet. Nach dem Abziehen des Lösungsmittels wird das erhaltene Rohprodukt durch Säulenchromatographie an Kieselgel gereinigt. Man erhält 3,19 g (73,4%) der Titelverbindung.
¹H-NMR (CDCl₃): δ = 1,07 (3H), 1,86-2,08 (4H), 2,32-2,40 (2H), 2,55-2,65 (2H), 3,88 (2H) ppm.

### C) 1-(1-Oxopropyl)cyclobutancarbaldehyd

Analog zu Beispiel Ce) werden aus 3,19 g (22,4 mmol) der unter Cf) beschriebenen Verbindung durch Oxidation 3,14 g (100%) der Titelverbindung erhalten.
¹H-NMR (CDCl₃): δ = 1,07 (3H), 1,85-2,00 (2H), 2,40-2,53 (6H), 9,70 (1H) ppm.

### Beispiel 1:

### (R)-4,4-Dimethyl-3-[3-[[dimethyl(1,1-dimethylethyl)silyl]oxy]-5-oxo-heptansäure

Zu einer Lösung von 190 mg des unter Beispiel 1c) hergestellten Silylethers in 2.5 ml einer Mischung aus Tetrahydrofuran und Wasser im Verhältnis 4:1 gibt man bei 0°C 0.17 ml einer 30%igen Wasserstoffperoxid-Lösung. Nach 5 Minuten Rühren wird dann eine Lösung von 15.8 mg Lithiumhydroxid in 0.83 ml Wasser hinzugegeben, und die Reaktionsmischung für 3 Stunden bei 25°C gerührt. Anschließend wird mit einer Lösung von 208 mg Natriumsulfit in 1.24 ml Wasser versetzt und mit 10 ml Methylenchlorid extrahiert. Die wäßrige Phase wird mit 5N Salzsäure auf pH=1 eingestellt und dreimal mit je 10 ml Essigester extrahiert. Nach dem Trocknen über Natriumsulfat und Filtration wird im Vakuum eingeengt. Zusätzlich wird die obige Methylenchlorid-Phase mit 5N Salzsäure gewaschen und dann diese wäßrige Phase dreimal mit je 10 ml Essigester extrahiert. Nach dem Trocknen über Natriumsulfat und Filtration wird im Vakuum eingeengt und eine zusätzliche Menge an Rohprodukt erhalten. Die vereinigten, so erhaltenen Rückstande reinigt man durch Chromatographie an Kieselgel. Mit Hexan / 0-50% Essigester erhält man neben 70 mg (4R,5S)-4-Methyl-5-phenyloxazolidin-2-on 93 mg der Titelverbindung als farbloses Öl. [α]_{D}=+15.5° (CHCl₃)
¹H-NMR (CDCl₃): d = 0.03 - 0.08 (6H), 0.86 (9H), 1.01 (3H), 1.10 (3H), 1.15 (3H), 2.35 (1H), 2.4 - 2.7 (3H), 4.48 (1H) ppm.

### 1a) (4R,5S)-3-(Bromacetyl)-4-methyl-5-phenyloxazolidin-2-on

Zu einer Lösung von 30.1 g (4*R*,5S)-4-Methyl-5-phenyloxazolidin-2-on in 500 ml Tetrahydrofuran gibt man innerhalb von 30 Minuten bei -70°C unter Stickstoff 117 ml einer 1.6 molaren Lösung von Butyllithium in Hexan zu. Anschließend wird eine Lösung von 26.8 g Bromacetylchlorid in 250 ml Tetrahydrofuran so zugetropft, daß die Temperatur nicht über -65°C steigt. Nach 1.75 Stunden Rühren bei -70°C gibt man eine gesättigte Ammoniumchlorid-Lösung hinzu, gefolgt von 60 ml einer gesättigten Natriumhydrogencarbonat-Lösung und läßt auf 25°C kommen. Nach Trennung der Phasen wird die wäßrige Phase zweimal mit je 100 ml Ether extrahiert. Die vereinigten organischen Phasen werden mit halbkonzentrierter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-50% Ether erhält man 34.8 g der Titelverbindung als farbloses Öl.
¹H-NMR (CDCl₃): δ = 0.95 (3H), 4.57 (2H), 4.80 (2H), 5.76 (2H), 7.2 - 7.5 (5H) ppm.

### 1b)[4R-[3(R*),4α,5α]]-3-[4 4-Dimethyl-1,5-dioxo-3-hydroheptyl-4-methyl-5-phenyl-oxazolidin-2-on

Zu einer Suspension von 5.0 g wasserfreim Chrom(II)chlorid in 60 ml Tetrahydrofuran gibt man unter Argon 218 mg Lithiumiodid. Anschließend wird eine Mischung von 2.09 g des literaturbekannten 2,2-Dimethyl-3-oxo-pentanal (siehe unter "Ausgangsprodukte" Ab) und 5.34 g der vorstehend hergestellten Bromverbindung in 10 ml Tetrahydrofuran hinzugegeben. Nach 2 Stunden Reaktionszeit wird mit 30 ml gesättigter Natriumchlorid-Lösung versetzt und 15 Minuten gerührt. Die wäßrige Phase wird dreimal mit je 200 ml Ether extrahiert. Die vereinigten organischen Phasen werden mit halbkonzentrierter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan / 0-30% Essigester erhält man 1.55 g der Titelverbindung als farbloses Öl.
¹H-NMR (CDCl₃): δ = 0.92 (3H), 1.06 (3H), 1.18 (3H), 1.23 (3H), 2.58 (2H), 3.07 (2H), 3.28 (1H), 4.35 (1H), 4.79 (1H), 5.70 (2H), 7.2 - 7.5 (5H) ppm.

### 1c) [4R-[3(R*),4α,5α]]-3-[4,4-Dimethyl-3-[[dimethyl(1,1-dimethylethyl)silyl]oxy]-1,5-dioxoheptyl]-4-methyl-5-phenyloxazolidin-2-on

Zu einer Lösung von 347 mg des vorstehend hergestellten Alkohols in 3 ml Methylenchlorid gibt man unter Argon bei -70°C 150 mg 2,6-Lutidin. Nach 5 Minuten Rühren werden 344 mg tert.Butyldimethylsilyltrifluormethansulfonat hinzugegeben und für weitere 45 Minuten bei -70°C gerührt. Man versetzt mit 1 ml gesättigter Natriumchlorid-Lösung und läßt auf 25°C kommen. Anschließend wird mit Ether verdünnt und die organische Phase mit gesättigter Natriumchlorid-Lösung gewaschen. Nach dem Trocknen über Natriumsulfat und Filtration wird im Vakuum eingeengt. Den so erhaltenen Rückstand reinigt man durch Chromatographie an Kieselgel. Mit Hexan / 0-30% Essigester erhält man 192 mg der Titelverbindung als farblose kristalline Verbindung mit einem Schmelzpunkt von 111-112°C.
¹H-NMR (CDCl₃): δ = 0.01 - 0.12 (6H), 0.86 (9H), 0.90 (3H), 1.00 (3H), 1.13 (3H), 1.17 (3H), 2.56 (2H), 3.05 (2H), 4.65 - 4.80 (2H), 5.68 (1H), 7.2 - 7.5 (5H) ppm.

### Beispiel 2

### (S)-4,4-Dimethyl-3-[3-[[dimethyl(1,1-dimethylethyl)silyl]oxy]-5-oxo-heptansäure

Die Verbindung wird in Analogie zu Beispiel 1 hergestellt. Als Ausgangsprodukt dient (4S,5R)-4-Methyl-5-phenyloxazolidin-2-on. NMR ist deckungsgleich mit Beispiel 1.
[α]_{D}= -15.7° (CHCl₃)

### 2a) (4S,5R)-3-(Bromacetyl)-4-methyl-5-phenyloxazolidin-2-on

Die Darstellung erfolgt analog zu Beispiel 1a) ausgehend von (4S,5R)-4-Methyl-5-phenyloxazolidin-2-on. NMR ist deckungsgleich mit 1a).

### Beispiel 3

### (S)-3-[3-[[Dimethyl(1,1-dimethyl)silyl]oxyl-3-[1-(1-oxopropyl)cyclobut-1-yl]propansäure

Analog zu Beispiel 1 werden aus 2,79 g (5,9 mmol) der unter 3b) beschriebenen Verbindung 1,49 g (80 %) der Titelverbindung und 941 mg zurückgewonnenes (4*S*,5*R*)-4-Methyl-5-phenyloxazolidin-2-on erhalten. Die Titelverbindung und das zurückzugewinnende chirale Auxiliar lassen sich durch Chromatographie (analog Beispiel 1) oder auch fraktionierte Kristallisation trennen und danach durch Chromatographie gewünschtenfalls aufreinigen.
¹H-NMR (CDCl₃): δ = 0.09 (3H), 0.19 (3H), 0.90 (9H), 1.08 (3H), 1.70 - 2.00 (3H), 2.20 - 2.40 (4H), 2.47 (1H), 2.50 - 2.70 (2H), 4.45 (1H) ppm.

### 3a)[4S-[3(R*),4α,5α]]-3-[3-Hydroxy-1-oxo-3-[1-(1-oxopropyl)cyclobut-1-yl]propyl]-4-methyl-5-phenyloxazolidin-2-on

Analog zu Beispiel 1b) werden aus 3,14 g (22,4 mmol) der unter C) beschriebenen Verbindung, 9,7 g (78,8 mmol) wasserfreiem Chrom(II)chlorid, 9,69 g (32,5 mmol) 2a) und 300 mg (2,2 mmol) wasserfreiem Lithiumiodid in Tetrahydrofuran nach Säulenchromatographie an Kieselgel 3,0 g (37,4%) der Titelverbindung als farbloses Öl erhalten.
¹H-NMR (CDCl₃): δ = 0,93 (3H), 1,10 (3H), 1,80-2,03 (2H), 2,10-2,21 (1H), 2,26-2,35 (3H), 2,54-2,70 (2H), 3,03-3,08 (2H), 3,34 (1H), 4,39 (1H), 4,74-4,85 (1H), 5,69 (1H), 7,27-7,34 (2H), 7,36-7,49 (3H) ppm.

### 3b) [4S-[3(R*),4α,5α]]-3-[3-[[Dimethyl(1,1-dimethylethyl)silyl]oxy]-1-oxo-3-[1-(1-oxopropyl)cyrclobut-1-yl]propyl]-4-methyl-5-phenyloxazolidin-2-on

Analog zu Beispiel 1c) werden aus 3,0 g (8,35 mmol) der unter Beispiel 3a) beschriebenen Verbindung, tert.Butyldimethylsilyltrifluormethansulfonat und 2,6-Lutidin nach Umkristallisation aus Diisopropylether 2,79 g (70,6 %) der Titelverbindung erhalten.
¹H-NMR (CDCl₃): δ = 0,10 (3H), 0,21 (3H), 0,92 (3H), 0,95 (9H), 1,10 (3H), 1,70-1,92 (2H), 2,02-2,16 (1H), 2,20-2,40 (3H), 2,50-2,72 (2H), 2,98-3,10 (2H), 4,63-4,75 (1H), 5,69 (1H), 7,28-7,35 (2H), 7,36-7,48 (3H) ppm.

### Beispiel 4

### (R)-3-[3-[[Dimethyl(1,1-dimethyl)silyl]oxy]-3-[1-(1-oxopropyl)cyclobut-1-yl]propansäure

Die Verbindung wird in Analogie zu Beispiel 3 hergestellt. Als Ausgangsprodukt dient (4R,5S)-3-(Bromacetyl)-4-methyl-5-phenyloxazolidin-2-on.
Das NMR-Spektrum ist deckungsgleich mit Beispiel 3.

Durch die Wahl der Stereochemie an C4 und C5 des chiralen Auxiliars 4-Methyl-5-phenyl-2-oxazolidon läßt sich die Stereochemie in Position 3 steuern.
Die Struktur des Intermediats 1 b) wurde durch eine Röntgenstrukturanalyse belegt.

### Beispiele für die Herstellung des Bausteines C

### Beispiel 1

### (S)-Dihydro-3-hydroxy-2(3H)-furanon

10 g L-(-)-Äpfelsäure werden in 45 ml Trifluoressigsäureanhydrid 2 Stunden bei 25°C gerührt. Danach engt man im Vakuum ein, addiert zu dem Rückstand 7 ml Methanol und läßt 12 Stunden nachrühren. Anschließend wird im Vakuum eingeengt. Der erhaltene Rückstand wird in 150 ml absolutem Tetrahydrofuran gelöst. Man kühlt auf 0°C und addiert 150 ml Boran-Tetrahydrofuran-Komplex und läßt 2,5 Stunden bei 0°C nachrühren. Danach werden 150 ml Methanol addiert. Man läßt eine Stunde bei Raumtemperatur nachrühren und engt dann im Vakuum ein. Das erhaltene Rohprodukt wird in 80 ml Toluol gelöst. Man addiert 5 g Dowex^{®} (aktiviert, sauer)und kocht eine Stunde unter Rückfluß. Anschließend wird das Dowex^{®} abfiltriert und das Filtrat im Vakuum eingeengt. Das erhaltene Rohprodukt (7,61 g; 99,9%) wird ohne Aufreinigung in die Folgestufe eingesetzt.

### Beispiel 2

### (S)-Dihydro-3-[[(1,1-dimethylethyl)diphenylsilyl]oxy]2(3H)-furanon

Zu einer Lösung von 7,61 g der unter Beispiel 1 beschriebenen Substanz und 10 g Imidazol in 100 ml *N,N*-Dimethylformamid werden 24 ml *tert*.Butyldiphenylsilylchlorid addiert. Man läßt zwei Stunden bei 25°C nachrühren und gießt dann das Reaktionsgemisch auf eiskalte gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 13,4 g (52,8%) der Titelverbindung erhalten.
¹H-NMR (CDCl₃): δ = 7,72 (2H), 7,70 (2H), 7,40-7,50 (6H), 4,30-4,42 (2H), 4,01 (1H), 2,10-2,30 (2H), 1,11 (9H) ppm.

### Beispiel 3

### (2RS,3S)-3-[[(I, I-Dimethylethyl)diphenylsilyl]oxy]tetrahydro-2-furanol

Zu einer Lösung von 13,4 g der unter Beispiel 2 beschriebenen Substanz in 150 ml absolutem Tetrahydrofuran werden 80 ml einer 1 molaren Lösung von Diisobutylaluminiumhydrid in Hexan bei -78°C addiert. Man rührt 45 Minuten bei -78°C nach und quencht dann mit Wasser. Man extrahiert mit Ethylacetat, wäscht die organische Phase mit gesättigter Natriumchloridlösung, trocknet über Natriumsulfat und engt im Vakuum ein. Man erhält 13,46 g (99,4%) der Titelverbindung, welche ohne Reinigung in die Folgestufe eingesetzt wird.

### Beispiel 4

### (2RS,3S)-3-[[(1,1-Dimethylethyl)diphenylsilyl]oxy],4-pentandiol

Zu 20 ml einer 3 molaren Lösung von Methylmagnesiumchlorid in Tetrahydrofuran wird bei 0°C eine Lösung von 13,46 g der unter Beispiel 3 beschriebenen Substanz in 150 ml absolutem Tetrahydrofuran getropft. Man läßt eine Stunde bei 0°C nachrühren und gießt dann auf gesättigte wäßrige Ammoniumchloridlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 11,42 g (81,6%) der Titelverbindung erhalten.
¹H-NMR (CDCl₃): δ = 7,65-7,75 (4H), 7,40-7,55 (6H), 5,20 (1H), 4,30 (2H), 3,70 (1H), 1,80 (2H), 1,05 (9H) ppm.

### Beispiel 5

### (2RS,3S)-5-[[Dimethyl(1,1-dimethylethyl)silyl]oxy]-3-[[(1,1-dimethylethyl)diphenylsilyl]oxy]-2-pentanol

Zu einer Lösung von 11,42 g der unter Beispiel 4 beschriebenen Substanz und 3,25 g 1*H*-Imidazol in 120 ml *N*,*N*-Dimethylformamid werden 4,9 g *tert*-Butyldimethylsilylchlorid addiert. Man läßt 2 Stunden bei 25°C nachrühren und gießt dann das Reaktionsgemisch auf eiskalte gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 10,64 g (70,5%) der Titelverbindung erhalten.
¹H-NMR (CDCl₃): δ = 7,60-7,70 (4H), 7,30-7,45 (6H), 3,70-3,80 (2H), 3,40 (1H), 3,00 (1H), 1,80 (1H), 1,60 (1H), 1,05-1,12 (12H), 0,82 (9H), 0,02 (6H) ppm.

### Beispiel 6

### (3S)-5-[[Dimethyl(1,1-dimethylethyl)silyl]oxy]-3-[[(1,1-dimethylethyl)diphenylsilyl]oxy]-2-pentanon

Zu 7,37 ml Oxalylchlorid in 80 ml Dichlormethan werden bei -78°C 13 ml Dimethylsulfoxid addiert. Man läßt 3 Minuten nachrühren und addiert dann 10,64 g der unter Beispiel 5 beschriebenen Substanz in 100 ml Dichlormethan. Nach weiteren 15 Minuten Nachrührzeit werden 52 ml Triethylamin hinzugetropft. Anschließend läßt man auf 0°C erwärmen. Danach wird das Reaktionsgemisch auf gesättigte Natriumhydrogencarbonatlösung gegossen. Man extrahiert mit Dichlormethan, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 9,3 g (26,5% bezogen auf die eingesetzte Äpfelsäure) der Titelverbindung erhalten.
¹H-NMR (CDCl₃): δ = 7,60-7,70 (4H), 7,32-7,50 (6H), 4,25 (1H), 3,72 (1H), 3,58 (1H), 2,05 (3H), 1,90 (1H), 1,75 (1H), 1,13 (9H), 0,89 (9H), 0,01 (6H) ppm.

### Beispiel 7

### (R)-Dihydro-3-hydroxy-2(3H)-furanon

10 g D-(+)-Äpfelsäure werden analog zu Beispiel 1 umgesetzt. Man erhält 7,26 g der Titelverbindung. Das ¹ H-NMR-Spektrum ist deckungsgleich mit 1.

### Beispiel 8

### (R)-Dihydro-3-[[(1,1-dimethylethyl)diphenylsilyl]oxy]-2(3H)-furanon

Analog zu Beispiel 2 werden aus 7,26 g der unter Beispiel 7 beschriebenen Substanz 12,9 g der Titelverbidnung erhalten. Das ¹H-NMR-Spektrum ist deckungsgleich mit 2.

### Beispiel 9

### (2RS,3R)-3-[[(1,1-Dimethylethyl)diphenylsilyl]oxy]tetrahydro-2-furanol

Analog zu Beispiel 3 werden aus 12,9 g der unter Beispiel 8 beschriebenen Substanz 12,95 g der Titelverbindung erhalten. Das ¹ H-NMR-Spektrum ist deckungsgleich mit 3.

### Beispiel 10

### (2RS,3R)-3-[[(1,1-Dimethylethyl)diphenylsilyl]oxy]-1,4-pentandiol

Analog zu Beispiel 4 werden aus 12,95g der unter Beispiel 9 beschriebenen Substanz 11 g der Titelverbindung erhalten. Das ¹H-NMR-Spektrum ist deckungsgleich mit 4.

### Beispiel 11

### (2RS,3R)-5-[[Dimethyl(1,1-dimethylethyl)silyl]oxy]-3-[[(1,1-dimethylethyl)diphenylsilyl]oxy]-2-pentanol

Analog zu Beispiel 5 werden aus 11 g der unter Beispiel 10 beschriebenen Substanz 10,11 g der Titelverbindung erhalten. Das ¹H-NMR-Spektrum ist deckungsgleich mit 5.

### Beispiel 12

### (R)-5-[[Dimethyl(1,1-dimethylethyl)silyl]oxy]-3-[[(1,1-dimethylethyl)diphenylsilyl]oxy]-2-pentanon

Analog zu Beispiel 6 werden aus 10,11 g der unter Beispiel 11 beschriebenen Substanz 8,85 g der Titelverbindung erhalten. Das ¹H-NMR-Spektrum ist deckungsgleich mit 6.

### Beispiel 13

### (3RS)-Dihydro-3-hydroxy-2(3H)-furanon

5 g racemische Äpfelsäure werden analog zu Beispiel 1 umgesetzt. Man erhält 3,68 g der Titelverbindung. Das 1H-NMR-Spektrum ist deckungsgleich mit 1.

### Beispiel 14

### (3RS)-Dihydro-3-[[(1,1-dimethylethyl)diphenylsilyl]oxy]-2(3H)-furanon

Analog zu Beispiel 2 werden aus 3,68 g der unter Beispiel 13 beschriebenen Substanz 6,5 g der Titelverbindung erhalten. Das ¹H-NMR-Spektrum ist deckungsgleich mit 2.

### Beispiel 15

### (2RS,3RS)-3-[[(1,1-Dimethylethyl)diphenylsilyl]oxy]tetrahydro-2-furanol

Analog zu Beispiel 3 werden aus 6,5 g der unter Beispiel 14 beschriebenen Substanz 6,51 g der Titelverbindung erhalten. Das ¹H-NMR-Spektrum ist deckungsgleich mit 15.

### Beispiel 16

### (2RS,3RS)-3-[[(1,1-Dimethylethyl)diphenylsilyl]oxy]-1,4-pentandiol

Analog zu Beispiel 4 werden aus 6,51 g der unter Beispiel 15 beschriebenen Substanz 5,5 g der Titelverbindung erhalten. Das ¹ H-NMR-Spektrum ist deckungsgleich mit 4.

### Beispiel 17

### (2RS,3RS)-5-[[Dimethyl(1,1-dimethylethyl)silyl]oxy]-3-[[(1,1-dimethylethyl)diphenylsilyl]oxy]-2-pentanol

Analog zu Beispiel 5 werden aus 5,5 g der unter Beispiel 16 beschriebenen Substanz 5,05 g der Titelverbindung erhalten. Das ¹ H-NMR-Spektrum ist deckungsgleich mit 5.

### Beispiel 18

### (3RS)-5-[[Dimethyl(1,1-dimethylethyl)silyl]oxy]-3-[[(1,1-dimethylethyl)diphenylsilyl)oxy]-2-pentanon

Analog zu Beispiel 6 werden aus 5,05 g der unter Beispiel 17 beschriebenen Substanz 4,3 g der Titelverbindung erhalten. Das ¹H-NMR-Spektrum ist deckungsgleich mit 6.

### Beispiel 19

### (E,3S)-1-[[Dimethyl(1,1-dimethylethyl)silyl]oxy]-3-[[(1,1-dimethylethyl)diphenylsilyl]oxy]-4-methyl-5-(2-methylthiazol-4-yl)-pent-4-en

Die Lösung von 6,82g Diethyl(2-methylthiazol-4-yl)methanphosphonat in 300 ml wasserfreiem Tetrahydrofuran kühlt man unter einer Atmosphäre aus trockenem Argon auf -5°C, versetzt mit 16,2 ml einer 1,6 molaren Lösung von n-Buthyllithium in n-Hexan, läßt auf 23°C erwärmen und 2 Stunden rühren. Anschließend kühlt man auf -78°C, tropft die Lösung von 6,44 g (13,68 mmol) der nach Beispiel 6 dargestellten Verbindung in 150 ml Tetrahydrofuran zu, läßt auf 23°C erwärmen und 16 Stunden rühren. Man gießt in gesättigte Ammoniumchloridlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 6,46 g (11,4 mmol, 83%; Ausbeute bezogen auf die eingesetzte Äpfelsäure: 22%) der Titelverbindung als farbloses Öl.
¹H-NMR (CDCl₃): δ = -0,04 (6H), 0,83 (9H), 1,10 (9H), 1,79 (1H), 1,90 (1H), 1,97 (3H), 2,51 (3H), 3,51 (2H), 4,38 (1H), 6,22 (1H), 6,74 (1H), 7,23-7,47 (6H), 7,63 (2H), 7,70 (2H) ppm.

### Beispiel 20

### (E,3 S)-3-[[(1,1-dimethylethyl)diphenylsilyl]oxy]-4-methyl-5-(2-methylthiazol-4-yl)-pent-4-en-1-ol

Die Lösung von 4,79 g (8,46 mmol) der nach Beispiel 19 dargestellten Verbindung in 48 ml Tetrahydrofuran versetzt man mit 48 ml eines 65:35:10-Gemisches aus Eisessig/Wasser/Tetrahydrofuran und rührt 2,5 Tage bei 23°C. Man gießt in gesättigte Natriumcarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 3,42 g (7,57 mmol, 90%) der Titelverbindung als farbloses Öl.
¹H-NMR (CDCl₃): δ = 1,10 (9H), 1,53 (1H), 1,81 (2H), 1,96 (3H), 2,71 (3H), 3,59 (2H), 4,41 (1H), 6,38 (1H), 6,78 (1H), 7,26-7,49 (6H), 7,65 (2H), 7,72 (2H) ppm.

### Beispiel 21

### (E,3S)-1-Brom-3-[[(1,1-dimethylethyl)diphenylsilyl]oxy]-4-methyl-5-(2-methylthiazol-4-yl)-pent-4-en

Die Lösung von 378 mg (0,84 mmol) der nach Beispiel 20 dargestellten Verbindung in 9 ml Dichlormethan versetzt man bei 0°C unter einer Atmosphäre aus trockenem Argon mit 90 µl Pyridin, 439 mg Triphenylphosphin, 556 mg Tetrabrommethan und rührt 1 Stunde bei 0°C. Die Lösung chromatographiert man an feinem Kieselgel mit einem Gradientensystem aus n-Hexan und Ethylacetat. Isoliert werden 362 mg (0,70 mmol, 84%) der Titelverbindung als farbloses Öl.
¹H-NMR (CDCl₃): δ = 1,09 (9H), 1,95 (3H), 2,01-2,23 (2H), 2,71 (3H), 3,15-3,35 (2H), 4,35 (1H), 6,30 (1H), 6,79 (1H), 7,25-7,49 (6H), 7,63 (2H), 7,69 (2H) ppm.

### Beispiel 22

### (E,3S)-1-Iod-3-[[(1,1-dimethylethyl)diphenylsilyl]oxy]4-methyl-5-(2-methylthiazol-4-yl)-pent-4-en

Die Lösung von 8,41 g Triphenylphosphin in 120 ml Dichlormethan versetzt man bei 23°C unter einer Atmosphäre aus trockenem Argon mit 2,19 g Imidazol, 8,14 g Iod, tropft die Lösung von 12,2 g (27,0 mmol) der nach Beispiel 20 dargestellten Verbindung in 30 ml Dichlormethan zu und rührt 0,5 Stunden. Die Lösung chromatographiert man an feinem Kieselgel mit einem Gradientensystem aus n-Hexan und Ethylacetat. Isoliert werden 12,15 g (21,6 mmol, 80%) der Titelverbindung als farbloses Öl.
¹H-NMR (CDCl₃): δ = 1,08 (9H), 1,96 (3H), 2,10 (2H), 2,70 (3H), 2,87-3,08 (2H), 4,24 (1H), 6,32 (1H), 6,79 (1H), 7,28-7,48 (6H), 7,60-7,72 (4H) ppm.

### Beispiel 23

### (5E,3S)-[3-[[(1,1-Dimethylethyl)diphenylsilyl]oxy]4-methyl-5-(2-methylthiazol-4-yl)-pent-4-en-1-yl]-triphenylphosphoniumiodid

Die Suspension aus 12,55 g (22,3 mmol) der nach Beispiel 22 dargestellten Verbindung, 85 g Triphenylphosphin und 11,6 ml N-Ethyldiisopropylamin rührt man unter einer Atmosphäre aus trockenem Argon 16 Stunden bei 80°C. Nach dem Erkalten versetzt man mit Diethylether, filtriert und wäscht den Rückstand mehrfach mit Diethylether nach und kristallisiert aus Ethylacetat um. Isoliert werden 15,7 g (19,1 mmol, 74%) der Titelverbindung als kristalliner Feststoff.
¹H-NMR (CDCl₃): δ = 1,07 (9H), 1,68-1,92 (2H), 1,98 (3H), 2,70 (3H), 2,93 (1H), 3,30 (1H), 4,53 (1H), 6,62 (1H), 7,03 (1H), 7,23-7,47 (6H), 7,48-7,72 (16H), 7,73-7,85 (3H) ppm.

### Beispiel 24

### (E,3R)-1-[[Dimethyl(1,1-dimethylethyl)silyl]oxy]-3-[[(1,1-dimethylethyl)diphenylsilyl]oxy]-4-methyl-5-(2-methylthiazol-4-yl)-pent-4-en

Analog zu Beispiel 19 werden aus 8,85 g der unter Beispiel 12 beschriebenen Verbindung 8,56 g (80%) der Titelverbindung erhalten. Das ¹H-NMR-Spektrum ist deckungsgleich mit 19

### Beispiel 25

### (E,3R)-3-[[(1,1-dimethylethyl)diphenylsilyl]oxy]-4-methyl-5-(2-methylthiazol-4-yl)-pent-4-en-1-ol

Analog zu Beispiel 20 werden aus 8,56 g der unter Beispiel 24 beschriebenen Verbindung 6,25 g (92 %) der Titelverbindung erhalten. Das ¹H-NMR-Spektrum ist deckungsgleich mit 20.

### Beispiel 26

### (E,3R)-1-lod-3-[[(1,1-dimethylethyl)diphenylsilyl]oxy]-4-methyl-5-(2-methylthiazol-4-yl)-pent-4-en

Analog zu Beispiel 22 werden aus 6,25 g der unter Beispiel 25 beschriebenen Verbindung 6,22 g (80%) der Titelverbindung erhalten. Das ¹ H-NMR-Spektrum ist deckungsgleich mit 22.

### Beispiel 27

### (5E,3R)-[3-[[(1,1-Dimethylethyl)diphenylsilyl]oxy]-4-methyl-5-(2-methylthiazol-4-yl)-pent-4-en-1-yl]-triphenylphosphoniumiodid

Analog zu Beispiel 23 werden aus 6,22 g der unter Beispiel 26 beschriebenen Verbindung 7,36 g (70 %) der Titelverbindung erhalten. Das ¹ H-NMR-Spektrum ist deckungsgleich mit 23.

### Beispiel 28

### (E,3RS)-1-[[Dimethyl(1,1-dimethylethyl)silyl]oxy]-3-[[(1,1-dimethylethyl)diphenylsilyl]oxy]-4-methyl-5-(2-methylthiazol-4-yl)-pent-4-en

Analog zu Beispiel 19 werden aus 4,3 g der unter Beispiel 18 beschriebenen Verbindung 4,52 g (87 %) der Titelverbindung erhalten. Das ¹H-NMR-Spektrum ist deckungsgleich mit 19.

### Beispiel 29

### (E,3RS)-3-[[(1,1-dimethylethyl)diphenylsilyl]oxy]-4-methyl-5-(2-methylthiazol-4-yl)-pent-4-en-1-ol

Analog zu Beispiel 20 werden aus 4,52 g der unter Beispiel 28 beschriebenen Verbindung 3,16 g (88 %) der Titelverbindung erhalten. Das ¹H-NMR-Spektrum ist deckungsgleich mit 20.

### Beispiel 30

### (E,3RS)-1-Iod-3-[[(1,1-dimethylethyl)diphenylsilyl]oxy]-4-methyl-5-(2-methylthiazol-4-yl)-pent-4-en

Analog zu Beispiel 22 werden aus 3,16 g der unter Beispiel 25 beschriebenen Verbindung 3,34 g (85 %) der Titelverbindung erhalten. Das ¹H-NMR-Spektrum ist deckungsgleich mit 22.

### Beispiel 31

### (5E,3RS)-[3-[[(1,1- Dimethylethyl)diphenylsilyl]oxy]-4-methyl-5-(2-methylthiazol-4-yl)-pent-4-en-1-yl]-triphenylphosphoniumiodid

Analog zu Beispiel 23 werden aus 3,34 g der unter Beispiel 26 beschriebenen Verbindung 4,35 g (77 %) der Titelverbindung erhalten. Das ¹H-NMR-Spektrum ist deckungsgleich mit 23.

### Beispiel 32

### (E,3S)-1-[[Dimethyl(1,1-dimethylethyl)silyl]oxy]-3-[[(1,1-dimethylethyl)diphenylsilyl]oxy]-4-methyl-5-(2-pyridyl)-pent-4-en

In Analogie zu Beispiel 19 setzt man 2 g (4,23 mmol) der nach Beispiel 6 dargestellten Verbindung unter Verwendung von Diethyl(2-pyridyl)methanphosphonat um und isoliert nach Aufarbeitung und Reinigung 2 g (3,68 mmol, 87%) der Titelverbindung als farbloses Öl.
¹H-NMR (CDCl₃): δ = -0,06 (6H), 0,80 (9H), 1,09 (9H), 1,81 (1H), 1,90 (1H), 2,00 (3H), 3,53 (2H), 4,40 (1H), 6,22 (1H), 6,99 (1H), 7,06 (1H), 7,25-7,45 (6H), 7,58 (1H), 7,65-7,77 (4H), 8,58 (1H) ppm.

### Beispiel 33

### (E,3S)-3-[[(1,1-Dimethylethyl)diphenylsilyl]oxy]-4-methyl-5-(2-pyridyl)-pent-4-en-1-ol

Analog zu Beispiel 20 werden 2 g (3,68 mmol) der unter Beispiel 32 hergestellten Verbindung mit einem 65:35:10-Gemische aus Eisessig/Wasser/Tetrahydrofuran umgesetzt. Man erhält nach Aufreinigung 1,38 g (3,20 mmol, 87%) der Titelverbindung.
¹H-NMR (CDCl₃): δ = 1,12 (9H), 1,85 (2H), 2,00 (3H), 3,62 (2H), 4,45 (1H), 6,44 (1H), 7,03 (1H), 7,08 (1H), 7,25-7,48 (6H), 7,59 (1H), 7,65-7,77 (4H), 8,58 (1H) ppm.

### Beispiel 34

### (Z,3S)-1-[[Dimethyl(1,1-dimethylethyl)silyl]oxy]-3-[[(1,1-dimethylethyl)diphenylsilyl]oxy]-4-methyl-5-(3-pyridyl)-pent-4-en (A) und (E,3S)-1-[[Dimethyl(1,1-dimethylethyl)silyl] oxy]-3-[[(1,1-dimethylethyl)diphenylsilyl]oxy]-4-methyl-5-(3-pyridyl)-pent-4-en (B)

In Analogie zu Beispiel 19 setzt man 4,8 g (10,2 mmol) der nach Beispiel 6 dargestellten Verbindung unter Verwendung von Diethyl(3-pyridyl)methanphosphonat um und isoliert nach Aufarbeitung und Reinigung 448 mg (0,82 mmol, 8%) der Titelverbindung A sowie 3,5 g (6,41 mmol, 63%) der Titelverbindung B jeweils als farbloses Öl.
¹H-NMR (CDCl₃) von A: δ = -0,06 (6H), 0,81 (9H), 1,01 (9H), 1,75 (1H), 1,97 (4H), 3,48 (2H), 4,83 (1H), 6,11 (1H), 6,97 (1H), 7,11-7,30 (5H), 7,30-7,39 (2H), 7,39-7,50 (4H), 8,08 (1H), 8,33 (1H) ppm.
¹H-NMR (CDCl₃) von B: δ = -0,01 (6H), 0,85 (9H), 1,11 (9H), 1,78 (3H), 1,83 (1H), 1,97 (1H), 3,58 (2H), 4,42 (1H), 6,03 (1H), 7,21 (1H), 7,28-7,50 (7H), 7,62-7,75 (4H), 8,29 (1H), 8,41 (1H) ppm.

### Beispiel 35

### (E,3S)-3-[[(1,1-Dimethylethyl)diphenylsilyl]oxy]-4-methyl-5-(3-pyridyl)-pent-4-en-1-ol

Analog zu Beispiel 20 werden 3,5 g (6,41 mmol) der unter Beispiel 34B hergestellten Verbindung mit einem 65:35:10-Gemische aus Eisessig/Wasser/Tetrahydrofuran umgesetzt. Man erhält nach Aufreinigung 2,1 g (4,86 mmol, 76%).
¹H-NMR (CDCl₃): δ = 1,12 (9H), 1,75 (3H), 1,88 (2H), 3,65 (2H), 4,45 (1H), 6,25 (1H), 7,21 (1H), 7,28-7,50 (7H), 7,60-7,75 (4H), 8,30 (1H), 8,44 (1H) ppm.

### Beispiel 36

Analog zu Beispiel 22 werden aus 2,1 g der unter Beispiel 35 beschriebenen Verbindung 1,98 g (75%) der Titelverbindung erhalten.
¹H-NMR (CDCl₃): δ= 1,11 (9H), 1,78 (3H), 2,17 (2H), 3,03 (2H), 4,29 (1H), 6,19 (1H), 7,22 (1H), 7,30-7,50 (7H), 7,63-7,75 (4H), 8,32 (1H), 8,44 (1H) ppm.

### Beispiel 37

Analog zu Beispiel 23 werden aus 1,98 g der unter Beispiel 36 beschriebenen Verbindung 2,35 g (80%) der Titelverbindung erhalten.
¹H-NMR (CDCl₃): δ= 1,08 (9H), 1,80 (3H), 3,27 (1H), 3,56 (1H), 4,66 (1H), 6,52 (1H), 7,25-7,90 (27H), 8,35 (1H), 8,46 (1H) ppm.

### Beispiel 38

### (Z,3S)-1-[[Dimethyl1(1,1-dimethylethyl)silyl]oxy]-3-[[(1,1-dimethylethyl)diphenylsilyl]oxy]-4-methyl-5-(4-pyridyl)-pent-4-en (A) und (E,3S)-1-[[Dimethyl(1,1-dimethylethyl)silyl] oxy]-3-[[(1,1-dimethylethyl)diphenylsilyl]oxy]-4-methyl-5-(4-pyridyl)-pent-4-en (B)

In Analogie zu Beispiel 19 setzt man 4,59 g (9,75 mmol) der nach Beispiel 6 dargestellten Verbindung unter Verwendung von Diethyl(4-pyridyl)methanphosphonat um und isoliert nach Aufarbeitung und Reinigung 605 mg (1,11 mmol, 11%) der Titelverbindung A sowie 4,34 g (7,95 mmol, 82%) der Titelverbindung B jeweils als farbloses Öl.
¹H-NMR (CDCl₃) von A: δ = -0,05 (6H), 0,82 (9H), 1,02 (9H), 1,78 (1H), 1,96 (3H), 3,48 (2H), 4,92 (1H), 6,08 (1H), 6,73 (2H), 7,20-7,30 (4H), 7,32-7,40 (2H), 7,41-7,49 (4H), 8,30 (2H) ppm.
¹H-NMR (CDCl₃) von B: δ = -0,04 (6H), 0,80 (9H), 1,08 (9H), 1,78 (3H), 1,91 (1H), 3,55 (2H), 4,39 (1H), 6,02 (1H), 6,93 (2H), 7,26-7,48 (6H), 7,60-7,72 (4H), 8,50 (2H) ppm.

### Beispiel 39

### (E,3S)-3-[[(1,1-Dimethylethyl)diphenylsilyl]oxy]-4-methyl-5-(4-pyridyl)-pent-4-en-1-ol

Analog zu Beispiel 20 werden 4,34 g (7,95 mmol) der unter Beispiel 38B hergestellten Verbindung mit einem 65:35:10-Gemische aus Eisessig/Wasser/Tetrahydrofuran umgesetzt. Man erhält nach Aufreinigung 2,92 g (6,76 mmol, 85%) der Titelverbindung.
¹H-NMR (CDCl₃): δ = 1,12 (9H), 1,78 (3H), 1,87 (2H), 3,65 (2H), 4,42 (1H), 6,26 (1H), 6,97 (2H), 7,26-7,48 (6H), 7,60-7,72 (4H), 8,52 (2H) ppm.

### Beispiel 40

Analog zu Beispiel 22 werden aus 2,92 g (6,76 mmol) der unter Beispiel 39 beschriebenen Verbindung 2,82 g (77%) der Titelverbindung erhalten.
¹H-NMR (CDCl₃): δ = 1,08 (6H), 1,78 (3H), 2,15 (2H), 3,00 (2H), 4,26 (1H), 6,17 (1H), 6,95 (2H), 7,30-7,50 (6H), 7,60-7,70 (4H), 8,50 (2H) ppm.

### Beispiel 41

Analog zu Beispiel 23 werden aus 2,82 g (5,21 mmol) der unter Beispiel 40 beschriebenen Verbindung 3,27 g (4,06 mmol, 78%) der Titelverbindung erhalten.
¹H-NMR (CDCl₃): δ = 1,09 (6H), 1,82 (3H), 3,15 (1H), 3,50 (1H), 4,65 (1H), 6,53 (1H), 7,05 (2H), 7,25-7,48 (6H), 7,50-7,70 (4H), 8,50 (2H) ppm.

### Herstellung der Epothilon-Derivate der allgemeinen Formel 1:

### Beispiel 1

### (4S,7R,8S,9S,13(Z),16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion

### Beispiel 1a

### (3S)-1-Oxa-2-oxo-3-(tetrahydropyran-2(RS)-yloxy)-4,4-dimethyl-cyclopentan

Die Lösung von 74,1 g (569 mmol) D-(-)-Pantolacton in 1l wasserfreiem Dichlormethan versetzt man unter einer Atmosphäre aus trockenem Argon mit 102 ml 3,4-Dihydro-2H-pyran, 2 g p-Toluolsulfonsäure-Pyridiniumsalz und rührt 16 Stunden bei 23°C. Man gießt in eine gesättigte Natriumhydrogencarbonatlösung, trennt die organische Phase ab und trocknet über Natriumsulfat. Nach Filtration und Lösungsmittelabzug chromatographiert man den Rückstand an ca. 5 kg feinem Kieselgel mit einem Gemisch aus n-Hexan und Ethylacetat. Isoliert werden 119,6 g (558 mmol, 98%) der Titelverbindung als farbloses Öl.
¹H-NMR (CDCl₃): δ = 1,13 (3H), 1,22 (3H), 1,46-1,91 (6H), 3,50-3,61 (1H), 3,86 (1H), 3,92 (1H), 4,01 (1H), 4,16 (1H), 5,16 (1H) ppm.

### Beispiel 1b

### (2RS,3S)-1-Oxa-2-hydroxy-3-(tetrahydropyran-2(RS)-yloxy)-4,4-dimethyl-cyclopentan

Die Lösung von 117,5 g (548 mmol) der nach Beispiel 1a dargestellten Verbindung in 2,4 I wasserfreiem Toluol kühlt man unter einer Atmosphäre aus trockenem Argon auf -70°C, versetzt innerhalb 1 Stunde mit 540 ml einer 1,2 molaren Lösung von Diisobutylaluminiumhydrid in Toluol und rührt noch 3 Stunden bei -70°C. Man läßt auf - 20°C erwärmen, versetzt mit gesättigter Ammoniumchloridlösung, Wasser und trennt die ausgefallenen Aluminiumsalze durch Filtration über Celite ab. Das Filtrat wird mit Wasser und gesättigter Natriumchloridlösung gewaschen und über Magnesiumsulfat getrocknet. Isoliert werden nach Filtration und Lösungsmittelabzug 111,4 g (515 mmol, 94%) der Titelverbindung als farbloses Öl, das man ohne Reinigung weiter umsetzt.
IR (CHCl₃): 3480, 3013, 2950, 2874, 1262, 1133, 1074, 1026 und 808 cm⁻¹.

### Beispiel 1c

### (3S)-2,2-Dimethyl-3-(tetrahydropyran-2(R)-yloxy)-pent-4-en-1-ol und (3S)-2,2-Dimethyl-3-(tetrahydropyran-2(S)-yloxy)-pent-4-en-1-ol

Die Aufschlämmung von 295 g Methyl-triphenylphosphoniumbromid in 2,5 1 wasserfreiem Tetrahydrofuran versetzt man unter einer Atmosphäre aus trockenem Argon bei -60°C mit 313 ml einer 2,4 molaren Lösung von n-Butyllithium in n-Hexan, läßt auf 23°C erwärmen, eine Stunde nachrühren und kühlt auf 0°C. Man versetzt mit der Lösung von 66,2 g (306 mmol) der nach Beispiel 1b dargestellten Verbindung in 250 ml Tetrahydrofuran, läßt auf 23°C erwärmen und 18 Stunden rühren. Man gießt in eine gesättigte Natriumhydrogencarbonatlösung, extrahiert mehrfach mit Dichlormethan und trocknet die vereinigten organischen Extrakte über Natriumsulfat. Nach Filtration und Lösungsmittelabzug chromatographiert man den Rückstand an ca. 5 I feinem Kieselgel mit einem Gradientensystem aus n-Hexan und Ethylacetat. Isoliert werden 36,5 g (170 mmol, 56%) des unpolaren, 14,4 g (67,3 mmol, 22%) des polaren THP-Isomeren der Titelverbindung, sowie 7,2 g (33,3 mmol; 11%) des Ausgangsmaterials jeweils als farbloses Öl.
¹H-NMR (CDCl₃), unpolares Isomer: δ = 0,78 (3H), 0,92 (3H), 1,41-1,58 (4H), 1,63-1,87 (2H), 3,18 (1H), 3,41 (1H), 3,48 (1H), 3,68 (1H), 3,94 (1H), 4,00 (1H), 4,43 (1H), 5,19 (1H), 5,27 (1H), 5,75 (1H) ppm.
¹H-NMR (CDCl₃), polares Isomer: δ = 0,83 (3H), 0,93 (3H), 1,42-1,87 (6H), 2,76 (1H), 3,30 (1H), 3,45 (1H), 3,58 (1H), 3,83 (1H), 3,89 (1H), 4,65 (1H), 5,12-5,27 (2H), 5,92 (1H) ppm.

### Beispiel 1 d

### (3S)-1-(tert.-Butyldiphenylsilyloxy)-2,2-dimethyl-pentan-3-(tetrahydropyran-2-yloxy)-pent-4-en

Die Lösung von 59,3 g (277 mmol) des nach Beispiel 1c dargestellten THP-IsomerenGemisches in 1000 ml wasserfreiem Dimethylformamid versetzt man unter einer Atmosphäre aus trockenem Argon mit 28 g Imidazol, 85 ml tert.-Butyldiphenylchlorsilan und rührt 16 Stunden bei 23°C. Man gießt in Wasser, extrahiert mehrfach mit Dichlormethan, wäscht die vereinigten organischen Extrakte mit Wasser und trocknet über Natriumsulfat. Nach Filtration und Lösungsmittelabzug chromatographiert man den Rückstand an feinem Kieselgel mit einem Gradientensystem aus n-Hexan und Ethylacetat. Isoliert werden 106,7 g (236 mmol, 85%) der Titelverbindung als farbloses Öl.
¹H-NMR (CDCl₃): δ =0,89 (3H), 0,99 (3H), 1,08 (9H), 1,34-1,82 (6H), 3,40 (1H), 3,51 (2H), 3,76 (1H), 4,02 (1H), 4,67 (1H), 5,18 (1H), 5,23 (1H), 5,68 (1H), 7,30-7,48 (6H), 7,60-7,73 (4H) ppm.

### Beispiel 1 e

### (3S)-1-(tert.-Butyldiphenylsilyloxy)-2,2-dimethyl-3-(tetrahydropyran-2-yloxy)-pentan-5-ol

Die Lösung von 3,09 g (6,83 mmol) der nach Beispiel 1d dargestellten Verbindung in 82 ml Tetrahydrofuran versetzt man man unter einer Atmosphäre aus trockenem Argon bei 23°C mit 13,1 ml einer 1 molaren Lösung von Boran in Tetrahydrofuran und läßt 1 Stunde reagieren. Anschließend versetzt man unter Eiskühlung mit 16,4 ml einer 5%- igen Natronlauge sowie 8,2 ml einer 30%-igen Wasserstoffperoxidlösung und rührt weitere 30 Minuten. Man gießt in Wasser, extrahiert mehrfach mit Ethylacetat, wäscht die vereinigten organischen Extrakte mit Wasser, gesättigter Natriumchloridlösung und trocknet über Magnesiumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 1,78 g (3,78 mmol, 55%) der Titelverbindung als chromatographisch trennbares Gemisch der beiden THP-Epimeren sowie 0,44g (1,14 mmol, 17%) der Titelverbindung aus Beispiel 6 jeweils als farbloses Öl.
¹H-NMR (CDCl₃), unpolares THP-Isomer: δ = 0,80 (3H), 0,88 (3H), 1,10 (9H), 1,18-1,80 (9H), 3,27 (1H), 3,39 (1H), 3,48 (1H), 3,64 (1H), 3,83 (1H), 3,90-4,08 (2H), 4,49 (1H), 7,31-7,50 (6H), 7,58-7,73 (4H) ppm.
¹H-NMR (CDCl₃), polares THP-Isomer: δ = 0,89 (3H), 0,98 (3H), 1,08 (9H), 1,36-1,60 (4H), 1,62-1,79 (3H), 1,88 (1H), 2,03 (1H), 3,37 (1H), 3,50 (1H), 3,57 (1H), 3,62-3,83 (4H), 4,70 (1H), 7,30-7,48 (6H), 7,61-7,73 (4H) ppm.

### Beispiel 1f

### (35)-1-(tert.-Butyldiphenylsilyloxy)-2,2-dimethyl-3-hydroxy-pent-4-en

Die Lösung von 106,7 g (236 mmol) der nach Beispiel 1 d dargestellten Verbindung in 1,5 I wasserfreiem Ethanol versetzt man unter einer Atmosphäre aus trockenem Argon mit 5,9 g Pyridinium-p-Toluolsulfonat und erhitzt 6 Stunden auf 50°C. Nach Lösungsmittelabzug chromatographiert man den Rückstand an feinem Kieselgel mit einem Gemisch aus n-Hexan und Ethylacetat. Isoliert werden 82,6 g (224 mmol, 95%) der Titelverbindung als farbloses Öl, in dem noch zusätzlich ca. 5g Ethoxy-tetrahydropyran enthalten sind.
¹H-NMR (CDCl₃) einer analytischen Probe: δ = 0,89 (6H), 1,08 (9H), 3,45 (1H), 3,49 (1H), 3,58 (1H), 4,09 (1H), 5,21 (1H), 5,33 (1H), 5,93 (1H), 7,34-7,51 (6H), 7,63-7,73 (4H) ppm.

### Beispiel 1 g

### (35)-1-(tert.-Butyldiphenylsilyloxy)-2,2-dimethyl-pentan-3,5-diol

Die Lösung von 570 mg (1,55 mmol) der nach Beispiel 1f dargestellten Verbindung setzt man in Analogie zu Beispiel 1e um und isoliert nach Aufarbeitung und Reinigung 410 mg (1,06 mmol, 68%) der Titelverbindung als farbloses Öl.
¹H-NMR (CDCl₃): δ = 0,82 (3H), 0,93 (3H), 1,08 (9H), 1,56-1,79 (2H), 3,11 (1H), 3,50 (2H), 3,78-3,92 (3H), 4,02 (1H), 7,34-7,51 (6H), 7,61-7,71 (4H) ppm.

### Beispiel 1 h

### 4(S)-[2-Methyl-1-(tert.-butyldiphenylsilyloxy)-prop-2-yl]-2,2-dimethyl-[1,3]dioxan

Die Lösung von 100 mg (0,212 mmol) der nach Beispiel 1e dargestellten Verbindungen in 2,6 ml wasserfreiem Aceton versetzt man unter einer Atmosphäre aus trockenem Argon mit 78,9 mg Kupfer(II)sulfat, einer Spatelspitze p-Toluolsulfonsäure-Monohydrat und rührt 16 Stunden bei 23°C. Man versetzt mit gesättigter Natriumhydrogencarbonatlösung, extrahiert mehrfach mit Diethylether, wäscht mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 24 mg (56 µmol, 27%) der Titelverbindung als farbloses Öl.
¹H-NMR (CDCl₃): δ = 0,83 (3H), 0,89 (3H), 1,07 (9H), 1,30 (1H), 1,36 (3H), 1,44 (3H), 1,71 (1H), 3,24 (1H), 3,62 (1H), 3,86 (1H), 3,91-4,03 (2H), 7,31-7,48 (6H), 7,61-7,74 (4H) ppm.

### Variante II

320 mg (0,88 mmol) der nach Beispiel 1g dargestellten Verbindung setzt man in Analogie zu Beispiel 1h;Variante I um und isoliert nach Aufarbeitung und Reinigung 234 mg (0,548 mmol, 62%) der Titelverbindung.

### Variante III

Die Lösung von 5,60 g (14,5 mmol) der nach Beispiel 1g dargestellten Verbindung in 250 ml wasserfreiem Dichlormethan versetzt man unter einer Atmosphäre aus trockenem Argon mit 10 ml 2,2-Dimethoxypropan, 145 mg Campher-10-sulfonsäure und rührt 6 Stunden bei 23°C. Man versetzt mit Triethylamin, verdünnt mit Ethylacetat, wäscht mit gesättigter Natriumhydrogencarbonatlösung und trocknet über Natriumsulfat. Nach Filtration und Lösungsmittelabzug chromatographiert man den Rückstand an feinem Kieselgel mit einem Gemisch aus n-Hexan und Ethylacetat. Isoliert werden 5,52 g (12,9 mmol, 89%) der Titelverbindung als farbloses Öl.

### Beispiel 1i

### (4S)-4-(2-Methyl-1-hydroxy-prop-2-yl)-2,2-dimethyl-[1,3]dioxan

Die Lösung von 5,6 g (13,1 mmol) der nach Beispiel 1 h dargestellten Verbindung in 75 ml wasserfreiem Tetrahydrofuran versetzt man unter einer Atmosphäre aus trockenem Argon mit 39 ml einer 1 molaren Lösung von Tetrabutylammoniumfluorid in Tetrahydrofuran und erwärmt 16 Stunden auf 50°C. Man versetzt mit gesättigter Natriumhydrogencarbonatlösung, extrahiert mehrfach mit Ethylacetat, wäscht mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 2,43 g (12,9 mmol, 99%) der Titelverbindung als farbloses Öl.
¹H-NMR (CDCl₃): δ = 0,87 (3H), 0,90 (3H), 1,35 (1H), 1,37 (3H), 1,43 (3H), 1,77 (1H), 2,93 (1H), 3,36 (1H), 3,53 (1H), 3,79 (1H), 3,87 (1H), 3,96 (1H) ppm.

### Beispiel 1 k

### (4S)-4-(2-Methy)-1-oxo-prop-2-yl)-2,2-dimethyl-[1,3]dioxan

Die Lösung von 0,13 ml Oxalylchlorid in 5,7 ml wasserfreiem Dichlormethan kühlt man unter einer Atmosphäre aus trockenem Argon auf -70°C, versetzt mit 0,21 ml Dimethylsulfoxid, der Lösung von 200 mg (1,06 mmol) der nach Beispiel 1i dargestellten Verbindung in 5,7 ml wasserfreiem Dichlormethan und rührt 0,5 Stunden. Anschließend versetzt man mit 0,65 ml Triethylamin, läßt 1 Stunde bei -30°C reagieren und versetzt mit n-Hexan und gesättigter Natriumhydrogencarbonatlösung. Die organische Phase wird abgetrennt, die wässrige noch mehrfach mit n-Hexan extrahiert, die vereinigten organischen Extrakte mit Wasser gewaschen und über Magnesiumsulfat getrocknet. Den nach Filtration und Lösungsmittelabzug erhaltenen Rückstand setzt man ohne Reinigung weiter um.

### Beispiel 1l

### (4S)-4-((3RS)-2-Methyl-3-hydroxy-hex-2-yl)-2,2-dimethyl-[1,3]dioxan

Die Lösung von 450 mg (2,42 mmol) der nach Beispiel 1 k dargestellten Verbindung in 7 ml wasserfreiem Diethylether versetzt man unter einer Atmosphäre aus trockenem Argon bei 0°C mit 1,21 ml einer 2,4 molaren Lösung von Propylmagnesiumbromid in Diethylether, läßt auf 23°C erwärmen und 16 Stunden rühren. Man versetzt mit gesättigter Ammoniumchloridlösung, trennt die organische Phase ab und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 244 mg (1,06 mmol, 44%) der chromatographisch trennbaren 3R- und 3S- Epimeren der Titelverbindung sowie 191 mg der in Beispiel 1i beschriebenen Titelverbindung jeweils als farbloses Öl.
¹H-NMR (CDCl₃) unpolares Isomer: δ = 0,87 (3H), 0,89 (3H), 0,94 (3H), 1,25-1,52 (4H), 1,38 (3H), 1,45 (3H), 1,66 (1H), 1,85 (1H), 3,46 (1H), 3,80-4,02 (4H) ppm.
¹H-NMR (CDCl₃) polares Isomer: δ = 0,73 (3H), 0,92 (3H), 0,95 (3H), 1,19-1,84 (6H), 1,37 (3H), 1,49 (3H), 3,49 (1H), 3,60 (1H), 3,80-4,03 (3H) ppm.

### Beispiel 1 m

### (4S)-4-(2-Methyl-3-oxo-hex-2-yl)-2,2-dimethyl-[1,3]dioxan

Die Lösung von 207 mg (0,90 mmol) eines Gemisches der nach Beispiel 11 dargestellten Verbindungen in 18 ml wasserfreiem Dichlormethan versetzt man mit Molekularsieb (4A, ca. 20 Kugeln), 176 mg N-Methylmorpholino-N-oxid, 18 mg Tetrapropylammoniumperruthenat und rührt 16 Stunden bei 23°C unter einer Atmosphäre aus trockenem Argon. Man engt ein und reinigt das erhaltene Rohprodukt durch Chromatographie an ca. 100 ml feinem Kieselgel mit einem Gradientensystem aus n-Hexan und Ethylacetat. Isoliert werden 185 mg (0,81 mmol, 90%) der Titelverbindung als farbloses Öl.
¹H-NMR (CDCl₃): δ = 0,88 (3H), 1,04 (3H), 1,12 (3H), 1,22-1,37 (1H), 1,31 (3H), 1,40 (3H), 1,48-1,71 (3H), 2,46 (2H), 3,83 (1H), 3,96 (1H), 4,04 (1H) ppm.

### Beispiel 1 n

### 4-Tert.-butyldimethylsilyloxy-but-2-in-1-ol

Zu einer Lösung von 100 g 2-Butin-1-ol und 158 g Imidazol in 300 ml Dimethylformamid tropft man bei 0°C unter Stickstoff langsam eine Lösung von 175 g tert.-Butyldimethylsilylchlorid in 100 ml eines 1:1 Gemisches von Hexan und Dimethylformamid und rührt 2 Stunden bei 0°C und 16 Stunden bei 22°C. Man verdünnt die Reaktionsmischung mit 2.5 I Ether, wäscht einmal mit Wasser, einmal mit 5%iger Schwefelsäure, einmal mit Wasser, einmal mit gesättigter Natriumhydrogencarbonat- Lösung und mit halbgesättigter Natriumchlorid-Lösung neutral. Nach Trocknung über Natriumsulfat und Filtration wird im Vakuum eingeengt. Den so erhaltenen Rückstand reinigt man durch Chromatographie an Kieselgel. Mit Hexan / 0-40% Ether erhält man 74.3 g der Titelverbindung als farbloses Öl.
IR (Film): 3357, 2929, 2858, 1472, 1362, 1255, 1132, 1083, 1015, 837, 778 cm⁻¹.

### Beispiel 1o

### (4R,5S,2'S)-4-Methyl-5-phenyl-3-[1-oxo-2-methyl-6-(tert.-butyldimethylsilyloxy)-hex-4-in-1-yl]-2-oxazolidinon

Zu 21 g einer Lösung des nach Beispiel 1 n hergestellten Silylethers in 125 ml Toluol gibt man unter Stickstoff 11.3 ml Lutidin. Anschließend kühlt man auf -40°C und tropft bei dieser Temperatur 17.7 ml Trifluormethansulfonsäureanhydrid zu. Dann verdünnt man mit 100 ml Hexan und rührt 10 Minuten. Diese Lösung wird unter Stickstoff über eine Umkehrfritte zu einer Lösung gegeben, die aus 17.8 g Hexamethyldisilazan in 140 ml Tetrahydrofuran mit 73.5 ml einer 1.6 M Lösung von Butyllithium in Hexan bei -60°C (10 Minuten Nachrührzeit) und 23.3 g (4R,5S)-4-Methyl-5-phenyl-3-propionyl-2-oxazolidinon in 62 ml Tetrahydrofuran (30 Minuten Nachrührzeit) hergestellt wurde. Man läßt 1 Stunde bei -60°C Nachrühren, versetzt dann mit 6 ml Essigsäure in 5 ml Tetrahydrofuran und läßt die Reaktionsmischung auf 22°C erwärmen. Man gibt auf 80 ml Wasser und extrahiert dreimal mit Ether. Die vereinigten organischen Phasen werden zweimal mit gesättigter Natriumchlorid-Lösung gewaschen und über Natriumsulfat getrocknet. Nach Filtration wird im Vakuum eingeengt. Den so erhaltenen Rückstand reinigt man durch Chromatographie an Kieselgel. Mit Hexan / 0-20% Ether erhält man 16.0 g der Titelverbindung als farbloses Öl.
¹H-NMR (CDCl₃): δ= 0.10 (6H), 0.90 (9H), 0.92 (3H), 1.28 (3H), 2.47 (1H), 2.61 (1H), 3.96 (1H), 4.26 (2H), 4.78 (1H), 5.68 (1H), 7.31 (1H), 7.3-7.5 (3H) ppm.

### Beispiel 1p

### (2S)-2-Methyl-6-(tert.-butyldimethylsilyloxy)-4-hexinsäureethylester

Zu einer Lösung von 39.3 g des nach Beispiel 1o hergestellten Alkylierungsproduktes in 120 ml Ethanol gibt man unter Stickstoff 9.0 ml Titan(IV)ethylat und erhitzt unter Rückfluß für 4 Stunden. Die Reaktionsmischung wird im Vakuum eingeengt und der Rückstand in 100 ml Essigester gelöst. Man gibt 3 ml Wasser hinzu, rührt für 20 Minuten, saugt vom Niederschlag ab und wäscht gut mit Essigester nach. Das Filtrat wird eingeengt, mit 200 ml Hexan versetzt und vom Niederschlag abfiltriert. Der Niederschlag wird gut mit Hexan gewaschen. Das Filtrat wird im Vakuum eingeengt und der so erhaltene Rückstand durch Chromatographie an Kieselgel gereinigt. Mit Hexan / 0-20% Ether erhält man 25.4 g der Titelverbindung als farbloses Öl.
¹H-NMR (CD₂Cl₂): δ= 0.10 (3H), 0.90 (9H), 1.2-1.3 (6H), 2.37 (1H), 2.54 (1H), 2.60 (1H), 4.12 (2H), 4.27 (2H) ppm.

### Beispiel 1 q

### (2S)-2-Methyl-6-(tert.-butyldimethylsilyloxy)-hexansäureethylester

Eine Lösung von 10.5 g des nach Beispiel 1p hergestellten Esters in 200 ml Essigester versetzt man mit 1 g 10% Palladium auf Kohle und rührt 3 Stunden bei 22°C in einer Wasserstoffatmosphäre. Anschließend filtriert man vom Katalysator ab, wäscht gut mit Essigester nach und engt das Filtrat im Vakuum ein. Den so erhaltenen Rückstand reinigt man durch Chromatographie an Kieselgel. Mit Hexan / 0-10% Ether erhält man 9.95 g der Titelverbindung als farbloses Öl.
¹H-NMR (CD₂Cl₂): δ= 0.01 (6H), 0.84 (9H), 1.07 (3H), 1.18 (3H), 1.2 -1.7 (6H), 2.38 (1H), 3.57 (2H), 4.05 (2H) ppm.

### Beispiel 1 r

### (2S)-2-Methyl-6-(tert.-butyldimethylsilyloxy)-hexan-1-ol

Zu einer Lösung aus 9.94 g des nach Beispiel 1q hergestellten Esters in 130 ml Toluol gibt man bei -40°C unter Stickstoff 63 ml einer 1.2 M Lösung von Diisobutylaluminiumhydrid in Toluol und rührt 1 Stunde bei dieser Temperatur. Anschließend gibt man vorsichtig 15 ml Isopropanol und nach 10 Minuten 30 ml Wasser hinzu, läßt auf 22°C kommen und rührt bei dieser Temperatur 2 Stunden. Man filtriert vom Niederschlag ab, wäscht gut mit Essigester nach und engt das Filtrat im Vakuum ein. Der so erhaltene Rückstand wird durch Chromatographie an Kieselgel gereinigt. Mit Hexan / 0-30% Ether erhält man 7.9 g der Titelverbindung als farbloses Öl. [a]_{D} -8.1 ° (c = 0.97, CHCl₃)
¹H-NMR (CDCl₃): δ= 0.07 (3H), 0.89 (9H), 0.91 (3H), 1.0-1.7 (7H), 3.48 (2H), 3.52 (2H) ppm.

### Beispiel 1 s

### (2S)-2-Methyl-6-(tert.-butyldimethylsilyloxy)-1-(tetrahydro-2H-pyran-2-yloxy)-hexan

Zu 6.4 g des nach Beispiel 1r hergestellten Alkohols in 26 ml Methylenchlorid gibt man bei 0°C unter Argon 3.52 ml Dihydropyran gefolgt von 49 mg p-Toluolsulfonsäure-Monohydrat. Nach 1.5 Stunden Rühren bei 0°C wird mit 10 ml gesättigte Natriumhydrogencarbonat-Lösung versetzt und mit Ether verdünnt. Die organische Phase wird zweimal mit halbgesättigter Natriumchlorid-Lösung gewaschen und über Natriumsulfat getrocknet. Nach Filtration wird im Vakuum eingeengt und der so erhaltene Rückstand durch Chromatographie an Kieselgel gereinigt. Mit Hexan / 0-5% Ether erhält man 4.75 g der Titelverbindung als farbloses Öl.
¹ H-NMR (CDCl₃): δ= 0.05 (6H), 0.89 (9H), 0.92 (3H), 1.0-1.9 (13H), 3.19 (1H), 3.50 (1H), 3.55-3.65 (3H), 4.87 (1H), 4.57 (1H) ppm.

### Beispiel 1t

### (5S)-5-Methyl-6-(tetrahydro-2H-pyran-2-yloxy)-hexan-1-ol

Zu einer Lösung von 4.7 g des nach Beispiel 1s hergestellten THP-Ethers in 170 ml Tetrahydrofuran gibt man unter Stickstoff 13.5 g Tetrabutylammoniumfluorid-Trihydrat und rührt 3 Stunden. Anschließend verdünnt man die Reaktionsmischung mit 800 ml Ether und wäscht dreimal mit je 20 ml halbgesättigter Natriumchlorid-Lösung und trocknet über Natriumsulfat. Nach Filtration wird im Vakuum eingeengt und der so erhaltene Rückstand durch Chromatographie an Kieselgel gereinigt. Mit Hexan / 0-50% Essigester erhält man 2.88 g der Titelverbindung als farbloses Öl.
¹H-NMR (CD₂Cl₂): δ= 0.90 / 0.92 (3H), 1.1-1.9 (13H), 3.18 (1H), 3.40-3.65 (4H), 3.82 (1H), 4.53 (1H) ppm.

### Beispiel 1 u

### (5S)-5-Methyl-6-(tetrahydro-2H-pyran-2-yloxy)-hexanal

Zu 1.08 ml Oxalylchlorid gelöst in 10 ml Methylenchlorid tropft man unter Stickstoff vorsichtig bei -70°C 1.9 ml Dimethylsulfoxid gelöst in 7 ml Methylenchlorid und rührt 10 Minuten bei dieser Temperatur. Anschließend tropft man eine Lösung von 2.0 g des nach Beispiel 1t hergestellten Alkohols in 7 ml Methylenchlorid zu und rührt 2 Stunden zwischen -60°C und -70°C. Dann gibt man 3.86 ml Triethylamin zu und nach 1 Stunde Rühren bei -60°C wird die Reaktionsmischung auf 30 ml Wasser gegeben. Nach Phasentrennung wird die wäßrige Pase zweimal mit je 30 ml Methylenchlorid extrahiert. Die vereinigten organischen Phasen werden dreimal mit gesättigter Natriumchlorid- Lösung gewaschen. Nach dem Trocknen über Natriumsulfat und Filtration wird im Vakuum eingeengt. Man erhält 1.99 g des Aldehyds, der ohne weitere Reinigung verwendet wird.

### Beispiel 1v

### (2RS,6S)-6-Methyl-7-(tetrahydro-2H-pyran-2-yloxy)-heptan-2-ol

Zu einer Lösung von 1.98 g des nach Beispiel 1 u hergestellten Aldehyds in 30 ml Ether tropft man unter Stickstoff bei 0°C langsam 6.16 ml einer 3M Methylmagnesiumbromid-Lösung in Ether. Nach 60 Minuten gießt man langsam auf 50 ml eiskalte gesättigte Ammoniumchlorid-Lösung und extrahiert dreimal mit Ether. Die vereinigten organischen Phasen werden einmal mit Wasser zweimal mit gesättigter Natriumchlorid-Lösung gewaschen und über Natriumsulfat getrocknet. Nach Filtration wird im Vakuum eingeengt und der so erhaltene Rückstand durch Chromatographie an Kieselgel gereinigt. Mit Hexan /0-60% Ether erhält man 1.57 g der Titelverbindung als farbloses Öl.
¹H-NMR (CD₂Cl₂): δ= 0.90 / 0.93 (3H), 1.15 (3H), 1.0-1.9 (13H), 3.18 (1H), 3.4-3.6 (2H), 3.7-3.9 (2H), 4.53 (1H) ppm.

### Beispiel 1 w

### (2S,6RS)-2-Methyl-6-(tert.-butyl-diphenylsilyloxy)-1-(tetrahydro-2H-pyran-2-yloxy)-heptan

Zu einer Lösung von 1.57 g des nach Beispiel 1v hergestellten Alkohols und 1.11 g Imidazol in 20 ml Dimethylformamid gibt man bei 0°C unter Stickstoff 2.13 ml tert.-Butyldiphenylsilylchlorid, rührt 15 Minuten bei 0°C und 16 Stunden bei 22°C. Man verdünnt die Reaktionsmischung mit 200 ml Ether, wäscht einmal mit Wasser, einmal mit 10%iger Schwefelsäure, einmal mit gesättigter Natriumhydrogencarbonat-Lösung und mit gesättigter Natriumchlorid-Lösung neutral. Nach Trocknung über Natriumsulfat und Filtration wird im Vakuum eingeengt. Den so erhaltenen Rückstand reinigt man durch Chromatographie an Kieselgel. Mit Hexan / 0-10% Ether erhält man 2.87 g der Titelverbindung als farbloses Öl.
¹H-NMR (CDCl₃): δ= 0.87 / 0.89 (3H), 1.04 (9H), 0.9-1.9 (16H), 3.15 (1H), 3.4-3.6 (2H), 3.8-3.9 (2H), 4.56 (1H), 7.3-7.5 (6H), 7.69 (4H) ppm.

### Beispiel 1 x

### (2S,6RS)-2-Methyl-6-(tert.-butyl-diphenylsilyloxy)-heptan-1-ol

Zu einer Lösung von 2.3 g des nach Beispiel 1w hergestellten Silylethers in 100 ml Ethanol gibt 131 mg Pyridinium-p-toluolsulfonat und rührt 4 Stunden bei 40°C. Anschließend wird im Vakuum eingeengt und der so erhaltene Rückstand durch Chromatographie an Kieselgel gereinigt. Mit Hexan / 20% Ether erhält man 1.68 g der Titelverbindung als farbloses Öl.

### Beispiel 1y

### (2S,6RS)-2-Methyl-6-(tert.-butyl-diphenylsilyloxy)-heptanal

2,13 g des unter Beispiel 1x dargestellten Alkohols oxidiert man in Analogie zu Beispiel 1u und isoliert nach Aufarbeitung und chromatographischer Reinigung 2,10 g der Titelverbindung als farbloses Öl.
¹H-NMR (CDCl₃): δ= 1,00-1,12 (15H), 1,18-1,63 (6H), 2,22 (1H), 3,83 (1H), 7,32-7,47 (6H), 7,61-7,72 (4H), 9,54 (1H) ppm.

### Beispiel 1 z

### (S)-Dihydro-3-hydroxy-2(3H)-furanon

10 g L-(-)-Äpfelsäure werden in 45 ml Trifluoressigsäureanhydrid 2 Stunden bei 25°C gerührt. Danach engt man im Vakuum ein, addiert zu dem Rückstand 7 ml Methanol und läßt 12 Stunden nachrühren. Anschließend wird im Vakuum eingeengt. Der erhaltene Rückstand wird in 150 ml absolutem Tetrahydrofuran gelöst. Man kühlt auf 0°C und addiert 150 ml Boran-Tetrahydrofuran-Komplex und läßt 2,5 Stunden bei 0°C nachrühren. Danach werden 150 ml Methanol addiert. Man läßt eine Stunde bei Raumtemperatur nachrühren und engt dann im Vakuum ein. Das erhaltene Rohprodukt wird in 80 ml Toluol gelöst. Man addiert 5 g Dowex^{###} (aktiviert, sauer)und kocht eine Stunde unter Rückfluß. Anschließend wird das Dowex^{###} abfiltriert und das Filtrat im Vakuum eingeengt. Das erhaltene Rohprodukt (7,61 g) wird ohne Aufreinigung in die Folgestufe eingesetzt.

### Beispiel 1aa

### (S)-Dihydro-3-[[(1,1-dimethylethyl)diphenylsilyl]oxy]2(3H)-furanon

Zu einer Lösung von 7,61 g der unter Beispiel 1z beschriebenen Substanz und 10 g Imidazol in 100 ml *N,N*-Dimethylformamid werden 24 ml *tert.*Butyldiphenylsilylchlorid addiert. Man läßt zwei Stunden bei 25°C nachrühren und gießt dann das Reaktionsgemisch auf eiskalte gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 13,4 g der Titelverbindung erhalten.
¹H-NMR (CDCl₃): δ = 7,72 (2H), 7,70 (2H), 7,40-7,50 (6H), 4,30-4,42 (2H), 4,01 (1H), 2,10-2,30 (2H), 1,11 (9H) ppm.

### Beispiel 1 ab

### (2RS,3S)-3-[[(1,1-Dimethylethyl)diphenylsilyl]oxy]tetrahydro-2-furanol

Zu einer Lösung von 13,4 g der unter Beispiel 1aa beschriebenen Substanz in 150 ml absolutem Tetrahydrofuran werden 80 ml einer 1 molaren Lösung von Diisobutylaluminiumhydrid in Hexan bei -78°C addiert. Man rührt 45 Minuten bei -78°C nach und quencht dann mit Wasser. Man extrahiert mit Ethylacetat, wäscht die organische Phase mit gesättigter Natriumchloridlösung, trocknet über Natriumsulfat und engt im Vakuum ein. Man erhält 13,46 g der Titelverbindung, welche ohne Reinigung in die Folgestufe eingesetzt wird.

### Beispiel 1 ac

### (2RS,3S)-3-[[(1,1-Dimethylethyl)diphenylsilyl]oxy]-1,4-pentandiol

Zu 20 ml einer 3 molaren Lösung von Methylmagnesiumchlorid in Tetrahydrofuran wird bei 0°C eine Lösung von 13,46 g der unter Beispiel 1ab beschriebenen Substanz in 150 ml absolutem Tetrahydrofuran getropft. Man läßt eine Stunde bei 0°C nachrühren und gießt dann auf gesättigte wäßrige Ammoniumchloridlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 11,42 g der Titelverbindung erhalten.
¹H-NMR (CDCl₃) : δ = 7,65-7,75 (4H), 7,40-7,55 (6H), 5,20 (1H), 4,30 (2H), 3,70 (1H), 1,80 (2H), 1,05 (9H) ppm.

### Beispiel 1 ad

### (2RS,3S)-5-[[Dimethyl(1,1-dimethylethyl)silyl]oxy]-3-[[(1,1-dimethylethyl)diphenylsilyl]oxy]-2-pentanol

Zu einer Lösung von 11,42 g der unter Beispiel 1ac beschriebenen Substanz und 3,25 g 1*H*-Imidazol in 120 ml N,N-Dimethylformamid werden 4,9 g *tert*-Butyldimethylsilylchlorid addiert. Man läßt 2 Stunden bei 25°C nachrühren und gießt dann das Reaktionsgemisch auf eiskalte gesättigte Natriumhydrogencarbonatlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 10,64 g der Titelverbindung erhalten.
¹H-NMR (CDCl₃): δ = 7,60-7,70 (4H), 7,30-7,45 (6H), 3,70-3,80 (2H), 3,40 (1H), 3,00 (1H), 1,80 (1H), 1,60 (1H), 1,05-1,12 (12H), 0,82 (9H), 0,02 (6H) ppm.

### Beispiel 1 ae

### (3S)-5-[[Dimethyl(1,1-dimethylethyl)silyl]oxy]-3-[[(1,1-dimethylethyl)diphenylsilyl]oxy]-2-pentanon

Zu 7,37 ml Oxalylchlorid in 80 ml Dichlormethan werden bei -78°C 13 ml Dimethylsulfoxid addiert. Man läßt 3 Minuten nachrühren und addiert dann 10,46 g der unter Beispiel 1 ad beschriebenen Substanz in 100 ml Dichlormethan. Nach weiteren 15 Minuten Nachrührzeit werden 52 ml Triethylamin hinzugetropft. Anschließend läßt man auf 0°C erwärmen. Danach wird das Reaktionsgemisch auf gesättigte Natriumhydrogencarbonatlösung gegossen. Man extrahiert mit Dichlormethan, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat werden 9,3 g der Titelverbindung erhalten.
¹H-NMR (CDCl₃): δ = 7,60-7,70 (4H), 7,32-7,50 (6H), 4,25 (1H), 3,72 (1H), 3,58 (1H), 2,05 (3H), 1,90 (1H), 1,75 (1H), 1,13 (9H), 0,89 (9H), 0,01 (6H) ppm.

### Beispiel 1 af

### (E,3S)-1-[[Dimethyl(1,1-dimethylethyl)silyl]oxy]-3-[[(1,1-dimethylethyl)diphenylsilyl]oxy]-4-methyl-5-(2-methylthiazol-4-yl)-pent-4-en

Die Lösung von 6,82g Diethyl(2-methylthiazol-4-yl)methanphosphonat in 300 ml wasserfreiem Tetrahydrofuran kühlt man unter einer Atmosphäre aus trockenem Argon auf -5°C, versetzt mit 16,2 ml einer 1,6 molaren Lösung von n-Buthyllithium in n-Hexan, läßt auf 23°C erwärmen und 2 Stunden rühren. Anschließend kühlt man auf -78°C, tropft die Lösung von 6,44 g (13,68 mmol) der nach Beispiel 1ae dargestellten Verbindung in 150 ml Tetrahydrofuran zu, läßt auf 23°C erwärmen und 16 Stunden rühren. Man gießt in gesättigte Ammoniumchloridlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 6,46 g (11,4 mmol, 83%) der Titelverbindung als farbloses Öl.
¹H-NMR (CDCl₃): δ = -0,04 (6H), 0,83 (9H), 1,10 (9H), 1,79 (1H), 1,90 (1H), 1,97 (3H), 2,51 (3H), 3,51 (2H), 4,38 (1H), 6,22 (1H), 6,74 (1H), 7,23-7,47 (6H), 7,63 (2H), 7,70 (2H) ppm.

### Beispiel 1ag

### (E,3S)-3-[[(1,1-dimethylethyl)diphenylsilyl]oxy]-4-methyl-5-(2-methylthiazol-4-yl)-pent-4-en-1-ol

Die Lösung von 4,79 g (8,46 mmol) der nach Beispiel 1af dargestellten Verbindung in 48 ml Tetrahydrofuran versetzt man mit 48 ml eines 65:35:10-Gemisches aus Eisessig/Wasser/Tetrahydrofuran und rührt 2,5 Tage bei 23°C. Man gießt in gesättigte Natriumcarbonatlösung, extrahiert mehrfach mit Ethylacetat, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an feinem Kieselgel mit einem Gradientensystem aus n-Hexan und Ethylacetat. Isoliert werden 3,42 g (7,57 mmol, 90%) der Titelverbindung als farbloses Öl.
¹ H-NMR (CDCl₃): δ = 1,10 (9H), 1,53 (1H), 1,81 (2H), 1,96 (3H), 2,71 (3H), 3,59 (2H), 4,41 (1H), 6,38 (1H), 6,78 (1H), 7,26-7,49 (6H), 7,65 (2H), 7,72 (2H) ppm.

### Beispiel 1 ah

### (E,3S)-1 -lod-3-[[(1,1 -dimethylethyl)diphenylsilyl]oxy]-4-methyl-5-(2-methylthiazol-4-yl)-pent-4-en

Die Lösung von 8,41 g Triphenylphosphin in 120 ml Dichlormethan versetzt man bei 23°C unter einer Atmosphäre aus trockenem Argon mit 2,19 g Imidazol, 8,14 g lod, tropft die Lösung von 12,2 g (27,0 mmol) der nach Beispiel 1ag dargestellten Verbindung in 30 ml Dichlormethan zu und rührt 0,5 Stunden. Die Lösung chromatographiert man an feinem Kieselgel mit einem Gradientensystem aus n-Hexan und Ethylacetat. Isoliert werden 12,15 g (21,6 mmol, 80%) der Titelverbindung als farbloses Öl.
¹H-NMR (CDCl₃): δ = 1,08 (9H), 1,96 (3H), 2,10 (2H), 2,70 (3H), 2,87-3,08 (2H), 4,24 (1H), 6,32 (1H), 6,79 (1H), 7,28-7,48 (6H), 7,60-7,72 (4H) ppm.

### Beispiel 1 ai

### (5E,3S)-[3-[[(1,1-Dimethylethyl)diphenylsilyl]oxy]-4-methyl-5-(2-methylthiazol-4-yl)-pent-4-en-1-yl]-triphenylphosphoniumiodid

Die Suspension aus 12,55 g (22,3 mmol) der nach Beispiel 1ah dargestellten Verbindung, 85 g Triphenylphosphin und 11,6 ml N-Ethyldiisopropylamin rührt man unter einer Atmosphäre aus trockenem Argon 16 Stunden bei 80°C. Nach dem Erkalten versetzt man mit Diethylether, filtriert und wäscht den Rückstand mehrfach mit Diethylether nach und kristallisiert aus Ethylacetat um. Isoliert werden 15,7 g (19,1 mmol, 74%) der Titelverbindung als kristalliner Feststoff.
¹H-NMR (CDCl₃): δ = 1,07 (9H), 1,68-1,92 (2H), 1,98 (3H), 2,70 (3H), 2,93 (1H), 3,30 (1H), 4,53 (1H), 6,62 (1H), 7,03 (1H), 7,23-7,47 (6H), 7,48-7,72 (16H), 7,73-7,85 (3H) ppm.

### Beispiel 1 ak

### (4S(4R,5S,6S,10RS))-4-(2,6-Dimethyl-10-[[(1,1-dimethylethyl)diphenylsilyl]oxy]-4-ethyl-5-hydroxy-3-oxo-undec-2-yl)-2,2-dimethyl-[1 ,3]dioxan (A) und (4S(4S,5R,6S,10RS))-4-(2,6-Dimethyl-10-[[(1,1-dimethylethyl)diphenylsilyl]oxy]-4-ethyl-5-hydroxy-3-oxo-undec-2-yl)-2,2-dimethyl-[1,3]dioxan (B)

Die Lösung von 1,96 ml Diisopropylamin in 44 ml wasserfreiem Tetrahydrofuran kühlt man unter einer Atmosphäre aus trockenem Argon auf -30°C, versetzt mit 6,28 ml einer 2,4 molaren Lösung von n-Butyllithium in n-Hexan und rührt noch 15 Minuten. Bei -78°C tropft man die Lösung von 3,08 g (13,47 mmol) der nach Beispiel 1 m dargestellten Verbindung in 44 ml Tetrahydrofuran zu und läßt 1 Stunde reagieren. Anschließend versetzt man mit der Lösung von 5,77 g (15,1 mmol) der nach Beispiel 1y dargestellten Verbindung in 44 ml Tetrahydrofuran und gießt nach 45 Minuten in gesättigte Ammoniumchloridlösung. Man verdünnt mit Wasser, extrahiert mehrfach mit Ethylacetat, wäscht die vereinigten organischen Extrakte mit gesättigter Natriumchloridlösung, trocknet über Natriumsulfat und engt im Vakuum ein. Nach Säulenchromatographie an Kieselgel mit einem Gradientensystem aus n-Hexan und Ethylacetat werden neben 13% Ausgangsmaterial 4,03 g (5,92 mmol, 44%) der Titelverbindung A sowie 1,58 g (2,32 mmol, 17%) eines Diastereomeren B erhalten.
¹H-NMR (CDCl₃) von A: δ = 0,79 (3H), 0,85 (3H), 0,90-1,10 (16H), 1,19-1,79 (10H), 1,26 (3H), 1,32 (3H), 1,38 (3H), 2,79 (1H), 3,18 (1H), 3,42 (1H), 3,78-3,92 (2H), 3,98 (1H), 4,17 (1H), 7,30-7,46 (6H), 7,62-7,72 (4H) ppm.
¹H-NMR (CDCl₃) von B: δ = 0,83 (3H), 0,91 (3H), 0,94-1,12 (16H), 1,19 (3H), 1,15-1,80 (10H), 1,31 (3H), 1,41 (3H), 2,54 (1H), 3,18 (1H), 3,47 (1H), 3,78-3,91 (2H), 3,97 (1H), 4,14 (1H), 7,31-7,47 (6H), 7,62-7,73 (4H) ppm.

### Beispiel 1 al

### (4S(4R,5S,6S,10RS))-4-(2,6-Dimethyl-10-[[(1,1-dimethylethyl)diphenylsilyl]oxy]-4-ethyl-3-oxo-5-(tetrahydropyran-2-yloxy)-undec-2-yl)-2,2-dimethyl-[1,3]dioxan

Die Lösung von 4,02 g (6,58 mmol) der nach Beispiel 1 ak dargestellten Verbindung setzt man in Analogie zu Beispiel 1a um und isoliert nach Aufarbeitung und Reinigung 4,26 g (6,13 mmol, 93%) der Titelverbindung als farbloses Öl.
¹H-NMR (CDCl₃): δ = 0,67-1,97 (47H), 3,02+3,12 (1H), 3,38 (1H), 3,48-4,04 (5H), 4,18+4,26 (1H), 4,42+4,50 (1H), 7,30-7,46 (6H), 7,61-7,72 (4H) ppm.

### Beispiel 1 am

### (4S(4R,5S,6S,10RS))-4-(2,6-Dimethyl-4-ethyl-10-hydroxy-3-oxo-5-(tetrahydropyran-2-yloxy)-undec-2-yl)-2,2-dimethyl-[1,3]dioxan

Die Lösung von 4,26 g (6,13 mmol) der nach Beispiel 1al dargestellten Verbindung setzt man in Analogie zu Beispiel 1i um und isoliert nach Aufarbeitung und Reinigung 2,38 g (5,21 mmol, 85%) der Titelverbindung als farbloses Öl.
¹H-NMR (CDCl₃): δ = 0,78+0,84 (3H), 0,92-1,10 (6H), 1,13-1,98 (29H), 2,43 (1H), 3,06+3,18 (1H), 3,42 (1H), 3,60-4,04 (5H), 4,21+4,28 (1H), 4,42+4,54 (1H) ppm.

### Beispiel 1 an

### (4S(4R,5S,6S))-4-(3,10-Dioxo-2,6-dimethyl-4-ethyl-5-(tetrahydropyran-2-yloxy)-undec-2-yl)-2,2-dimethyl-[1,3]dioxan

Die Lösung von 2,49 g (5,45 mmol) der nach Beispiel 1 am dargestellten Verbindung setzt man in Analogie zu Beispiel 1m um und isoliert nach Aufarbeitung und Reinigung 2,24 g (4,93 mmol, 90%) der Titelverbindung als farbloses Öl.
¹H-NMR (CDCl₃): δ = 0,78+0,86 (3H), 0,90-1,37 (15H), 1,37-1,95 (15H), 2,13 (3H), 2,42 (2H), 3,07+3,18 (1H), 3,42 (1H), 3,60-4,04 (4H), 4,22+4,27 (1H), 4,41+4,53 (1H) ppm.

### Beispiel 1 ao

### (4S(4R,5S,6S,10E/Z,13S,14E))-4-(13-[[(1,1-Dimethylethyl)dimethylsilyl]oxy]-4-ethyl-15-(2-methyl-4-thiazolyl)-3-oxo-5-(tetrahydropyran-2-yloxy)-2,6,10,14-tetramethyl-pentadeca-10,14-dien-2-yl)-2,2-dimethyl-[1,3]dioxan

Die Suspension von 4,92 g (5,97 mmol) der in Analogie zu Beispiel 1ai dargestellten Verbindung (5E,3S)-[3-[[(1,1-Dimethylethyl)dimethylsilyl]oxy]-4-methyl-5-(2-methyl-thiazol-4-yl)-pent-4-en-1-yl]-triphenylphosphoniumiodid in 14 ml wasserfreiem Tetrahydrofuran versetzt man bei 0°C unter einer Atmosphäre aus trockenem Argon mit 5,96 ml einer 1 M Lösung von Natrium-bis-(trimethylsilyl)-amid in Tetrahydrofuran und läßt auf 23°C erwärmen. Zu der roten Lösung tropft man langsam die Lösung von 877 mg (1,93 mmol) der nach Beispiel 1an dargestellten Verbindung in 14 ml Tetrahydrofuran, läßt 2 Stunden rühren, gießt auf gesättigte Ammmoniumchloridlösung und extrahiert mehrfach mit Ethylacetat. Die vereinigten organischen Extrakte trocknet man über Natriumsulfat und engt im Vakuum ein. Nach Säulenchromatographie an Kieselgel mit einem Gradientensystem aus n-Hexan und Ethylacetat werden neben 29% Ausgangsmaterial 732 mg (0,98 mmol, 51%) der Titelverbindung erhalten.
¹H-NMR (CDCl₃): δ = 0,01 (3H), 0,05 (3H), 0,79 (3H), 0,81-1,02 (6H), 0,90 (9H), 1,04-1,38 (11H), 1,38-2,08 (19H), 1,60 (3H), 2,01 (3H), 2,16-2,34 (2H), 2,72 (3H), 3,06 + 3,17 (1H), 3,42 (1H), 3,68 (1H), 3,80-4,03 (3H), 4,03-4,32 (2H), 4,46 + 4,54 (1H), 5,13 (1H), 6,45 (1H), 6,92 (1H) ppm.

### Beispiel 1 ap

### (3S,6R,7S,8S,12E/Z,15S,16E)-6-Ethyl-17-(2-methyl-4-thiazolyl)-5-oxo-4,4,8,12,16-pentamethyl-heptadeca-12,16-dien-1,3,7,15-tetraol (A) und (3S,6R,7S,8S,12E/Z,15S,16E)-15-[[(1,1-Dimethylethyl)dimethylsilyl]oxy]-6-ethyl-17-(2-methyl-4-thiazolyl)-5-oxo-4,4,8,12,16-pentamethyl-heptadeca-12,16-dien-1,3,7-triol (B)

Die Lösung von 732 mg (0,98 mmol) der nach Beispiel 1ao dargestellten Verbindung setzt man in Analogie zu Beispiel 1f um und isoliert nach Aufarbeitung und Reinigung 98 mg (0,19 mmol, 20%) der Titelverbindung A sowie 380 mg (0,61 mmol, 62%) der Titelverbindung B jeweils als farbloses Öl.
¹H-NMR (CDCl₃) von A: δ = 0,79-0,95 (6H), 0,98-1,19 (4H), 1,21-1,86 (15H), 1,92-2,17 (5H), 2,33 (2H), 2,74 (3H), 2,87-3,23 (3H), 3,31-3,50 (1H), 3,65-3,92 (3H), 4,05-4,20 (2H), 5,10-5,25 (1H), 6,53 (1H), 6,96 (1H) ppm.
¹H-NMR (CDCl₃) von B: δ = 0,01+0,05 (6H), 0,80-0,96 (15H), 1,01-1,17 (4H), 1,20-1,68 (4H), 1,68-1,90 (10H), 1,90-2,16 (5H), 2,25 (2H), 2,73+2,77 (3H), 2,91 (1H), 3,19 (1H), 3,42 (1H), 3,61 (1H), 3,79-3,93 (3H), 3,99-4,19 (2H), 5,10+5,20 (1H), 6,42 (1H), 6,94 (1H) ppm.

### Beispiel 1 aq

### (3S,6R,7S,8S,12E/Z,15S,16E)-6-Ethyl-17-(2-methyl-4-thiazolyl)4,4,8,12,16-pentamethyl-1,3,7,15-tetrakis-[[dimethyl(1,1-dimethylethyl)silyl]oxy]-heptadeca-12,16-dien-5-on

Die Lösung von 520 mg (ca. 0,86 mmol) eines Gemisches der nach Beispiel 1ap dargestellten Verbindungen A und B in 25 ml wasserfreiem Dichlormethan kühlt man unter einer Atmosphäre aus trockenem Argon auf -78°C, versetzt mit 2,6 ml 2,6-Lutidin, 2,57 ml Trifluormethansulfonsäure-tert.butyldimethylsilylester und rührt 16 Stunden. Man gießt in gesättigte Natriumhydrogencarbonatlösung und extrahiert mehrfach mit Dichlormethan. Die vereinigten organischen Extrakte trocknet man über Natriumsulfat und engt im Vakuum ein. Nach Säulenchromatographie an Kieselgel mit einem Gradientensystem aus n-Hexan und Ethylacetat isoliert man 1,14 g (max. 0,86 mmol, max. 100%) der Titelverbindung, die noch Silanol enthält.
¹H-NMR (CDCl₃) einer analytisch aufgereinigten Probe: ¹H-NMR (CDCl₃) δ = -0,04-0,11 (24H), 0,78-0,96 (42H), 1,13 (3H), 1,20 (3H), 1,02-1,65 (6H), 1,58+1,68 (3H), 1,72 (1H), 1,88-2,07 (2H), 2,00 (3H), 2,23 (2H), 2,71 (3H), 3,01 (1H), 3,52-3,73 (2H), 3,82 (1H), 3,91 (1H), 4,09 (1H), 5,13 (1H), 6,45 (1H), 6,91 (1H) ppm.

### Beispiel 1 ar

### (3S,6R,7S,8S,12E/Z.15S,16E)-3,7,15-6-Ethyl-tris-[[dimethyl(1,1-dimethylethyl)silyl]oxy]-1- hydroxy-17-(2-methyl-4-thiazolyl)-4,4,8,12,16-pentamethyl-heptadeca-12,16-dien-5-on

Die Lösung von 1,14g (max. 0,86 mmol) der nach Beispiel 1 aq dargestellten Verbindung in einem Gemisch aus 8 ml Dichlormethan und 8 ml Methanol versetzt man bei 0°C unter einer Atmosphäre aus trockenem Argon mit 204 mg Campher-10-sulfonsäure, läßt auf 23°C erwärmen und rührt noch 1,5 Stunden. Man versetzt mit Triethylamin, gießt in eine gesättigte Natriumhydrogencarbonatlösung und extrahiert mehrfach mit Dichlormethan. Die vereinigten organischen Extrakte trocknet man über Natriumsulfat und engt im Vakuum ein. Nach Säulenchromatographie an feinem Kieselgel mit einem Gradientensystem aus n-Hexan und Ethylacetat isoliert man 618 mg (0,78 mmol, 90%) der Titelverbindung.
¹H-NMR (CDCl₃): δ= -0,02-0,13 (18H), 0,77-0,98 (33H), 1,01-1,80 (10H), 1,08 (3H), 1,19 (3H), 1,55+1,66 (3H), 1,74-2,05 (2H), 2,00 (3H), 2,25 (2H), 2,70 (3H), 3,00 (1H), 3,68 (2H), 3,85 (1H), 4,08 (2H), 5,14 (1H), 6,44 (1H), 6,90 (1H) ppm.

### Beispiel 1as

### (35,6R,75,85,12E/Z,15S,16E)-6-Ethyl-3,7,15-tris-[[dimethyl(1,1-dimethylethyl)silyl]oxy]-4,4,8,12,16-pentamethyl-17-(2-methyl-4-thiazolyl)-5-oxo-heptadeca-12,16-dienal

510 mg (0,64 mmol) der nach Beispiel 1ar dargestellten Verbindung setzt man in Analogie zu Beispiel 1 k um und isoliert nach Aufarbeitung 545 mg (max. 0,64 mmol) der Titelverbindung als Rohprodukt, das man ohne Reinigung weiter umsetzt.

### Beispiel 1 at

### (3S,6R,7S,8S,12E/Z,15S,16E)-6-Ethyl-3,7,15-tris-[[dimethyl(1,1-dimethylethyl)silyl]oxy]-4,4,8,12,16-pentamethyl-17-(2-methyl-4-thiazolyl)-5-oxo-heptadeca-12,16-diensäure

Die Lösung von 545 mg (max. 0,64 mmol) der nach Beispiel 1 as dargestellten Verbindung in 15 ml Aceton kühlt man auf -30°C, versetzt mit 460 µl einer standardisierten, 8N Chromschwefelsäurelösung und rührt 1 Stunde. Man gießt in ein Gemisch aus Wasser und Diethylether, wäscht die organische Phsse mit gesättigter Natriumchloridlösung und trocknet über Natriumsulfat. Nach Filtration und Lösungsmittelabzug isoliert man 410 mg (0,47 mmol, 74% bezogen auf Edukt in Beispiel 1as) der Titelverbindungen, die chromatographisch getrennt werden können, als schwach gelbes Öl.
¹H-NMR (CDCl₃) des Z-Isomeren: δ= -0,02-0,15 (18H), 0,80-0,95 (33H), 1,03-2,28 (12H), 1,17 (3H), 1,18 (3H), 1,69 (3H), 1,96 (3H), 2,35 (1H), 2,54 (1H), 2,71 (3H), 3,03 (1H), 3,81 (1H), 4,16 (1H), 4,41 (1H), 5,20 (1H), 6,53 (1H), 6,94 (1H) ppm.
¹H-NMR (CDCl₃) des E-Isomeren: δ= -0,03-0,16 (18H), 0,79-0,95 (33H), 0,99-2,06 (10H), 1,17 (3H), 1,19 (3H), 1,57 (3H), 1,97 (3H), 2,26 (2H), 2,32 (1H), 2,61 (1H), 2,70 (3H), 3,09 (1H), 3,85 (1H), 4,09 (1H), 4,36 (1H), 5,12 (1H), 6,48 (1H), 6,94 (1H) ppm.

### Beispiel 1 au

### (3S,6R,7S,8S,12E/Z,15S,16E)-3,7-Bis-[[dimethyl(1,1-dimethylethyl)silyl]oxy]-15-hydroxy-6-ethyl-17-(2-methyl-4-thiazolyl)-5-oxo-4,4,8,12,16-pentamethyl -heptadeca-12,16-diensäure

### Variante I:

Die Lösung von 310 mg (0,36 mmol) der nach Beispiel 1at dargestellten Säure in 30 ml wasserfreiem Tetrahydrofuran versetzt man unter einer Atmosphäre aus trockenem Argon mit 500 µl eines Fluorwasserstoff-Pyridin-Komplexes, 7,1 ml einer 1,1 M Lösung von Tetrabutylammoniumfluorid in Tetrahydrofuran und rührt 3 Tage bei 50°C. Man gießt in eine gesättigte Ammoniumchloridlösung, extrahiert mehrfach mit Ethylacetat, wäscht die vereinigten organischen Extrakte mit gesättigter Natriumchloridlösung und trocknet über Natriumsulfat. Nach Filtration und Lösungsmittelabzug reinigt man den Rückstand durch Chromatographie an ca. 200 ml feinem Kieselgel mit einem Gradientensystem aus Dichlormethan und Methanol. Isoliert werden 125 mg (max. 0,24 mmol, max. 66%), die noch Tetrabutylammoniumsalze enthält.

### Variante II:

In Analogie zu Beispiel 1t setzt man 32 mg (37 µmol) der nach Beispiel 1 at dargestellten Säure um und isoliert nach Aufarbeitung und Reinigung 16 mg (31 µmol, 83%) der Titelverbindung als farbloses Öl.
¹H-NMR (CDCl₃) des Z-Isomeren: δ= 0,01-0,14 (12H), 0,80-0,99 (24H), 1,02-1,67 (7H), 1,18 (3H), 1,19 (3H), 1,70 (1H). 1,73 (3H), 1,97 (1H). 2,01 (3H), 2,14 (1H), 2,27-2,40 (3H), 2,53 (1H), 2,71 (3H), 2,81 (1H), 3,01 (1H). 3,82 (1H), 4,17 (1H). 4,48 (1H). 5,19 (1H), 6,69 (1H). 6,95 (1H) ppm.
¹H-NMR (CDCl₃) des E-Isomeren: δ= -0,02-0,11 (12H), 0,73-0,95 (24H), 1,00-1,63 (7H), 1,12 (3H), 1,17 (3H), 1,60 (3H), 1,71 (1H), 1,89-2,06 (2H), 2,00 (3H), 2,22-2,39 (3H), 2,53 (1H), 2,69 (3H), 2,79 (1H), 3,02 (1H), 3,79 (1H), 4,15 (1H), 4,34 (1H), 5,15 (1H), 6,56 (1H), 6,92 (1H) ppm.

### Beispiel 1 aw

### (4S,7R,8S,9S,13E/Z,16S(E))-4,8-Bis-[[dimethyl(1,1-dimethylethyl)silyl]oxy]-7-ethyl-16-(1-methyl-2-(2-methyl-4-thiazolyl)ethenyl)-1-oxa-5,5,9,13-tetramethyl-cyclohexadec-13-en-2,6-dion

Die Lösung von 55 mg (73 µmol) der nach Beispiel 1au dargestellten Verbindung in 0,8 ml wasserfreiem Tetrahydrofuran versetzt man unter einer Atmosphäre aus trockenem Argon mit 46 µl Triethylamin, 44 µl 2,4,6-Trichlorbenzoylchlorid und rührt 20 Minuten. Man verdünnt mit 20 ml Tetrahydrofuran, versetzt mit 68 mg 4-Dimethylaminopyridin und rührt 30 Minuten bei 23°C. Man engt ein, nimmt in wenig Dichlormethan auf und reinigt durch Chromatographie an 100 ml feinem Kieselgel mit einen Gradientensystem aus n-Hexan und Ethylacetat. Isoliert werden 49 mg (65 µmol, 89%) der Titelverbindungen - als farbloses Öl.
¹H-NMR (CDCl₃) des Z-Isomeren: δ=-0,12 (3H), 0,08 (3H), 0,10 (3H), 0,13 (3H), 0,73 (3H), 0,79-1,78 (7H), 0,85 (9H), 0,93 (9H), 0,99 (3H), 1,10 (3H), 1,18 (3H), 1,67 (3H), 1,88 (1H), 2,05 (1H), 2,09 (3H), 2,45 (1H), 2,54-2,74 (2H), 2,69 (3H), 2,77 (1H), 3,08 (1H), 4,00 (2H), 4,56 (1H), 5,16 (1H), 6,56 (1H), 6,95 (1H) ppm.
¹H-NMR (CDCl₃) des E-Isomeren: δ= 0,02-0,16 (12H), 0,78-1,00 (24H), 1,09 (3H), 1,14-1,93 (8H), 1,20 (3H), 1,59 (3H), 2,09-2,21 (1H), 2,13 (3H), 2,39 (1H), 2,43-2,64 (3H), 2,70 (3H), 2,98 (1H), 3,95 (1H), 4,40 (1H), 5,21 (1H), 5,29 (1H), 6,51 (1H), 6,92 (1H) ppm.

### Beispiel 1

### (4S,7R,8S,9S,13Z,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion (A) und (4S,7R,8S,9S,13E,16S(E))-4,8-Dihydroxy-7-ethyl-16-(1-methy)-2-(2-methyl-4-thiazolyl)ethenyl)-1-oxa-5,5,9,13-tetramethyl-cyclohexadec-13-en-2,6-dion (B)

Die Lösung von 48 mg (64 µmol) der nach Beispiel 1aw dargestellten Verbindung in 3 ml wasserfreiem Dichlormethan versetzt man bei -20°C unter einer Atmosphäre aus trockenem Argon mit 220 µl einer ca. 20%igen Trifluoressigsäure und rührt 1 Stunde. Man gießt in eine gesättigte Natriumhydrogencarbonatlösung, extrahiert mit Dichlormethan und trocknet die organische Phase über Natriumsulfat. Nach Filtration und Lösungsmittelabzug reinigt man den Rückstand durch mehrfache Chromatographie an analytischen Dünnschichtplatten. Als Laufmittel dient ein Gemisch aus n-Hexan und Ethylacetat, als Elutionsmittel Ethylacetat. Isoliert werden 13 mg (25 µmol, 39%) der Titelverbindung A sowie 12 mg (23 µmol, 36%) der Titelverbindung B jeweils als farbloses Öl.
¹H-NMR (CDCl₃) von A: δ= 0,89 (3H), 1,04 (3H), 1,09 (3H), 1,19-1,94 (8H), 1,33 (3H), 1,70 (3H), 2,07 (3H), 2,15-2,33 (2H), 2,38 (1H), 2,44-2,74 (3H), 2,70 (3H), 3,23 (1H), 3,62 (1H), 3,72 (1H), 4,24 (1H), 5,12 (1H), 5,22 (1H), 6,57 (1H), 6,95 (1H) ppm.
¹H-NMR (CDCl₃) von B: δ= 0,84 (3H), 1,01 (6H), 1,29 (3H), 1,38-2,00 (8H), 1,61 (3H), 2,07 (3H), 2,20 (1H), 2,22-2,50 (3H), 2,58 (1H), 2,70 (3H), 3,37 (1H), 3,73 (1H), 4,02 (1H), 4,12 (1H). 4,41 (1H), 5,05 (1H), 5,38 (1H), 6,57 (1H), 6,99 (1H) ppm.

### Beispiel 2

### (1S,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion (A) und (1R,3S(E),7S,10R,11S,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion (B)

Die Lösung von 10 mg (19 µmol) der nach Beispiel 1 dargestellten Verbindung A in 1 ml Dichlormethan versetzt man unter einer Atmosphäre aus trockenem Argon bei -10°C mit 10 mg einer ca. 80%igen meta-Chlorperbenzoesäure und rührt 4 Stunden bei 0°C. Man gießt in eine gesättigte Natriumhydrogencarbonatlösung, extrahiert mit Dichlormethan und trocknet die organische Phase über Natriumsulfat. Nach Filtration und Lösungsmittelabzug reinigt man den Rückstand durch wiederholte Chromatographie an analytischen Dünnschichtplatten. Als Laufmittel dienen Gemische aus n-Hexan und Ethylacetat sowie Dichlormethan und Methanol, als Elutionsmittel Ethylacetat. Isoliert werden 4,5 mg (8,4 µmol, 44%) der Titelverbindung A sowie 1 mg (1,9 µmol, 10%) der Titelverbindung B als farbloser Schaum.
¹H-NMR (CDCl₃) von A: δ= 0,86 (3H), 1,00 (3H), 1,05 (3H), 1,28 (3H), 1,33-2,12 (10H), 1,38 (3H), 2,11 (3H), 2,41 (1H), 2,57 (1H). 2,70 (3H), 2,77-2,85 (2H), 3,38 (1H), 3,49 (1H), 3,67 (1H), 4,27 (1H), 4,56 (1H), 5,46 (1H), 6,57 (1H), 6,97 (1H) ppm.
¹H-NMR (CDCl₃) von B: δ= 0,85 (3H), 0,95 (3H), 1,03 (3H), 1,22-1,73 (10H), 1,30 (3H), 1,38 (3H), 2,08 (1H), 2,61 (3H), 2,41-2,59 (2H), 2,71 (3H), 2,91 (1H), 2,99 (1H), 3,24 (1H), 3,43 (1H), 3,96 (1H), 4,30 (1H), 5,60 (1H), 6,60 (1H), 6,98 (1H) ppm.

### Beispiel 3

### (1R,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5.9-dion (A) und (1S,3S(E).7S,10R,11S,12S,16S1-7,11-Dihydroxy-3-(1-methyl-2-12-methyl-4-thiazolyl)ethenyl)-10-ethyl-8.8,12.16-tetramethyl-4,17-dioxabicyclo[14.1.0]heptadecan-5,9-dion (B)

10 mg (19 µmol) der nach Beispiel 1 hergestellten Verbindung B setzt man in Analogie zu Beispiel 2 um und isoliert nach Aufarbeitung und Reinigung 6 mg (11 µmol, 59%) eines Gemisches der beiden Titelverbindungen als farblosen Schaum.
¹H-NMR (CDCl₃) von A oder B: δ= 0,86 (3H), 0,96 (3H), 1,03 (3H), 1,06-2,08 (11H), 1,28 (3H), 1,38 (3H), 2,09 (3H), 2,46-2,59 (2H), 2,70 (3H), 2,87 (1H), 3,02 (1H), 3,33 (1H), 3,79 (1H), 4,22 (1H), 4,34 (1H), 5,49 (1H), 6,65 (1H), 7,00 (1H) ppm.
¹H-NMR (CDCl₃) von B oder A: δ= 0,86 (3H), 0,96 (3H), 1,09 (3H), 1,21-1,94 (9H), 1,25 (3H), 1,37 (3H), 2,03 (2H), 2,09 (3H), 2,50-2,61 (2H), 2,71 (3H), 2,87 (1H). 2,94 (1H), 3,28 (1H), 3,67 (1H). 3,72 (1H), 4,27 (1H), 5,46 (1H), 6,59 (1H), 6,97 (1H) ppm.

### Beispiel 4

### (4S,7S,8R,9S,13Z,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion (A) und (4S,7S,8R,9S,13E,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion (B)

Die nach Beispiel 1 ak hergestellte diastereomere Verbindung B setzt man in Analogie zu den Beispielen 1 al bis 1 aw und 1 zu den Titelverbindungen A und B um.

### Beispiel 5

### (1S,3S(E),7S,10S,11 R,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion (A) und (1R,3S(E),7S,10S,11R,12S,16S)-7,11-Dihydroxy-3-(1-methyl-2-(2-methyl-4-th iazolyl)ethenyl)-10-ethyl-8,8,12,16-tetramethyl-4,17-dioxabicyclo[14.1.0]heptadecan-5,9-dion (B)

Die nach Beispiel 4 hergestellte Verbindung A setzt man in Analogie zu Beispiel 2 zu den trennbaren Titelverbindungen A und B um.

### Beispiel 6

### (1S,3S(E),7S,10S,11R,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und (1R,3S(E),7S,10S,11R,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion

Die nach Beispiel 4 hergestellte Verbindung B setzt man in Analogie zu Beispiel 2 zu einem Gemisch der Titelverbindungen um.

### Beispiel 7

### (4S,7R,8S,9S,13(Z),16S(E))-4,8-Dihydroxy-5,5,7,9,13-pentamethyl-16-((3-pyridyl)ethenyl)-1-oxa-cyclohexadec-13-en-2,6-dion (A) und (4S,7R,8S,9S,13E,16S(E))-4,8-Dihydroxy-5,5,7,9,13-pentamethyl-16-((3-pyridyl)ethenyl)-1-oxa-cyclohexadec-13-en-2,6-dion (B)

### Beispiel 7a

### (Z,3S)-1-[[Dimethyl(1,1-dimethylethyl)silyl]oxy]-3-[[(1,1-dimethylethyl)diphenylsilyl]oxy]-4-methyl-5-(3-pyridyl)-pent-4-en (A) und (E,3S)-1-[[Dimethyl(1,1-dimethylethyl)silyl]oxy]-3-[[(1,1-dimethylethyl)diphenylsilyl]oxy]4-methyl-5-(3-pyridyl)-pent-4-en (B)

In Analogie zu Beispiel 1af setzt man 4,8 g (10,2 mmol) der nach Beispiel 1ae dargestellten Verbindung unter Verwendung von Diethyl(3-pyridyl)methanphosphonat um und isoliert nach Aufarbeitung und Reinigung 448 mg (0,82 mmol, 8%) der Titelverbindung A sowie 3,5 g (6,41 mmol, 63%) der Titelverbindung B jeweils als farbloses Öl.
¹H-NMR (CDCl₃) von A: δ = -0,06 (6H), 0,81 (9H), 1,01 (9H), 1,75 (1H), 1,97 (4H), 3,48 (2H), 4,83 (1H), 6,11 (1H), 6,97 (1H), 7,11-7,30 (5H), 7,30-7,39 (2H), 7,39-7,50 (4H), 8,08 (1H), 8,33 (1H) ppm.
¹H-NMR (CDCl₃) von B: δ = -0,01 (6H), 0,85 (9H), 1,11 (9H), 1,78 (3H), 1,83 (1H), 1,97 (1H), 3,58 (2H), 4,42 (1H), 6,03 (1H). 7,21 (1H), 7,28-7,50 (7H), 7,62-7,75 (4H), 8,29 (1H), 8,41 (1H) ppm.

### Beispiel 7b

### (E,3S)-3-[[(1,1-Dimethylethyl)diphenylsilyl]oxy]-4-methyl-5-(3-pyridyl)-pent-4-en-1-ol

Analog zu Beispiel 1ag werden 3,5 g (6,41 mmol) der unter Beispiel 7aB hergestellten Verbindung mit einem 65:35:10-Gemische aus Eisessig/Wasser/Tetrahydrofuran umgesetzt. Man erhält nach Aufreinigung 2,1 g (4,86 mmol, 76%).
¹H-NMR (CDCl₃): δ = 1,12 (9H), 1,75 (3H), 1,88 (2H), 3,65 (2H), 4,45 (1H), 6,25 (1H), 7,21 (1H), 7,28-7,50 (7H), 7,60-7,75 (4H), 8,30 (1H), 8,44 (1H) ppm.

### Beispiel 7c

### (E,35)-1-Iod-3-[[(1,1-dimethylethyl)diphenylsilyl]oxy]-4-methyl-5-(3-pyridyl)-pent-4-en

Analog zu Beispiel 1 ah werden aus 2,1 g (4,86 mmol) der unter Beispiel 7b beschriebenen Verbindung 1,98 g (3,66 mmol, 75%) der Titelverbindung erhalten.
¹H-NMR (CDCl₃): δ= 1,11 (9H), 1,78 (3H), 2,17 (2H), 3,03 (2H), 4,29 (1H), 6,19 (1H), 7,22 (1H), 7,30-7,50 (7H), 7,63-7,75 (4H), 8,32 (1H), 8,44 (1H) ppm.

### Beispiel 7d

### (5E,3S)-[3-[[(1,1-dimethylethyl)diphenylsilyl]oxy]-4-methyl-5-(3-pyridyl)-pent-4-en-1-yl]-triphgenylphosphoniumiodid

Analog zu Beispiel 1ai werden aus 1,98 g (3,66 mmol) der unter Beispiel 7c beschriebenen Verbindung 2,35 g (2,93 mmol, 80%) der Titelverbindung erhalten.
¹H-NMR (CDCl₃): δ= 1,08 (9H), 1,80 (3H), 3,27 (1H), 3,56 (1H), 4,66 (1H), 6,52 (1H), 7,25-7,90 (27H), 8,35 (1H), 8,46 (1H) ppm.

### Beispiel 7e

### (4S(4R,5S,6S,10E/Z,13S,14E))-4-(13-[[(1,1-Dimethylethyl)diphenylsilyl]oxy]-2,4,6,10,14-pentamethyl-1 5-(3-pyridyl)-3-oxo-5-(tetrahydropyran-2-yloxy)-pentadeca-10,14-dien-2-yl)-2,2-dimethyl-[1,3]dioxan

Analog zu Beispiel 1 ao werden 800 mg (1,76 mmol) der in Analogie zu den Beispielen 1l (Umsetzung mit Ethylmagnesiumbromid) bis 1 an hergestellten Verbindung (4S(4R,5S,6S))-4-(3,10-Dioxo-2,4,6-trimethyl-5-(tetrahydropyran-2-yloxy)-undec-2-yl)-2,2-dimethyl-[1,3]dioxan mit 4,24 g (5,28 mmol) der unter Beispiel 7d beschriebenen Verbindung und 5,44 ml einer 1 M Lösung von Natrium-bis-(trimethylsilyl)-amid in Tetrahydrofuran umgesetzt. Man erhält 684 mg (0,79 mmol, 45%) der Titelverbindung.
¹H-NMR (CDCl₃): δ= 0,86-0,98 (3H), 0,98-1,94 (45H), 2,20-2,42 (2H), 3,22 (1H), 3,42 (1H), 3,58-4,02 (4H), 4,08-4,22 (2H), 4,46 + 4,52 (1H), 5,00 (1H), 6,03 (1H), 7,19 (1H), 7,24-7,47 (7H), 7,60-7,73 (4H), 8,28+8,40 (2H) ppm.

### Beispiel 7f

### (3S,6R,7S,8S,12E/Z,15S,16E)-15-[[(1,1-Dimethylethyl)diphenylsilyl]oxy]-4,4,6,8,12,16-hexamethyl-17-(3-pyridyl)-5-oxo-heptadeca-1 2,16-dien-1,3,7-triol

Analog zu Beispiel 1ap werden aus 684 mg (0,79 mmol) der unter Beispiel 7e beschriebenen Verbindung 542 mg (0,73 mmol, 92%)der Titelverbindung erhalten.

### Beispiel 7g

### (3S,6R,7S,8S,12E/Z,15S,16E)-15-[[(1,1-Dimethylethyl)diphenylsilyl]oxy]-4,4,6,8,12,16-hexamethyl-17-(3-pyridyl)-1,3,7-tris-[[dimethyl(1,1-dimethylethyl)silyl]oxy]-heptadeca-12,16-dien-5-on

Analog zu Beispiel 1 aq werden aus 542 mg (0,73 mmol) der unter Beispiel 7f beschriebenen Verbindung 995 mg (max. 0,73 mmol, max. 100%) der Titelverbindung erhalten, die mit Silanol verunreinigt ist.

### Beispiel 7h

### (3S,6R,7S,8S,12E/Z,15S,16E)-3,7-Bis-[[dimethyl(1,1-dimethylethyl)silyl]oxy]-15-[[(1,1-dimethylethyl)diphenylsilyl]oxy]-1-hydroxy-4,4,6,8,12,16-hexamethyl-17-(3-pyridyl)-heptadeca-12,16-dien-5-on

Analog zu Beispiel 1ar werden aus 995 mg (max. 0,73 mmol) der unter Beispiel 7g beschriebenen Verbindung 604 mg (0,62 mmol, 85%) der Titelverbindung erhalten.

### Beispiel 7i

### (3S,6R,7S,8S,12E/Z,15S,16E)-3,7-Bis-[[dimethyl(1,1-dimethylethyl)sityl]oxy]-15-[[(1,1-dimethylethyl)diphenylsilyl]oxy]-4,4,6,8,12,16-hexamethyl-17-(3-pyridyl)-5-oxo-heptadeca-12,16-diensäure

Analog zu den Beispielen 1 as und 1 at werden aus 604 mg (0,62 mmol) der unter Beispiel 7h beschriebenen Verbindung 550 mg (0,56 mmol, 90%) der Titelverbindung erhalten.

### Beispiel 7k

### (3S,6R,7S,8S,12E/Z,15S,16E)-4,4,6,8,12,16-Hexamethyl-17-(3-pyridyl)-5-oxo-3,7,15-trihydroxy-heptadeca-12,16-diensäure

Analog zu Beispiel 1au werden aus 550 mg (0,56 mmol) der unter Beispiel 7i beschriebenen Verbindung 269 mg (0,49 mmol, 88%) der Titelverbindung erhalten.

### Beispiel 71

### (3S,6R,8S,12E/Z,15S,16E)-3,7-Bis[[dimethyl(1,1-dimethylethyl)silyl]oxy]-4,4,6,8,12,16-hexamethyl-15-hydroxy-17-(3-pyridyl)-5-oxy-heptadeca-12,16-diensäure

Analog zu Beispiel 1 av werden aus 269 mg (0,49 mmol) der unter Beispiel 7k beschriebenen Verbindung 127 mg (0,17 mmol, 35%) der Titelverbindung erhalten.

### Alternative Darstellung von 71 über 7n bis 7r:

### Beispiel 7n

### (4S(4R,5S,6S,10E/Z,13S,14E))-4-(13-hydroxy-2,4,6,10,14-pentamethyl-15-(3-pyridinyl)-3-oxo-5-(tetrahydropyran-2-yloxy)-pentadeca-10,14-dien-2-yl)-2,2-dimethyl-[1,3]dioxan

Analog zu Beispiel 1i werden aus 710 mg (0,85 mmol) der unter 7e beschriebenen Verbindung 486 mg (0,81 mmol, 95%) der Titelverbindung erhalten.
¹H-NMR (CDCl₃): δ= 0,90-1,00 (3H), 1,05-1,90 (36H), 2,38 (2H), 3,27 (1H), 3,46 (1H), 3,63 + 3,80-4,00 (4H), 4,10-4,20 (2H), 4,46 + 4,55 (1H), 5,15 (1H), 6,49 (1H), 7,24 (1H), 7,57 (1H), 8,47 (1H), 8,54 (1H) ppm.

### Beispiel 7o

### (3S,6R,7S,8S,12E/Z,15S,16E)-4,4,6,8,12,16-Hexamethyl-17-(3-pyridyl)-1,3,7,15-tetrahydroxy-heptadeca-12,16-dien-5-on

Analog zu Beispiel 1f werden aus 486 mg (0,81 mmol) der unter 7n beschriebenen Verbindung 335 mg (0,71 mmol, 87%) der Titelverbindung erhalten.
¹H-NMR (CDCl₃): δ= 0,82 + 0,86 (3H), 1,08 + 1,10 (3H), 1,13 (3H), 1,22 (3H), 1,68 + 1,72 (3H), 1,90 (3H), 2,40 (2H), 3,30 (1H), 3,35-3,48 (2H), 3,85-3,96 (2H), 4,17 (1H), 4,20 (1H), 5,05 (1H), 6,50 (1H), 7,25 (1H), 7,61 (1H), 8,45 (1H), 8,53 (1H) ppm.

### Beispiel 7p

### (3S,6R,7S,8S,12E/Z,15S,16E)-3,7,15-Tris-[[dimethyl(1,1-dimethylethyl)silyl]oxy]-1-hydroxy-4,4,6,8,12,16-hexamethyl-17-(3-pyridyl)-heptadeca-12,16-dien-5-on

Analog zu Beispiel 1aq werden aus 335 mg (0,71 mmol) der unter 7o beschriebenen Verbindung 730 mg (max. 0,71 mmol, max. 100%) der Titelverbindung erhalten, die mit Silanol verunreinigt ist.
¹H-NMR (CDCl₃): δ= 0,05-1,16 (24H), 0,85-0,97 (39H), 1,02 + 1,04 + 1,07 (6H), 1,22 (3H), 1,60 (3H), 1,70 + 1,83 (3H), 2,29 (1H), 3,13 (1H), 3,05-3,80 (2H), 3,76 (1H), 3,89 (1H), 4,11 (1H), 5,13 (1H), 6,46 (1H), 7,23 (1H), 7,54 (1H), 8,42 (1H), 8,50 (1H9 ppm.

### Beipiel 7q

### (3S,6R,7S,8S,12E/Z,15S,16E)-3,7,15-Tris-[[dimethyl(1,1-dimethylethyl)silyl]oxyl-1-hydroxy-4,4,6,8,12,16-hexamethyl-17-(3-pyridyl)-heptadeca-12,16-dien-5-on

Analog zu Beispiel 1ar werden aus 730 mg (max. 0,71 mmol) der unter 7p beschriebenen Verbindung 441 mg (0,54 mmol, 76%) der Titelverbindung erhalten.
¹H-NMR (CDCl₃): δ= 0,05-0,18 (18H), 0,90-1,10 (30H), 1,11 (6H), 1,25 (3H), 1,62 + 1,70 (3H), 1,82 (3H), 2,38 (1H), 3,13 (1H), 3,63 (2H), 3,81 (1H), 4,05-4,15 (2H), 5,17 (1H), 6,38 (1H), 7,22 (1H), 7,53 (1H), 8,45 (1H), 8,52 (1H) ppm.

### Beispiel 7r

### (3S,6R,7S,8S,12E/Z,15S,16E)-3,7,15-Tris-[[dimethyl(1,1-dimethylethyl)silyl]oxy]-4,4,6,8,12,16-hexamethyl-1 7-(3-pyridyl)-5-oxo-heptadeca-1 2,16-diensäure

Analog zu den Beispielen 1as und 1 at werden aus 441 mg (0,38 mmol) der unter 7q beschriebenen Verbindung 316 mg (0,38 mmol, 70%) der Titelverbindung erhalten.
¹H-NMR (CDCl₃): δ= 0,00-0,18 (18H), 0,90-1,00 (30H), 1,12 (3H), 1,13 + 1,14 (3H), 1,19 (3H), 1,62 + 1,70 (3H), 1,79 + 1,80 (3H), 3,18 (1H), 3,75 + 3,80 (1H), 4,19 (1H), 4,44 + 4,48 (1H), 5,12 + 5,14 (1H), 6,32 + 6,35 (1H), 7,30 (1H), 7,60 + 7,62 (1H), 8,38 + 8,40 (1H) , 8,58 ppm.

### Beispiel 71

### (3S,6R,8S,12E/Z,15S,16E)-3,7-Bis[[dimethyl(1,1-dimethylethyl)sillyl]oxy]-4,4,6,8,12,16-hexamethyl-15-hydroxy-17-(3-pyridyl)-5-oxy-heptadeca-12,16-diensäure

Analog zu Beispiel 1i werden aus 316 mg (0,38 mmol) der unter 7r beschriebenen Verbindung 295 mg (max. 0,38 mmol, max. 100%) der Titelverbindung erhalten.
¹H-NMR (CDCl₃): δ= 0,00-0,18 (12H), 0,88-1,00 (21H), 1,10 (3H), 1,15 (3H), 1,18 (3H), 1,63 + 1,70 (3H), 1,84 + 1,86 (3H), 2,30-2,50 (3H), 3,10 (1H), 3,75 + 3,78 (1H), 4,20 +4,25 (1H), 4,45 (1H), 5,14 (1H), 6,49 (1H), 7,33 (1H), 7,68 (1H), 8,41 (1H), 8,60 (1H) ppm.

### Beispiel 7m

### (4S,7R,8S,13E/Z,16S(E))-4,8-Bis-[[dimethyl(1,1-dimethylethyl)silyl]oxy]-4,4,6,8,12,16-hexamethyl-16-((3-pyridyl)ethenyl)1-oxa-cyclohexadec-13-en-2,6-dion

Analog zu Beispiel 1 aw werden aus 127 mg (0,17 mmol) der unter Beispiel 71 beschriebenen Verbindung 104 mg (0,14 mmol, 85%) der Titelverbindung erhalten.
¹H-NMR (CDCl₃): δ= -0,05-0,13 (12H), 0,82-1,00 (21H), 1,12 (3H), 1,15 (3H), 1,23 (3H), 1,60 + 1,69 (3H), 1,90 + 1,92 (3H), 2,40-2,60 (4H), 3,02 (1H), 3,88 + 3,90 (1H), 4,10 (1H), 4,48 (1H), 5,07 + 5,14 (1H), 5,18 + 5,25 (1H), 6,47 + 6,50 (1H), 7,25 (1H), 7,55 + 7,60 (1H), 8,45 (1H), 8,50 + 8,53 (1H) ppm.

### Beispiel 7

### (4S,7R,8S,9S,13Z,16S(E))-4,8-Dihydroxy-5,5,7,9,13-pentamethyl-16-((3-pyridyl)ethenyl)-1-oxa-cyclohexadec-13-en-2,6-dion (A) und b(4S,7R,8S,9S,13E,16S(E))-4,8-Dihydroxy-5,5,7,9,13-pentamethyl-16-((3-pyridyl)ethenyl)-1-oxa-cyclohexadec-13-en-2,6-dion (B)

Analog zu Beispiel 1 werden aus 104 mg (0,14 mmol) der unter Beispiel 7m beschriebenen Verbindung 24 mg (48 µmol, 34%) der Titelverbindung A sowie 25 mg (50 µmol, 36%) der Titelverbindung B erhalten.
¹H-NMR (CDCl₃)
Verbindung A: δ= 1,03 (3H), 1,10 (3H), 1,21 (3H), 1,32 (3H), 1,62 (3H), 1,92 (3H), 2,18-2,80 (6H), 3,14 (1H), 3,73 (1H). 4,16 (1H), 5,17 (1H). 5,29 (1H), 6,51 (1H), 7,25 (1H), 7,58 (1H), 8,47 (1H), 8,53 (1H) ppm.
Verbindung A: δ= 1,00 (3H), 1,05 (3H), 1,16 (3H), 1,30 (3H), 1,63 (3H), 1,91 (3H), 2,18-2,65 (6H), 3,22 (1H), 3,65 (1H), 4,20 (1H), 5,11 (1H), 5,43 (1H), 6,49 (1H), 7,27 (1H), 7,59 (1H), 8,49 (1H), 8,52 (1H) ppm.

### Beispiel 8

### (1S,3S(E),7S,10R,11S,12S,16R)-7,11-Dihydroxy-8,8,10,12,16-pentamethy-3-((3-pyridyl)ethenyl)-4,17-dioxabicyclo[14,1,0]heptadecan-5,9-dion (A)und (1S,3S(E),7S,10R,11S,12S,16S)-7,11-Dihydroxy-8,8,10,12,16-pentamethyl-3-((3-pyridyl)ethenyl)-4,17-dioxabicyclo[14,1,0]heptadecan-5,9-dion (B) und (1S,3S(E),7S,10R,11 S,12S,16R)-7,11-Dihydroxy-8,8,10,12,16-pentamethyl-3-((3-N-oxypyridyl)ethenyl)-4,17-dioxabicyclo[14.1.0]heptadecan-5,9-dion (C) und (1S,3S(E),7S,10R,11 S,12S,16S)-7,11-Dihydroxy-8,8,10,12,16-pentamethyl-3-((3-N-oxvpyridyl)ethenyl)-4,17-dioxabicyclo[14.1.0]heptadecan-5,9-dion (D)

Analog zu Beispiel 2 werden aus 15 mg (30 µmol) der unter Beispiel 7 beschriebenen Verbindung A 7,4 mg (14 µmol, 46%) der Titelverbindung A, 1,6 mg (3 µmol, 10%) der Titelverbindung B, 2,4 mg der Titelverbindung C sowie 0,9 mg (4,4 mmol, 15%) der Titelverbindung D (1,7 mg, 6%) erhalten.
¹H-NMR (CDCl₃): Verbindung C:
δ= 1,03 (3H), 1,10 (3H), 1,17 (3H), 1,28 (3H), 1,22 (3H), 1,91 (3H), 2,40-2,63 (3H), 2,79 (1H), 3,33 (1H), 3,68 (1H), 3,77 (1H), 4,12 (3H), 5,46 (1H), 6,46 (1H), 7,18 (1H), 7,25 (1H), 8,11 (1H), 8,18 (1H) ppm.
Verbindung D:
δ= 0,97 (3H), 1,10 (3H), 1,13 (3H), 1,28 (3H), 1,40 (3H), 1,95 (3H), 2,50 (1H), 3,12 (1H). 3,34 (1H), 3,80 (1H), 4,08 (1H), 4,16 (1H), 5,69 (1H), 6,47 (1H), 7,17 (1H), 7,26 (1H), 8,11 (1H), 8,18 (1H) ppm.

### Beispiel 9

### (4S,7R,8S,9S,13(Z),16S(E))-4,8-Dihydroxy-5,5,7,9,13-pentamethyl-16-((4-pyridyl)ethenyl)-1-oxa-cyclohexadec-13-en-2,6-dion (A) und (4S,7R,8S,9S,13E,16S(E))-4,8-Dihydroxy-5,5,7,9,13-pentamethyl-16-((4-pyridyl)ethenyl)-1-oxa-cyclohexadec-13-en-2,6-dion (B)

### Beispiel 9a

### (4S(4R,5S,6S,10E/Z,13S,14E))-4-(13-[[(1,1-Dimethylethyl)diphenylsilyl]oxy-2,4,6,10,14-pentamethyl-15-(4-pyridyl)-3-oxo-5-(tetrahydropyran-2-yloxy)-pentadeca-10,14-dien-2-yl)-2,2-dimethyl-[1,3]dioxan

Analog zu Beispiel 7e werden 2,08 g (4,70 mmol) der in Analogie zu den Beispielen 11 (Umsetzung mit Ethylmagnesiumbromid) bis 1 an hergestellten Verbindung (4S(4R,5S,6S))-4-(3,10-Dioxo-2,4,6-trimethyl-5-(tetrahydropyran-2-yloxy)-undec-2-yl)-2,2-dimethyl-[1,3]dioxan mit 11,4 g (14,2 mmol) (5E,3S)-[3-[[(1,1-dimethylethyl) diphenylsilyl]oxy]-4-methyl-5-(4-pyridyl)-pent-4-en-1-yl]-triphenylphosphoniumiodid, das man in Analogie zu den Beispielen 7a bis 7d unter Verwendung von Diethyl(4-pyridyl)methanphosphonat hergestellt hat, umgesetzt. Nach Aufarbeitung und Reinigung isoliert man 2,10 g (2,5 mmol, 53%) der Titelverbindung.
¹H-NMR (CDCl₃): δ= 0,81-1,95 (49H), 2,20-2,42 (2H), 3,23 (1H), 3,42 (1H), 3,58-4,02 (3H), 4,06-4,21 (2H), 4,46+4,52 (1H), 4,99 (1H), 6,03 (1H), 6,94 (2H), 7,22-7,48 (6H), 7,59-7,73 (4H), 8,49 (2H) ppm.

### Beispiel 9b

### (4S(4R,5S,6S,10E/Z,13S,14E))-4-(13-hydroxy-2,4,6,10,14-pentamethyl-15-(4-pyridyl)-3-oxo-5-(tetrahydropyran-2-yloxy)-pentadeca-10,14-dien-2-yl)-2,2-dimethyl-[1,3]dioxan

Analog zu Beispiel 1i werden aus 780 mg (0,93 mmol) der unter Beispiel 9a beschriebenen Verbindung 550 mg (0,91 mmol, 98%)der Titelverbindung erhalten.
¹H-NMR (CDCl₃): δ= 0,80-1,85 (33H), 1,91 (3H), 1,94-2,11 (5H), 2,36 (2H), 3,27 (1H), 3,43 (1H), 3,61-4,01 (3H), 4,08-4,21 (2H), 4,46+4,54 (1H), 5,16 (1H), 6,48 (1H), 7,18 (2H), 8,55 (2H) ppm.

### Beispiel 9c

### (3S,6R,7S,8S,12E/Z,15S,16E)-4,4,6,8,12,16-Hexamethyl-17-(4-pyridyl)-1,3,7,15-tetrahydroxy-heptadeca-12,16-dien-5-on

Analog zu Beispiel 1f werden aus 600 mg (1,00 mmol) der unter Beispiel 9b beschriebenen Verbindung unter Verwendung von p-Toluolsulfonsäure 340 mg (0,71 mmol, 71%) der Titelverbindung erhalten.
¹H-NMR (CDCl₃): δ= 0,82 (3H), 1,06 (3H), 1,12 (3H), 1,22 (3H), 1,73 (3H), 0,90-1,83 (9H), 1,91 (3H), 1,95-2,13 (3H), 2,30-2,47 (2H), 3,19-3,35 (2H), 3,42 (1H), 3,81-3,97 (2H), 4,04 (1H), 4,19 (1H), 5,18 (1H), 6,46 (1H), 7,18 (2H), 8,52 (2H) ppm.

### Beispiel 9d

### (3S,6R,7S,8S,12E/Z,15S,16E)-3,7,15-Tris-[[dimethyl(1,1-dimethylethyl)silyl]oxy]-1-hydroxy-4,4,6,8,12,16-hexamethyl-17-(4-pyridyl)-heptadeca-12,16-dien-5-on

Analog zu Beispiel 1 aq werden aus 300 mg (0,63 mmol) der unter Beispiel 9c beschriebenen Verbindung 435 mg (0,47 mmol, 74%) der Titelverbindung erhalten.
¹H-NMR (CDCl₃): δ= -0,01-0,14 (24H), 0,82-0,97 (37H), 1,02 (3H), 1,04 (3H), 1,21 (3H), 0,98-1,70 (12H), 1,87 (3H), 1,90-2,03 (2H), 2,25 (2H), 3,13 (1H), 3,51-3,71 (2H), 3,76 (1H), 3,88 (1H), 4,03-4,14 (1H), 5,13 (1H), 6,34 (1H), 7,13 (2H), 8,52 (2H) ppm.

### Beispiel 9e

### (3S,6R,7S,8S,12E/Z,15S,16E)-3,7,15-Tris-[[dimethyl(1,1-dimethylethyl)silyl]oxy]-1-hydroxy-4,4,6,8,12,16-hexamethyl-17-(4-pyridyl)-heptadeca-12,16-dien-5-on

Analog zu Beispiel 1 ar werden aus 410 mg (0,44 mmol) der unter Beispiel 9d beschriebenen Verbindung 339 mg (0,41 mmol, 94%) der Titelverbindung erhalten.
¹H-NMR (CDCl₃): δ= -0,01-0,14 (18H), 0,80-0,95 (31H), 0,97-1,70 (7H), 1,06 (6H), 1,21 (3H), 1,59+1,69 (3H), 1,87 (3H), 1,90-2,06 (2H), 2,26 (2H), 3,12 (1H), 3,65 (2H), 3,80 (1H), 4,09 (2H), 5,14 (1H), 6,36 (1H), 7,13 (2H), 8,53 (2H) ppm.

### Beispiel 9f

### (3S,6R,7S,8S,12E/Z,15S,16E)-3,7,15-Tris-[[dimethyl(1,1-dimethylethyl)silyl]oxy-4,4,6,8,12,16-hexamethyl-17-(4-pyridyl)-5-oxo-heptadeca-12,16-diensäure

Analog zu den Beispielen 1as und 1 at werden aus 280 mg (0,34 mmol) der unter Beispiel 9e beschriebenen Verbindung 204 mg (0,25 mmol, 72%) der Titelverbindung erhalten.
¹H-NMR (CDCl₃): δ= 0,00-0,14 (18H), 0,78-0,98 (30H), 1,06 (3H), 1,08 (3H), 1,24 (3H), 1,05-1,55 (5H), 1,60+1,69 (3H), 1,87 (3H), 1,98 (2H), 2,20-2,37 (3H), 2,10-3,10 (1H), 2,51 (1H), 3,14 (1H), 3,79 (1H), 4,11 (1H), 4,40 (1H), 5,13 (1H), 6,36 (1H), 7,17 (2H), 8,53 (2H) ppm.

### Beispiel 9g

### (3S,6R,8S,12E/Z,15S,16E)-3,7-Bis[[dimethyl(1,1-dimethylethyl)silyl]oxy]-4,4,6,8,12,16-hexamethyl-15-hydroxy-17-(4-pyridyl)-5-oxy-heptadeca-12,16-diensäure

Analog zu Beispiel 1 av werden aus 198 mg (0,24 mmol) der unter Beispiel 9f beschriebenen Verbindung 132 mg (0,18 mmol, 77%) der Titelverbindung erhalten.
¹H-NMR (CDCl₃): δ= 0,00-0,15 (12H), 0,85-1,00 (18H), 1,10-1,18 (6H), 1,20-1,28 (6H), 1,62 + 1,73 (3H), 2,05 (1H), 2,20-2,50 (4H), 2,85 (1H), 3,15 (1H), 3,79 (1H), 4,18 (1H), 4,42 (1H), 5,18 (1H), 6,50 (1H), 7,15-7,25 (2H), 8,50-8,60 (2H) ppm.

### Beispiel 9h

### (4S,7R,8S,13E/Z,16S(E))-4,8-Bis-[[dimethyl(1,1-dimethylethyl)silyl]oxy]-4,4,6,8,12,16-hexamethyl-16-((4-pyridyl)ethenyl)1-oxa-cyclohexadec-13-en-2,6-dion

Analog zu Beispiel 1aw werden aus 130 mg (0,18 mmol) der unter Beispiel 9g beschriebenen Verbindung 98 mg (0,14 mmol, 76%) der Titelverbindung erhalten.

### Beispiel 9

### (45,7R,8S,9S,13Z,16S(E))-4,8-Dihydroxy-5,5,7,9,13-pentamethyl-16-((4-pyridyl)ethenyl)-1-oxa-cyclohexadec-13-en-2,6-dion (A) und

### (45,7R,8S,9S,13E,165(E))-4,8-Dihydroxy-5,5,7,9,13-pentamethyl-16-((4-pyridyl)ethenyl)-1-oxa-cyclohexadec-13-en-2,6-dion (B)

Analog zu Beispiel 1 werden aus 98 mg (0,14 mmol) der unter Beispiel 9h beschriebenen Verbindung 24 mg (49 µmol, 35%) der Titelverbindung A sowie 21 mg (43 µmol, 31%) der Titelverbindung B erhalten.

### Beispiel 10

### (1S,3S(E),7S,10R,11S,12S,16R)-7,11-Dihydroxy-8,8,10,12,16-pentamethyl-3-((4-pyridyl)ethenyl)-4,17-dioxabicyclo[14.1.0]heptadecan-5,9-dion (A)und (1S,3S(E),7S,10R,11S,12S,16S)-7,11-Dihydroxy-8,8,10,12,16-pentamethyl-3-((4-pyridyl)ethenyl)-4,17-dioxabicyclo[14.1.0]heptadecan-5,9-dion (B)

Analog zu Beispiel 2 werden aus 18 mg (37 µmol) der unter Beispiel 9 beschriebenen Verbindung A 11 mg (22 µmol, 59%) der Titelverbindung A bzw. aus sowie 15 mg (31 µmol) Verbindung B 9 mg (18 µmol, 58%) der Titelverbindung B erhalten.

### Beispiel 11

### (1S,3S(E),7S,10R,11S,12S,16R)-7,11-Dihydroxy-3-(1-methyl-2-(3-N-oxido-2-methyl-4-thiazolyl)ethenyl)-10-ethyl-8,8,12,16-tetramethyl-4,17-dioxabicyclo[14.1.0]heptadecan-5,9-dion

In Analogie zu Beispiel 2 setzt man 10 mg (19 µmol) der nach Beispiel 2 dargestellten Verbindung A bei 23°C um und isoliert nach Aufarbeitung und Reinigung 3,5 mg (6,5 µmol, 34%) der Titelverbindung als farbloses Öl.
¹H-NMR (CDCl₃): δ= 0,90 (3H), 1,03 (3H), 1,07 (3H), 1,10-2,03 (9H), 1,31 (3H), 1,43 (3H), 2,03 (1H), 2,09 (3H), 2,19-2,26 (2H), 2,52 (1H), 2,61 (3H), 2,68-2,81 (2H), 3,34 (1H), 3,65 (1H), 4,59 (1H), 5,39 (1H), 6,61 (1H), 6,81 (1H), 7,08 (1H) ppm.

### Beispiel 12

### (4S,7R,8S,9S,13Z,16S(E))-4,8-Dihydroxy-7-benzyl-16-(1-methyl-2-(2-methyl-4-thiazolyllethenyl)-1-oxa-5,5,9,13-tetramethyl-cyclohexadec-13-en-2,6-dion

### Beispiel 12a

### (4S)-4-((3RS)-2-Methyl-3-hydroxy-5-phenyl-pent-2-yl)-2,2-dimethyl-[1,3]dioxan

In Analogie zu Beispiel 11 setzt man 2,97 g (15,9 mmol) der nach Beispiel 1 k dargestellten Verbindung unter Verwendung von Phenethylmagnesiumbromid um und isoliert nach Aufarbeitung und Reinigung 3,27 g (11,2 mmol, 70%) der Titelverbindung als farbloses Öl.
¹H-NMR (CDCl₃): δ= 0,72+0,88 (3H), 0,89+0,93 (3H), 1,33 (1H), 1,39+1,42 (3H), 1,47+1,50 (3H), 1,58-1,93 (3H), 2,61 (1H), 3,00 (1H), 3,48-3,60 (1H), 3,72-4,03 (4H), 7,13-7,35 (5H) ppm.

### Beispiel 12b

### (4S)-4-(2-Methyl-3-oxo-5-phenyl-pent-2-yl)-2,2-dimethyl-[1,3]dioxan

In Analogie zu Beispiel 1m setzt man 2,71 g (9,3 mmol) der nach Beispiel 12a dargestellten Verbindung um und isoliert nach Aufarbeitung und Reinigung 2,35 g (8,1 mmol, 87%) der Titelverbindung als farbloses Öl.
¹H-NMR (CDCl₃): δ= 1,03 (3H), 1,12 (3H), 1,28 (1H), 1,31 (3H), 1,38 (3H), 1,60 (1H), 2,77-2,92 (4H), 3,83 (1H), 3,93 (1H), 4,02 (1H), 7,12-7,22 (3H), 7,22-7,32 (2H) ppm.

### Beispiel 12c

### (4S(4R,5S,6S,10RS))-4-(2,6-Dimethyl-10-[[(1,1-dimethylethyl)diphenylsilyl]oxy]-4-benzyl-5-hydroxy-3-oxo-undec-2-yl)-2,2-dimethyl-[1,3]dioxan (A) und (4S(4S,5R,6S,10RS))-4-(2,6-Dimethyl-10-[[(1,1-dimethylethyl)diphenylsilyl]oxy]-4-benzyl-5-hydroxy-3-oxo-undec-2-yl)-2,2-dimethyl-[1,3]dioxan (B)

In Analogie zu Beispiel 1ak setzt man 2,34 g (8,06 mmol) der nach Beispiel 12b dargestellten Verbindung um und isoliert nach Aufarbeitung und Reinigung 2,91 g (4,32 mmol, 54%) der Titelverbindung A sowie 1,72 g (2,55 mmol, 32%) der Titelverbindung B jeweils als farbloses Öl.
¹H-NMR (CDCl₃) von A: δ= 0,38 (3H), 0,83-1,82 (31H), 2,66-3,02 (3H), 3,47 (1H), 3,58 (1H), 3,74-3,94 (4H), 7,05-7,28 (5H), 7,31-7,46 (6H), 7,61-7,72 (4H) ppm.
¹H-NMR (CDCl₃) von B: δ= 0,78 (3H), 0,82-1,66 (21H), 0,98 (3H), 1,29 (3H), 1,36 (3H), 2,78 (1H), 2,94 (1H), 3,05 (1H), 3,44 (1H), 3,54 (1H), 3,72-3,91 (4H), 7,04-7,29 (5H), 7,31-7,48 (6H), 7,63-7,75 (5H) ppm.

### Beispiel 12d

### (4S(4R,5S,6S,10RS))-4-(2,6-Dimethyl-10-[[(1,1-dimethylethyl)diphenylsilyl]oxy]-4-benzyl-3-oxo-5-(tetrahydropyran-2-yloxy)-undec-2-yl)-2,2-dimethyl-[1,3]dioxan

In Analogie zu Beispiel 1a setzt man 2,90 g (4,4 mmol) der nach Beispiel 12c dargestellten Verbindung A um und isoliert nach Aufarbeitung und Reinigung 3,18 g (4,2 mmol, 95%) der Titelverbindung als farbloses Öl.

### Beispiel 12e

### (4S(4R,5S,6S,1 ORS))-4-(2,6-Dimethyl-4-benzyl-10-hydroxy-3-oxo-5-(tetrahydropyran-2-yloxy)-undec-2-yl)-2,2-dimethyl-[1,3]dioxan

In Analogie zu Beispiel 1i setzt man 3,18 g (4,20 mmol) der nach Beispiel 12d dargestellten Verbindung um und isoliert nach Aufarbeitung und Reinigung 1,39 g (2,68 mmol, 64%) der Titelverbindung als farbloses Öl.
¹H-NMR (CDCl₃): δ= 0,28+0,47+0,49 (3H), 0,92-1,14 (7H), 1,14-1,95 (24H), 2,79+2,99-3,13 (2H), 3,34-4,27 (8H), 4,45+4,56 (1H), 7,05-7,29 (5H) ppm.

### Beispiel 12f

### (4S(4R,5S,6S))-4-(2,6-Dimethyl-4-benzyl-3,10-dioxo-5-(tetrahydropyran-2-yloxy)-undec-2-yl)-2,2-dimethyl-[1,3]dioxan

In Analogie zu Beispiel 1m setzt man 1,39 g (2,68 mmol) der nach Beispiel 12e dargestellten Verbindung um und isoliert nach Aufarbeitung und Reinigung 1,18 g (2,28 mmol, 85%) der Titelverbindung als farbloses Öl.
¹H-NMR (CDCl₃): δ= 0,26+0,47 (3H), 0,96-1,11 (7H), 1,27+1,31 (3H), 1,39+1,41 (3H), 1,20-1,90 (12H), 2,15 (3H), 2,45 (2H), 2,79+2,97-3,12 (2H), 3,36-4,07 (6H), 4,15+4,21 (1H), 4,43+4,54 (1H), 7,08-7,28 (5H) ppm.

### Beispiel 12g

### (4S(4R,5S,6S,10E/Z,13S,14E))-4-(13-[[(1,1-Dimethylethyl)diphenylsilyl]oxy]-4-benzyl-15-(2-methyl-4-thiazolyl)-3-oxo-5-(tetrahydropyran-2-yloxy)-2,6,10,14-tetramethyl-pentadeca-10,14-dien-2-yl)-2,2-dimethyl-[1,3]dioxan

In Analogie zu Beispiel 1ao setzt man 477 mg (923 µmol) der nach Beispiel 12f dargestellten Verbindung unter Verwendung von n-Butyllithium als Base um und isoliert nach Aufarbeitung und Reinigung 367 mg (393 µmol, 43%) der Titelverbindung als farbloses Öl.
¹H-NMR (CDCl₃): δ= 0,23+0,46 (3H), 0,92-1,10 (19H), 1,10-1,92 (22H), 1,99 (3H), 2,13-2,40 (2H), 2,70 (3H), 2,80+2,94-3,14 (2H), 3,35-4,25 (6H), 4,47+4,53 (1H), 4,98 (1H), 6,22 (1H), 6,77 (1H), 7,07-7,24 (5H), 7,25-7,45 (6H), 7,60-7,73 (4H) ppm.

### Beispiel 12h

### (4S(4R,5S,6S,10E/Z,13S,14E))-4-(4-Benzyl-13-hydroxy-15-(2-methyl-4-thiazolyl)-3-oxo-5-(tetrahydropyran-2-yloxy)-2,6,10,14-tetramethyl-pentadeca-10,14-dien-2-yl)-2,2-dimethyl-[1,3]dioxan

In Analogie zu Beispiel 1i setzt man 548 mg (586 µmol) der nach Beispiel 12g dargestellten Verbindung um und isoliert nach Aufarbeitung und Reinigung 330 mg (474 µmol, 81%) der Titelverbindung als farbloses Öl.
¹H-NMR (CDCl₃): δ= 0,25+,046 (3H), 0,92-1,10 (6H), 1,10-1,90 (13H); 1,28+1,32 (3H), 1,39+1,41 (3H), 1,68+1,74 (3H), 1,99-2,13 (2H), 2,06 (3H), 2,36 (2H), 2,71 (3H), 2,81+3,00-3,14 (2H), 3,37-4,26 (9H), 4,48+4,57 (1H), 5,20 (1H), 6,58 (1H), 6,94 (1H), 7,08-7,26 (5H) ppm.

### Beispiel 12i

### 3S,6R,7S,8S,12E/Z,15S,16E)-6-Benzyl-17-(2-methyl-4-thiazolyl)-5-oxo-4,4,8,12,16-pentamethyl-heptadeca-12,16-dien-1,3,7,15-tetraol

In Analogie zu Beispiel 1f setzt man 330 mg (474 µmol) der nach Beispiel 12h dargestellten Verbindung um und isoliert nach Aufarbeitung und Reinigung 224 mg (392 µmol, 83%) der Titelverbindung als farbloses Öl.
¹H-NMR (CDCl₃): δ= 0,40 (3H), 0,93-1,04 (6H), 1,08-1,87 (8H), 1,63+1,71 (3H), 1,92-2,11 (5H), 2,33 (2H), 2,67-3,06 (3H), 2,72 (3H), 3,11 (1H), 3,23-3,50 (2H), 3,54 (1H), 3,65-3,92 (3H), 4,13 (1H), 5,18 (1H), 6,53 (1H), 6,94 (1H), 7,06-7,29 (5H) ppm.

### Beispiel 12k

### (3S,6R,7S,8S,12E/Z,15S,16E)-6-Benzyl-17-(2-methyl-4-thiazolyl)-4,4,8,12,16-pentamethyl-1,3,7,15-tetrakis-[[dimethyl(1,1-dimethylethyl)silyl]oxy]-heptadeca-12,16-dien-5-on

In Analogie zu Beispiel 1 aq setzt man 224 mg (392 µmol) der nach Beispiel 12i dargestellten Verbindung um und isoliert nach Aufarbeitung und Reinigung 323 mg (314 µmol, 80%) der Titelverbindung als farbloses Öl.
¹H-NMR (CDCl₃): δ= -0,03-0,12 (24H), 0,79-1,73 (53H), 1,61+1,69 (3H), 1,91-2,07 (2H), 2,00 (3H), 2,26 (2H), 2,71 (3H), 2,86 (1H), 2,98 (1H), 3,33-3,55 (2H), 3,66 (1H), 3,80 (1H), 4,10 (1H), 5,17 (1H), 6,47 (1H), 6,91 (1H), 7,06-7,29 (H) ppm.

### Beispiel 121

### (3S,6R,7S,8S,12E/Z,15S,16E)-6-Benzyl-1-hydroxy-17-(2-methyl-4-thiazolyl)-4,4,8,12,16-pentamethyl-3,7,15-tris-[[dirnethyl(1,1-dimethylethyl)silyl]oxy]-heptadeca-12,16-dien-5-on

In Analogie zu Beispiel 1ar setzt man 432 mg (420 µmol) der nach Beispiel 12k dargestellten Verbindung um und isoliert nach Aufarbeitung und Reinigung 264 mg (289 µmol, 69%) der Titelverbindung als farbloses Öl.
¹H-NMR (CDCl₃): δ= -0,03-0,12 (18H), 0,53 (1H), 0,78-1,40 (41H), 1,62+1,71 (3H), 1,42-1,81 (2H), 2,00 (3H), 1,92-2,10 (2H), 2,27 (2H), 2,70 (3H), 2,852 (1H), 3,09 (1H), 3,30 (2H), 3,40 (1H), 3,70 (1H), 3,81 (1H), 4,11 (1H), 5,17 (1H), 6,46 (1H), 6,91 (1H), 7,11-7,30 (5H) ppm.

### Beispiel 12m

### (3S,6R,7S,8S,12E/Z,15S,16E)-6-Benzyl-3,7,15-tris-[[dimethyl(1,1-dimethylethyl)silyl]oxy]-4,4,8,12,16-pentamethyl-17-(2-methyl-4-thiazolyl)-5-oxo-heptadeca-1 2,16-diensäure

In Analogie zu Beispiel 1 k setzt man 264 mg (289 µmol) der nach Beispiel 121 dargestellten Verbindung um und isoliert nach Aufarbeitung 255 mg (279 µmol, 97%) der Titelverbindung als farbloses Öl, das man ohne Reinigung weiter umsetzt.

### Beispiel 12n

### (3S,6R,7S,8S,12Z,15S,16E)-6-Benzyl-17-(2-methyl-4-thiazolyl)-5-oxo-4,4,8,12,16-pentamethyl-3,7,15-tris-[[dimethyl(1,1-dimethylethyl)silyl]oxy]-heptadeca-12,16-diensäure (A) und (3S,6R,7S,8S,12E,15S,16E)-6-Benzyl-17-(2-methyl-4-thiazolyl)-5-oxo-4,4,8,12,16-pentamethyl-3,7,15-tris-[[dimethyl(1,1-dimethylethyl)silyl]oxy]-heptadeca-12,16-diensäure (B)

In Analogie zu Beispiel 1 at setzt man 255 mg (279 µmol) der nach Beispiel 12m dargestellten Verbindung um und isoliert nach Aufarbeitung und Reinigung 61 mg (66 µmol, 24%) der Titelverbindung A als farblosen Feststoff sowie 54 mg (58 µmol, 21%) der Titelverbindung B als farbloses Öl.
¹H-NMR (CDCl₃) von A: δ= -0,07-0,18 (18H), 0,60 (3H) 0,78 (3H), 0,82 (9H), 0,89 (9H), 0,92 (9H), 1,07 (3H), 1,72 (3H), 1,95 (3H), 0,74-2,33 (12H), 2,69 (3H), 2,91 (1H) 3,03 (1H), 3,41 (1H), 3,62 (1H), 4,20 (1H), 4,30 (1H), 5,23 (1H), 6,72 (1H), 6,96 (1H), 7,05-7,29 (5H) ppm.
¹H-NMR (CDCl₃) von B: δ= -0,08-0,14 (18H), 0,72 (3H), 0,82 (3H), 0,85 (9H), 0,90 (9H), 0,93 (9H), 0,98 (3H), 1,60 (3H), 0,65-2,08 (9H), 1,96 (3H), 2,12 (1H), 2,29 (2H), 2,71 (3H), 2,92 (2H), 3,47 (1H), 3,69 (1H), 4,09 (1H), 4,21 (1H), 5,12 (1H), 6,49 (1H), 6,95 (1H), 7,06-7,30 (5H) ppm.

### Beispiel 12o

### (3S,6R,7S,8S,12Z,15S,16E)-6-Benzyl-15-hydroxy-17-(2-methyl-4-thiazolyl)-5-oxo-4,4,8,12,16-pentamethyl-3,7-bis-[[dimethyl(1,1 -dimethylethyl)silyl]oxy]-heptadeca-12,16-diensäure

In Analogie zu Beispiel 1i setzt man 61 mg (66 µmol) der nach Beispiel 12n dargestellten Verbindung A bei 23°C um und isoliert nach Aufarbeitung und Reinigung 33 mg (41 µmol, 61%) der Titelverbindung als farbloses Öl.
¹H-NMR (CDCl₃): δ= -0,11 (3H), -0,08-0,05 (9H), 0,80 (9H), 0,88 (9H), 0,91 (3H), 0,94 (3H), 0,99 (3H), 1,72 (3H), 1,98 (3H), 0,77-2,22 (12H), 2,69 (3H), 2,70-2,91 (2H), 3,39 (1H), 3,62 (1H), 4,18 (1H), 4,33 (1H), 4,43-5,73 (1H), 5,13 (1H), 6,68 (1H), 6,91 (1H), 7,05-7,26 (5H) ppm.

### Beispiel 12p

### (4S,7R,8S,9S,13Z,16S(E))-4,8-Bis-[[dimethyl(1,1-dimethylethyl)silyl]oxy]-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1 aw setzt man 33 mg (40 µmol) der nach Beispiel 12o dargestellten Verbindung um und isoliert nach Aufarbeitung und Reinigung 17 mg (21 µmol, 53%) der Titelverbindung als farbloses Öl.
¹H-NMR (CDCl₃): δ= -0,06 (3H), 0,00 (3H), 0,07 (3H), 0,09 (3H), 0,98 (3H), 1,71 (3H), 2,10 (3H), 0,70-2,48 (34H), 2,63 (1H), 2,71 (3H), 2,81 (2H), 3,23 (1H), 3,76 (1H), 4,17 (1H), 5,13 (2H), 6,56 (1H), 6,95 (1H), 7,06-7,32 (5H) ppm.

### Beispiel 12

### (4S,7R,8S,9S,13Z,16S(E))-4,8-Dihydroxy-7-benzyl-6-(1-methy)-2-(2-methyl-4-thiazolyl)ethenyl)-1-oxa-5,5,9,13-tetramethyl-cyclohexadec-13-en-2,6-dion

In Analogie zu Beispiel 1 setzt man 12,2 mg (9,7 µmol) der nach Beispiel 12p dargestellten Verbindung um und isoliert nach Aufarbeitung und Reinigung 5,0 mg (8,8 µmol, 91%) der Titelverbindung als farbloses Öl.
¹H-NMR (CDCl₃): δ= 0,61 (3H), 0,83 (3H), 1,11 (3H), 1,22-2,00 (5H), 1,71 (3H), 2,05 (3H), 2,19-2,49 (5H), 2,61 (1H), 2,66 (3H), 2,89 (1H), 3,03 (1H), 3,59 (1H), 3,67 (1H), 4,21 (1H), 5,10 (1H), 5,24 (1H), 6,53 (1H), 6,92 (1H), 7,07-7,31 (5H) ppm.

### Beispiel 13

### (4S,7R,8S,9S,13E,16S(E1)-4,8-Dihydroxy-7-benzyl-16-(1-methyl-2-(2-methyl-4-thiazolyl)ethenyl)-1-oxa-5,5,9,13-tetramethyl-cyclohexadec-13-en-2,6-dion

### Beispiel 13a

### (3S,6R,7S,8S,12E,15S,16E)-6-Benzyl-15-hydroxy-17-(2-methyl-4-th iazolyl)-5-oxo-4,4,8,12,16-pentamethyl-3,7-bis-[[dimethyl(1,1-diniethylethyl)silyl]oxy]-heptadeca-12,16-diensäure

In Analogie zu Beispiel 1i setzt man 47 mg (51 µmol) der nach Beispiel 12n dargestellten Verbindung B bei 23°C um und isoliert nach Aufarbeitung und Reinigung 22 mg (27 µmol, 53%) der Titelverbindung als farbloses Öl.
¹H-NMR (CDCl₃): δ= -0,08 (3H), -0,03-0,09 (9H), 0,82 (9H), 0,89 (12H), 0,97 (6H), 1,64 (3H), 2,02 (3H), 0,78-2,10 (9H), 2,27-2,46 (2H), 2,70 (3H), 2,82 (2H), 2,92-3,34 (2H), 3,42 (1H), 3,67 (1H), 4,19 (1H), 4,32 (1H), 5,28 (1H), 6,63 (1H), 6,92 (1H), 7,02-7,27 (5H) ppm.

### Beispiel 13b

### (45,7R,85,95,13E,16S(E))-4,8-Bis-[[dimethyl(1,1-dimethylethyl)silyl]oxy]-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1 aw setzt man 22 mg (27 µmol) der nach Beispiel 13a dargestellten Verbindung um und isoliert nach Aufarbeitung und Reinigung 12 mg (15 µmol, 56%) der Titelverbindung als farbloses Öl.
¹H-NMR (CDCl₃): δ= -0,04 (3H), 0,06 (6H), 0,12 (3H), 0,80 (3H), 0,88 (9H), 0,90 (9H), 0,96 (3H), 1,08 (3H), 1,64 (3H), 0,74-1,72 (4H), 1,80-2,27 (5H), 2,09 (3H), 2,33 (1H), 2,53-2,82 (2H), 2,70 (3H), 2,96 (1H), 3,20 (1H), 3,74 (1H), 4,15 (1H), 5,19-5,32 (2H), 6,47 (1H), 6,90 (1H), 7,07-7,31 (5H) ppm.

### Beispiel 13

### (4S,7R,8S,9S,13E,16S(E))-4,8-Dihydroxy-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1 setzt man 12 mg (15 µmol) der nach Beispiel 13b dargestellten Verbindung um und isoliert nach Aufarbeitung und Reinigung 6,0 mg (11 µmol, 69%) der Titelverbindung als farbloses Öl.
¹H-NMR (CDCl₃): δ= 0,69 (3H), 0,72 (3H), 0,89 (1H), 1,08 (3H), 1,38-1,69 (3H), 1,61 (3H), 1,90-2,12 (2H), 2,02 (3H), 2,19 (1H), 2,25-2,44 (3H), 2,54 (1H), 2,69 (3H), 2,79 (1H), 2,99 (1H), 3,73 (2H), 4,25-4,39 (2H), 4,66 (1H), 5,03 (1H), 5,34 (1H), 6,52 (1H), 6,97 (1H), 7,04-7,29 (5H) ppm.

### Beispiel 14

### (1S,3S(E),7S,10R,11S,12S,16R)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5,9-dion (A) und (1R,3S(E),7S,10R,11S,12S,16S)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5,9-dion (B)

Die Lösung von 4,0 mg (7,0 µmol) der nach Beispiel 12 dargestellten Verbindung in 0,1 ml Acetonitril versetzt man mit 38 µl einer 1 M Lösung von Natriumethylendiamin-tetracetat, kühlt auf 0°C und versetzt mit 67 µl 1,1,1-Trifluoraceton sowie einem Gemisch aus 21 mg Oxon und 4,5 mg Natriumhydrogencarbonat. Man läßt 5 Stunden reagieren, gießt auf Natriumthiosulfatlösung und extrahiert mehrfach mit Ethylacetat. Die vereinigten organischen Extrakte wäscht man mit gesättigter Natriumchloridlösung und reinigt den nach Filtration und Lösungsmittelabzug erhaltenen Rückstand durch Chromatographie an einer analytischen Dünnschichtplatte. Als Laufmittel dient ein Gemisch aus n-Hexan und Ethylacetat. Isoliert werden 2,2 mg (3,8 µmol, 54%) der Titelverbindung A sowie 0,3 mg (0,5 µmol, 7%) der Titelverbindung B jeweils als farbloses Öl.
¹H-NMR (CDCl₃) von A: δ= 0,67 (3H), 0,80 (3H), 1,07 (3H), 1,29 (3H), 1,35-2,06 (9H), 2,09 (3H), 2,33 (1H), 2,49 (1H), 2,68 (3H), 2,72-2,85 (2H), 3,04 (1H), 3,40 (1H), 3,62 (1H), 3,77 (1H), 4,22 (1H), 4,51 (1H), 5,47 (1H), 6,51 (1H), 6,95 (1H), 7,06-7,30 (5H) ppm.
¹H-NMR (CDCl₃) von B: δ= 0,68 (3H), 0,76 (3H), 0,86 (1H), 1,07 (3H), 1,23-2,13 (7H), 1,30 (3H), 2,08 (3H), 2,30-2,49 (2H), 2,70 (3H), 2,87-3,11 (3H), 3,28 (2H), 3,57 (1H), 3,93 (1H), 4,21 (1H), 4,54-5,73 (1H), 5,58 (1H), 6,58 (1H), 6,97 (1H), 7,07-7,31 (5H) ppm.

### Beispiel 15

### (1S,3S(E),7S,10R,11S,12S,16S)-10-Benzyl-7,11-dihydroxy-3-(1-methyl-2-(2-methyl-4-thiazolyl)ethenyl)-8,8,12,16-tetramethyl-4,1 7-dioxabicyclo[14.1.0]heptadecan-5,9-dion (A) und (1R,3S(E),7S,10R,11S,12S,16R)-10-Benzyl-7,11-dihydroxy-3-(1-methyl-2-12-methyl-4-thiazolyl)ethenyl)-8,8,12,16-tetramethyl-4,17-dioxabicyclo[14.1.0]heptadecan-5,9-dion (B)

In Analogie zu Beispiel 14 setzt man 3,1 mg (5,4 µmol) der nach Beispiel 13 dargestellten Verbindung um und isoliert nach Aufarbeitung und Reinigung 0,7 mg (1,2 µmol, 22%) der Titelverbindung A oder B sowie 0,6 mg (1,0 µmol, 19%) der Titelverbindung B oder A als farbloses Öl.
¹H-NMR (CDCl₃) von A oder B: δ= 0,76 (3H), 0,88 (3H), 1,02 (3H), 1,24 (1H), 1,30 (3H), 1,38-1,78 (5H), 1,92-2,13 (3H), 2,07 (3H), 2,44 (2H), 2,70 (3H), 2,78-2,87 (2H), 3,04 (1H), 3,60 (1H), 3,71-3,80 (2H), 4,01 (1H), 4,28 (1H), 5,45 (1H), 6,62 (1H), 6,99 (1H), 7,11-7,31 (5H) ppm.
¹H-NMR (CDCl₃) von B oder A: δ= 0,70 (3H), 0,76 (3H), 1,06 (3H), 1,19-1,64 (5H), 1,22 (3H), 1,80 (1H), 1,90-2,12 (3H), 2,07 (3H), 2,46 (2H), 2,69 (3H), 2,79 (1H), 2,92 (1H), 3,08 (1H), 3,32 (1H), 3,57 (1H), 3,62 (1H), 3,71 (1H), 4,12 (1H), 5,42 (1H), 6,54 (1H), 6,96 (1H), 7,06-7,31 (5H) ppm.

### Beispiel 16

### (4S,7S,8R,9S,13Z,16S(E))-4,8-Dihydroxy-7-benzyl-16-(1-methyl-2-(2-methyl-4-thiazolyl)ethenyl)-1-oxa-5,5,9,13-tetramethyl-cyclohexadec-13-en-2,6-dion

### Beispiel 16a

### (4S(4S,5R,6S,10RS))-4-(2,6-Dimethyl-10-[[(1,1-dimethylethyl)diphenylsilyl]oxy]-4-benzyl-3-oxo-5-(tetrahydropyran-2-yloxy)-undec-2-yl)-2,2-dimethyl-[1,3]dioxan

In Analogie zu Beispiel 1a setzt man 1,71 g (2,59 mmol) der nach Beispiel 12c dargestellten Verbindung B um und isoliert nach Aufarbeitung und Reinigung 1,51 g (1,99 mmol, 77%) der Titelverbindung als farbloses Öl.

### Beispiel 16b

### (4S(4S,5R,6S,10RS))-4-(2,6-Dimethyl-4-benzyl-10-hydroxy-3-oxo-5-(tetrahydropyran-2-yloxy)-undec-2-yl)-2,2-dimethyl-[1,3]dioxan

In Analogie zu Beispiel 1i setzt man 1,51 g (1,99 mmol) der nach Beispiel 16a dargestellten Verbindung um und isoliert nach Aufarbeitung und Reinigung 855 mg (1,65 mmol, 83%) der Titelverbindung als farbloses Öl.
¹H-NMR (CDCl₃): δ= 0,88+0,92 (3H), 0,92-1,95 (32H), 2,82-3,10 (2H), 3,32-3,59 (2H), 3,71-3,98 (5H), 4,43-4,59 (1H), 7,11-7,31 (5H) ppm.

### Beispiel 16c

### (4S(4S,5R,6S))-4-(2,6-Dimethyl-4-benzyl-3,10-dioxo-5-(tetrahydropyran-2-yloxy)-undec-2-yl)-2,2-dimethyl-[1,3]dioxan

In Analogie zu Beispiel 1m setzt man 850 mg (1,64 mmol) der nach Beispiel 16b dargestellten Verbindung um und isoliert nach Aufarbeitung und Reinigung 741 mg (1,43 mmol, 88%) der Titelverbindung als farbloses Öl.
¹H-NMR (CDCl₃): δ= 0,84+0,90 (3H), 0,95+1,05 (3H), 0,97 (3H), 1,8-1,88 (19H), 2,15 (3H), 2,42 (2H), 2,79-3,08 (2H), 3,31-3,57 (2H), 3,69-3,96 (5H), 4,43+4,52 (1H), 7,10-7,29 (5H) ppm.

### Beispiel 16d

### (4S(4S,5R,6S,10E/Z,13S,14E))-4-(13-[[(1,1-Dimethylethyl)diphenylsilyl]oxy]-4-benzyl-15-(2-methyl-4-thiazolyl)-3-oxo-5-(tetrahydropyran-2-yloxy)-2,6,10,14-tetramethyl-pentadeca-10,14-dien-2-yl)-2,2-dimethyl-[1,3]dioxan

In Analogie zu Beispiel 1ao setzt man 737 mg (1,43 mmol) der nach Beispiel 16c dargestellten Verbindung unter Verwendung von n-Butyllithium als Base um und isoliert nach Aufarbeitung und Reinigung 491 mg (525 µmol, 37%) der Titelverbindung als farbloses Öl.

### Beispiel 16e

### (4S(4S,5R,6S,10E/Z,13S,14E))-4-(4-Benzy)-13-hydroxy-15-(2-methy)-4-thiazolyl)-3-oxo-5-(tetrahydropyran-2-yloxy)-2,6,10,14-tetramethyl-pentadeca-10,14-dien-2-yl)-2,2-dimethyl-[1,3]dioxan

In Analogie zu Beispiel 1i setzt man 1,09 g (1,17 mmol) der nach Beispiel 16d dargestellten Verbindung um und isoliert nach Aufarbeitung und Reinigung 677 mg (973 µmol, 83%) der Titelverbindung als farbloses Öl.
¹H-NMR (CDCl₃): δ= 0,78-2,12 (31H), 1,67+1,73 (3H), 2,06 (3H), 2,36 (2H), 2,71 (3H), 2,81-3,08 (2H), 3,30-3,52 (2H), 3,69-3,96 (5H), 4,14 (1H), 4,43+4,51 (1H), 5,20 (1H), 6,57 (1H), 6,95 (1H). 7,08-7,30 (5H) ppm.

### Beispiel 16f

### (3S,6S,7R,8S,12E/Z,15S,16E)-6-Benzyl-17-(2-methyl-4-thiazolyl)-5-oxo-4,4,8,12,16-pentamethyl-heptadeca-12,16-dien-1,3,7,15-tetraol

In Analogie zu Beispiel 1f setzt man 675 mg (970 µmol) der nach Beispiel 16e dargestellten Verbindung um und isoliert nach Aufarbeitung und Reinigung 495 mg (866 µmol, 89%) der Titelverbindung als farbloses Öl.
¹H-NMR (CDCl₃): δ= 0,73-0,86 (6H), 0,96-1,10 (3H), 1,21-1,79 (7H), 1,67+1,76 (3H), 1,98-2,17 (5H), 2,28-2,50 (3H), 2,70 (3H), 2,85 (1H). 2,97 (1H), 3,09 (1H), 3,40-3,87 (7H), 4,16 (1H). 5,27 (1H), 6,51+6,57 (1H), 6,94 (1H), 7,07-7,30 (5H) ppm.

### Beispiel 16g

### (3S,6S,7R,8S,12E/Z,15S,16E)-6-Benzyl-17-(2-methyl-4-thiazolyl)-4,4,8,12,16-pentamethyl-1,3,7,15-tetrakis-[[dimethyl(1, 1-dimethylethyl)silyl]oxy]-heptadeca-12, 16-dien-5-on

In Analogie zu Beispiel 1aq setzt man 337 mg (589 µmol) der nach Beispiel 16f dargestellten Verbindung um und isoliert nach Aufarbeitung und Reinigung 444 mg (432 µmol, 73%) der Titelverbindung als farbloses Öl.
¹H-NMR (CDCl₃): δ= -0,08-0,13 (24H), 0,42 (3H), 0,79-1,03 (42H), 1,11-1,73 (8H), 1,60+1,67 (3H), 1,90-2,08 (4H), 2,26 (2H), 2,71 (3H), 2,91 (2H), 3,22 (1H), 3,50-3,72 (3H), 3,85 (1H), 4,09 (1H), 5,16 (1H), 6,46 (1H), 6,91 (1H), 7,07-7,27 (5H) ppm.

### Beispiel 16h

### (3S,6S,7R,8S,12E/Z,15S,16E)-6-Benzyl-1-hydroxy-17-(2-methyl-4-thiazolyl)-4,4,8,12,16-pentamethyl-3,7,15-tris-[[dimethyl(1,1-dimethylethyl)silyl]oxy]-heptadeca-12,16-dien-5-on

In Analogie zu Beispiel 1ar setzt man 444 mg (432 µmol) der nach Beispiel 16g dargestellten Verbindung um und isoliert nach Aufarbeitung und Reinigung 272 mg (297 µmol, 69%) der Titelverbindung als farbloses Öl.
¹H-NMR (CDCI₃): δ= -0,07-0,18 (18H), 0,48 (3H), 0,79-1,72 (40H), 1,61+1,68 (3H), 1,81 (1H), 1,90-2,09 (5H), 2,26 (2H), 2,70 (3H), 2,86-3,04 (2H), 3,23 (1H), 3,59 (2H), 3,70 (1H), 3,91 (1H), 4,10 (1H), 5,16 (1H), 6,44 (1H), 6,91 (1H), 7,08-7,29 (5H) ppm.

### Beispiel 16i

### (3S,6S,7R,8S,12E/Z,15S,16E)-6-Benzyl-3,7,15-tris-[[dimethyl(1,1-dimethylethyl)silyl]oxy]-4,4,8,12,16-pentamethyl-17-(2-methyl-4-thiazolyl)-5-oxo-heptadeca-12,16-diensäure

In Analogie zu Beispiel 1k setzt man 272 mg (297 µmol) der nach Beispiel 16h dargestellten Verbindung um und isoliert nach Aufarbeitung 264 mg (289 µmol, 97%) der Titelverbindung als farbloses Öl, das man ohne Reinigung weiter umsetzt.

### Beispiel 16k

### (3S,6S,7R,8S,12Z,15S,16E)-6-Benzyl-17-(2-methyl-4-thiazolyl)-5-oxo-4,4,8,12,16-pentamethyl-3,7,15-tris-[[dimethyl(1,1-dimethylethyl)silyl]oxy]-heptadeca-12,16-diensäure (A) und (3S,6S,7R,8S,12E,15S,16E)-6-Benzyl-17-(2-methyl-4-thiazolyl)-5-oxo-4,4,8,12,16-pentamethyl-3,7,15-tris-[[dimethyl(1,1-dimethylethyl)silyl]oxy]-heptadeca-12,16-diensäure (B)

In Analogie zu Beispiel 1at setzt man 264 mg (289 µmol) der nach Beispiel 16i dargestellten Verbindung um und isoliert nach Aufarbeitung und Reinigung 87 mg (94 µmol, 32%) der Titelverbindung A sowie 67 mg (73 µmol, 25%) der Titelverbindung B jeweils als farbloses Öl.
¹H-NMR (CDCl₃) von A: δ= -0,09 (3H), -0,02-0,13 (15H), 0,69 (3H), 0,80-1,48 (32H), 1,03 (3H), 1,63-1,79 (1H), 1,68 (3H), 2,00 (3H), 1,91-2,09 (2H), 2,12-2,33 (3H), 2,72 (3H), 2,77-3,20 (6H), 3,31 (1H), 3,70 (1H), 4,10 (1H), 4,43 (1H), 5,16 (1H), 6,47 (1H), 6,91 (1H), 7,08-7,29 (5H) ppm.
¹H-NMR (CDCl₃) von B: δ= -0,10 (3H), -0,03-0,17 (15H), 0,68 (3H), 0,80-1,50 (33H), 1,02 (3H), 1,61 (3H), 1,71 (2H), 1,88-2,07 (2H), 2,00 (3H), 2,11-2,68 (4H), 2,71 (3H), 2,86 (2H), 3,30 (1H), 3,69 (1H), 3,75-4,08 (1H), 4,11 (1H), 4,43 (1H), 5,16 (1H), 6,47 (1H), 6,91 (1H), 7,08-7,30 (5H) ppm.

### Beispiel 16l

### (3S,6S,7R, 8S,12Z,15S,16E)-6-Benzyl-15-hydroxy-17-(2-methyl-4-thiazolyl)-5-oxo-4,4,8,12,16-pentamethyl-3,7-bis-[[dimethyl(1,1-dimethylethyl)silyl]oxy]-heptadeca-12,16-diensäure

In Analogie zu Beispiel 1i setzt man 87 mg (94 µmol) der nach Beispiel 16k dargestellten Verbindung A bei 23°C um und isoliert nach Aufarbeitung und Reinigung 76 mg (93 µmol, 99%) der Titelverbindung als farbloses Öl.
¹H-NMR (CDCl₃): δ= -0,03-0,13 (12H), 0,52 (3H), 0,78-1,80 (28H), 1,73 (3H), 1,91-2,17 (2H), 2,00 (3H), 2,21 (2H), 2,34 (2H), 2,69-3,01 (3H), 2,73 (3H), 3,19 (1H), 3,31 (1H), 3,74 (1H), 4,13 (1H), 4,28-5,68 (1H), 4,36 (1H), 5,18 (1H), 6,62 (1H). 6,97 (1H), 7,08-7,31 (5H) ppm.

### Beispiel 16m

### (4S,7S,8R,9S,13Z,16S(E))-4,8-Bis-[[dimethyl(1,1-dimethylethyl)silyl]oxy]-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1aw setzt man 76 mg (93 µmol) der nach Beispiel 161 dargestellten Verbindung um und isoliert nach Aufarbeitung und Reinigung 68 mg (85 µmol, 92%) der Titelverbindung als farbloses Öl.
¹H-NMR (CDCl₃): δ= -0,02 (3H), 0,01 (3H), 0,16 (3H), 0,30 (3H), 0,54 (3H), 0,64 (3H), 0,85 (9H), 0,97 (9H), 0,99 (3H), 0,80-1,75 (5H), 1,69 (3H), 1,89 (1H), 1,98-2,31 (3H), 2,13 (3H), 2,37 (1H), 2,52 (1H), 2,70 (1H), 2,72 (3H), 3,10 (1H), 3,46 (1H), 3,96 (1H), 4,05 (1H), 5,10 (1H), 5,15 (1H), 6,48 (1H), 7,02 (1H), 7,09-7,31 (5H) ppm.

### Beispiel 16

### (4S,7S,8R,9S,13Z,16S(E))-4,8-Dihydroxy-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1 setzt man 10 mg (13 µmol) der nach Beispiel 12p dargestellten Verbindung um und isoliert nach Aufarbeitung und Reinigung 6,3 mg (11 µmol, 89%) der Titelverbindung als farbloses Öl.
¹H-NMR (CDCl₃): δ= 0,47 (3H), 0,84 (1H), 0,97 (3H), 1,04 (3H), 1,22-1,70 (4H), 1,76 (3H), 1,94 (1H), 1,93 (3H), 2,22-2,49 (4H), 2,61-2,77 (1H), 2,71 (3H), 2,83 (1H), 2,90 (1H), 3,02 (1H), 3,08 (1H), 3,59 (1H), 3,62 (1H), 4,18 (1H), 5,19 (1H), 5,53 (1H), 6,50 (1H), 6,96 (1H), 7,08-7,31 (5H) ppm.

### Beispiel 17

### (4S,7S 8R,9S.13E 16S(E))-4.8-Dihydroxv-7-benzyl-16-(1-methyl-2-(2-methyl-4-thiazolyl)ethenyl)-1-oxa-5,5,9,13-tetramethyl-cyclohexadec-13-en-2,6-dion

### Beispiel 17a

### (3S,6S,7R,8S,12E,15S,16E)-6-Benzyl-15-hydroxy-17-(2-methyl-4-thiazolyl)-5-oxo-4,4,8,12,16-pentamethyl-3,7-bis-[[dimethyl(1,1-dimethylethyl)silyl]oxy]-heptadeca-12,16-diensäure

In Analogie zu Beispiel 1i setzt man 67 mg (72 µmol) der nach Beispiel 16k dargestellten Verbindung B bei 23°C um und isoliert nach Aufarbeitung und Reinigung 57 mg (70 µmol, 97%) der Titelverbindung als farbloses Öl.
¹H-NMR (CDCl₃): δ= -0,06-0,13 (12H), 0,47 (3H), 0,77-1,76 (28H), 1,64 (3H), 1,90-2,07 (2H), 2,00 (3H), 2,28 (2H), 2,39 (2H), 2,66-2,89 (2H), 2,73 (2H), 2,91-3,05 (3H), 3,19 (1H), 3,29 (1H), 3,76 (1H), 4,20 (1H), 4,36 (1H), 5,16 (1H), 6,58 (1H), 6,94 (1H), 7,07-7,31 (5H) ppm.

### Beispiel 17b

### (4S,7S,8R,9S,13E,16S(E))-4,8-Bis-[[dimethyl(1,1-dimethylethyl)silyl]oxy]-7-benzyl-16-(1-methyl-2-(2-rnethyl-4-thiazolyl)ethenyl)-1-oxa-5,5,9,13-tetramethyl-cyclohexadec-13-en-2,6-dion

In Analogie zu Beispiel 1aw setzt man 57 mg (70 µmol) der nach Beispiel 17a dargestellten Verbindung um und isoliert nach Aufarbeitung und Reinigung 32 mg (40 µmol, 57%) der Titelverbindung als farblosen Feststoff.
¹H-NMR (CDCl₃): δ= 0,07 (9H), 0,23 (3H), 0,53 (3H), 0,72 (3H), 0,88 (9H), 0,93 (9H), 0,98 (3H), 1,08-1,30 (2H), 1,39 (1H), 1,48-1,86 (3H), 1,61 (3H), 2,10 (3H), 2,07-2,27 (2H), 2,31-2,58 (3H), 2,63-2,78 (1H), 2,71 (3H), 3,08 (1H), 3,41 (1H), 3,82 (1H), 4,19 (1H), 5,08 (1H), 5,15 (1H), 6,51 (1H), 7,02 (1H), 7,08-7,30 (5H) ppm.

### Beispiel 17

### (4S,7S,8R,9S,13E,16S(E))-4,8-Dihydroxy-7-benzyl-16-(1-methyl-2-(2-methyl-4-thiazolyl)ethenyl)-1-oxa-5,5,9,13-tetramethyl-cyclohexadec-13-en-2,6-dion

In Analogie zu Beispiel 1 setzt man 32 mg (40 µmol) der nach Beispiel 17b dargestellten Verbindung um und isoliert nach Aufarbeitung und Reinigung 16,6 mg (29 µmol, 73%) der Titelverbindung als farbloses Öl.
¹H-NMR (CDCl₃): δ= 0,35 (3H), 0,91 (3H), 0,93 (3H), 1,61 (3H), 0,83-1,72 (5H), 1,94-2,20 (2H), 2,09 (3H), 2,32 (1H), 2,46 (1H), 2,51 (2H), 2,69 (3H), 2,90-3,02 (3H), 3,13 (1H), 3,55-3,68 (2H), 4,23 (1H), 5,11 (1H), 5,43 (1H), 6,47 (1H), 6,92 (1H), 7,07-7,31 (5H) ppm.

### Beispiel 18

### (1S,35(E),75,10S,11R,12S,16R)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5.9-dion (A) und (1R,3S(E)7S,10S,11R,12S,16S)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5.9-dion (B)

In Analogie zu Beispiel 14 setzt man 1,4 mg (2,5 µmol) der nach Beispiel 16 dargestellten Verbindung um und isoliert nach Aufarbeitung und Reinigung 0,3 mg (0,5 µmol, 21%) der Titelverbindungen A und B als farbloses Öl.
¹H-NMR (CDCl₃): δ= 0,27 (3H), 0,98 (3H), 1,08 (3H), 1,23 (3H), 1,15-2,46 (10H), 2,19 (3H), 2,71 (3H), 2,82 (1H), 2,91 (1H), 2,95 (1H), 3,10 (1H), 3,47 (1H), 3,95 (1H), 4,12 (1H), 4,42 (1H), 4,70-5,30 (1H), 5,60 (1H), 6,65 (1H), 7,00 (1H), 7,12-7,32 (5H) ppm.

### Beispiel 19

### (1S,3S(E),7S,10S,11R,12S,16S)-10-Benzyl-7,11-dihydroxy-3-(1-methyl-2-(2-methyl-4-thiazolyl)ethenyl)-8 8,12,16-tetramethyl-4,17-dioxabicyclo[14.1.0]heptadecan-5.9-dion (A) und (1R,3S(E),7S,10S 11R,12S,16R)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5.9-dion (B)

In Analogie zu Beispiel 14 setzt man 7,4 mg (13 µmol) der nach Beispiel 17 dargestellten Verbindung um und isoliert nach Aufarbeitung und Reinigung 1,9 mg (3,3 µmol, 25%) der Titelverbindung A sowie 1,7 mg (2,9 µmol, 22%) der Titelverbindung B jeweils als farbloses Öl.
¹H-NMR (CDCl₃) von A: δ= 0,40 (3H), 0,89 (3H), 0,97 (3H), 1,08-1,77 (6H), 1,22 (3H), 1,90-2,07 (3H), 2,08 (3H), 2,38 (1H), 2,57 (1H), 2,70 (3H), 2,83 (1H), 2,92-3,06 (3H), 3,19 (1H), 3,54 (1H), 3,77 (1H), 4,19 (1H), 5,53 (1H), 6,52 (1H), 6,97 (1H), 7,08-7,31 (5H) ppm.
¹H-NMR (CDCl₃) von B: δ= 0,17 (3H), 0,89 (3H), 1,00 (3H), 1,21-1,97 (8H), 1,28 (3H), 2,06 (1H), 2,10 (3H), 2,27-2,44 (3H), 2,71 (3H), 2,90 (1H), 2,99-3,11 (2H), 3,36 (1H), 3,96 (1H), 4,20 (1H), 4,29 (1H), 5,77 (1H), 6,57 (1H), 6,98 (1H), 7,08-7,31 (5H) ppm.

### Beispiel 20

### (4S,7R,8S,9S,13(Z),16S(E))-4,8-Dihydroxy-16-(1-methyl-2-(2-pyridyl)ethenyl)1-oxa-5,5,7,9,13-pentamethyl-cyclohexadec-13-en-2,6-dion

### Beispiel 20a

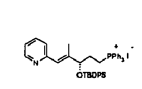

### (5E,3S)-[3-[[(1,1-dimethylethyl)diphenylsilyl]oxy]-4-methyl-5-(2-pyridyl)-pent-4-en-1-yl]-triphenylphosphoniumiodid

In Analogie zu den Beispielen 7a bis 7d erhält man unter Verwendung von Diethyl(2-pyridyl)methanphosphonat die Titelverbindung als kristallinen Feststoff.
¹H-NMR (CDCl₃): δ= 1,08 (9H), 1,70-1,95 (2H), 1,99 (1H), 3,00 (1H), 3,31 (1H), 4,59 (1H), 6,68 (1H), 7,10 (1H), 7,18-7,46 (8H), 7,50-7,74 (18H), 7,74-7,87 (3H), 8,57 (1H) ppm.

### Beispiel 20b

### (4S(4R,5S,6S,10E/Z,13S,14E))-4-(13-[[(1,1-Dimethylethyl)diphenylsilyl]oxy]15-(2-pyridyl)-3-oxo-5-(tetrahydropyran-2-yloxy)-2,4,6,10,14-pentamethyl-pentadeca-10,14-dien-2-yl)-2,2-dimethyl-[1,3]dioxan

Analog zu Beispiel 1ao werden 2,9 g (6,58 mmol) der in Analogie zu den Beispielen 11 (Umsetzung mit Ethylmagnesiumbromid) bis 1 an hergestellten Verbindung (4S(4R,5S,6S))-4-(3,10-Dioxo-2,4,6-trimethyl-5-(tetrahydropyran-2-yloxy)-undec-2-yl)-2,2-dimethyl-[1,3]dioxan mit 8,0 g (9,95 mmol) der unter Beispiel 20a beschriebenen Verbindung und 7,54 ml einer 1,6 M Lösung von n-Buthyllithium in n-Hexan umgesetzt. Man erhält neben Ausgangsmaterial 1,71 g (2,0 mmol, 31%) der Titelverbindung.
¹H-NMR (CDCl₃): δ= 0,84-0,98 (3H), 0,99-1,97 (42H), 2,01 (3H), 2,29 (2H), 3,22 (1H), 3,41 (1H), 3,58-4,01 (4H), 4,07-4,22 (2H), 4,47+4,51 (1H), 5,01 (1H), 6,24 (1H), 7,07 (1H), 7,22-7,46 (7H), 7,52-7,75 (5H), 8,57(1H) ppm.

### Beispiel 20c

### (4S(4R,5S,6S,10E/Z,13S,14E))-4-(13-Hydroxy-15-(2-pyridyl)-3-oxo-5-(tetrahydropyran-2-yloxy)-2,4,6,10,14-pentamethyl-pentadeca-10,14-dien-2-yl)-2,2-dimethyl-[1,3]dioxan

In Analogie zu Beispiel 1i setzt man 1,76 g (2,11 mmol) der nach Beispiel 20b dargestellten Verbindung um und isoliert nach Aufarbeitung und Reinigung 1,17 g (1,95 mmol, 93%) der Titelverbindung als farbloses Öl.
¹H-NMR (CDCl₃): δ= 0,88-2,13 (37H), 2,09 (3H), 2,39 (2H), 3,26 (1H), 3,44 (1H), 3,75-4,02 (3H), 4,08-4,22 (2H), 4,48+4,55 (1H). 5,21 (1H), 6,60 (1H), 7,10 (1H), 7,25 (1H), 7,64 (1H), 8,60 (1H) ppm.

### Beispiel 20d

### (3S,6R,7S,8S,12E/Z,15S,16E)-1,3,7,15-Tetrahydroxy-4,4,6,8,12,16-hexamethyl-17-(2-pyridyl)-heptadeca-12,16-dien-5-on

In Analogie zu Beispiel 1f setzt man 1,17 g (1,95 mmol) der nach Beispiel 20c dargestellten Verbindung unter Verwendung von p-Toluolsulfonsäure-Monohydrat um und isoliert nach Aufarbeitung und Reinigung 852 mg (1,79 mmol, 92%) der Titelverbindung als farbloses Öl.
¹H-NMR (CDCl₃): δ= 0,83+0,88 (3H), 1,06 (3H), 1,12 (3H), 1,22 (3H), 1,63+1,72 (3H), 0,98-1,82 (7H), 1,96-2,21 (3H), 2,07 (3H), 2,39 (2H), 2,90-3,80 (2H), 3,28 (1H), 3,32-3,48 (2H), 3,89 (2H), 4,06 (1H), 4,18 (1H), 5,20 (1H), 6,59 (1H), 7,11 (1H), 7,28 (1H), 7,64 (1H), 8,59 (1H) ppm.

### Beispiel 20e

### (3S,6R,7S,8S,12E/Z,15S,16E)-1,3,7,15-Tetrakis-[[dimethyl(1,1-dirnethylethyl)silyl]oxy]-4,4,6,8,12,16-hexamethyl-17-(2-pyridyl)-heptadeca-12,16-dien-5-on

In Analogie zu Beispiel 1aq setzt man 847 mg (1,78 mmol) der nach Beispiel 20d dargestellten Verbindung um und isoliert nach Aufarbeitung und Reinigung 1,32 g (1,42 mmol, 80%) der Titelverbindung als farbloses Öl.
¹H-NMR (CDCl₃): δ= -0,02-0,13 (24H), 0,80-0,97 (39H), 1,02 (3H), 1,04 (3H), 1,21 (3H), 1,59+1,68 (3H), 1,08-1,70 (7H), 1,89-2,08 (2H), 2,06 (3H), 2,28 (2H), 3,13 (1H), 3,52-3,74 (2H), 3,77 (1H), 3,89 (1H), 4,11 (1H), 5,18 (1H), 6,48 (1H), 7,08 (1H), 7,21 (1H), 7,62 (1H), 8,60 (1H) ppm.

### Beispiel 20f

### (3S,6R,7S,8S,12E/Z,15S,16E)-3,7,15-Tris-[[dimethyl(1,1-dimethylethyl)silyl]oxy]-4,4,6,8,12,16-hexamethyl-1-hydroxy-17-(2-pyridyl)-heptadeca-12,16-dien-5-on

In Analogie zu Beispiel 1ar setzt man 1,32 g (1,42 mmol) der nach Beispiel 20e dargestellten Verbindung um und isoliert nach Aufarbeitung und Reinigung 1,06 g (1,29 mmol, 91%) der Titelverbindung als farbloses Öl.
¹H-NMR (CDCl₃): δ= 0,00-0,13 (18H), 0,80-0,97 (30H), 1,06 (6H), 1,00-1,63 (7H), 1,21 (3H), 1,58+1,68 (3H), 1,89-2,08 (3H), 2,04 (3H), 2,28 (2H), 3,12 (1H), 3,63 (2H), 3,79 (1H), 4,02-4,16 (2H), 5,18 (1H), 6,48 (1H), 7,08 (1H), 7,21 (1H), 7,61 (1H), 8,60 (1H) ppm.

### Beispiel 20g

### (3S,6R,7S,8S,12E/Z,15S,16E)-3,7,15-Tris-[[dimethyl(1,1-dimethylethyl)silyl]oxy]-4,4,6,8,12,16-hexamethyl-17-(2-pyridyl)-5-oxo-heptadeca-12,16-dienal

In Analogie zu Beispiel 1k setzt man 1,14 g (1,39 mmol) der nach Beispiel 20f dargestellten Verbindung um und isoliert nach Aufarbeitung 1,10 g (1,35 mmol, 97%) der Titelverbindung als farbloses Öl, das man ohne Reinigung weiter umsetzt.

### Beispiel 20h

### (3S,6R,7S,8S,12E,15S,16E)-3,7,15-Tris-[[dimethyl(1,1-dimethylethyl)silyl]oxy]-4,4,6,8,12,16-hexamethyl-17-(2-pyridyl)-5-oxo-heptadeca-12,16-diensäure (A) und (3S,6R,7S,8S,12Z,15S,16E)-3,7,15-Tris-[[dimethyl(1,1-dimethylethyl)silyl]oxy]-4,4,6,8,12,16-hexamethyl-17-(2-pyridyl)-5-oxo-heptadeca-12,16-diensäure (B)

In Analogie zu Beispiel 1at setzt man 1,10 g (1,35 mmol) der nach Beispiel 20g dargestellten Verbindung um und isoliert nach Aufarbeitung und Reinigung 467 mg (0,56 mmol, 42%) der Titelverbindung B sowie 374 mg (0,45 mmol, 33%) der Titelverbindung A jeweils als farbloses Öl.
¹H-NMR (CDCl₃) von A: δ= 0,00-0,19 (18H), 0,85 (3H), 0,90 (27H), 1,01-1,50 (6H), 1,07 (3H), 1,15 (3H), 1,21 (3H), 1,57 (3H), 1,81-2,08 (1H), 1,96 (3H), 2,24-2,41 (4H), 2,60 (1H), 3,18 (1H), 3,83 (1H), 4,13 (1H), 4,38 (1H), 5,13 (1H), 6,50 (1H), 7,16 (1H), 7,36 (1H), 7,71 (1H), 8,61 (1H) ppm.
¹H-NMR (CDCl₃) von B: δ= -0,02-0,17 (18H), 0,80-0,98 (30H), 1,00-1,59 (6H), 1,05 (3H), 1,13 (3H), 1,18 (3H), 1,69 (3H), 1,81-1,98 (1H), 1,91 (3H), 2,10-2,40 (4H), 2,49 (1H), 3,10 (1H), 3,79 (1H), 4,15 (1H), 4,42 (1H), 5,21 (1H), 6,63 (1H), 7,17 (1H), 7,31 (1H), 7,70 (1H), 8,58 (1H) ppm.

### Beispiel 20i

### (3S,6R,7S,8S,12Z,15S,16E)-3,7-Bis-[[dimethyl(1,1-dimethylethyl)silyl]oxy]-4,4,6,8,12,16-hexamethyl-15-hydroxy-17-(2-pyridyl)-5-oxo-heptadeca-12,16-diensäure

In Analogie zu Beispiel 1i setzt man 405 mg (0,49 mmol) der nach Beispiel 20h dargestellten Verbindung B um und isoliert nach Aufarbeitung und Reinigung 338 mg (0,47 mmol, 96%) der Titelverbindung als farbloses Öl.
¹H-NMR (CDCl₃): δ= 0,00-0,15 (12H), 0,80-0,99 (21H), 1,02-1,60 (6H), 1,07 (3H), 1,14 (3H), 1,19 (3H), 1,72 (3H), 1,90-2,08 (1H), 1,99 (3H), 2,17 (1H), 2,31 (1H), 2,38 (2H), 2,49 (1H), 3,00-4,00 (1H), 3,12 (1H), 3,81 (1H), 4,19 (1H), 4,43 (1H), 5,24 (1H), 6,73 (1H), 7,18 (1H), 7,32 (1H), 7,71 (1H), 8,60 (1H) ppm.

### Beispiel 20j

### (4S,7R,8S,9S,13(Z),16S(E))-4,8-Bis-[[dimethyl(1,1-dimethylethyl]silyl]oxy]-16-(1-methyl-2-(2-pyridyl)ethenyl)-1-oxa-5,5,7,9,13-pentamethyl-cyclohexadec-13-en-2,6-dion

In Analogie zu Beispiel 1aw setzt man 287 mg (0,40 mmol) der nach Beispiel 20i dargestellten Verbindung um und isoliert nach Aufarbeitung und Reinigung 144 mg (0,21 mmol, 51%) der Titelverbindung als farbloses Öl.
¹H-NMR (CDCl₃): δ= -0,09 (3H), 0,01-0,18 (9H), 0,79-1,32 (4H), 0,85 (9H), 0,94 (9H), 0,98 (3H), 1,10 (3H), 1,14 (3H), 1,20 (3H), 1,46-1,82 (3H), 1,69 (3H), 2,03-2,21 (1H), 2,15 (3H), 2,49 (1H), 2,62-2,88 (2H), 3,03 (1H), 3,90 (1H), 4,05 (1H), 5,02 (1H), 5,19 (1H). 6,58 (1H), 7,11 (1H), 7,27 (1H), 7,65 (1H), 8,61 (1H) ppm.

### Beispiel 20

### (4S,7R,8S,9S,13(Z),16S(E))-4,8-Dihydroxy-16-(1-methyl-2-(2-pyridyl)ethenyl)-1-oxa-5,5,7,9,13-pentamethyl-cyclohexadec-13-en-2,6-dion

In Analogie zu Beispiel 1 setzt man 144 mg (206 µmol) der nach Beispiel 20j dargestellten Verbindung um und isoliert nach Aufarbeitung und Reinigung 90 mg (191 µmol, 93%) der Titelverbindung als farbloses Öl.
¹H-NMR (CDCl₃): δ= 1,02 (3H), 1,08 (3H), 1,20 (3H), 1,24-1,43 (4H), 1,38 (3H), 1,67 (3H), 1,60-1,98 (2H), 2,06 (3H), 2,23 (1H), 2,31 (2H), 2,45 (1H), 2,64 (1H), 3,11-3,27 (2H), 3,73 (1H), 4,41 (1H), 4,50-4,77 (1H), 5,09-5,23 (2H), 6,62 (1H), 7,14 (1H), 7,31 (1H), 7,69 (1H), 8,52 (1H) ppm.

### Beispiel 21

### (1S,3S(E),7S,10R,11S,12S,16R)-7.11-Dihydroxy-3-(1-methyl-2-(2-pyridyl)ethenyl)-8,8,10,12,16-pentamethyl-4,17-dioxabicyclo[14.1.0]hectadecan-5,9-dion (A) und (1R,3S(E),7S,10R,11S,12S,16S)-7,11-Dihydroxy-3-(1-methyl-2-(2-pyridyl)ethenyl)-8,8,10,12,16-pentamethyl-4,17-dioxabicyclo[14.1.0]heptadecan-5,9-dion (B) und (4S,7R,8S,9S,13(Z),16S(E))-4,8-Dihydroxy-16-(1-methyl-2-(2-N-oxypyridyl) ethenyl)-1-oxa-5,5,7,9,13-pentamethyl-cyclohexadec-13-en-2,6-dion (C) und (1S,3S(E),7S,10R,11S,12S,16R)-7,11 -Dihydroxy-3-(1-methyl-2-(2-N-oxypyridyl) ethenyl)-8,8,10,12,16-pentamethyl-4.17-dioxabicyclo[14.1.0]heptadecan-5,9-dion (D) und (1R,3S(E),7S,10R,11S.12S,16S)-7,11-Dihydroxy-3-(1-methyl-2-(2-N-oxypyridyl)ethenyl)-8,8,10,12,16-pentamethyl-4,17-dioxabicyclo[14.1.0] heptadecan-5,9-dion (E)

In Analogie zu Beispiel 14 setzt man 40 mg (84 µmol) der nach Beispiel 20 dargestellten Verbindung um und isoliert nach Aufarbeitung und Reinigung 8,5 mg (17 µmol, 21%) der Titelverbindung A, 2,0 mg (4 µmol, 5%) der Titelverbindung B, 2,9 mg (6 µmol, 7%) der Titelverbindung C, 12,6 mg (25 µmol, 30%) der Titelverbindung D, sowie 2,5 mg (5 µmol, 6%) der Titelverbindung E.
¹H-NMR (CDCl₃) von A: δ= 1,00 (3H), 1,08 (3H), 1,16 (3H), 1,21-1,98 (9H), 1,29 (3H), 1,38 (3H), 2,07 (3H), 2,19 (1H), 2,30 (1H), 2,53 (1H), 2,81 (1H), 2,89 (1H), 3,29 (1H), 3,76 (1H), 4,37 (1H), 5,40 (1H), 6,53 (1H), 7,16 (1H), 7,29 (1H), 7,70 (1H), 8,53 (1H) ppm.
¹H-NMR (CDCl₃) von B: δ= 0,94 (3H), 1,03 (3H), 1,11 (3H), 1,28 (3H), 1,38 (3H), 1,00-1,95 (8H), 2,14 (3H), 2,08-2,20 (1H), 2,41 (1H), 2,49 (1H), 2,83 (1H), 3,09 (1H), 3,33 (1H), 3,95 (1H), 4,06 (1H), 4,17 (1H), 5,70 (1H), 6,64 (1H), 7,12 (1H), 7,25 (1H), 7,67 (1H), 8,59 (1H) ppm.
¹H-NMR (CDCl₃) von C: δ= 1,01 (3H), 1,04 (3H), 1,20 (3H), 1,43 (3H), 1,68 (3H), 1,12-1,93 (6H), 2,02-2,64 (5H), 2,13 (3H), 3,22 (1H), 3,38 (1H), 3,69 (1H), 4,56 (1H), 5,11 (1H), 5,18 (1H), 6,28 (1H), 7,03 (1H), 7,21 (1H), 7,37 (1H), 7,48 (1H), 8,29 (1H) ppm.
¹H-NMR (CDCl₃) von D: δ= 1,01 (3H), 1,06 (3H), 1,18 (3H), 1,30 (3H), 1,46 (3H), 1,13-1,89 (8H), 2,14 (3H), 2,09-2,30 (2H), 2,52 (1H), 2,78 (1H), 3,17 (1H), 3,29 (1H), 3,71 (1H), 4,54 (1H), 5,37 (1H), 6,24 (1H), 6,96 (1H), 7,22 (1H), 7,37 (1H), 7,42 (1H), 8,28 (1H) ppm.
¹H-NMR (CDCl₃) von E: δ= 0,96 (3H), 1,06 (3H), 1,10 (3H), 1,29 (3H), 1,43 (3H), 1,22-1,77 (6H), 1,78-2,18 (3H), 2,11 (3H), 2,35-2,52 (2H), 2,96 (1H), 3,31 (1H), 3,43 (1H), 3,91 (1H), 4,49 (1H), 5,42 (1H), 5,49 (1H), 7,02 (1H), 7,19 (1H), 7,33 (1H), 7,45 (1H), 8,28 (1H) ppm.

### Beispiel 22

### (4S,7R,8S,9S,13(E),16S(E))-4,8-Dihydroxy-16-(1-methyl-2-(2-pyridyl)ethenyl)-1-oxa-5,5,7,9,13-pentamethyl-cyclohexadec-13-en-2,6-dion

### Beispiel 22a

### (3S,6R,7S,8S,12E,15S,16E)-3,7-Bis-[[dimethyl(1,1-dimethylethyl)silyl]oxy]-4,4,6,8,12,16-hexamethyl-15-hydroxy-17-(2-pyridyl)-5-oxo-heptadeca-12,16-diensäure

In Analogie zu Beispiel 1i setzt man 370 mg (444 µmol) der nach Beispiel 20h dargestellten Verbindung A um und isoliert nach Aufarbeitung und Reinigung 309 mg (430 µmol, 97%) der Titelverbindung als farbloses Öl.

### Beispiel 22b

### (4S,7R,8S,9S,13(E),16S(E))-4,8-Bis-[[dimethyl(1,1-dimethylethyl)silyl]oxy]-16-(1-methyl-2-(2-pyridyl)ethenyl)1-oxa-5,5,7,9,13-pentamethyl-cyclohexadec-13-en-2,6-dion

In Analogie zu Beispiel 1aw setzt man 309 g (430 µmol) der nach Beispiel 22a dargestellten Verbindung um und isoliert nach Aufarbeitung und Reinigung 233 mg (333 µmol, 77%) der Titelverbindung als farbloses Öl.
¹H-NMR (CDCl₃): δ= 0,02-0,17 (12H), 0,88 (18H), 0,93 (3H), 1,09 (3H), 1,12 (3H), 1,16-1,37 (2H), 1,19 (3H), 1,45-1,64 (3H), 1,59 (3H), 1,93 (1H), 2,08-2,21 (1H), 2,18 (3H), 2,50 (1H), 2,54-2,70 (3H), 3,07 (1H), 3,90 (1H), 4,51 (1H), 5,20 (1H), 5,30 (1H), 6,58 (1H), 7,10 (1H), 7,19 (1H), 7,63 (1H), 8,60 (1H) ppm.

### Beispiel 22

### (4S,7R,8S,9S,13(E),16S(E))-4,8-Dihydroxy-16-(1-methyl-2-(2-pyridyl)ethenyl)-1-oxa-5,5,7,9,13-pentamethyl-cyclohexadec-13-en-2,6-dion

In Analogie zu Beispiel 1 setzt man 228 mg (326 µmol) der nach Beispiel 22b dargestellten Verbindung um und isoliert nach Aufarbeitung und Reinigung 131 mg (278 µmol, 85%) der Titelverbindung als farbloses Öl.
¹H-NMR (CDCl₃): δ= 0,98 (3H), 1,07 (3H), 1,17 (3H), 1,31 (3H), 1,20-1,46 (3H), 1,52-1,83 (2H), 1,61 (3H), 1,98 (1H), 2,08 (3H), 2,17 (1H), 2,39 (1H), 2,41-2,66 (3H), 3,18-3,39 (2H), 3,66 (1H), 3,87 (1H), 4,38 (1H), 5,14 (1H), 5,42 (1H), 6,60 (1H), 7,13 (1H), 7,32 (1H), 7,69 (1H), 8,56 (1H) ppm.

### Beispiel 23

### (1R,3S(E),7S,10R,11S,12S,16R)-7,11-Dihydroxy-3-(1-methyl-2-(2-pyridyl)ethenyl)-8,8,10,12,16-pentamethyl-4,17-dioxabicyclo[14.1.0]heptadecan-5,9-dion (A) und (1S,3S(E),7S,10R,11S,12S,16S)-7,11-Dihydroxy-3-(1-methyl-2-(2-pyridyl)ethenyl)-8,8,10,12,16-pentamethyl-4,17-dioxabicyclo[14.1.0]heptadecan-5,9-dion (B) (1R,3S(E),7S,10R,11S,12S,16R)-7,11-Dihydroxy-3-(1-methyl-2-(2-N-oxidopyridyl)ethenyl)-8,8,10,12,16-pentamethyl-4,17-dioxabicyclo[14.1.0]heptadecan-5,9-dion (C) und (1S,3S(E),7S,10R,11S,12S,16S)-7,11-Dihydroxy-3-(1-methyl-2-(2-N-oxidopyridyl)ethenyl)-8,8,10,12,16-pentamethyl-4,17-dioxabicyclo[14.1.0]heptadecan-5,9-dion (D)

In Analogie zu Beispiel 14 setzt man 50 mg (106 µmol) der nach Beispiel 20 dargestellten Verbindung um und isoliert nach Aufarbeitung und Reinigung 5,3 mg (11 µmol, 10%) der Titelverbindung A (oder B), 4,4 mg (9 µmol, 9%) der Titelverbindung B (oder A), 9,6 mg (10 µmol, 9%) der Titelverbindung C (oder D) und 11,1 mg (11 µmol, 11 %) der Titelverbindung D (oder C).
¹H-NMR (CDCl₃) von A oder B: δ= 0,94 (3H), 1,04 (3H), 1,13 (3H), 1,28 (3H), 1,39 (3H), 2,11 (3H), 1,01-2,15 (9H), 2,44 (1H), 2,58 (1H), 2,74 (1H), 2,91 (1H), 3,31 (1H), 3,73 (1H), 4,21 (1H), 4,30 (1H), 5,53 (1H), 6,53 (1H), 7,13 (1H), 7,30 (1H), 7,67 (1H), 8,57 (1H) ppm.
¹H-NMR (CDCl₃) von B oder A: .δ= 0,93 (3H), 1,09 (3H), 1,14 (3H), 1,28 (3H), 1,37 (3H), 1,22-2,16 (9H), 2,09 (3H), 2,46 (1H), 2,57 (1H), 2,96 (1H), 3,08 (1H), 3,26 (1H), 3,72 (1H), 3,89 (1H), 4,37 (1H), 5,47 (1H), 6,62 (1H), 7,13 (1H), 7,28 (1H), 7,68 (1H), 8,57 (1H) ppm.
¹H-NMR (CDCl₃) von C oder D: δ= 0,93 (3H), 1,06 (3H), 1,19 (3H), 1,21 (3H), 1,44 (3H), 1,15-2,01 (8H), 2,10 (3H), 2,12-2,26 (2H), 2,49 (1H), 2,89 (1H), 3,26 (1H), 3,48 (1H), 3,67 (1H), 4,63 (1H), 5,45 (1H), 5,76 (1H), 7,09 (1H), 7,21 (1H), 7,36 (1H), 7,45 (1H), 8,29 (1H) ppm.
¹H-NMR (CDCl₃) von D oder C: δ= 0,96 (3H), 1,06 (3H), 1,15 (3H), 1,24 (3H), 1,43 (3H), 1,02-2,19 (9H), 2,08 (3H), 2,23 (1H), 2,56 (1H), 2,96 (1H), 3,29 (1H), 3,68 (2H), 4,53 (1H), 5,60-5,72 (2H), 7,10 (1H), 7,21 (1H), 7,37 (1H), 7,52 (1H), 8,29 (1H) ppm.

### Beispiel 24

### (4S,7R,8S,9S,13(Z),16S(E1)-4,8-Dihydroxy-7-ethyl-16-(1-methyl-2-(2-pyridyl)ethenyl]-1-oxa-5,5,9,13-tetramethyl-cyclohexadec-13-en-2,6-dion

### Beispiel 24h, Variante l

### (3S,6R,7S,8S,12E/Z,15S,16E)-6-Ethyl-1,3,7,15-tetrakis-[[dimethyl(1,1-dimethylethyl)silyl]oxy]-4,4,8,12,16-pentamethyl-17-(2-pyridyl)-heptadeca-12,16-dien-5-on

### Beispiel 24a/I

### (2S)-2-Methyl-1-(tetrahydropyran-2-yloxy)-heptan-6-on

In Analogie zu Beispiel 1m setzt man 9,0 g (39,1 mmol) der nach Beispiel 1v dargestellten Verbindung um und isoliert nach Aufarbeitung und Reinigung 8,05 g (35,3 mmol, 90%) der Titelverbindung als farbloses Öl.
¹H-NMR (CDCl₃): δ= 0,93 (3H), 1,12 (1H), 1,32-1,89 (10H), 2,14 (3H), 2,42 (2H), 3,19 (1H), 3,45-3,63 (2H), 3,84 (1H), 4,56 (1H) ppm.

### Beispiel 24b/l

### (2S,6E/Z,9S,10E)-9-[[(1,1-Dimethylethyl)diphenylsilyl]oxy]-11-(2-pyridyl)-1-(tetrahydropyran-2-yloxy)-2,6,10-trimethyl-undeca-6,10-dien

In Analogie zu Beispiel 7ao bzw. 20b setzt man 1,89 g (8,28 mmol) der nach Beispiel 24a/l dargestellten Verbindung mit 10,0 g (12,4 mmol) der nach Beispiel 20a dargestellten Verbindung unter Verwendung von n-Buthyllithium als Base um und isoliert nach Aufarbeitung und Reinigung 1,98 g (3,2 mmol, 38%) der Titelverbindung als farbloses Öl.
¹H-NMR (CDCl₃): δ= 0,81-0,92 (3H), 1,08 (9H), 1,18-1,92 (16H), 2,02 (3H), 2,19-2,42 (2H), 3,02-3,62 (3H), 3,83 (1H), 4,20 (1H), 4,55 (1H), 5,00 (1H), 6,24 (1H), 6,98-7,10 (2H), 7,22-7,46 (6H), 7,57 (1H), 7,62-7,75 (4H), 8,58 (1H) ppm.

### Beispiel 24c/l

### (2S,6E/Z,9S,10E)-11-(2-Pyridyl)-1-(tetrahydropyran-2-yloxy)-2,6,10-trimethyl-undeca-6,10-dien-9-ol

In Analogie zu Beispiel 1i setzt man 1,98 g (3,2 mmol) der nach Beispiel 24b/l dargestellten Verbindung um und isoliert nach Aufarbeitung und Reinigung 1,16 g (3,0 mmol, 94%) der Titelverbindung als farbloses Öl.
¹H-NMR (CDCl₃): δ= 0,87-1,00 (3H), 1,12 (1H), 1,32-1,95 (11H), 1,67+1,73 (3H), 1,98-2,18 (2H), 2,10 (3H), 2,40 (2H), 3,08-3,28 (1H), 3,42-3,65 (2H), 3,84 (1H), 4,19 (1H), 4,55 (1H), 5,19 (1H), 6,59 (1H), 7,10 (1H), 7,24 (1H), 7,63 (1H), 8,60 (1H) ppm.

### Beispiel 24d/l

### (2S,6E/Z,9S,10E)-9-[[Dimethyl(1,1-dimethylethyl)silyl]oxy]-11-(2-pyridyl)-1- (tetrahydropyran-2-yloxy)-2,6,10-trimethyl-undeca-6,10-dien

In Analogie zu Beispiel 1n setzt man 1,15 g (2,97 mmol) der nach Beispiel 24c/l dargestellten Verbindung um und isoliert nach Aufarbeitung und Reinigung 1,43 g (2,85 mmol, 96%) der Titelverbindung als farbloses Öl.
¹H-NMR (CDCl₃): δ= 0,03 (3H), 0,08 (3H), 0,81-0,98 (12H), 1,11 (1H), 1,28-2,10 (12H), 1,60+1,69 (3H), 2,06 (3H), 2,28 (2H), 3,07-3,27 (1H), 3,42-3,63 (2H), 3,85 (1H), 4,12 (1H), 4,56 (1H), 5,18 (1H), 6,48 (1H), 7,08 (1H), 7,22 (1H), 7,62 (1H), 8,60 (1H) ppm.

### Beispiel 24e/I

### (2S,6E/Z,9S,10E)-9-[[Dimethyl(1,1-dimethylethyl)silyl]oxyl-11-(2-pyridyl)-2,6,10-trimethyl-undeca-6,10-dien-1-ol

In Analogie zu Beispiel 1f setzt man 1,43 g (2,85 mmol) der nach Beispiel 24d/l dargestellten Verbindung unter Verwendung von p-Toluolsulfonsäure-Monohydrat bei 23°C um und isoliert nach Aufarbeitung und Reinigung 1,11 g (2,66 mmol, 93%) der Titelverbindung als farbloses Öl.
¹H-NMR (CDCl₃): δ= 0,03 (3H), 0,08 (3H), 0,82-0,96 (12H), 0,97-1,71 (6H), 1,59+1,69 (3H), 1,90-2,14 (2H), 2,04 (3H), 2,30 (2H), 3,35-3,56 (2H), 4,13 (1H), 5,13+5,21 (1H), 6,48 (1H), 7,10 (1H), 7,25 (1H), 7,63 (1H), 8,58 (1H) ppm.

### Beispiel 24f/l

### (2S,6E/Z,9S,10E)-9-[[Dimethyl(1,1-dimethylethyl)silyl]oxy]-11-(2-pyridyl)-2,6,10-trimethyl-undeca-6,10-dienal

In Analogie zu Beispiel 1 k setzt man 1,01 g (2,42 mmol) der nach Beispiel 24e/l dargestellten Verbindung um und isoliert nach Aufarbeitung und Reinigung 921 mg (2,22 mmol, 92%) der Titelverbindung als farbloses Öl.
¹H-NMR (CDCl₃): δ= 0,03 (3H), 0,08 (3H), 0,92 (9H), 1,05+1,09 (3H), 1,22-1,75 (4H), 1,60+1,68 (3H), 1,95-2,11 (2H), 2,07 (3H), 2,23-2,38 (3H), 4,12 (1H). 5,19 (1H), 6,48 (1H). 7,08 (1H), 7,22 (1H). 7,63 (1H), 8,60 (1H), 9,57+9,61 (1H) ppm.

### Beispiel 24g/l

### (3S,6R,7S,8S,12E/Z,15S,16E)-6-Ethyl-7-hydroxy-1,3,15-tris-[[dimethyl(1,1-dimethylethyl)silyl]oxy]-4,4,8,12,16-pentamethyl-17-(2-pyridyl)-heptadeca-12,16-dien-5-on

**(A) und (3S,6S,7R,8S,12E/Z,15S,16E)-6-Ethyl-7-hydroxy-1,3,15-tris-[[dimethyl(1,1-dimethylethyl)silyl]oxy]-4,4,8,12,16-pentamethyl-17-(2-pyridyl)-heptadeca-12,16-dien-5-on**
**(B)**
   In Analogie zu Beispiel 1ak setzt man 1,0 g (2,41 mmol) der nach Beispiel 24f/l dargestellten Verbindung mit 1,16 g (2,78 mmol) der nach Beispiel 1m dargestellten Verbindung um und isoliert nach Aufarbeitung und Reinigung 972 mg (1,17 mmol, 48%) der Titelverbindung A sowie 178 mg (0,21 mmol, 9%) der Titelverbindung B jeweils als farbloses Öl.
   ¹H-NMR (CDCl₃) von A: δ= 0,00-0,14 (18H), 0,80-0,95 (33H), 1,00-1,81 (9H), 1,11 (3H), 1,17 (3H), 1,60+1,68 (3H), 1,90-2,11 (2H), 2,04 (3H), 2,29 (2H), 3,03 (1H), 3,18 (1H), 3,32 (1H), 3,54-3,77 (2H), 3,99 (1H), 4,12 (1H), 5,18 (1H), 6,48 (1H), 7,09 (1H), 7,23 (1H), 7,62 (1H), 8,60 (1H) ppm.
   ¹H-NMR (CDCl₃) von B: δ= -0,02-0,14 (18H), 0,83-1,01 (33H), 1,02-1,80 (9H), 1,10 (3H), 1,16 (3H), 1,62+1,70 (3H), 1,92-2,10 (2H), 2,06 (3H), 2,30 (2H), 3,02 (1H), 3,15 (1H), 3,42 (1H), 3,53-3,74 (2H), 4,02 (1H), 4,12 (1H), 5,19 (1H), 6,49 (1H), 7,09 (1H), 7,23 (1H), 7,63 (1H), 8,60 (1H) ppm.

### Beispiel 24h/l

### (3S,6R,7S,8S,12E/Z,15S,16E)-6-Ethyl-1,3,7,15-tetrakis-[[dimethyl(1,1-dimethylethyl)silyl]oxyl-4,4,8,12,16-pentamethyl-17-(2-pyridyl)-heptadeca-12,16-dien-5-on

In Analogie zu Beispiel 1aq setzt man 972 mg (1,17 mmol) der nach Beispiel 24g/l dargestellten Verbindung A um und isoliert nach Aufarbeitung und Reinigung 1,02 g (1,08 mmol, 92%) der Titelverbindung als farbloses Öl.
¹H-NMR (CDCl₃): δ= 0,00-0,12 (24H), 0,78-0,97 (42H), 1,00-1,80 (9H), 1,03 (3H), 0,21 (3H), 1,60+1,68 (3H), 1,90-2,10 (2H), 2,05 (3H), 2,28 (2H), 3,02 (1H), 3,52-3,73 (2H), 3,82 (1H), 3,91 (1H), 4,11 (1H), 5,19 (1H), 6,49 (1H), 7,08 (1H), 7,22 (1H), 7,61 (1H), 8,60 (1H) ppm.

### Beispiel 24h, Variante II

### (3S,6R,7S,8S,12E/Z,15S,16E)-6-Ethyl-1,3,7,15-tetrakis-[[dimethyl(1,1-dimethylethyl)silyl]oxy]-4,4,8,12,16-pentamethyl-17-(2-pyridyl)-heptadeca-12,16-dien-5-on

### Beispiel 24a/II

### (4S(4R,5S,6S,10E/Z,13S,14E))-4-(13-[[(1,1-Dimethylethyl)diphenylsilyl]oxy]-4-ethyl-15-(2-pyridyl)-3-oxo-5-(tetrahyd ropyran-2-yloxy)-2,6,10,14-tetramethyl-pentadeca-10,14-d ien-2-yl)-2,2-dimethyl-[1,3]dioxan

In Analogie zu Beispiel 1ao bzw. 20b setzt man 724 mg (1,59 mmol) der nach Beispiel 1an dargestellten Verbindung mit 1,93 g (2,40 mmol) der nach Beispiel 20a dargestellten Verbindung unter Verwendung von n-Buthyllithium als Base um und isoliert nach Aufarbeitung und Reinigung 478 mg (0,56 mmol, 35%) der Titelverbindung als farbloses Öl.
¹H-NMR (CDCl₃): δ= 0,72-1,96 (48H), 2,01 (3H), 2,16-2,41 (2H), 3,03+3,13 (1H), 3,41 (1H), 3,59-4,04 (3H), 4,12-4,32 (2H), 4,43+4,52 (1H), 5,01 (1H), 6,23 (1H), 6,97-7,10 (2H), 7,21-7,46 (6H), 7,58 (1H), 7,62-7,74 (4H), 8,57 (1H) ppm.

### Beispiel 24b/II

### (4S(4R,5S,6S,10E/Z,13S,14E))-4-(4-Ethyl-13-hydroxy-15-(2-pyridyl)-3-oxo-5-(tetrahyd ropyran-2-yloxy)-2,6,10,14-tetramethyl-pentadeca-10,14-d ien-2-yl)-2,2-d imethyl-[1,3]dioxan

In Analogie zu Beispiel 1i setzt man 660 mg (0,77 mmol) der nach Beispiel 24a/II dargestellten Verbindung um und isoliert nach Aufarbeitung und Reinigung 475 mg (0,77 mmol, 100%) der Titelverbindung als farbloses Öl.
¹H-NMR (CDCl₃): δ= 0,72-2,12 (39H), 2,09 (3H), 2,39 (2H), 3,07+3,17 (1H), 3,42 (1H), 3,62-4,32 (6H), 4,43+4,54 (1H), 5,20 (1H), 6,61 (1H), 7,10 (1H), 7,25 (1H), 7,63 (1H), 8,60 (1H) ppm.

### Beispiel 24c/II

### (3S,6R,7S,8S,12E/Z,15S,16E)-6-Ethyl-1,3,7,15-tetrahydroxy-4,4,8,12,16-pentamethyl-17-(2-pyridyl)-heptadeca-12,16-dien-5-on

In Analogie zu Beispiel 1f setzt man 472 mg (0,77 mmol) der nach Beispiel 24b/II dargestellten Verbindung unter Verwendung von p-Toluolsulfonsäure-Monohydrat bei 23°C um und isoliert nach Aufarbeitung und Reinigung 348 mg (0,71 mmol, 92%) der Titelverbindung als farbloses Öl.
¹H-NMR (CDCl₃): δ= 0,75-0,92 (6H), 1,07 (3H), 1,11-2,47 (13H), 1,26 (3H), 1,63 (3H), 1,72 (3H), 2,04+2,05 (3H), 2,96 (1H), 3,18 (1H), 3,41 +3,48 (1H), 3,86 (2H), 4,04-4,23 (2H), 5,18+5,23 (1H), 6,57 (1H), 7,12 (1H), 7,29 (1H), 7,67 (1H), 8,59 (1H) ppm.

### Beispiel 24h/II

### (3S,6R,7S,8S,12E/Z,15S,16E)-6-Ethyl-1,3,7,15-tetrakis-[[dimethyl(1,1-dimethylethyl)silyl]oxy]-4,4,8,12,16-pentamethyl-1 7-(2-pyridyl)-heptadeca-1 2,16-dien-5-on

In Analogie zu Beispiel 1aq setzt man 343 mg (0,70 mmol) der nach Beispiel 24c/ll dargestellten Verbindung um und isoliert nach Aufarbeitung und Reinigung 497 mg (0,52 mmol, 75%) der Titelverbindung als farbloses Öl.
¹H-NMR (CDCl₃): deckungsgleich mit dem unter Beispiel 24h/l beschriebenen.

### Beispiel 24i

### (3S,6R,7S,8S,12E/Z,15S,16E)-6-Ethyl-3,7,15-tris-[[dimethyl(1,1-dimethylethyl)silyl] oxy]-4,4,8,12,16-pentamethyl-1-hydroxy-17-(2-pyridyl)-heptadeca-12,16-dien-5-on

In Analogie zu Beispiel 1ar setzt man 1,71 g (1,81 mmol) der nach Beispiel 24h/l oder Beispiel 24h/ll dargestellten Verbindung um und isoliert nach Aufarbeitung und Reinigung 1,38 g (1,66 mmol, 97%) der Titelverbindung als farbloses Öl.
¹H-NMR (CDCl₃): δ= 0,00-0,15 (18H), 0,80-0,98 (33H), 1,02-2,10 (11H), 1,09 (3H), 1,21 (3H), 1,59+1,68 (3H), 2,05 (3H), 2,29 (2H), 3,01 (1H), 3,69 (2H), 3,84 (1H), 4,02-4,19 (3H), 5,18 (1H), 6,48 (1H), 7,09 (1H), 7,22 (1H), 7,62 (1H), 8,59 (1H) ppm.

### Beispiel 24k

### (3S,6R,7S,8S,12E/Z,15S,16E)-6-Ethyl-3,7,15-tris-[[dimethyl(1,1-dimethylethyl)silyl] oxy]-4,4,8,12,16-pentamethyl-17-(2-pyridyl)-5-oxo-heptadeca-12,16-dienal

In Analogie zu Beispiel 1k setzt man 1,38 g (1,66 mmol) der nach Beispiel 24i dargestellten Verbindung um und isoliert nach Aufarbeitung und Reinigung 1,34 g (1,61 mmol, 97%) der Titelverbindung als farbloses Öl.
¹H-NMR (CDCl₃): δ= 0,01-0,13 (18H), 0,78-0,97 (35H), 1,09 (3H), 1,13-1,79 (5H), 1,21 (3H), 1,60+1,68 (3H), 1,91-2,10 (2H), 2,05 (3H), 2,28 (2H), 2,40 (1H), 2,57 (1H), 3,02 (1H), 3,82 (1H), 4,12 (1H), 4,48 (1H), 5,18 (1H), 6,48 (1H), 7,08 (1H), 7,22 (1H), 7,62 (1H), 8,60 (1H), 9,79 (1H) ppm.

### Beispiel 24l

### (3S,6R,7S,8S,12E,15S,16E)-6-Ethyl-3,7,15-tris-[[dimethyl(1,1-dimethylethyl)silyl] oxy]-4,4,8,12,16-pentamethyl-17-(2-pyridyl)-5-oxo-heptadeca-12,16-diensäure (A) und (3S,6R,7S,8S,12Z,15S,16E)-6-Ethyl-3,7,15-tris-[[dimethyl(1,1-dimethylethyl) silyl]oxy]-4,4,8,12,16-pentamethyl-17-(2-pyridyl)-5-oxo-heptadeca-12,16-diensäure (B)

In Analogie zu Beispiel 1at setzt man 1,34 g (1,61 mmol) der nach Beispiel 24k dargestellten Verbindung um und isoliert nach Aufarbeitung und Reinigung 433 mg (0,51 mmol, 32%) der Titelverbindung A sowie 662 mg (0,78 mmol, 49%) der Titelverbindung B jeweils als farbloses Öl.
¹H-NMR (CDCl₃) von A: δ= 0,00-0,16 (18H), 0,78-0,93 (35H), 0,98-1,71 (6H), 1,12 (3H), 1,21 (3H), 1,56 (3H), 1,80-2,07 (2H), 1,93 (3H), 2,23-2,41 (3H), 2,67 (1H), 3,05 (1H), 3,86 (1H), 4,12 (1H). 4,33 (1H), 5,11 (1H), 6,48 (1H), 7,24 (1H), 7,33 (1H), 7,69 (1H), 8,61 (1H) ppm.
¹H-NMR (CDCl₃) von B: δ= -0,01-0,17 (18H), 0,81-0,96 (35H), 1,00-1,78 (6H), 1,15 (3H), 1,21 (3H), 1,70 (3H), 1,89 (1H), 1,96 (3H), 2,11-2,42 (4H), 2,59 (1H), 3,00 (1H), 3,82 (1H), 4,17 (1H), 4,41 (1H), 5,24 (1H), 6,63 (1H), 7,19 (1H), 7,33 (1H), 7,71 (1H), 8,64 (1H) ppm.

### Beispiel 24m

### (3S,6R,7S,8S,12Z,15S,16E)-3,7-Bis-[[dimethyl(1,1-dimethylethyl)silyl]oxy]-6-ethyl-4,4,8,12,16-pentamethyl-15-hyd roxy-17-(2-pyridyl)-5-oxo-heptadeca-12,16-diensäu re

In Analogie zu Beispiel 1i setzt man 662 mg (0,78 mmol) der nach Beispiel 241 dargestellten Verbindung B bei 23°C um und isoliert nach Aufarbeitung 680 mg der Titelverbindung als Rohprodukt, das man ohne Reinigung weiter umsetzt.

### Beispiel 24n

### (4S,7R,8S,9S,13(Z),16S(E))-4,8-Bis-[[dimethyl(1,1-dimethylethyl)silyl]oxy]-16-(1-methyl-2-(2-pyridyl)ethenyl)-7-ethyl-1 -oxa-5,5,9,13-tetramethyl-cyclohexadec-1 3-en-2,6-dion

In Analogie zu Beispiel 1 aw setzt man 680 mg (max. 0,78 mmol) der nach Beispiel 24m dargestellten Verbindung um und isoliert nach Aufarbeitung und Reinigung 287 mg (402 µmol, 52%) der Titelverbindung als farbloses Öl.
¹H-NMR (CDCl₃): δ= -0,11 (3H), 0,03-0,15 (9H), 0,72 (3H), 0,80-1,78 (23H), 0,83 (3H), 0,92 (3H), 0,98 (3H), 1,11 (3H), 1,18 (3H), 1,68 (3H), 1,85 (1H), 2,09 (1H), 2,12 (3H), 2,46 (1H), 2,55-2,82 (3H), 3,05 (1H), 4,01 (1H), 4,03 (1H), 4,99 (1H), 5,16 (1H), 6,54 (1H), 7,08 (1H), 7,23 (1H), 7,61 (1H), 8,58 (1H) ppm.

### Beispiel 24

### (4S,7R,8S,9S,13(Z),16S(E))-4,8-Dihydroxy-7-ethyl-16-(1-methyl-2-(2-pyridyl)ethenyl)-1-oxa-5,5,9,13-tetramethyl-cyclohexadec-1 3-en-2,6-dion

In Analogie zu Beispiel 1 setzt man 282 mg (395 µmol) der nach Beispiel 24n dargestellten Verbindung um und isoliert nach Aufarbeitung und Reinigung 115 mg (237 µmol, 60%) der Titelverbindung als farbloses Öl.
¹H-NMR (CDCl₃): δ= 0,89 (3H), 1,04 (3H), 1,09 (3H), 1,22-2,11 (8H), 1,36 (3H), 1,70 (3H), 2,07 (3H), 2,20-2,39 (3H), 2,49 (1H), 2,65 (1H), 2,69 (1H), 3,23 (1H), 3,70 (1H), 4,35 (1H), 4,59 (1H), 5,12 (1H), 5,19 (1H), 6,61 (1H), 7,13 (1H), 7,29 (1H), 7,69 (1H), 8,53 (1H) ppm.

### Beispiel 25

### (1S,3S(E),78,10R,11S,128,16R)-7,11-Dihydroxy-10-ethyl-3-(1-methyl-2-(2-pyridyl)ethenyl)-8,8,12,16-tetramethyl-4,17-dioxabicyclo[14,1,0]heptadecan-5,9-dion (A) und (1R,3S(E),7S,10R,11S,12S,16S)-7,11-Dihydroxy-10-ethyl-3-(1-methyl-2-(2-Pyridyl)ethenyl)-8,8,12,16-tetramethyl-4,17-dioxabicyclo[14.1.0]heptadecan-5,9-dion (B) und (4S,7R,8S,9S,13(Z),16S(E))-4,8-Dihydroxy-9-ethyl-16-(1-methyl-2-(2-N-oxypyridyl) ethenyl)-1-oxa-5,5,7,13-tetramethyl-cyclohexadec-13-en-2,6-dion (C) und (1S,3S(E),7S,10R,11S,12S,16R)-7,11-Dihydroxy-10-ethyl-3-(1-methyl-2-(2-N-oxypyridyl)ethenyl)-8,8,12,16-tetramethyl-4,17-dioxabicyclo[14,1,0]heptadecan-5,9-dion (D) und (1R,3S(E),7S,10R,11S,12S,16S)-7,11-Dihydroxy-10-ethyl-3-(1-methyl-2-(2-N-oxypyridyl)ethenyl)-8,8,12,16-tetramethyl-4,17-dioxabicyclo[14.1.0] heptadecan-5,9-dion (E)

In Analogie zu Beispiel 14 setzt man 50 mg (103 µmol) der nach Beispiel 24 dargestellten Verbindung um und isoliert nach Aufarbeitung und Reinigung 15,3 mg (30 µmol, 30%) der Titelverbindung A, 2 mg (4 µmol, 4%) der Titelverbindung B, 2 mg (4 µmol, 4%) der Titelverbindung C, 21 mg (42 µmol, 41%) der Titelverbindung D und 3,3 mg (7 µmol, 6%) der Titelverbindung E jeweils als farblosen Feststoff.
¹H-NMR (CDCl₃) von A: δ 0,87 (3H), 0,99 (3H), 1,06 (3H), 1,21-2,03 (10H), 1,30 (3H), 1,39 (3H), 2,03 (3H), 2,15 (1H), 2,37 (1H), 2,56 (1H), 2,81 (1H), 2,83 (1H), 3,32 (1H), 3,66 (1H), 4,36 (1H), 5,24 (1H), 5,45 (1H), 6,61 (1H), 7,16 (1H), 7,29 (1H), 7,70 (1H), 8,53 (1H) ppm.
¹H-NMR (CDCl₃) von B: δ= 0,85 (3H), 0,95 (3H), 1,04 (3H), 1,20-1,93 (10H), 1,30 (3H), 1,38 (3H), 2,08 (1H). 2,11 (3H), 2,42-2,61 (2H), 2,95 (1H), 2,98 (1H), 3,22 (1H), 3,63 (1H), 3,93 (1H), 4,33 (1H), 5,59 (1H), 6,66 (1H), 7,13 (1H), 7,28 (1H), 7,67 (1H), 8,58 (1H) ppm.
¹H-NMR (CDCl₃) von C: δ= 0,80-1,92 (8H), 0,92 (3H), 1,03 (3H), 1,08 (3H), 1,44 (3H), 1,70 (3H), 2,08-2,64 (5H), 2,12 (3H), 2,82 (1H), 3,29 (1H), 3,67 (1H), 4,53 (1H), 5,09 (1H), 5,17 (1H), 6,19 (1H), 6,99 (1H), 7,19 (1H), 7,35 (1H), 7,44 (1H), 8,29 (1H) ppm.
¹H-NMR (CDCl₃) von D: δ= 0,87 (3H), 1,00 (3H), 1,04 (3H), 1,09-2,03 (10H), 1,29 (3H), 1,42 (3H), 2,10 (3H), 2,18-2,32 (2H), 2,53 (1H), 2,67-2,82 (2H), 3,31 (1H), 3,62 (1H), 4,52 (1H), 5,41 (1H), 6,16 (1H), 6,93 (1H), 7,21 (1H), 7,37 (1H), 7,42 (1H), 8,28 (1H) ppm.
¹H-NMR (CDCl₃) von E: δ= 0,83 (3H), 0,94 (3H), 1,08 (3H), 1,20-2,08 (11H), 1,29 (3H), 1,45 (3H), 2,12 (3H), 2,39-2,56 (2H), 2,87 (1H), 3,24 (1H), 3,29 (1H), 3,87 (1H), 4,52 (1H), 5,41 (1H), 5,56 (1H), 7,03 (1H), 7,19 (1H), 7,34 (1H), 7,46 (1H), 8,29 (1H) ppm.

### Beispiel 26

### (4S,7R,8S,9S,13(E),16S(E))-4,8-Dihydroxy-7-ethyl-16-(1-methyl-2-(2-pyridyl)ethenyl)1-oxa-5,5,7,13-tetramethyl-cyclohexadec-13-en-2,6-dion

### Beispiel 26a

### (3S,6R,7S,8S,12E,15S,16E)-3,7-Bis-[[dimethyl(1,1-dimethylethyl)silyl]oxyl-6-ethyl-4,4,8,12,16-pentamethyl-15-hydroxy-17-(2-pyridyl)-5-oxo-heptadeca-12,16-diensäure

In Analogie zu Beispiel 1i setzt man 433 mg (0,51 mmol) der nach Beispiel 24l dargestellten Verbindung A um und isoliert nach Aufarbeitung 447 mg der Titelverbindung als Rohprodukt, das man ohne Reinigung weiter umsetzt.

### Beispiel 26b

### (4S,7R,8S,9S,13(E),16S(E))-4,8-Bis-[[dimethyl(1,1-dimethylethyl)silyl]oxy]-7-ethyl-16-(1-methyl-2-(2-pyridyl)ethenyl)-1-oxa-5,5,9,13-tetramethyl-cyclohexadec-13-en-2,6-dion

In Analogie zu Beispiel 1aw setzt man 447 mg (511 µmol) der nach Beispiel 26a dargestellten Verbindung um und isoliert nach Aufarbeitung und Reinigung 264 mg (370 µmol, 72%) der Titelverbindung als farbloses Öl.
¹H-NMR (CDCl₃): δ= 0,06-0,15 (12H), 0,85 (3H), 0,89 (9H), 0,91 (9H), 0,94 (3H), 1,08-1,92 (11H), 1,12 (3H), 1,21 (3H), 2,10-2,23 (1H), 2,16 (3H), 2,40 (1H), 2,46-2,68 (3H), 2,98 (1H), 3,95 (1H), 4,41 (1H), 5,23 (1H), 5,30 (1H), 6,57 (1H), 7,10 (1H), 7,21 (1H), 7,63 (1H), 8,60 (1H) ppm.

### Beispiel 26

### (4S,7R,8S,9S,13(E),16S(E))-4,8-Dihydroxy-7-ethyl-16-(1-methyl-2-(2-pyridyl)ethenyl)-1-oxa-5,5,9,13-tetramethyl-cyclohexadec-13-en-2,6-dion

In Analogie zu Beispiel 1 setzt man 260 mg (364 µmol) der nach Beispiel 26b dargestellten Verbindung um und isoliert nach Aufarbeitung und Reinigung 121 mg (249 µmol, 68%) der Titelverbindung als farbloses Öl.
¹H-NMR (CDCl₃): δ= 0,83 (3H), 0,90 (1H), 0,98 (3H), 1,01 (3H), 1,31 (3H), 1,37-2,00 (7H), 1,61 (3H), 2,08 (3H), 2,18 (1H), 2,37-2,52 (3H), 2,60 (1H), 3,35 (1H), 3,70 (1H), 3,83-4,32 (2H), 4,45 (1H), 5,08 (1H), 5,39 (1H), 6,58 (1H), 7,13 (1H), 7,35 (1H), 7,68 (1H), 8,53 (1H) ppm.

### Beispiel 27

### (1R,3S(E),7S,10R,11S,12S,16R)-7,11-Dihydroxy-10-ethyl-3-(1-methyl-2-(2-pyridyl)ethenyl)-8,8,12,16-tetramethyl-4,17-dioxabicyclo[14,1,0]heptadecan-5,9-dion (A) und (1S,3S(E),7S,10R,11S,12S,16S)-7,11-Dihydroxy-10-ethyl-3-(1-methyl-2-(2-pyridyl)ethenyl)-8,8,12,16-tetramethyl-4,17-dioxabicyclo[14,1,0]heptadecan-5,9-dion (B) (1R,3S(E),7S,10R,11S,12S,16R)-7,11-Dihydroxy-10-ethyl-3-(1-methyl-2-(2-N-oxidopyridyl)ethenyl)-8,8,12,16-tetramethyl-4,17-dioxabicyclo[14,1,0]heptadecan-5,9-dion (C) und (1S,3S(E),7S,10R,11S,12S,16S)-7,11-Dihydroxy-10-ethyl-3-(1-methyl-2-(2-N-oxidopyridyl)ethenyl)-8,8,12,16-pentamethyl-4,17-dioxabicyclo[14,1,0]heptadecan-5,9-dion (D)

In Analogie zu Beispiel 14 setzt man 59 mg (121 µmol) der nach Beispiel 26 dargestellten Verbindung um und isoliert nach Aufarbeitung und Reinigung 5 mg (10 µmol, 8%) der Titelverbindung A oder B, 2 mg (4 µmol, 3%) der Titelverbindung B oder A, 14 mg (27 µmol, 22%) der Titelverbindung C oder D und 6,9 mg (13 µmol, 11 %) der Titelverbindung D oder C jeweils als farblosen Feststoff.
¹H-NMR (CDCl₃) von A oder B: δ= 0,83 (3H), 0,92 (3H), 1,02 (3H), 1,09-2,19 (12H), 1,27 (3H), 1,37 (3H), 2,11 (3H), 2,43-2,61 (2H), 2,88 (1H), 3,31 (1H), 3,78 (1H), 4,26 (1H), 4,33 (1H), 5,48 (1H), 6,64 (1H), 7,12 (1H), 7,30 (1H), 7,67 (1H), 8,57 (1H) ppm.
¹H-NMR (CDCl₃) von B oder A: δ= 0,86 (3H), 0,93 (3H), 1,09 (3H), 1,19-2,19 (11H), 1,27 (3H), 1,38 (3H), 2,10 (3H), 2,50-2,63 (2H), 2,87 (1H), 2,98 (1H), 3,28 (1H), 3,71 (1H), 3,88 (1H), 4,31 (1H), 5,48 (1H), 6,62 (1H), 7,13 (1H), 7,28 (1H), 7,67 (1H), 8,58 (1H) ppm.
¹H-NMR (CDCl₃) von C oder D: δ= 0,84 (3H), 0,91 (3H), 1,06 (3H), 1,11-2,08 (10H), 1,26 (3H), 1,38 (3H), 2,02 (3H), 2,19 (1H), 2,37 (1H), 2,53 (1H), 2,92 (1H), 3,34 (1H), 3,56-3,72 (2H), 4,53 (1H), 5,05 (1H), 5,60 (1H), 6,99 (1H), 7,21 (1H), 7,33 (1H), 7,45 (1H), 8,28 (1H) ppm.
¹H-NMR (CDCl₃) von D oder C: δ= 0,84 (3H), 0,89 (3H), 1,07 (3H), 1,15-2,23 (11H), 1,22 (3H), 1,43 (3H), 2,09 (3H), 2,36 (1H), 2,53 (1H), 2,97 (1H), 3,02 (1H), 3,32 (1H), 3,58 (1H), 4,58 (1H), 5,44 (1H), 5,58 (1H), 7,06 (1H), 7,21 (1H), 7,36 (1H), 7,44 (1H), 8,29 (1H) ppm.

### Beispiel 28

### (4S,7R,8S,9S,13(Z),16S(E))-4,8-Dihydroxy-16-(1-methyl-2-(2-methyl-4-thiazolyl)ethenyl)-1-oxa-7,9,13-trimethyl-5,5-trimethylen-cyclohexadec-13-en-2,6-dion

### Beispiel 28a

### 1,1-Cyclobutandimethanol

Zu einer Lösung von 20 g (99,9 mmol) 1,1-Cyclobutandicarbonsäurediethylester in 200 ml absolutem Tetrahydrofuran werden bei 0°C 170 ml einer 1,2 molaren Lösung von Diisobutylaluminiumhydrid getropft. Man läßt eine Stunde bei 0°C nachrühren und addiert dann 30 ml Wasser. Es wird über Celite filtriert. Das Filtrat wird mit Natriumsulfat getrocknet und im Vakuum eingeengt. Das erhaltene Rohprodukt (9,9 g, 85,2 mmol, 85%) wird ohne Aufreinigung in die Folgestufe eingesetzt.

### Beispiel 28b

### 1-[[[Dimethyl(1,1-dimethylethyl)silyl]oxy]methyl]cyclobutanmethanol

Zu einer Suspension von 3,4 g Natriumhydrid (60%ig in Öl) in 35 ml absolutem Tetrahydrofuran wird bei 0°C eine Lösung von 9,9 g (85 mmol) der nach Beispiel 28a dargestellten Verbindung in 100 ml absolutem Tetrahydrofuran gegeben. Man läßt 30 Minuten nachrühren und addiert dann eine Lösung von 12,8 g *tert*.Butyldimethylsilylchlorid in 50 ml Tetrahydrofuran. Man läßt eine Stunde bei 25°C nachrühren und gießt dann das Reaktionsgemisch auf gesättigte wäßrige Natriumhydrogencarbonatlösung. Es wird mit Ethylacetat extrahiert. Die organische Phase wird mit gesättigter Natriumchloridlösung gewaschen und über Natriumsulfat getrocknet. Nach Abziehen des Lösungsmittels im Vakuum wird das erhaltene Rohprodukt durch Säulenchromatographie an Kieselgel mit einem Gemisch aus Hexan/Ethylacetat gereinigt. Man erhält 13,5 g (58,6 mmol, 69%) der Titelverbindung.
¹H-NMR (CDCl₃): δ = 0,04 (6H), 0,90 (9H), 1,70-2,00 (6H), 3,70 (4H) ppm.

### Beispiel 28c

### 1-[[[Dimethyl(1,1-dimethylethyl)silyl]oxy]methyl]cyclobutancarbaldehyd

Analog zu Beispiel 1 k werden aus 13,5 g (58,6 mmol) der unter 28b beschriebenen Verbindung nach Aufreinigung 7,7 g (33,7 mmol, 58%) der Titelverbindung erhalten.
¹ H-NMR (CDCl₃): δ = 9,70 s (1H), 3,83 s (2H), 2,20-2,30 m (2H), 1,85-2,00 m (4H), 0,90 s (9H), 0,03 s (6H) ppm.

### Beispiel 28d

### [1R-[1α(R*),2β]]-2-Phenylcyclohexyl 3-[1-[[[dimethyl(1,1-dimethylethyl)silyl]oxy] methyl]cyclobutyl]-3-hydroxypropanoat (A) und [1R-[1α(S*),2β]]-2-Phenylcyclohexyl 3-[1-[[[dimethyl(1,1-dimethylethyl)silyl]oxy]methyl]cyclobutyl]-3-hydroxypropanoat (B)

Aus 7,2 ml Diisopropylamin und Butyllithium (32 ml einer 1,6 molaren Lösung in Hexan) wird in absolutem Tetrahydrofuran Lithiumdiisopropylamid hergestellt. Dann addiert man bei -78°C eine Lösung von 11,2 g (1R-trans)-2-Phenylcyclohexyl acetat in 100 ml absolutem Tetrahydrofuran und läßt 30 Minuten bei dieser Temperatur nachrühren. Anschließend wird eine Lösung von 7,7 g (33,7 mmol) der nach Beispiel 28c dargestellten Verbindung in 50 ml Tetrahydrofuran addiert. Man läßt 1,5 Stunden bei -78°C nachrühren und gießt danach das Reaktionsgemisch auf gesättigte wäßrige Ammoniumchloridlösung. Man extrahiert mit Ethylacetat, wäscht die organische Phase mit gesättigter Natriumchloridlösung, trocknet über Natriumsulfat und engt im Vakuum ein. Nach Säulenchromatographie des Rohprodukts an Kieselgel mit einem Gemisch aus Hexan/Ethylacetat erhält man 6,34 g (14,2 mmol, 42%) der Titelverbindung A und 4,22 g (9,4 mmol, 28%) der Titelverbindung B.
¹H-NMR (CDCl₃) von A: δ = 0,04 (6H), 0,98 (9H), 2,69 (1H), 3,08 (1H), 3,60 (1H), 3,67 (1H), 3,78-3,84 (1H), 4,97 (1H), 7,15-7,30 (5H) ppm.
¹H-NMR (CDCl₃) von B: δ = 0,03 (6H) 0,90 (9H), 2,68 (1H), 2,80 (1H), 3,56 (2H), 3,68-3,72 (1H), 4,99 (1H), 7,18-7,30 m (5H) ppm.

### Beispiel 28e

### (S)-1-[1-[[[Dimethyl(1,1-dimethylethyl)silyl]oxy]methyl]cyclobutyl]-1,3-propandiol

Zu einer Lösung von 1 g (2,24 mmol) der nach Beispiel 28d dargestellten Verbindung A in 10 ml absolutem Toluol werden bei 0°C 4 ml einer 1,2 molaren Lösung von Diisobutylaluminiumhydrid in Toluol getropft. Man läßt 1,5 Stunden bei 0°C nachrühren und addiert dann 5 ml Wasser. Es wird über Celite filtriert. Das Filtrat wird über Natriumsulfat getrocknet und im Vakuum eingeengt. Man erhält nach Säulenchromatographie des Rohprodukts an Kieselgel mit einem Gemisch aus Hexan/Ethylacetat 370 mg (1,35 mmol, 60%) der Titelverbindung.
¹H-NMR (CDCl₃): δ = 0,05 (6H), 0,90 (9H), 1,55-1,60 (2H), 1,80 (2H), 1,90 (3H), 2,10 (1H), 3,75 (1H), 3,85-3,95 (4H) ppm.

### Beispiel 28f

### (S)-2,2-Dimethyl-4-[1-[[[dimethyl(1,1-dimethylethyl)silyl]oxy]methyl]cyclobutyl]-1,3-dioxan

Analog zu Beispiel 1 h werden aus 370 mg (1,35 mmol) der unter 28e beschriebenen Verbindung nach Aufreinigung 338 mg (1,07 mmol, 79%) der Titelverbindung erhalten.
¹H-NMR (CDCl₃): δ = 0,03 (6H), 0,88 (9H), 1,38 (3H), 1,42 (3H), 1,50-1,80 (4H), 2,00 (1H), 3,52 (1H), 3,62 (1H), 3,85-4,00 (3H) ppm.

### Beispiel 28g

### (S)-1-(2,2-Dimethyl-1,3-dioxan-4-yl)cyclobutanmethanol

1,27 g (4,04 mmol) der nach Beispiel 28f hergestellten Verbindung werden analog zu 1i mit 6 ml einer 1 molaren Lösung von Tetrabutylammoniumchlorid in Tetrahydrofuran umgesetzt. Man erhält nach Säulenchromatographie 794 mg (98%) der Titelverbindung.
¹H-NMR (CDCl₃): δ = 1,38 (3H), 1,46 (3H), 1,55-1,67 (2H), 1,75-2,05 (6H), 2,97 (1H), 3,62 (1H), 3,84-4,10 (4H) ppm.

### Beispiel 28h

### (S)-1-(2,2-Dimethyl-1,3-dioxan-4-yl)cyclobutancarbaldehyd

In Analogie zu Beispiel 1k setzt man 794 mg (3,97 mmol) 28g um und isoliert 786 mg (100%) der Titelverbindung als Rohprodukt, welches ohne Reinigung in die Folgestufe eingesetzt wird.

### Beispiel 28i

### (S)-1-(2,2-Dimethyl-1,3-dioxan-4-yl)-α-ethylcyclobutanmethanol

In Analogie zu Beispiel 11 setzt man 786 mg (3,97 mmol) der unter 28h beschriebenen Verbindung mit einer 2 molaren Lösung von Ethylmagnesiumchlorid in Tetrahydrofuran um. Man erhält nach Aufreinigung 835 mg (95%) der Titelverbindung.
¹H-NMR (CDCl₃): 8 = 1,05 (3H), 1,38 (3H), 1,49 (3H), 1,60-2,10 (8H), 2,60 (1H), 2,83 (1H), 3,50 (1H), 3,85-4,15 (3H) ppm.

### Beispiel 28k

### (S)-1-[1-(2,2-Dimethyl-1,3-dioxan-4-yl)cyclobutyl] propan-1-on

In Analogie zu Beispiel 1 m werden aus 835 mg (3,67 mmol) der unter 28i beschrieben Verbindung nach Aufreinigung 689 mg (83%) der Titelverbindung erhalten.
¹H-NMR (CDCl₃): δ = 1,03 (3H), 1,35 (1H), 1,36 (3H), 1,45 (3H), 1,55 (1H), 1,65-1,90 (2H), 2,02 (1H), 2,14-2,30 (2H), 2,33 (1H), 2,45-2,60 (2H), 3,80-4,00 (2H), 4,10 (1H) ppm.

### Beispiel 28l

### (S)-1-[1-(1,3-Dihydroxypropyl)cyclobutyl]propan-1-on

680 mg (3 mmol) der unter 28k beschriebenen Verbindung werden in 30 ml Tetrahydrofuran gelöst. Man addiert 1 ml Wasser und 30 mg p-Toluolsulfonsäure und läßt 30 Minuten bei 50°C nachrühren. Nach Aufarbeitung und Reinigung werden 471 mg (84%) der Titelverbindung erhalten.
¹H-NMR (CDCl₃): δ = 1,05 (3H), 1,10 (1H), 1,53 (1H), 1,65 (1H), 1,80-2,00 (3H), 2,15 (1H), 2,40-2,70 (3H), 3,35 (1H), 3,55 (1H), 3,88 (1H), 4,10 (1H) ppm.

### Beispiel 28m

### (S)-1-(1,3-Bis[[dimethyl(1,1-dimethylethyl)silyl]oxy]cyclobutyl)propan-1-on

Analog zu Beispiel 1aq werden aus 470 mg (2,54 mmol) der unter 281 beschrieben Verbindung nach Aufreinigung 709 mg (68%) der Titelverbindung erhalten.
¹H-NMR (CDCl₃): δ = 0,02 (6H), 0,15 (3H), 0,17 (3H), 0,90 (9H), 0,94 (9H), 1,05 (3H), 1,30-1,53 (2H), 1,70-1,85 (2H), 1,98 (1H), 2,23 (3H), 2,45-2,53 (2H), 3,54 (2H), 4,11 (1H) ppm.

### Beispiel 28n

### (2S,6E/Z,9S,10E)-9-[[(1,1-Dimethylethyl)diphenylsilyl]oxy]-11-(2-methylthiazol-4-yl)-1-(tetrahydropyran-2-yloxy)-2,6,10-trimethyl-undeca-6,10-dien

Analog zu Beispiel 24b/I werden aus 2,24 g (9,84 mmol) der unter 24a/I beschriebenen Verbindung und 12,2 g (14,81 mmol) der unter 1ai beschriebenen Verbindung unter Verwendung von Butyllithium als Base 3,01 g (47%) der Titelverbindung nach Aufreinigung erhalten.
¹H-NMR (CDCl₃): δ = 0,86 (3H), 1,04 (9H), 1,55+1,60 (3H), 1,30 (2H), 1,99 (3H), 2,25 (2H), 2,70 (3H), 1,10-3,20 (1H), 3,45-3,60 (2H), 3,86 (1H), 4,14 (1H), 4,54 (1H), 4,97 (1H), 6,22 (1H), 6,78 (1H), 7,30-7,50 (6H), 7,60-7,70 (4H) ppm.

### Beispiel 28o

### (2S,6E/Z,9S,10E)-11-(2-Methylthiazolyl-4-yl)-1-(tetrahydropyran-2-yloxy)-2,6,10-trimethyl-undeca-6,10-dien-9-ol

Analog zu Beispiel 1i werden aus 7,65 g (11,84 mmol) der unter 28n beschriebenen Verbindung nach Aufreinigung 4,53 g (94%) der Titelverbindung erhalten.
¹H-NMR (CDCl₃): δ= 0,91 (3H), 1,10 (1H), 1,65+1,71 (3H), 2,04 (3H), 2,39 (2H), 2,70 (3H), 3,12+3,21 (1H), 3,50+3,58 (2H), 3,85 (1H), 4,14 (1H), 4,55 (1H), 5,15 (1H), 6,56 (1H), 6,93 (1H) ppm.

### Beispiel 28p

### (2S,6E/Z,9S,10E)-9-[[Dimethyl(1,1-dimethylethyl)silyl]oxy]-11-(2-methylthiazol-4-yl)-1-(tetrahydropyran-2-yloxy)-2,6,10-trimethyl-undeca-6,10-dien

Analog zu Beispiel 1 ad werden aus 4,53 g der unter 28o beschriebenen Verbindung nach Aufreinigung 5,68 g (98%) der Titelverbindung erhalten.
¹H-NMR (CDCl₃): δ= 0,00 (3H), 0,03 (3H), 0,90 (12H), 1,56+1,64 (3H), 1,99 (3H), 2,21 (2H), 3,10+3,20 (1H), 3,45-3,60 (2H), 3,85 (1H), 4,10 (1H), 4,57 (1H), 5,12 (1H), 6,45 (1H), 6,90 (1H) ppm.

### Beispiel 28q

### (2S,6E/Z,9S,10E)-9-[[Dimethyl(1,1-dimethylethyl)silyl]oxy]-11-(2-methylthiazol-4-1)-2,6,10-trimethyl-undeca-6,10-dien-1-ol

Analog zu Beispiel 1f werden (2 Stunden Reaktionszeit bei 50°C) aus 5,68 g (10,88 mmol) der unter 28p beschriebenen Verbindung nach Aufreinigung 4,02 g (84%) der Titelverbindung erhalten.
¹H-NMR (CDCl₃): δ = 0,00 (3H), 0,05 (3H), 0,90 (12H), 1,60+1,65 (3H), 2,00 (3H), 2,23 (2H), 2,71 (3H), 3,38-3,55 (2H), 4,10 (1H), 5,09+5,14 (1H), 6,45+6,48 (1H), 6,91+6,93 (1H) ppm.

### Beispiel 28r

### (2S,6E/Z,9S,10E)-9-[[Dimethyl(1,1-dimethylethyl)silyl]oxy]-11-(2-methylthiazol-4-yl)-2,6,10-trimethyl-undeca-6,10-dien-1-al

Analog zu Beispiel 1 k werden aus 667 mg (1,5 mmol) der unter 28q beschriebenen Verbindung nach Filtration über Kieselgel 648 mg (98%) der Titelverbindung erhalten.
¹H-NMR (CDCl₃): δ = 0,01 (3H), 0,06 (3H), 0,90 (9H), 1,06+1,09 (3H), 1,58+1,66 (3H), 2,00 (3H), 4,10 (1H), 5,13 (1H), 6,46 (1H), 6,91+6,93 (1H) ppm.

### Beispiel 28s

### (3S,6R,7S,8S,12Z,15S,16E)-7-hydroxy-1,3,15-tris-[[dimethyl(1,1-dimethylethyl)silyl]oxy]-6,8,12,16-tetramethyl-4,4-trimethylen-17-(2-methylthiazol-4-yl)-heptadeca-12,16-dien-5-on (A) und (3S,6R,7S,8S,12E,15S,16E)-7-hydroxy-1,3,15-tris-[[dimethyl(1,1-dimethylethyl)silyl]oxy]-6,8,12,16-tetramethyl-4,4-trimethylen-17-(2-methylthiazol-4-yl)-heptadeca-1 2,16-dien-5-on (B)

Analog zu Beispiel 1ak werden aus 709 mg (1,71 mmol) der unter 28 m beschriebenen Verbindung und 667 mg (1,52 mmol) der unter 28r beschriebenen Verbindung nach Aufreinigung 352 mg (27%) der Titelverbindung A und 227 mg (17%) der Titelverbindung B erhalten.
¹H-NMR (CDCl₃) von Verbindung A: δ = 0,00 (3H), 0,04 (9H), 0,14 (3H), 0,16 (3H), 0,80 (3H), 0,88 (18H), 0,91 (9H), 1,03 (3H), 1,68 (3H), 2,00 (3H), 2,20-2,40 (3H), 2,72 (3H), 3,25 (1H), 3,44 (1H), 3,58 (3H), 4,10 (2H), 5,13 (1H), 6,42 (1H), 6,93 (1H) ppm.
¹H-NMR (CDCl₃) von Verbindung B: δ = 0,00 (3H), 0,04 (6H), 0,08 (3H), 0,15 (3H), 0,18 (3H), 0,80 (3H), 0,89 (18H), 0,92 (9H), 1,05 (3H), 1,60 (3H), 2,00 (3H), 2,20-2,40 (3H), 2,70 (3H), 3,25 (1H), 3,45 (1H), 3,60 (3H), 4,10 (2H), 5,15 (1H), 6,45 (1H), 6,91 (1H) ppm.

### Beispiel 28t

### (3S,6R,7S,8S, 12Z, 15S, 1 6E)-1,3,7,15-tetrakis-[[dimethyl(1, 1-dimethylethyl)silyl]oxy]-6,8,12,16-tetramethyl-4,4-trimethylen-17-(2-methylthiazol-4-yl)-heptadeca-12,16-dien-5-on

Analog zu Beispiel 1 aq werden aus 352 mg (0,41 mmol) der unter 28s beschriebenen Verbindung A 381 mg (95%) der Titelverbindung erhalten.
¹ H-NMR (CDCl₃): δ = 0,00 (3H), 0,02 (6H), 0,04 (3H), 0,07 (3H), 0,09 (3H), 0,13 (3H), 0,16 (3H), 0,90 (18H), 0,94 (18H), 0,95 (3H), 1,09 (3H), 1,68 (3H), 2,20-2,40 83H), 2,71 (3H), 3,10 (1H), 3,58 (2H), 3,78 (1H), 4,10 (2H), 5,13 (1H), 6,47 (1H), 6,90 (1H) ppm.

### Beispiel 28u

### (3S,6R,7S,8S,12Z,15S,16E)-3,7,15-tris-[[dimethyl(1,1-dimethylethyl)silyl]oxy]-1-hydroxy-6,8,12,16-tetramethyl-4,4-triniethylen-17-(2-methylthiazol-4-yl)-heptadeca-12,16-dien-5-on

Analog zu Beispiel 1 ar werden aus 381 mg (0,39 mmol) der unter 28t beschriebenen Verbindung 289 mg (86%) der Titelverbindung erhalten.
¹H-NMR (CDCl₃): δ= 0,01 (3H), 0,05 (3H), 0,08 (3H), 0,11 (3H), 0,16 (3H), 0,18 (3H), 0,90-1,00 (30H), 1,10 (3H), 1,67 (3H), 1,99 (3H), 2,20-2,40 (3H), 2,71 (3H), 3,14 (1H), 3,63 (2H), 3,82 (1H), 4,09 (2H), 5,12 (1H), 6,46 (1H), 6,92 (1H) ppm.

### Beispiel 28v

### (3S,6R,7S,8S,12Z,15S,16E)-3,7,15-tris-[[dimethyl(1,1-dirnethylethyl)silyl]oxyl-6,8,12,16-tetramethyl-4,4-trimethylen-17-(2-methylthiazol-4-yl)-5-oxo-heptadeca-12,16-dien-1-al

Analog zu Beispiel 1 k werden aus 285 mg (0,34 mmol) der unter 28u beschriebenen Verbindung nach Filtration über Kieselgel 284 mg (100%) der Titelverbindung erhalten.

### Beispiel 28w

### (3S,6R,7S,8S,12Z,15S,16E)-3,7,15-tris-[[Dimethyl(1,1-dimethylethyl)silyl]oxy]-6,8,12,16-tetramethyl-4,4-trimethylen-17-(2-methylthiazol-4-yl)-5-oxo-heptadeca-12,16-diensäure

Analog zu Beispiel 1 at werden aus 284 mg (0,34 mmol) der unter 28v beschriebenen Verbindung nach Aufreinigung 235 mg (81%) der Titelverbindung erhalten.
¹H-NMR (CDCl₃): δ = 0,00 (3H), 0,02 (3H), 0,04 (3H), 0,09 (3H), 0,14 (3H9, 0,19 (3H9, 0,87-0,96 (30H), 1,13 (3H), 1,70 (3H), 1,95 (3H), 2,12-2,30 (3H), 2,70 (3H), 3,00 (1H), 3,80 (1H), 4,13 (1H), 4,49 (1H), 5,18 (1H), 6,63 (1H), 6,93 (1H) ppm.

### Beispiel 28x

### (3S,6R,7S,8S,12Z,15S,16E)-3,7-Bis-[[diµethyl(1,1-dirnethylethyl)silyl]oxy]-15-hydroxy-17-(2-methylthiazol-4-yl)-6,8,12,16-tetramethyl-4,4-trimethylen-5-oxo heptadeca-12,16-diensäure

Analog zu Beispiel 1 1 werden aus 230 mg (0,27 mmol) der unter 28w beschriebenen Verbindung 200 mg (100%) der Titelverbindung erhalten, die ohne Aufreinigung in die Folgestufe eingesetzt wird.
¹H-NMR (CDCl₃): δ = 0,05 (3H), 0,10 (6H), 0,19 (3H), 0,90 (18H), 0,95 (3H), 1,12 (3H), 1,70 (3H), 2,00 (3H), 2,70 (3H), 3,00 (1H), 3,84 (1H), 4,15 (1H), 4,49 (1H), 5,15 (1H), 6,67 (1H), 6,91 (1H) ppm.

### Beispiel 28y

### (4S,7R,8S,9S,13(Z),16S(E))-4,8-Bis-[[dimethyl(1,1-dimethylethyl)silyl]oxy]-16-(1-methyl-2-(2-methylthiazol-4-yl)ethenyl)-1-oxa-7,9,13-trimethyl-5,5-trimethylen-cyclohexadec-13-en-2,6-dion

Analog zu Beispiel 1aw werden aus 200 mg (0,27 mmol) der unter 28x beschriebenen Verbindung nach Aufreinigung 101 mg (52%) der Titelverbindung erhalten.
¹H-NMR (CDCl₃): δ = -0,05 (3H), 0,12 (3H), 0,15 (6H), 0,82 (9H), 0,98 (9H), 1,00 (3H), 1,24 (3H), 1,68 (3H), 2,11 (3H), 2,28 (1H), 2,47 (1H), 2,60-2,70 (2H), 2,72 (3H), 2,98 (1H), 3,93 (1H), 4,41 (1H), 5,03 (1H), 5,17 (3H), 6,58 (1H), 6,98 (1H) ppm.

### Beispiel 28

### (4S,7R,8S,9S,13(Z),16S(E))-4,8-Dihydroxy-16-(1-methyl-2-(2-methyl-4-thiazolyl)ethenyl)-1-oxa-7,9,13-trimethyl-5,5-trimethylen-cyclohexadec-13-en-2,6-dion

Analog zu Beispiel 1 werden aus 101 mg (0,14 mmol) der unter 28y beschriebenen Verbindung 51 mg (73 %) der Titelverbindung erhalten.
¹H-NMR (CDCl₃): δ = 1,01 (3H), 1,28 (3H), 1,67 (3H), 2,09 (3H), 2,70 (3H), 3,01 (1H), 3,73 (1H), 4,46 (1H), 5,14 (1H), 5,19 (1H), 6,60 (1H), 6,96 (1H) ppm.

### Beispiel 29

### (1S,3S(E),7S,10R,11 S,12S,16R)-7,11-Dihydroxy-3-(1-methyl-2-(2-methyl-4-thiazolyl)ethenyl)-10,12,16-trimethyl-8,8-trimethylen-4,17-dioxabicyclo[14.1.0]heptadecan-5,9-dion (A) und (1R,3S(E),7S,10R,11S,12S,16S)-7,11-Dihydroxy-3-(1-methyl-2-(2-methyl-4-thiazolyl)ethenyl)-10,12,16-trimethyl-8,8-trimethylen-4,17-dioxabicyclo[14.1.0]heptadecan-5,9-dion (B)

Analog zu Beispiel 14 werden aus 47 mg (0,09 mmol) der unter 28 beschriebenen Verbindung 29 mg (59%) der Titelverbindung A und 7 mg (14%) der Titelverbindung B nach Trennung erhalten.
¹H-NMR (CDCl₃) von Verbindung A: δ= 1,01 (3H), 1,24 (3H), 1,28 (3H), 2,09 (3H), 2,72 (3H), 2,78 (1H), 3,05 (1H), 3,72 (1H), 4,20 (1H), 4,45 (1H), 5,37 (1H), 6,59 (1H), 6,96 (1H) ppm.
¹H-NMR (CDCl₃) von Verbindung B: δ= 0,94 (3H), 1,20 (3H), 1,26 (3H), 2,12 (3H), 2,71 (3H), 2,99 (1H), 3,11 (1H), 4,41 (1H), 4,39 (1H), 5,60 (1H), 6,62 (1H), 6,99 (1H) ppm.

### Beispiel 30

### (4S,7R,8S,9S,13(E),16S(E))-4,8-Dihydroxy-16-(1-methyl-2-(2-methyl-4-thiazolyl)ethenyl)-1-oxa-7,9,13-trimethyl-5,5-trimethylen-cyclohexadec-13-en-2,6-dion

### Beispiel 30a

### (3S,6R,7S,8S,12E,15S,16E)-1,3,7,15-tetrakis-[[dimethyl(1,1-dimethytethyl)silyl]oxy]-6,8,12,16-tetramethyl-4,4-trimethylen-17-(2-methylthiazol-4-yl)-heptadeca-12,16-dien-5-on

Analog zu Beispiel 1aq werden aus 227 mg (0,27 mmol) der unter 28s beschriebenen Verbindung B 230 mg (90%) der Titelverbindung erhalten.
¹H-NMR (CDCl₃): δ = 0,01 (3H), 0,03 (3H), 0,04 (3H), 0,06 (3H), 0,08 (3H), 0,11 (3H), 0,15 (3H), 0,17 (3H), 0,87-0,98 (39 H), 1,06 (3H), 1,57 (3H), 2,00 (3H), 2,20-2,39 (3H), 2,70 (3H), 3,09 (1H), 3,61 (2H), 3,78 (1H), 4,10 (2H), 5,14 (3H), 6,45 (1H), 6,91 (1H) ppm.

### Beispiel 30b

### (3S,6R,7S,8S,12E,15S,16E)-3,7,15-tris-[[dimethyl(1,1-dimethylethyl)silyl)oxy]-1-hydroxy-6,8,12,16-tetramethyl-4,4-trimethylen-17-(2-methylthiazol-4-yl)-heptadeca-12,16-dien-5-on

Analog zu Beispiel 1 ar werden aus 230 mg (0,24 mmol) der unter 30a beschriebenen Verbindung 170 mg (84%) der Titelverbindung erhalten.
¹H-NMR (CDCl₃): δ= 0,01 (3H), 0,06 (3H), 0,08 (3H), 0,10 (3H), 0,17 (3H), 0,19 (3H), 0,85-1,00 (30H), 1,10 (3H), 1,62 (3H), 2,15-2,40 (3H), 2,71 (3H), 3,12 (1H), 3,63 (2H), 3,79 (1H), 4,09 (2H), 5,13 (1H), 6,42 (1H), 6,90 (1H) ppm.

### Beispiel 30c

### (3S,6R,7S,8S,12E,15S,16E)-3,7,15-tris-[[dimethyl(1,1-dimethylethyl)silyl]oxy]-6,8,12,16-tetramethyl-4,4-trimethylen-17-(2-methylthiazol-4-yl)-5-oxo-heptadeca-12,16-dien-1-al

Analog zu Beispiel 1 k werden aus 170 mg (0,20 mmol) der unter 30b beschriebenen Verbindung nach Filtration über Kieselgel 170 mg (100%) der Titelverbindung erhalten.

### Beispiel 30d

### (3S,6R,7S,8S,12E,15S,16E)-3,7,15-tris-[[Dimethyl(1,1-dimethylethyl)silyl]oxy]-4,4,6,8,12,16-tetramethyl-4,4-trimethylen-17-(2-methylthiazol-4-yl)-5-oxo-heptadeca-12,16-diensäure

Analog zu Beispiel 1 at werden aus 170 mg (0,20 mmol) der unter 30c beschriebenen Verbindung nach Aufreinigung 144 mg (83%) der Titelverbindung erhalten.
¹H-NMR (CDCl₃): δ = 0,01 (3H), 0,05 (3H), 0,06 (3H), 0,09 (3H), 0,15 (3H), 0,20 (3H), 0,85-1,00 (30H), 1,12 (3H), 1,55 (3H), 1,97 (3H), 2,71 (3H), 3,09 (1H), 3,82 (1H), 4,10 (1H), 4,41 (1H), 5,11 (1H), 6,46 (1H), 6,95 (1H) ppm.

### Beispiel 30e

### (3S,6R,7S,8S,12E,15S,16E)-3,7-Bis-[[dimethyl(1,1-dimethylethyl)silyl]oxy]-15-hydroxy-17-(2-methylthiazol-4-yl)-6,8,12,16-tetramethyl-4,4-trimethylen-5-oxo heptadeca-12,16-diensäure

Analog zu Beispiel 1i werden aus 140 mg (0,16 mmol) der unter 30d beschriebenen Verbindung 121 mg (100%) der Titelverbindung erhalten, die ohne Aufreinigung in die Folgestufe eingesetzt werden.
¹H-NMR (CDCl₃): δ= 0,05 (3H), 0,09 (6H), 0,18 (3H), 0,85-0,95 (18H), 0,98 (3H), 1,11 (3H), 1,61 (3H), 2,00 (3H), 2,69 (3H), 3,02 (1H), 3,82 (1H), 4,15 (1H), 4,40 (1H), 5,15 (1H), 6,54 (1H), 6,91 (1H) ppm.

### Beispiel 30f

### (4S,7R,8S,9S,13(E),16S(E))-4,8-Bis-[[dimethyl(1,1-dimethylethyl)silyl]oxy]-16-(1-methyl-2-(2-methylthiazol-4-yl)ethenyl)-1 -oxa-7,9,13-trimethyl-5,5-trimethylen-cyclohexadec-1 3-en-2,6-dion

Analog zu Beispiel 1aw werden aus 121 mg (0,16 mmol) der unter 30e beschriebenen Verbindung nach Aufreinigung 55 mg (48%) der Titelverbindung erhalten.
¹H-NMR (CDCl₃): δ = 0,01 (3H), 0,09 (3H), 0,15 (6H), 0,92 (9H), 0,96 (9H), 0,98 (3H), 1,26 (3H), 1,50 (3H), 2,19 (3H), 2,73 (3H), 2,91 (1H), 4,18 (1H), 4,63 (1H), 5,09 (1H), 5,31 (1H), 6,53 (1H), 6,93 (1H) ppm.

### Beispiel 30

### (4S,7R,8S,9S,13(E),16S(E))-4,8-Dihydroxy-16-(1 -methyl-2-(2-methyl-4-thiazolyl)ethenyl)-1 - oxa-7,9,13-trimethyl-5,5-trimethylen-cyclohexadec-13-en-2,6-dion

Analog zu Beispiel 1 werden aus 55 mg (0,08 mmol) der unter 30f beschriebenen Verbindung 27 mg (67 %) der Titelverbindung erhalten.
¹H-NMR (CDCl₃): δ= 1,03 (3H), 1,23 (3H), 1,55 (3H), 2,07 (3H), 2,72 (3H), 3,04 (1H), 3,32 (1H), 3,51 (1H), 3,70 (1H), 4,46 (1H), 5,06 (1H), 5,49 (1H) 6,59 (1H), 7,02 (1H) ppm.

### Beispiel 31

### (1R,3S(E),7S,10R,11S,12S,16R)-7,11-Dihydroxy-3-(1-methyl-2-(2-methyl-4-thiazolyl)ethenyl)-10,12,16-trirnethyl-8,8-trirnethylen-4,17-dioxabicyclo[14.1.0]heptadecan-5,9-dion (A) und (1S,3S(E),7S,10R,11S,12S,16S)-7,11-Dihydroxy-3-(1-methy)-2-(2-methyl-4-thiazolyl)ethenyl)-10,12,16-trimethyl-8,8-trimethylen-4,17-dioxabicyclo[14.1.0]heptadecan-5,9-dion (B)

Analog zu Beispiel 14 werden aus 25 mg (0,05 mmol) der unter 30 beschriebenen Verbindung 10 mg (39%) der Titelverbindung A und 8 mg (31 %) der Titelverbindung B nach Trennung erhalten.
¹H-NMR (CDCl₃) von Verbindung A: δ = 1,02 (3H), 1,25 (3H), 1,27 (3H), 2,08 (3H), 2,71 (3H), 2,84 (1H), 3,13 (1H), 3,72 (1H), 4,93 (1H), 5,51 (1H), 6,68 (1H), 7,04 (1H) ppm.
¹H-NMR (CDCl₃) von Verbindung B: δ= 0,98 (3H), 1,27 (3H), 1,28 (3H), 2,11 (3H), 2,89 (1H), 3,08 (1H), 3,70 (1H), 4,48 (1H), 5,43 (1H), 6,58 (1H), 6,97 (1H) ppm.

### Beispiel 32

### (4S,7R,8S,9S,13(Z),16S(E))-4,8-Dihydroxy-9,13-dimethyl-7-ethyl-16-(1-methyl-2-(2-methyl-4-thiazolyl)ethenyl)-1-oxa-5,5-trimethylen-cyclohexadec-13-en-2,6-dion

### Beispiel 32a

### 1-[[[Dimethyl(1,1-dimethylethyl)silyl]oxy]methyl]-α-propylcyclobutanmethanol

Analog zu Beispiel 11 werden aus 24,15 g (105,8 mmol) der unter 28c beschriebenen Verbindung nach Aufreinigung 20,81 g (72%) der Titelverbindung erhalten.
¹H-NMR (CDCl₃): δ = 0,09 (6H), 0,93 (9H), 0,95 (3H), 1,36 (3H), 1,48-1,80 (3H), 1,87 (3H), 2,08 (1H), 3,18 (1H), 3,56 (1H), 3,72 (1H), 3,86 (1H) ppm.

### Beispiel 32b

### 1-[1-[[[Dimethyl(1,1-dimethylethyl)silyl]oxy]methyl]cyclobut 1-yl]-1-butanon

Analog zu Beispiel 1k werden aus 20,81 g (76,34 mmol) der unter 32a beschriebenen Verbindung nach Filtration über Kieselgel 20,7 g (100%) der Titelverbindung erhalten.
¹H-NMR (CDCl₃): δ = 0,05 (6H), 0,88 (9H), 0,92 (3H), 1,59 (2H), 1,75-1,95 (4H), 2,23-2,34 (2H), 2,43 (2H), 3,81 (2H) ppm.

### Beispiel 32c

### 1-[1-(Hydroxymethyl)cyclobut 1-yl]-1-butanon

Analog zu Beispiel 1i werden aus 20,7 g (76,34 mmol) der unter 32b beschriebenen Verbindung nach Aufreinigung 11,57 g (97%) der Titelverbindung erhalten.
¹H-NMR (CDCl₃): δ = 0,94 (3H), 1,64 (2H), 1,85-2,10 (4H), 2,29-2,43 (2H), 2,53 (2H), 3,87 (2H) ppm.

### Beispiel 32d

### 1-(1-Oxobutyl)cyclobutancarbaldehyd

Analog zu Beispiel 1 k werden aus 2,34 g (15 mmol) der unter 32c beschriebenen Verbindung nach Filtration über Kieselgel 2,31 g (100%) der Titelverbindung erhalten.
¹H-NMR (CDCl₃): δ = 0,92 (3H), 1,62 (2H), 1,85-2,01 (4H), 2,38-2,55 (6H), 9,69 (1H) ppm.

### Beispiel 32e

### (4S,5R)-3-(Bromacetyl)-4-methyl-5-phenyloxazolidin-2-on

Zu einer Lösung von 33,06 g (186,6 mmol) (4S,5R)-4-Methyl-5-phenyloxazolidin-2-on in 500 ml Tetrahydrofuran gibt man innerhalb von 30 Minuten bei -70°C unter Argon 82 ml einer 2,5 molaren Lösung von Butyllithium in Hexan. Anschließend wird eine Lösung von 15,55 ml (187 mmol) Bromacetylchlorid in 250 ml Tetrahydrofuran so hinzugetropt, daß die Innentemperatur nicht über -65°C steigt. Anschließend wird eine Stunde bei -70°C nachgerührt. Danach gießt man das Reaktionsgemisch auf 50 ml gesättigte wäßrige Ammoniumchloridlösung. Man addiert danach 90 ml gesättigte wäßrige Natriumhydrogencarbonatlösung, läßt auf 25°C kommen, verdünnt mit Wasser und extrahiert mit Ethylacetat. Die organische Phase wird mit gesättigter wäßriger Natriumchloridlösung gewaschen, über Natriumsulfat getrocknet und über Kieselgel chromatographiert. Man erhält 42,32 g (76%) der Titelverbindung.
¹H-NMR (CDCl₃): δ = 0,95 (3H), 4,57 (2H), 4,80 (1H), 5,76 (1H), 7,2-7,5 (5H) ppm.

### Beispiel 32f

### [4S-[3(R*),4α,5α]]-3-[3-Hydroxy-1-oxo-3-[1-(1-oxobutyl)cyclobut-1-yl]propyl]-4-methyl-5-phenyloxazolidin-2-on

Zu einer Suspension von 5 g (40,68 mmol) wasserfreiem Chrom(ll)chlorid in 60 ml Tetrahydrofuran gibt man unter Argon 200 mg (1,5 mmol) wasserfreies Lithiumiodid. Anschließend wird eine Mischung von 5 g (16,77 mmol) der unter 32e beschriebenen Verbindung und 2,31 g (15 mmol) der unter 32d beschriebenen Verbindung in 10 ml Tetrahydrofuran addiert (exotherme Reaktion, die Innentemperatur sollte nicht über 35°C steigen). Man läßt eine Stunde bei 25°C nachrühren und addiert dann bei leichter Kühlung 50 ml gesättigte wäßrige Natriumchloridlösung. Es wird weitere 30 Minuten bei 25°C nachgerührt. Dann wir mit Ethylacetat extrahiert. Die organische Phase wird mit gesättigter wäßriger Natriumchloridlösung gewaschen, über Natriumsulfat getrocknet und über Kieselgel chromatographiert. Man erhält 3,89 g (69%) der Titelverbindung.
¹H-NMR (CDCl₃): δ = 0,90-0,99 (6H), 1,58-1,73 (4H), 1,79-2,05 (2H), 2,10-2,69 (7H), 3,00-3,12 (2H), 3,44 (1H), 4,39 (1H), 4,78 (1H), 5,70 (1H), 7,27-7,33 (2H), 7,35-7,48 (3H) ppm.

### Beispiel 32g

### [4S-[3(R*),4α,5α]]-3-[3-[[Dimethyl(1,1-dimethylethyl)silyl]oxy]-1-oxo-3-[1-(1-oxobutyl)cyclobut-1 -yl]propyl]-4-methyl-5-phenyloxazolidin-2-on

Analog zu Beispiel 1 aq werden aus 3,89 g (10,42 mmol) der unter Beispiel 32f beschriebenen Verbindung nach Aufreinigung 3,94 g (76%) der Titelverbindung erhalten.
¹H-NMR (CDCl₃): δ = 0,08 (3H), 0,20 (3H), 0,85-0,98 (15H), 1,55-1,93 (4H), 2,03 (1H), 2,20-2,38 (3H), 2,45-2,67 (2H), 2,91-3,13 (2H), 4,62-4,75 (2H), 5,67 (1H), 7,29-7,47 (5H) ppm.

### Beispiel 32h

### (S)-3-[3-[[Dimethyl(1,1-dimethyl)silylloxyl-3-[1-(1-oxopropyl)cyclobut-1-yl]propansäure

Zu einer Lösung von 3,94 g (8,08 mmol) der unter 32g beschriebenen Verbindung in 40 ml einer Mischung aus Tetrahydrofuran und Wasser (4:1) gibt man bei 0°C 3,29 ml (32,3 mmol) einer 30%igen Wasserstoffperoxidlösung (exotherme Reaktion, die Innentemperatur sollte nicht über 15°C steigen). Man läßt 5 Minuten bei 0°C nachrühren und addiert dann eine Lösung von 309 mg (32,3 mmol) Lithiumhydroxid in 16 ml Wasser. Anschließend wird 3 Stunden bei 0°C nachgerührt. Danach wird das Reaktionsgemisch vorsichtig auf eiskalte Natriumthiosulfatlösung gegossen. Es wird 5 Minuten bei 0°C und 15 Minuten bei 25°C nachgerührt. Danach wird das Tetrahydrofuran im Vakuum abgezogen und die verbleibende Lösung mit 5 N Salzsäure bis pH=1 angesäuert. Man extrahiert mit Dichlormethan. Die organische Phase wird mit gesättigter wäßriger Natriumchloridlösung gewaschen, über Natriumsulfat getrocknet und über Kieselgel chromatographiert. Man erhält 2,34 g (89%) der Titelverbindung und 1,04 g (4S,5R)-4-Methyl-5-phenyloxazolidin-2-on, welches erneut in Beispiel 32e eingesetzt werden kann.
¹H-NMR (CDCl₃): δ = 0,09 (3H), 0,18 (3H), 0,86-0,97 (12H), 1,59 (2H), 1,56-1,94 (3H), 2,05-2,36 (4H), 2,40-2,57 (3H), 4,44 (1H) ppm.

### Beispiel 32i

### (3S,6R,7S,8S,12Z,15S,16E)-3,7,15-tris-[[Dimethyl(1,1-dimethylethyl)silyl]oxy]-6-ethyl-8,12,16-trimethyl-4,4-trimethylen-17-(2-methylthiazol-4-yl)-5-oxo-heptadeca-12,16-diensäure (A) und (3S,6R,7S,8S,12E,15S,16E)-3,7,15-tris-[[Dimethyl(1,1-dimethylethyl)silyl]oxy]-6-ethyl-8,12,16-trimethyl-4,4-trimethylen-17-(2-methylthiazol-4-yl)-5-oxo-heptadeca-12,16-diensäure (B) und

### (3S,6S,7R,8S,12Z,15S,16E)-3,7,15-tris-[[Dimethyl(1,1-dimethylethyl)silyloxy]-6-ethyl-8,12,16-trimethyl-4,4-trimethylen-17-(2-methylthiazol-4-yl)-5-oxo-heptadeca-12,16-diensäure (C) und (3S,6S,7R,8S,12E,15S,16E)-3,7,15-tris-[[Dimethyl(1,1-dimethylethyl)silyl]oxy]-6-ethyl-8,12,16-trimethyl-4,4-trimethylen-17-(2-methylthiazol-4-yl)-5-oxo-heptadeca-12,16-diensäure (D)

Analog zu Beispiel 1ak wird aus 842 µl (5,99 mmol) Diisopropylamin und 3,74 ml (5,99 mmol) einer 1,6 molaren Lösung von Butyllithium in Hexan in 5 ml absolutem Tetrahydrofuran Lithiumdiisopropylamid hergestellt. Zu dieser Lösung wird bei -78°C eine Lösung von 787 mg (2,4 mmol) der unter 32h beschriebenen Verbindung in 5 ml absolutem Tetrahydrofuran addiert. Man rührt 1 Stunde bei -40°C nach. Anschließend kühlt man erneut auf -78°C und addiert eine Lösung von 524 mg (1,2 mmol) der unter 28r beschriebenen Verbindung in 5 ml absolutem Tetrahydrofuran. Es wird eine weitere Stunde bei -78°C nachgerührt. Anschließend gießt man das Reaktionsgemisch auf gesättigte wäßrige Ammoniumchloridlösung, addiert 0,45 ml Eisessig und läßt eine Stunde nachrühren. Danach wird mit Ethylacetat extrahiert. Die organische Phase wird mit gesättigter wäßriger Natriumchloridlösung gewaschen, über Natriumsulfat getrocknet und im Vakuum eingeengt. Das erhaltene Rohprodukt (max. 920 mg(100%)) wird in 10 ml Dichlormethan gelöst und analog zu Beispiel 1 aq in die persilylierte Verbindung überführt. Das so erhaltene Rohprodukt wird in 30 ml eines 1:1 Gemisches aus Dichlormethan und Methanol gelöst. Man addiert bei 0°C 280 mg (1,2 mmol) DL-Camphersulfonsäure und läßt 2,5 Stunden bei dieser Temperatur nachrühren. Danach werden 2,5 ml Triethylamin addiert. Anschließend engt man im Vakuum ein. Der Rückstand wird in Dichlormethan aufgenommen. Man wäscht mit 1 normaler Salzsäure und gesättigter wäßriger Natriumchloridlösung. Es wird über Natriumsulfat getrocknet und im Vakuum eingeengt. Das erhaltene Rohrpodukt wird durch mehrmalige Säulenchromatographie an Kieselgel getrennt. Man erhält: 229 mg (22%) Verbindung A 174 mg (17%) Verbindung B sowie 292 mg (28%) eines Gemisches der Verbindungen C und D.
¹H-NMR (CDCl₃) von Verbindung A: δ = 0,00 (3H), 0,02 (3H), 0,04 (3H), 0,08 (3H), 0,13 (3H), 0,18 (3H), 0,85-0,99 (33H), 1,79 (3H), 1,94 (3H), 2,10-2,28 (5H), 2,30-2,45 (2H), 2,48 (H), 2,70 (3H), 2,90 (1H), 3,78 (1H), 4,17 (1H), 4,46 (1H), 5,19 (1H), 6,64 (1H), 6,95 (1H) ppm.
¹H-NMR (CDCl₃) von Verbindung B: δ = 0,00 (3H), 0,03 (3H), 0,06 (3H), 0,07 (3H), 0,14 (3H), 0,19 (3H), 0,78-0,98 (33 H), 1,55 (3H), 1,92 (3H), 2,12-2,50 (10H), 2,69 (3H), 2,72 (1H), 3,00 (1H), 3,88 (1H), 4,08 (1H), 4,41 (1H), 5,10 (1H), 6,48 (1H), 6,94 (1H) ppm.

### Beispiel 32k

### (3S,6R,7S,8S,12Z,15S,16E)-3,7-Bis-[[dimethyl(1,1-dimethylethyl)silyl]oxy]-15-hydroxy-17-(2-methylthiazol-4-yl)-6-ethyl-8,12,16-trimethyl-4,4-trimethylen-5-oxo heptadeca-12,16-diensäure

Analog zu Beispiel 1i werden aus 229 mg (0,26 mmol) der unter 32i beschriebenen Verbindung A 200 mg (100%) der Titelverbindung erhalten, die ohne Aufreinigung in die Folgestufe eingesetzt werden.

### Beispiel 32l

### (4S,7R,8S,9S,13(Z),16S(E))-4,8-Bis-[[dimethyl(1,1-dimethylethyl)silyl]oxy]-16-(1-methy)-2-(2-methylthiazol-4-yl)ethenyl)-1-oxa-7-ethyl-9,13-trimethyl-5,5-trimethylen-cyclohexadec-13-en-2,6-dion

Analog zu Beispiel 1 aw werden aus 200 mg (0,26 mmol) der unter 32k beschriebenen Verbindung nach Aufreinigung 100 mg (51 %) der Titelverbindung erhalten.
¹H-NMR (CDCl₃): δ = 0,93 (3H), 0,11 (3H), 0,16 (6H), 0,83 (9H), 0,88 (3H), 0,96 (9H), 1,02 (3H), 1,68 (3H), 2,12 (3H), 2,30-2,70 (6H), 2,72 (3H), 3,03 (1H), 4,07 (1H), 4,43 (1H), 5,01 (1H), 5,17 (1H), 6,58 (1H), 6,98 (1H) ppm.

### Beispiel 32

### (4S,7R, 8S,9S,13(Z),16S(E))-4,8-Dihydroxy-9,13-dimethyl-7-ethyl-16-(1-methyl-2-(2-methyl-4-thiazolyl)ethenyl)-1 -oxa-5,5-trimethylen-cyclohexadec-1 3-en-2,6-dion

Analog zu Beispiel 1 werden aus 100 mg (0,13 mmol) der unter 32l beschriebenen Verbindung nach Aufreinigung 63 mg (90%) der Titelverbindung erhalten.
¹H-NMR (CDCl₃): δ = 0,95 (3H), 1,00 (3H), 1,68 (3H), 2,05 (3H), 2,72 (3H), 2,97 (1H), 3,67 (1H), 4,46 (1H), 5,08 (1H), 5,23 (1H), 6,59 (1H), 6,98 (1H) ppm.

### Beispiel 33

### (1S,3S(E),7S,10R,11S,12S,16R)-7,11-Dihydroxy-3-(1-methyl-2-(2-methyl-4-thiazolyl)ethenyl)-12,16-dimethyl-10-ethyl-8,8-trimethylen-4,17-dioxabicyclo[14.1.0]heptadecan-5,9-dion (A) und (1 R,3S(E),7S,10R,11 S,12S,16S)-7,11 -Dihydroxy-3-(1-methyl-2-(2-methyl)-4-thiazolyl)ethenyl)-12,16-dimethyl-10-ethyl-8,8-trimethylen-4,17-dioxabicyclo[14.1.0]heptadecan-5,9-dion (B)

Analog zu Beispiel 14 werden aus 50 mg (0,10 mmol) der unter 32 beschriebenen Verbindung 24 mg (47%) der Titelverbindung A und 6 mg (12%) der Titelverbindung B erhalten.
¹H-NMR (CDCl₃) von Verbindung A: δ = 0,95 (3H), 0,98 (3H), 1,30 (3H), 2,07 (3H), 2,71 (3H), 2,76 (1H), 3,03 (1H), 3,69 (1H), 4,44 (1H), 5,40 (1H), 6,58 (1H), 6,97 (1H) ppm.
¹H-NMR (CDCl₃) von Verbindung B: δ = 0,92 (3H), 0,95 (3H), 2,10 (3H), 2,71 (3H), 2,88 (1H), 3,04 (1H), 3,78 (1H), 4,49 (1H), 5,53 (1H), 6,64 (1H), 6,99 (1H) ppm.

### Beispiel 34

### (4S,7R,8S,9S,13(E),16S(E))-4,8-Dihydroxy-9,13-dimethyl-7-ethyl-16-(1-methyl-2-(2-methyl-4-thiazolyl)ethenyl)-1 -oxa-5,5-trimethylen-cyclohexadec-13-en-2,6-dion

### Beispiel 34a

### (3S,6R,7S,8S,12E,15S,16E)-3,7-Bis-((dimethyl(1,1-dimethylethyl)silyl]oxy]-15-hydroxy-17-(2-methylthiazol-4-yl)-6-ethyl-8,12,16-trimethyl-4,4-trimethylen-5-oxo heptadeca-12,16-diensäure

Analog zu Beispiel 1i werden aus 174 mg (0,20 mmol) der unter Beispiel 32i beschriebenen Verbindung B 151 mg (100%) der Titelverbindung erhalten, die ohne Aufreinigung in die Folgestufe eingesetzt werden.

### Beispiel 34b

### (4S,7R,8S,9S,13(E),16S(E))-4,8-Bis-[[dimethyl(1,1-dimethylethyl)silyl]oxy]-16-(1-methyl-2-(2-methylthiazol-4-yl)ethenyl)-1-oxa-7-ethyl-9,13-trimethyl-5,5-trimethylen-cyclohexadec-1 3-en-2,6-dion

Analog zu Beispiel 1 aw werden aus 151 mg (0,20 mmol) der unter 34a beschrieben Verbindung nach Aufreinigung 86 mg (58%) der Titelverbindung erhalten.
¹H-NMR (CDCl₃): δ = 0,04 (3H), 0,11 (6H), 0,13 (3H), 0,86 (3H), 0,88 (9H), 0,93 (9H), 1,01 (3H), 1,54 (3H), 2,17 (3H), 2,24-2,46 (3H), 2,72 (3H), 2,83 (1H), 3,03 (1H), 4,08 (1H), 4,53 (1H), 5,13 (1H), 5,27 (1H), 6,53 (1H), 6,96 (1H) ppm.

### Beispiel 34

### B(4S,7R,8S,9S,13(E),16S(E))-4,8-Dihydroxy-9,13-dimethyl-7-ethyl-16-(1 -methyl-2-(2-methyl-4-thiazolyl)ethenyl)-1 -oxa-5,5-trimethylen-cyclohexadec-1 3-en-2,6-dion

Analog zu Beispiel 1 werden aus 86 mg (0,12 mmol) der unter 34b beschriebenen Verbindung 39 mg (65%) der Titelverbindung erhalten.
¹H-NMR (CDCl₃): δ = 0,93 (3H), 1,06 (3H), 1,53 (3H), 2,03 (3H), 2,69 (3H), 3,09 (1H), 3,82 (1H), 4,52 (1H), 5,03 (1H), 5,36 (1H), 6,60 (1H), 7,03 (1H) ppm.

### Beispiel 35

### (1 R,3S(E),7S,10R,11S,12S,16R)-7,11-Dihydroxy-3-(1-methyl-2-(2-methyl-4-thiazolyl)ethenyl)-12,16-dimethyl-10-ethyl-8,8-trimethylen-4,17-dioxabicyclo[14.1.0]heptadecan-5,9-dion (A) und (1S,3S(E),7S,10R,11S,12S,16S)-7,11-Dihydroxy-3-(1-methyl-2-(2-methyl-4-thiazolyl)ethenyl)-12,16-dimethyl-10-ethyl-8,8-trimethylen-4,17-dioxabicyclo[14.1.0]heptadecan-5,9-dion (B)

Analog zu Beispiel 14 werden aus 30 mg (0,06 mmol) der unter Beispiel 34 beschriebenen Verbindung 10 mg (32%) der Titelverbindung A und 8 mg (26%) der Titelverbindung B erhalten.
¹H-NMR (CDCl₃) von Verbindung A: δ = 0,95 (3H), 1,03 (3H), 1,23 (3H), 2,08 (3H), 2,71 (3H), 2,84 (1H), 3,16 (1H), 3,82 (1H), 4,52 (1H), 5,50 (1H), 6,72 (1H), 7,06 (1H) ppm.
¹ H-NMR (CDCl₃) von Verbindung B: δ = 0,93 (3H), 0,98 (3H), 1,22 (3H), 2,06 (3H); 2,70 (3H), 2,88 (1H), 3,05 (1H), 3,62 (1H), 4,46 (1H), 5,41 (1H), 6,60 (1H), 6,96 (1H) ppm.

## Patentansprüche

1. Epothilon-Derivate der allgemeinen Formel I, 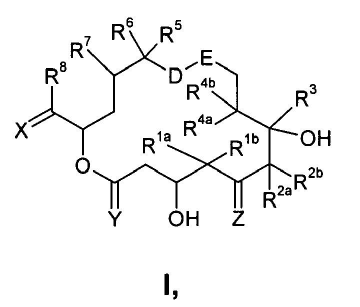 worin
R^{1a}, R^{1b} gleich oder verschieden sind und Wasserstoff, C₁-C₁₀-Alkyl, Aryl, C₇-C₂₀-Aralkyl, oder gemeinsam eine -(CH₂)ₘ-Gruppe mit m = 2, 3, 4 oder 5,
R^{2a}, R^{2b} gleich oder verschieden sind und Wasserstoff, C₁-C₁₀-Alkyl, Aryl, C₇-C₂₀-Aralkyl oder gemeinsam eine -(CH₂)ₙ-Gruppe mit n = 2, 3, 4 oder 5, wobei,
falls -D-E- für -CH₂-CH₂- oder Y für ein Sauerstoffatom steht,
R^{2a}/R^{2b} nicht Wasserstoff / Methyl sein können,
R³ Wasserstoff, C₁-C₁₀-Alkyl, Aryl, C₇-C₂₀-Aralkyl,
R^{4a}, R^{4b} gleich oder verschieden sind und Wasserstoff, C₁-C₁₀-Alkyl, Aryl, C₇-C₂₀-Aralkyl oder gemeinsam eine -(CH₂)ₚ-Gruppe mit p = 2, 3, 4 oder 5,
D-E eine Gruppe H₂C-CH₂, HC=CH , C≡C, 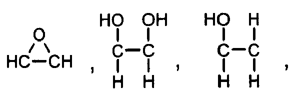
R⁵ Wasserstoff, C₁-C₁₀-Alkyl, Aryl, C₇-C₂₀-Aralkyl,
R⁶, R⁷ je ein Wasserstoffatom, gemeinsam eine zusätzliche Bindung oder ein Sauerstoffatom,
R⁸ Wasserstoff, C₁-C₂₀-Alkyl, Aryl, C₇-C₂₀-Aralkyl, die alle substituiert sein können,
X ein Sauerstoffatom, zwei Alkoxygruppen OR²³, eine C₂-C₁₀-Alkylenα,ω-dioxygruppe, die geradkettig oder verzweigt sein kann, H/OR⁹ oder eine Gruppierung CR¹⁰R¹¹,
wobei
R²³ für einen C₁-C₂₀-Alkylrest,
R⁹ für Wasserstoff oder eine Schutzgruppe PG^{X},
R¹⁰, R¹¹ gleich oder verschieden sind und für Wasserstoff, einen C₁-C₂₀-Alkyl-, Aryl-, C₇-C₂₀-Aralkylrest oder R¹⁰ und R¹¹ zusammen mit dem Methylenkohlenstoffatom gemeinsam für einen 5- bis 7-gliedrigen carbocyclischen Ring
stehen,
Y ein Sauerstoffatom oder zwei Wasserstoffatome,
Z ein Sauerstoffatom oder H/OR¹²,
wobei
R¹² Wasserstoff oder eine Schutzgruppe PG^{z}
ist,
bedeuten.

2. Epothilon-Derivate der allgemeinen Formel I nach Anspruch 1, worin Y, Z, R^{1a}, R^{1b}, R^{2a} und R^{2b} alle die in der allgemeinen Formel I angegebenen Bedeutungen haben können, und der Rest des Moleküls identisch ist mit dem natürlich vorkommenden Epothilon A oder B.

3. Epothilon-Derivate der allgemeinen Formel l nach Anspruch 1, worin R³, R^{4a}, R^{4b}, D-E, R⁵, R⁶ und R⁷ alle die in der allgemeinen Formel I angegebenen Bedeutungen haben können, und der Rest des Moleküls identisch ist mit dem natürlich vorkommenden Epothilon A oder B.

4. Epothilon-Derivate der allgemeinen Formel I nach Anspruch 1, worin R⁶, R⁷, R⁸ und X alle die in der allgemeinen Formel I angegebenen Bedeutungen haben können, und der Rest des Moleküls identisch ist mit dem natürlich vorkommenden Epothilon A oder B.

5. Epothilon-Derivate der allgemeinen Formel I nach Anspruch 1, worin Y, Z, R^{1a}, R^{1b}, R^{2a}, R^{2b}, R³, R^{4a}, R^{4b}, D-E, R⁵, R⁶ und R⁷ alle die in der allgemeinen Formel I angegebenen Bedeutungen haben können, und der Rest des Moleküls identisch ist mit dem natürlich vorkommenden Epothilon A oder B.

6. Epothilon-Derivate der allgemeinen Formel I nach Anspruch 1, worin Y, Z, R^{1a}, R^{1b}, R^{2a} , R^{2b}, R⁶, R⁷, R⁸ und X alle die in der allgemeinen Formel I angegebenen Bedeutungen haben können, und der Rest des Moleküls identisch ist mit dem natürlich vorkommenden Epothilon A oder B.

7. Epothilon-Derivate der allgemeinen Formel I nach Anspruch 1, worin R³, R^{4a}, R^{4b}, D-E, R⁵, R⁶, R⁷, R⁸ und X alle die in der allgemeinen Formel 1 angegebenen Bedeutungen haben können, und der Rest des Moleküls identisch ist mit dem natürlich vorkommenden Epothilon A oder B.

8. Verbindungen der allgemeinen Formel I, nämlich
(4S,7R,8S,9S,13(Z),16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion und
(4S,7R,8S,9S,13E,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion (B)
(1S,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
(1R,3S(E),7S,10R,11S,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(1S,3S(E),7S,10R,11S,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
(1R,3S(E),7S,10R,11S,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(4S,7S,8R,9S,13Z,16S(E))-4,8-Dihydroxy-7-ethyl-16-(1-methyl-2-(2-methyl-4-thiazolyl)ethenyl)-1-oxa-5,5,9,13-tetramethyl-cyclohexadec-13-en-2,6-dion und
(4S,7S, 8R,9S,13E,16S(E))-4,8-Dihydroxy-7-ethyl-16-(1-methyl-2-(2-methyl-4-thiazolyl)ethenyl)-1-oxa-5, 5,9,13-tetramethyl-cyclohexadec-13-en-2,6-dion
(1S,3S(E),7S,10S,11R,12S,16R)-7,11-Dihydroxy-3-(1-methyl-2-(2-methyl-4-thiazotyl)ethenyl)-10-ethyl-8,8,12,16-tetramethyl-4,17-dioxabicyclo[14.1.0]heptadecan-5,9-dion und
(1R,3S(E),7S,10S,11R,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(1S,3S(E),7S,10S,11R,12S,16R)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion und
(1R,3S(E),7S,10S,11R,12S,16S)-7,11-Dihydroxy-3-(1-methyl-2-(2-methyl-4-thiazolyl)ethenyl)-10-ethyl-8,8,12,16-tetramethyl-4,17-dioxabicyclo[14.1.0]heptadecan-5,9-dion
(4S,7R,8S,9S,13(Z),16S(E))-4,8-Dihydroxy-5,5,7,9,13-pentamethyl-16-((3-pyridyl)ethenyl)-1-oxa-cyclohexadec-13-en-2,6-dion und
(4S,7R,8S,9S,13E,16S(E))-4,8-Dihydroxy-5,5,7,9,13-pentamethyl-16-((3-pyridyl)ethenyl)-1-oxa-cyclohexadec-13-en-2,6-dion
(1S,3S(E),7S,10R,11S,12S,16R)-7,11-Dihydroxy-8,8,10,12,16-pentamethyl-3-((3-pyridyl)ethenyl)-4,17-dioxabicyclo[14.1.0]heptadecan-5,9-dion und
(1S,3S(E),7S,10R,11S,12S,16S)-7,11-Dihydroxy-8,8,10,12,16-pentamethyl-3-((3-pyridyl)ethenyl)-4,17-dioxabicyclo[14.1.0]heptadecan-5,9-dion
(4S,7R,8S,9S,13(Z),16S(E))-4,8-Dihydroxy-5,5,7,9,13-pentamethyl-16-((4-pyridyl)ethenyl)-1-oxa-cyclohexadec-13-en-2,6-dion und
(4S,7R,8S,9S,13E,16S(E))-4,8-Dihydroxy-5,5,7,9,13-pentamethyl-16-((4-pyridyl)ethenyl)-1-oxa-cyclohexadec-13-en-2,6-dion
(1S,3S(E),7S,10R,11S,12S,16R)-7,11-Dihydroxy-8,8,10,12,16-pentamethyl-3-((4-pyridyl)ethenyl)-4,17-dioxabicyclo[14.1.0]heptadecan-5,9-dion und
(1S,3S(E),7S, 10R, 11 S, 12S, 16S)-7,11-Dihydroxy-8,8,10,12,16-pentamethyl-3-((4-pyridyl)ethenyl)-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-1-oxa-7-phenyl-5,5,9,13-tetramethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-10-phenyl-8,8,12,16-tetramethyl-4,17-dioxabicyclo[14.1.0]heptadecan-5,9-dion
(1(R oder S),3S(E),7S,10R,11S,12S,16S)-7,11-Dihydroxy-3-(1-methyl-2-(2-methyl-4-thiazoly))ethenyl)-10-phenyl-8,8,12,16-tetramethyl-4,17-dioxabicyclo[14.1.0]heptadecan-5,9-dion
(4S,7R,8S,9S,13(E oder Z),16S(E))-7-Benzyl-4,8-dihydroxy-16-(1-methyl-2-(2-methyl-4-thiazolyl)ethenyl)-1-oxa-5,5,9,13-tetramethyl-cyclohexadec-13-en-2,6-dion
(1(S oder R),3S(E),7S,10R,11S,12S,16R)-10-Benzyl-7,11-dihydroxy-3-(1-methyl-2-(2-methyl-4-thiazolyl)ethenyl)-8,8,12,16-tetramethyl-4,17-dioxabicyclo[14.1.0]heptadecan-5,9-dion
(1(R oder S),3S(E),7S,10R,11S,12S,16S)-10-Benzyl-7,11-dihydroxy-3-(1-methyl-2-(2-methyl-4-thiazolyl)ethenyl)-8,8,10,12,16-tetramethyl-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5,7,13-tetramethyl-9-trifluormethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-8,8,10,16-tetramethyl-12-trifluormethyl-4,17-dioxabicyclo[14.1.0]heptadecan-5,9-dion
(1(R oder S),3S(E),7S,10R,11S,12S,16S)-7,11-Dihydroxy-3-(1-methyl-2-(2-methyl-4-thiazolyl)ethenyl)-8,8,10,16-tetramethyl-12-trifluormethyl-4,17-dioxabicyclo[14.1.0]heptadecan-5,9-dion
(4S,7R,8S,9S,11E/Z,13(E oder Z), 16S(E))-4,8-Dihydroxy-16-(1-methyl-2-(2-methyl-4-thiazolyl)ethenyl)-1-oxa-5,5,7,9,13-pentamethyl-cyclohexadec-11,13-dien-2,6-dion
(1(S oder R),3S(E),7S,10R,11S,12S,14E/Z,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-en-5,9-dion
(1(R oder S),3S(E),7S,10R,11S,12S,14E/Z,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-en-5,9-dion
(4S,7R.8S,9S,13(E oder Z),16S(E))-4,8-Dihydroxy-16-(1-methyl-2-(2-methyl-4-thiazolyl)ethenyl)-1-oxa-5,5,7,9,13-pentamethyl-cyclohexadec-13-en-11-in-2,6-dion
(1(S oder R),3S(E),7S,10R,11S,12S,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-in-5,9-dion
(1(R oder S),3S(E),7S,10R,11S,12S,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-14-in-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5,7,9-tetramethyl-13-trifluormethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-8,8,10,12-tetramethyl-16-trifluormethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-8,8,10,12-tetramethyl-16-trifluormethyl-4,17-dioxabicyclo[14.1.0]heptadeca-5,9-dion
(4S,7R,8S,9S,13(E oder Z),16S(E))-4,8-Dihydroxy-16-(1-methyl-2-(2-methyl-4-thiazolyl)ethenyl)-1-oxa-13-pentafluorethyl-5,5,7,9-tetramethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-16-pentafluorethyl-8,8,10,12-tetramethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-16-pentafluorethyl-8,8,10,12-tetramethyl-4,17-dioxabicydo[14.1.0]heptadeca-5,9-dion
(4S,7R,8S,9S,13(E oder Z),16S(E))-4,8-Dihydroxy-16-(1-methyl-2-(2-methyl-4-thiazolyl)ethenyl)-1-oxa-5,5-(1,3-trimethylen)-7,9,13-trimethyl-cyclohexadec-13-en-2,6-dion
(1(S oder R),3S(E),7S,10R,11S,12S,16R)-7,11-Dihydroxy-3-(1-methyl-2-(2-methyl-4-thiazolyl)ethenyl)-8,8-(1,3-trimethylen)-10,12,16-trimethyl-4,17-dioxabicyclo[14.1.0]heptadeca-5,9-dion
(1(R oder S),3S(E),7S,10R,11S,12S,16S)-7,11-Dihydroxy-3-(1-methyl-2-(2-methyl-4-thiazolyl)ethenyl)-8,8-(1,3-trimethylen)-10,12,16-trimethyl-4,17-dioxabicyclo[14.1.0]heptadeca-5,9-dion
(4S,7R,8S,9S,11E/Z,13(E oder Z), 16S(E))-4,8-Dihydroxy-13-ethyl-16-(1-methyl-2-(2-methyl-4-thiazolyl)ethenyl)-1-oxa-5,5,7,9-tetramethyl-cyclohexadec-11,13-dien-2,6-dion
(1(S oder R),3S(E),7S,10R,11S,12S,14E/Z,16R)-7,11-Dihydroxy-16-ethyl-3-(1-methyl-2-(2-methyl-4-thiazolyl)ethenyl)-8,8,10,12-tetramethyl-4,17-dioxabicyclo[14.1.0]heptadec-14-en-5,9-dion
(1(R oder S),3S(E),7S,10R,11S,12S,14E/Z,16S)-7,11-Dihydroxy-16-ethyl-3-(1-methyl-2-(2-methyl-4-thiazolyl)ethenyl)-8,8,10,12-tetramethyl-4,17-dioxabicyclo[14.1.0]heptadec-14-en-5,9-dion
(4S,7R,8S,9S,11E/Z,13(E oder Z),16S(E))-4,8-Dihydroxy-16-(1-methyl-2-(2-methyl-4-thiazolyl)ethenyl)-1-oxa-13-propyl-5,5,7,9-tetramethyl-cyclohexadec-11,13-dien-2,6-dion
(1(S oder R),3S(E),7S,10R,11S,12S,14E/Z,16R)-7,11-Dihydroxy-3-(1-methyl-2-(2-methyl-4-thiazolyl)ethenyl)-16-propyl-8,8,10,12-tetramethyl-4,17-dioxabicyclo[14.1.0]heptadec-14-en-5,9-dion
(1(R oder S),3S(E),7S,10R,11S,12S,14E/Z,16S)-7,11-Dihydroxy-3-(1-methyl-2-(2-methyl-4-thiazolyl)ethenyl)-16-propyl-8,8,10,12-tetramethyl-4,17-dioxabicyclo[14.1.0]heptadec-14-en-5,9-dion
(4S,7R,8S,9S,13(E oder Z),16S(E))-4,8-Dihydroxy-16-(1-methyl-2-(2-pyridyl)ethenyl)-1-oxa-5,5,7,9,13-pentamethyl-cyclohexadec-13-en-2,6-dion
(1(S oder R),3S(E),7S,10R,11S,12S,16R)-7,11-Dihydroxy-3-(1-methyl-2-(2-pyridyl)ethenyl)-8,8,10,12,16-pentamethyl-4,17-dioxabicyclo[14.1.0]heptadecan-5,9-dion
(1(R oder S),3S(E),7S,10R, 11S,12S,16S)-7,11-Dihydroxy-3-(1-methyl-2-(2-pyridyl)ethenyl)-8,8,10,12,16-pentamethyl-4,17-dioxabicyclo[14.1.0]heptadecan-5,9-dion
(4S,7R,8S,9S,13(E oder Z),16S(E))-4,8-Dihydroxy-16-(1-methyl-2-(4-pyridyl)ethenyl)-1-oxa-5,5,7,9,13-pentamethyl-cyclohexadec-13-en-2,6-dion
(1(S oder R),3S(E),7S,10R,11S,12S,16R)-7,11-Dihydroxy-3-(1-methyl-2-(4-pyridyl)ethenyl)-8,8,10,12,16-pentamethyl-4,17-dioxabicyclo[14.1.0]heptadecan-5,9-dion
(1(R oder S),3S(E),7S,10R,11S,12S,16S)-7,11-Dihydroxy-3-(1-methyl-2-(4-pyridyl)ethenyl)-8,8,10,12,16-pentamethyl-4,17-dioxabicyclo[14.1.0]heptadecan-5,9-dion
(4S,7R,8S,9S,13(E oder Z),16S(E))-4,8-Dihydroxy-16-(1-methyl-2-(2-methyl-4-thiazolyl)ethenyl)-5,5,7,913-pentamethyl-cyclohexadec-13-en-6-on
(1(S oder R),3S(E),7S,10R,11S,12S,16R)-7,11-Dihydroxy-3-(1-methyl-2-(2-methyl-4-thiazolyl)ethenyl)-8,8,10,12,16-pentamethyl-4,17-dioxabicyclo[14.1.0]heptadec-9-on
(1(R oder S),3S(E),7S, 10R,11S,12S,16S)-7,11-Dihydroxy-3-(1-methyl-2-(2-methyl-4-thiazolyl)ethenyl)-8,8,10,12,16-pentamethyl-4,17-dioxabicyclo[14.1.0]heptadec-9-on.

9. Verfahren zur Herstellung der Epothilon-Derivate der allgemeinen Formel I nach Anspruch 1 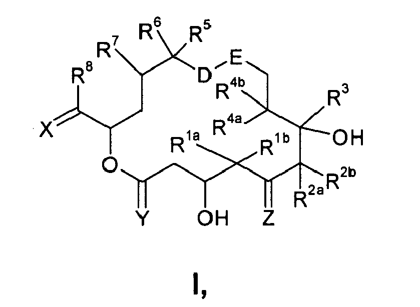 worin
die Substituenten die in der allgemeinen Formel I angegebenen Bedeutungen haben,
**dadurch gekennzeichnet, daß**
ein Fragment der allgemeinen Formel A 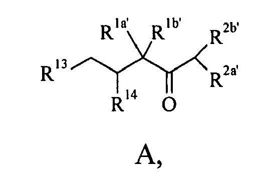 worin
R^{1a'}, R^{1b'}, R^{2a'} und R^{2b'} die bereits für R^{1a}, R^{1b}, R^{2a} und R^{2b} genannten Bedeutungen haben und
R¹³ CH₂OR^{13a}, CH₂-Hal, CHO, CO₂R^{13b}, COHal,
R¹⁴ Wasserstoff, OR^{14a}, Hal, OSO₂R^{14b},
R^{13a}, R^{14a} Wasserstoff, SO₂-Alkyl, SO₂-Aryl, SO₂-Aralkyl oder gemeinsam eine - (CH₂)ₒ-Gruppe oder gemeinsam eine CR^{15a}R^{15b}-Gruppe,
R^{13b}, R^{14b} Wasserstoff, C₁-C₂₀-Alkyl, Aryl, C₇-C₂₀-Aralkyl,
R^{15a}, R^{15b} gleich oder verschieden sind und Wasserstoff, C₁-C₁₀-Alkyl, Aryl, C₇-C₂₀-Aralkyl, oder gemeinsam eine -(CH₂)q-Gruppe,
Hal Halogen,
o 2 bis 4,
q 3 bis 6,
einschließlich aller Stereoisomeren sowie deren Gemische
bedeuten, sowie
freie Hydroxylgruppen in R¹³ und R¹⁴ verethert oder verestert, freie Carbonylgruppen in A und R¹³ ketalisiert, in einen Enolether überführt oder reduziert sowie freie Säuregruppen in A in deren Salze mit Basen überführt sein können.
mit einem Fragment der allgemeinen Formel B 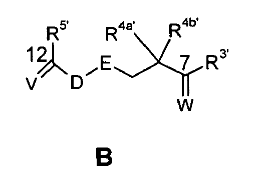 worin
R^{3'}, R^{4a'}, R^{4b'} und R^{5'} die bereits für R³, R^{4a}, R^{4b} und R⁵ genannten Bedeutungen haben, und
V ein Sauerstoffatom, zwei Alkoxygruppen OR¹⁷, eine C₂-C₁₀-Alkylen-α,ω-dioxygruppe, die geradkettig oder verzweigt sein kann oder H/OR¹⁶,
W ein Sauerstoffatom, zwei Alkoxygruppen OR¹⁹, eine C₂-C₁₀-Alkylen-α,ω-dioxygruppe, die geradkettig oder verzweigt sein kann oder H/OR¹⁸,
R¹⁶, R¹⁸ unabhängig voneinander Wasserstoff oder eine Schutzgruppe PG¹
R¹⁷, R¹⁹ unabhängig voneinander C₁-C₂₀-Alkyl,
bedeuten,
zu einem Teilfragment der allgemeinen Formel AB 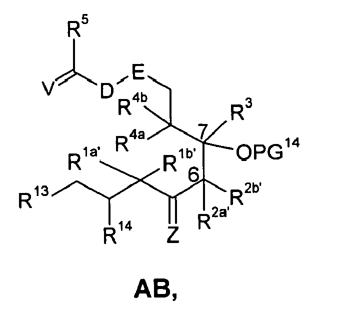 worin R^{1a'}, R^{1b'}, R^{2a'}, R^{2b'}, R³, R^{4a}, R^{4b}, R⁵, R¹³, R¹⁴, D, E, V und Z die bereits genannten Bedeutungen haben und PG¹⁴ ein Wasserstoffatom oder eine Schutzgruppe PG darstellt,
umgesetzt und dieses Teilfragment AB mit einem Fragment der allgemeinen Formel C 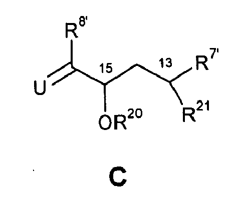 worin
R⁸' die bereits in der allgemeinen Formel I für R⁸ genannte Bedeutung hat und
R^{7'} ein Wasserstoffatom,
R²⁰ ein Wasserstoffatom oder eine Schutzgruppe PG²
R²¹ eine Hydroxygruppe, Halogen, eine geschützte Hydroxygruppe OPG³, ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
U ein Sauerstoffatom, zwei Alkoxygruppen OR²³, eine C₂-C₁₀-Alkylen-α,ω-dioxygruppe, die geradkettig oder verzweigt sein kann, H/OR⁹ oder eine Gruppierung CR¹⁰R¹¹,
wobei
R²³ für einen C₁-C₂₀-Alkylrest,
R⁹ für Wasserstoff oder eine Schutzgruppe PG³,
R¹⁰, R¹¹ gleich oder verschieden sind und für Wasserstoff, einen C₁-C₂₀-Alkyl-, Aryl-, C₇-C₂₀-Aralkylrest oder
R¹⁰ und R¹¹
zusammen mit dem Methylenkohlen-stoffatom gemeinsam für einen 5- bis 7-gliedrigen carbocyclischen Ring
stehen,
bedeuten,
zu einem Teilfragment der allgemeinen Formel ABC 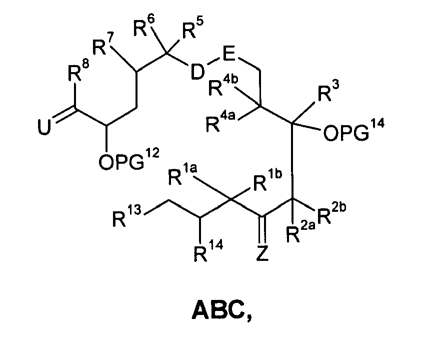 worin R^{1a'}, R^{1b'}, R^{2a'}, R^{2b'}, R³, R^{4a}, R^{4b}, R⁵, R⁶, R⁷, R⁸, R¹³, R¹⁴, D, E, U und Z die bereits genannten Bedeutungen haben,
umgesetzt und dieses Teilfragment der allgemeinen Formel ABC zu einem Epothilon-Derivat der allgemeinen Formel I cyclisiert wird.

10. Pharmazeutische Präparate enthaltend mindestens eine Verbindung der allgemeinen Formel I gemäß Anspruch 1 sowie einen pharmazeutisch verträglichen Träger.

11. Verwendung der Verbindungen der allgemeinen Formel 1 gemäß Anspruch 1 zur Herstellung von Arzneimitteln.

12. Verfahren zur Herstellung von Verbindungen der allgemeinen Formel A 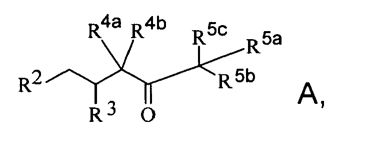 worin
R² CH₂OR^{2a}, CHO, CO₂R^{2b}, COX,
R^{2a}, R^{2b} Wasserstoff, C₁-C₂₀-Alkyl, Aryl, C₇-C₂₀-Aralkyl,
R³ Wasserstoff, OR^{3a}, X, OSO₂R^{3b},
R^{3a} Wasserstoff oder gemeinsam mit R^{2a} eine -(CH₂)ₙ-Gruppe oder eine CR^{6a}R^{6b}-Gruppe,
R^{3b} C₁-C₄-Alkyl, Aryl,
X Halogen,
n 2 bis 4,
R^{6a}, R^{6b} gleich oder verschieden sind und C₁-C₈-Alkyl, C₆-C₁₀-Aryl, oder gemeinsam eine -(CH₂)ₒ-Gruppe,
o 3 bis 6,
R^{6a} zusätzlich die Bedeutung von Wasserstoff annehmen kann,
R^{4a}, R^{4b} gleich oder verschieden sind und Wasserstoff, C₁-C₁₀-Alkyl, C₇-C₂₀-Aralkyl, oder gemeinsam eine -(CH₂)ₘ-Gruppe,
m 2 bis 5,
R^{5a}, R^{5b} gleich oder verschieden sind und Wasserstoff, C₁-C₁₀-Alkyl, C₇-C₂₀-Aralkyl, oder gemeinsam eine -(CH₂)p-Gruppe,
p 2 bis 5,
R^{5c} Wasserstoff,
einschließlich aller Stereoisomeren sowie deren Gemische
bedeuten sowie
freie Hydroxylgruppen in R² und R³ verethert oder verestert, freie Carbonylgruppen in A und R² ketalisiert, in einen Enolether überführt oder reduziert sowie freie Säuregruppen in A in deren Salze mit Basen überführt sein können,
**dadurch gekennzeichnet, daß**
a) ein Pantolacton der allgemeinen Formel lla 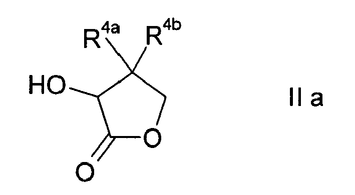 worin
R^{4a} und R^{4b} jeweils eine Methylgruppe ist
oder
b) ein Malonsäuredialkylester der allgemeinen Formel XXVIII 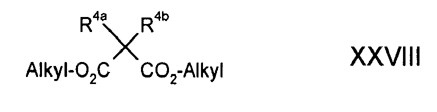 worin
R^{4a}, R^{4b} die in der allgemeinen Formel A angegebene Bedeutung haben, und Alkyl unabhängig voneinander einen C₁-C₂₀-Alkyl-, C₃-C₁₀-Cycloalkyl- oder C₄-C₂₀-Alkylcycloalkylrest bedeuten,
als Ausgangsprodukt verwendet wird.

13. Verbindungen der allgemeinen Formel A' 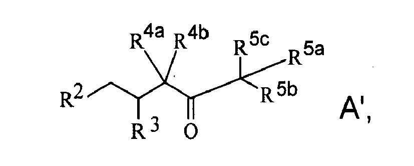 worin
R² CH₂OR^{2a}, CHO, CO₂R^{2b}, COX,
R^{2a}, R^{2b} Wasserstoff, C₁-C₂₀-Alkyl, Aryl, C₇-C₂₀-Aralkyl,
R³ Wasserstoff, OR^{3a}, X, OSO₂R^{3b},
R^{3a} Wasserstoff oder gemeinsam mit R^{2a} eine -(CH₂)ₙ-Gruppe oder eine CR^{6a}R^{6b}-Gruppe,
R^{3b} C₁-C₄-Alkyl, Aryl,
X Halogen,
n 2 bis 4,
R^{6a}, R^{6b} gleich oder verschieden sind und C₁-C₈-Alkyl, C₆-C₁₀-Aryl, oder gemeinsam eine -(CH₂)ₒ-Gruppe,
o 3 bis 6,
R^{6a} zusätzlich die Bedeutung von Wasserstoff annehmen kann,
R^{4a}, R^{4b} gleich oder verschieden sind und Wasserstoff, C₁-C₁₀-Alkyl, C₇-C₂₀-Aralkyl, oder gemeinsam eine -(CH₂)ₘ-Gruppe,
m 2 bis 5,
R^{5a}, R^{5b} gleich oder verschieden sind und Wasserstoff, C₁-C₁₀-Alkyl, C₇-C₂₀-Aralkyl, oder gemeinsam eine -(CH₂)p-Gruppe,
p 2 bis 5,
R^{5c} Wasserstoff,
einschließlich aller Stereoisomeren sowie deren Gemische
bedeuten sowie
freie Hydroxylgruppen in R² und R³ verethert oder verestert, freie Carbonylgruppen in A und R² ketalisiert, in einen Enolether überführt oder reduziert sowie freie Säuregruppen in A in deren Salze mit Basen überführt sein können,
ausgenommen der Verbindungen

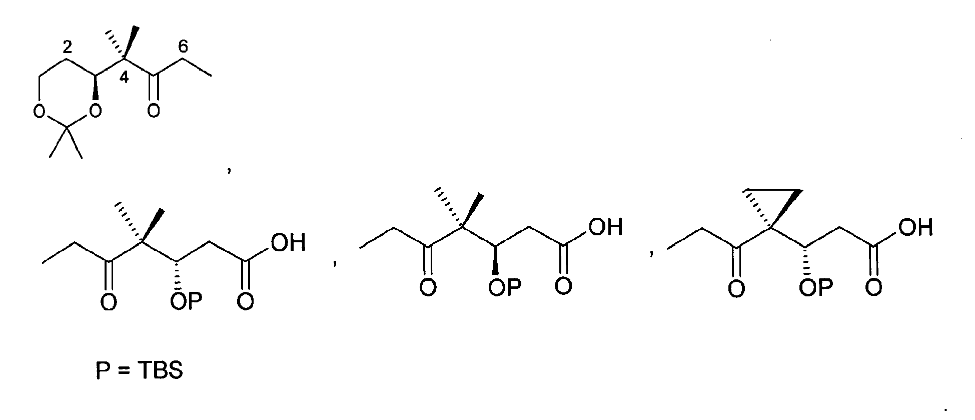

14. Verfahren zur Herstellung von Verbindungen der allgemeinen Formel A" 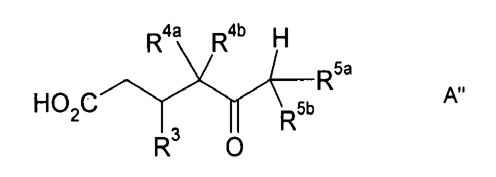 worin
R³ OR^{3a} und
R^{3a} Wasserstoff oder eine Schutzgruppe PG
R^{4a}, R^{4b} gleich oder verschieden sind und Wasserstoff, C₁-C₁₀-Alkyl, C₇-C₂₀-Aralkyl, oder gemeinsam eine -(CH₂)ₘ-Gruppe,
m 2 bis 5,
R^{5a}, R^{5b} gleich oder verschieden sind und Wasserstoff, C₁-C₁₀-Alkyl, C₇-C₂₀-Aralkyl, oder gemeinsam eine -(CH₂)p-Gruppe,
p 2 bis 5,
einschließlich aller Stereoisomeren sowie deren Gemische
bedeuten sowie
freie Carbonylgruppen in I ketalisiert sein können,
**dadurch gekennzeichnet, daß** eine Verbindung der allgemeinen Formel II 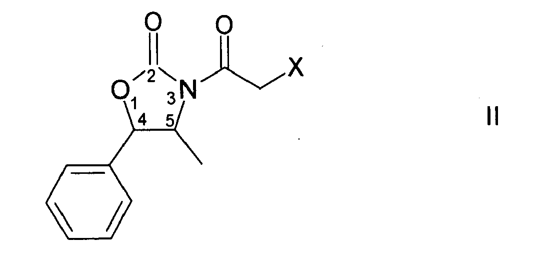 worin
X ein Chlor- oder Bromatom ist, und der 2-Oxazolidinon-Ring entweder (4R,5S)- oder (4S,5R)-Konformation aufweist,
mit einer Verbindung der allgemeinen Formel III 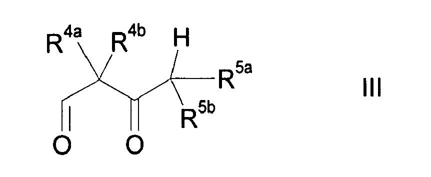 worin
R^{4a}, R^{4b} gleich oder verschieden sind und Wasserstoff, C₁-C₁₀-Alkyl, C₇-C₂₀-Aralkyl, oder gemeinsam eine -(CH₂)ₘ-Gruppe,
m 2 bis 5,
R^{5a}, R^{5b} gleich oder verschieden sind und Wasserstoff, C₁-C₁₀-Alkyl, C₇-C₂₀-Aralkyl, oder gemeinsam eine -(CH₂)ₚ-Gruppe,
p 2 bis 5,
bedeuten,
zu einer Verbindung der allgemeinen Formel IV umgesetzt 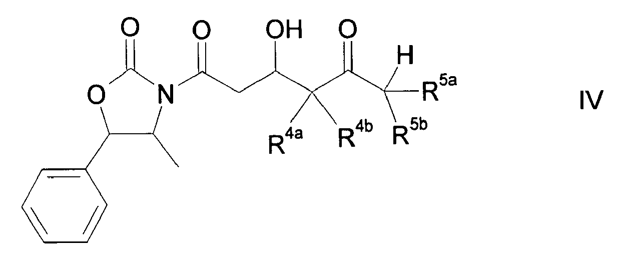 worin
der 2-Oxazolidinon-Ring (4R,5S)- und das 3'-Kohlenstoffatom R-Konformation oder
der 2-Oxazolidinon-Ring (4S,5R)- und das 3'-Kohlenstoffatom S-Konformation
aufweisen,
die 3'-Hydroxygruppe in IV mit einer Schutzgruppe PG geschützt, der Oxazolidinon-Ring abgespalten und gegebenenfalls die Schutzgruppe PG abgespalten wird.

15. Verfahren nach Anspruch 14, **dadurch gekennzeichnet, daß** die Verbindung der allgemeinen Formel II in Gegenwart von Chrom(II)chlorid mit einer Verbindung der allgemeinen Formel III umgesetzt wird.

16. Verfahren nach Anspruch 14 oder 15, **dadurch gekennzeichnet, daß** der abgespaltene Oxazolidinon-Ring enantiomerenrein wiedergewonnen wird.

17. Verbindungen der allgemeinen Formel C, 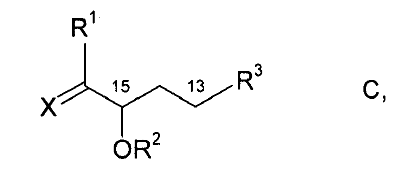 worin
R¹ Wasserstoff, C₁-C₂₀-Alkyl, Aryl, C₇-C₂₀-Aralkyl, die alle substituiert sein können,
R² Wasserstoff oder eine Schutzgruppe PG¹,
R³ eine Hydroxygruppe, Halogen, eine geschützte Hydroxygruppe OPG², ein Phosphoniumhalogenidrest PPh₃⁺Hal⁻ (Ph = Phenyl; Hal = F, Cl, Br, I), ein Phosphonatrest P(O)(OQ)₂ (Q = C₁-C₁₀-Alkyl oder Phenyl) oder ein Phosphinoxidrest P(O)Ph₂ (Ph = Phenyl),
X ein Sauerstoffatom, zwei Alkoxygruppen OR⁴, eine C₂-C₁₀-Alkylen-□,□-dioxygruppe, die geradkettig oder verzweigt sein kann, H/OR⁵ oder eine Gruppierung CR⁶R⁷,
wobei
R⁴ für einen C₁-C₂₀-Alkylrest,
R⁵ für Wasserstoff oder eine Schutzgruppe PG³,
R⁶, R⁷ gleich oder verschieden sind und für Wasserstoff, einen C₁-C₂₀- Alkyl-, Aryl-, C₇-C₂₀-Aralkylrest oder R⁶ und R⁷ zusammen mit dem Methylenkohlenstoffatom gemeinsam für einen 5- bis 7- gliedrigen carbocyclischen Ring
stehen,
bedeuten,
wobei nicht gleichzeitig
R¹ eine Methylgruppe, R² ein tert.-Butyldimethylsilyl- oder Benzylrest, R³ ein O-tert.-Butyldimethylsilylrest und X ein (2-Methylthiazol-4-yl)methylen-rest oder
R¹ eine Methylgruppe, R² ein tert.-Butyldimethylsilylrest, R³ ein Triphenylphosphoniumiodidrest und X ein (2-Methylthiazol-4-yl)methylenrest
sein können.

18. Verbindungen der allgemeinen Formel C nach Anspruch17, **dadurch gekennzeichnet, daß** R¹ für ein Wasserstoffatom, einen gegebenenfalls substituierten C₁-C₄-Alkylrest, einen gegebenenfalls mit 1 bis 3 Resten, ausgewählt aus der Gruppe der Substituenten Halogen, freie Hydroxygruppe oder geschützte Hydroxygruppe OPG⁴, C₁-C₄-Alkyl, Azido, Nitro, Nitril, Amino (NH₂), substituierten Phenylrest steht.

19. Verbindungen der allgemeinen Formel C nach Anspruch 17, **dadurch gekennzeichnet, daß** X für ein Sauerstoffatom steht.

20. Verbindungen der allgemeinen Formel C nach Anspruch 17, **dadurch gekennzeichnet, daß** der für R⁶ und/oder R⁷ stehende Arylrest für einen gegebenenfalls mit 1 bis 3 Resten, ausgewählt aus der Gruppe der Substituenten Halogen, freie Hydroxygruppe oder geschützte Hydroxygruppe OPG⁵, C₁-C₄-Alkyl, Azido, Nitro, Nitril, Amino (NH₂), substituierten Phenylrest oder für einen gegebenenfalls mit 1 bis 2 C₁-C₄-Alkylresten substituierten 5- oder 6-gliedrigen Heteroarylrest steht.

21. Verbindungen der allgemeinen Formel C nach Anspruch 20, **dadurch gekennzeichnet, daß** der für R⁶ und/oder R⁷ stehende Arylrest ausgewählt ist aus der Gruppe 2-, 3-Furanyl-, 2-, 3-, 4-Pyridinyl-, 2-, 4-, 5-Thiazolyl-, 2-, 4- und 5-Imidazolylrest, der gegebenenfalls durch 1 oder 2 C₁-C₄-Alkylreste substituiert ist.

22. Verbindungen der allgemeinen Formel C nach Anspruch 17, **dadurch gekennzeichnet, daß** die Schutzgruppen PG¹, PG² und PG³ aus der Gruppe der Substituenten Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-, Formyl, Acetyl-, Propionyl-, Isopropionyl-, Pivalyl-, Butyryl- oder Benzoylrest ausgewählt sind.

23. Verbindungen nach Anspruch 18, **dadurch gekennzeichnet, daß** die Schutzgruppe PG⁴ aus der Gruppe der Substituenten Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-, Formyl, Acetyl-, Propionyl-, Isopropionyl-, Pivalyl-, Butyryl- oder Benzoylrest ausgewählt ist.

24. Verbindungen nach Anspruch 20, **dadurch gekennzeichnet, daß** die Schutzgruppe PG⁵ aus der Gruppe der Substituenten Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl, para-Nitrobenzyl-, para-Methoxybenzyl-, Formyl, Acetyl-, Propionyl-, Isopropionyl-, Pivalyl-, Butyryl- oder Benzoylrest ausgewählt ist.

25. Verbindungen nach Anspruch 22, **dadurch gekennzeichnet, daß** die Schutzgruppe PG¹ ein tert.-Butyldiphenylsilyl-, tert.-Butyldimethylsilyl- oder Triisopropylsilyl-Rest ist.

26. Verbindungen nach Anspruch 22, **dadurch gekennzeichnet, daß** die Schutzgruppe PG² ein tert.-Butyldimethylsilyl-, Acetyl-, Benzoyl-, Benzyl- oder Tetrahydropyranyl-Rest ist.

27. Verfahren zur Herstellung der Verbindungen der allgemeinen Formel C' 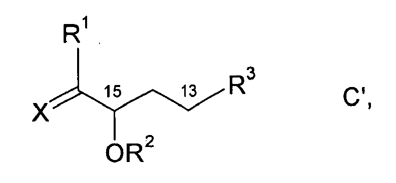 worin
R¹ Wasserstoff, C₁-C₂₀-Alkyl, Aryl, C₇-C₂₀-Aralkyl, die alle substituiert sein können,
R² Wasserstoff oder eine Schutzgruppe PG¹,
R³ eine Hydroxygruppe, Halogen, eine geschützte Hydroxygruppe OPG², ein Phosphoniumhalogenidrest PPh₃⁺Hal⁻ (Ph = Phenyl; Hal = F, Cl, Br, 1), ein Phosphonatrest P(O)(OQ)₂ (Q = C₁-C₁₀-Alkyl oder Phenyl) oder ein Phosphinoxidrest P(O)Ph₂ (Ph = Phenyl),
X ein Sauerstoffatom, zwei Alkoxygruppen OR⁴, eine C₂-C₁₀-Alkylen-□,□-dioxygruppe, die geradkettig oder verzweigt sein kann, H/OR⁵ oder eine Gruppierung CR⁶R⁷,
wobei
R⁴ für einen C₁-C₂₀-Alkylrest,
R⁵ für Wasserstoff oder eine Schutzgruppe PG³,
R⁶, R⁷ gleich oder verschieden sind und für Wasserstoff, einen C₁-C₂₀-Alkyl-, Aryl-, C₇C₂₀-Aralkylrest oder R⁶ und R⁷ zusammen mit dem Methylenkohlenstoffatom gemeinsam für einen 5- bis 7-gliedrigen carbocyclischen Ring
stehen,
bedeuten,
**dadurch gekennzeichnet ist, daß** L-(-)-Äpfelsäure, D-(+)-Äpfelsäure oder racemische Äpfelsäure als Ausgangsprodukt verwendet wird.

28. Verfahren nach Anspruch 27, **dadurch gekennzeichnet, daß** L-(-)-Äpfelsäure oder D-(+)-Äpfelsäure verwendet wird.

29. Zwischenverbindungen der allgemeinen Formel VI" 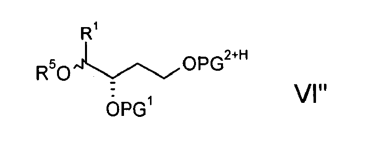 worin
R¹, PG¹ und R⁵ die in der allgemeinen Formel C angegebene Bedeutung haben und
PG^{2+H} für ein Wasserstoffatom oder eine Schutzgruppe PG² stehen.

30. Verfahren zur Herstellung der Verbindungen der allgemeinen Formel VI" gemäß Anspruch 29, **dadurch gekennzeichnet, daß** an eine Verbindung der allgemeinen Formel IV 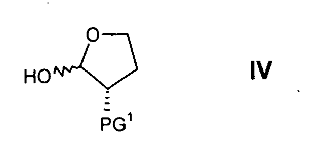 worin
PG¹ die in der allgemeinen Formel C angegebene Bedeutung hat,
unter Öffnung des Lactolringes eine Organometallverbindung der allgemeinen Formel
R¹Y
worin R¹ die in der allgemeinen Formel C' angegebeneBedeutung hat, und
Y für ein Alkalimetallatom oder MZ steht, wobei M ein zweiwertiges Metallatom und Z ein Halogenatom ist,
addiert und anschließend gegebenenfalls
die primäre Hydroxygruppe mit einer Schutzgruppe PG² und gegebenenfalls
die sekundäre Hydroxygruppe mit einer Schutzgruppe PG³
geschützt wird.
